**Europäisches Patentamt**

**European Patent Office**

**Office européen des brevets**

(19)

(11) Veröffentlichungsnummer: **0 221 005**
**A2**

(12) ## EUROPÄISCHE PATENTANMELDUNG

(21) Anmeldenummer: **86730058.4**

(22) Anmeldetag: **01.04.86**

(51) Int. Cl.⁴: **A 61 M 5/14**

(30) Priorität: **07.09.85 PC /DE85/003 13**

(43) Veröffentlichungstag der Anmeldung: **06.05.87**
**Patentblatt 87/19**

(84) Benannte Vertragsstaaten: **AT BE CH DE FR GB IT LI LU NL SE**

(71) Anmelder: **Wagner, Wolfgang, Dr.med., Exerzierstrasse 1, D-1000 Berlin 65 (DE)**

(72) Erfinder: **Wagner, Wolfgang, Dr.med., Exerzierstrasse 1, D-1000 Berlin 65 (DE)**

(54) **Injektionsvorrichtung mit Sensor.**

(57) Die Erfindung betrifft ein Gerät für die automatische Injektion, insbesondere für die Injektion von Insulin bei der Behandlung von Diabetes.

Das Kernstück der Erfindung ist mit einer sogenannten Sensor-Kanüle gegeben. Beim Einstich einer erfindungsgemässen Sensor-Kanüle unter die Haut bewirkt die Körperflüssigkeit eine elektro-chemische Beeinflussung der Sensor-Beschichtung. Es werden Ausführungsformen der Sensor-Kanüle sowohl als aktives als auch als passives Bauelement vorgeschlagen. Daneben wird eine photometrische Sensor-Kanüle beschrieben.

Das von der Sensor-Kanüle festgestellte Signal wird einem Rechner zur Auswertung zugeleitet. Der Rechner, wiederum, steuert eine Dosiereinheit, die die berechnete Dosis einem Spritzen-Zylinder aus einem Vorratsbehälter zur Injektion freigibt.

Die Vorteile der Erfindung sind darin zu sehen, dass die Injektion von Insulin auch individuellen Lebensgewohnheiten angepasst werden kann, dass die Messung der Glukose-Konzentration und die Injektion der erforderlichen Insulin-Menge mit nur einem einzigen Einstich erfolgt, und dass alle weiteren Funktionsabläufe weitgehend automatisch ablaufen.

0221005

# Injektionsvorrichtung mit Sensor

Die Erfindung betrifft eine Kanüle, welche durch Besonderheiten ihrer Gestaltung und im Zusammenwirken mit einer Meßanordnung vor oder während der Einspritzung von Flüssigkeiten in den Körper eines Säugers es gestattet, Stoffwechselzustände vornämlich bei Nieren- und vorallem Zuckerkranken zu beurteilen und zu registrieren. Wesentlich aber richtet sich die Idee der Erfindung auf einen Injektor, welcher nach oder während der Gewebszuckerbestimmung die ermittelten Meßwerte zu einer voreinstellbaren Dosierung und Dosiskorrektur in Beziehung setzt und gegebenenfalls -bei häufig notwendig werdenden Korrekturen- die Programmierung der Dosierung automatisch anpaßt. Vornehmlich erfolgt dies in einem Sauginjektor, welcher die Haut mittels Unterdruckes in die Kanüle hebt und so durch Steuerung der Wiederbelüftung der Saugglocke Meß- und Injektionsdauer sicher berücksichtigt. Neben Ausgestaltungen der Sensorkanüle hinsichtlich ihrer Form, Lokalisation und Art von Sensorbeschichtungen, elektrischer oder optischer Meßfühler, gilt das Augenmerk der Verbesserung der Sauginjektion hinsichtlich Handlichkeit, Einfachheit der Bedienung und Stimmigkeit mit
den Voraussetzungen einer Compliance. Art der Unterdruckerzeugung, der Dosierung und Besonderheiten der
Funktionssteuerungen werden in diesem Sinne behandelt.


A u f g a b e n s t e l l u n g :

Die Erfindung bezieht sich auch das Gebiet der Medizintechnik, spezieller auf die Injektionstechnik. Besonders stellt sie sich die Aufgabe, die Behandlung
der insulinbenötigenden Diabetiker zu verbessern.
Dabei soll die Stoffwechsellage des Kranken diesem häufig und auch möglichst regelmäßig erkennbar gemacht
werden, um ihm eine vernünftige Diäteinhaltung zu erleichtern und bei Abweichungen der Lebensführung von
einer starren Norm einen Ausgleich durch Anpassung der
Insulinbehandlung zu ermöglichen. Dem behandelnden Arzt
soll weitgehend Einblick in die Ernährungsweise des
Kranken und eine Anpassung seiner Dosierungsvorschläge
für Insulin ermöglicht werden. Aus diesem Vorsatz ergibt sich die Aufgabe, der Schaffung eines Injektors
nahezukommen, dessen Handhabung durch Automatisierung seiner Funktionen auch Behinderten möglich ist,
und welcher häufigere täglichere Anwendungen begünstigt. Hierzu soll der Injektor möglichst klein und
handlich, sicher in seinen Funktionen, ohne Gefahren
für die Gesundheit und möglichst in seiner Anwendung mit geringer Belästigung verbunden sein. Die Handhabung - auch hinsichtlich der Nachfüllung von Insulin oder Kanülen soll möglichst einfach, die Kosten sollen möglichst gering sein. Besonders günstig zur Lösung der Aufgabe, die Zuckermessung mit der Injektion zu verbinden, zeigt sich die Anwendung der Sauginjektion, bei welcher die Haut mittels Unterdruckes in die Kanüle gehoben und bis zur Wiederbelüftung der Saugglocke eine feste Verbindung zwischen Injektor und Haut gewährleistet wird. Gerade aber hinsichtlich der Sauginjektionstechnik sind Anpassungen an die Meßtechnik und weitere Verbesserungen bezüglich Lagerung, Mischung und Dosierung des Insulins, aber auch der Erzeugung und Anwendung des Unterdruckes erforderlich. Die über fünfzigjährigen Versuche, die Sauginjektion in nennenwertem Umgange zum Einsatz zu bringen, zeigen, daß nur eine komplexe Behandlung, bei welcher alle Funktionsteile und die menschliche(vorallem auch psychische) Konstitution aufeinander abgestimmt werden, zu einer vorteilhaften und auch gewerblich nutzbaren Lösung führen. Gegenüber der inzwischen einfacher zu handhabenden(aber teueren) Langhub-Einmalspritze mit eingebauter KLanüle muß ein vollautomatischer Injektor taschengängig-handlich, funktionssicher, leicht zu bedienen und kostengünstig sein. Hinsichtlich des Kanülendurchmessers muß das Minimum gehalten werden, um zusätzliche Verletzung und Belästigung zu vermeiden. Gegenüber der herkömmlichen Spritze müssen zusätzliche wesentliche Vorteile geboten werden, wie sie in der Dosisanpassung unter Zuckermessung gegeben sind und einschließlich der Kontroll-, Einübungs- und Erziehungsmöglichkeiten·dazu geeignet sind, die Compliance zu erhöhen.

Stoffwechseluntersuchungen direkt am Krankenbett über automatisierte Probeentnahmen sind schon länger bekannt, wobei auch Meßdaten gespeichert werden(z.B.Hudspeth,C.L.,Richardson,Ph.Cl.u.a.in DE 24 37 467). Beispielsweise mit dem Insulin-Infusions-Gerät Biostator der Firma Miles ist die ständige Zufuhr von Insulin unter Überwachung der Blutglukosekonzentration in den letzten Jahren möglich geworden. Die verhältnismäßig umfangreiche Apparatur ist aber nur am Krankenbett einsetzbar. Der "einpflanzbare elektrochemische Sensor", wie er von Leblanc jun.,u.a. angegeben wird(DE 26 45 048) wird innerhalb einer Ummantelung in ein Gefäß eingeführt und dient der Überwachung der Ionenaktivität. Er zeigt Flüssigkeitszufuhr mittels einer Tropfinfusion. Es wird auch vorgeschlagen über eine liegende Sonde Blutproben zur Analyse zu entnehmen (Clemens,Anton Hubert, E.Myers,R.Weston, DE 27 20 482) und die getrennte Zufuhr von Glukose und Insulin zu steuern. Am nächsten kommen der hier vorgestellten Erfindung Ash St.R.,Lafayette,Loeb M.P. mit ihrer "Anordnung zur bedarfweisen Verabreichung von Insulin" (D 30 50 155). Einer insulinzuführenden Kanüle wird ein ummantelter Sensor direkt zur gemeinsamen Einführung unter die Haut angelagert. Anstelle einer störanfälligeren spezifischen Fermentreaktion mit Glukose wird die Osmolarität des Blutes gemessen und unter gleichzeitiger Salzbestimmung von einem etwaigen Wasserentzug des Körpers in Abzug gebracht. Die Meßergebnisse werden rechnerisch mit einem Dosierprogramm abgestimmt. Die besonders für jugendliche und stoffwechsellabile Diabetiker weiterhin nützlichen Insulinpumpen, welche mit liegender sogenannter Dauerkanüle am Körper getragen werden, dienen der Zufuhr einer regelbaren Mindestinsulinmenge mit der Möglichkeit willkürlicher veränderlicher Zusatzdosen vor den Mahlzeiten durch den Patienten selbst.(Beispielsweise Haerten,R. und Kresse H, DE 25 13 467). Eine Blutzuckermeßvorrichtung ist hier nicht vorhanden. Eine bogig angeordnete auf dem Körper zu tragende Spritze unter Katheteranwendung geben Loeb M.P. und Olson A.M.(DE 32 41 313) an. Zur Einpflanzung in den Körper werden ebenfalls mehrere Pumpen mit Vorratsgefäßen beschrieben( z.B. Cummins R.D. und Park O.,DE 33 43 708). Man beschäftigt sich mit der drahtlosen Übertragung von Dosieranweisungen an ein solches Gerät(Prestek K. und Franzeski M. DE 30 35 670) sowie mit der Funktionskontrolle über Meßdaten aus dem Körperinneren, wobei auch eine Sperrvorrichtung bei Überschreitung eines Insulininfusionsgrenzwertes vorgesehen ist (Fischell R. und Ellentuch, DE 32 47 232). Auch der Titel "The Development Of A Needle-Type Glucose Sensor For Wearable Artificial Endocrine Pancreas"(Yamasaki.Y. Journal of Osaka Univ.,Voll-2,Sept.1984) sollte nicht darüber hinwegtäuschen, daß es sich hier um eine Hunden in Narkose eingepflanzte Knopfsonde mit kompliziertem Schichtenaufbau handelt und der Erfindungsgedanke der Kanülenbeschichtung nicht genannt wird. Praktikabler scheinen für unseren Zweck die auf Draht aufgeschichteten Sensoren von Wilkens E. und H.G., Daniel R.u.a. und Gough A.u.a., welche bei der Beschreibung der Sensorkanülen genauer zitiert werden. Aber insgesamt werfen solche "katalytische Sensoren" im Sinne stromerzeugender Zellen doch erhebliche Probleme auf, gerade weil ihr Dauereinsatz im Menschen angestrebt wird. Optische Systeme sind zwar hinsichtlich der Stoffkonkurrenz(z.B.die Polarisationsebene drehender Aminosäuren) ebenfalls nicht problemlos, jedoch erst in der miniaturisierten Form des Körperzuganges durch die Kanüle praktikabel. Müller A. beschäftigte sich mit in-vivo-Messungen(DE 2944113) durch die Ohrmuschel hindurch. So erhaltene Dauermeßwerte könnten durchaus zu einer verbesserten Dosiskorrektur bei der Sauginjektion eingesetzt werden. Weniger umständlich bliebe wohl die Messung über die Kanüle. Für die Bindung von Herzglykosiden und anderer Zuckerabkömmlinge wird Sepharose-Concanavalin A seit einiger Zeit verwendet. Andererseits ist bekannt, daß Zucker Nichtleiter darstellen und in höherer Konzentration den elektrischen Widerstand einer Elektrolytlösung erhöhen; weshalb in der Zuckerchemie Widerstandsmessungen zum Aufspüren von Zuckern benutzt werden( eine Tatsache, die ich der persönlichen Mitteilung von Herrn Prof.Dr.Mauch Berlin verdanke).

Die pflasteradhäsive Hautfixation während der Injektion hat ebenfalls Vorläufer, aber wohl noch ohne Hautanhebung.

Die Sauginjektion -das heißt die Einspritzung unter eine unter Sogeinwirkung innerhalb einer Saugglocke hochgezogene Haut-, welche schon am 7.11.1933 von M.Demarchi(US.Pat.1,934,046) beschrieben und von G.N.Hein(US.Pat.2,743,723 vom 1.5.1956) und von W.R.C.Geary(US.Pat.3,122,138 am 25.2.1964) und in mehreren eigenen Anmeldungen seit 1974(GB 1494325, 1494646,1513463,1517289,DE 25 51 991,25 51 992, 25 51 993,30 19 589, Australien 510,272,US.4.139, 008,4.169,474,4.284,077,4.393,870, Can.1189412 und vielen anderen) weiterentwickelt wurde, fand wegen technischer Unvollkommenheiten beim Menschen keine bekanntgewordene Anwendung, obwohl sich menschliche Haut für die Sauginjektion wegen ihrer besonderen Elastizität und feineren Behaarung gut eignet.

G.H.Hein gibt bereits eine Vorrichtung an, welche ein vorzeitiges Eindringen der Haut vor der Injektion sicher verhindert, aber die wichtigere Distanzierung von Haut und Kanüle n a c h der Injektion wurde vernachlässigt. Die Methoden der Unterdruckerzeugung sind ebenfalls noch unbefriedigend, wobei natürlich eine Einhandbedienung für die Selbstbehandlung der Diabetiker Voraussetzung ist. Auch die Dosiersysteme sind noch unbefriedigend; eine von Stein(DE 14 91 840) angegebene Injektionsvorrichtung benutzt zwar das Prinzip eines durch den dosierenden Spritzenkolben hindurch durch ein Ventil in demselben aus einer Vorratsflasche erfolgenden Arzneizufuhr, aber die Notwendigkeit der Trennung zwischen Außenluft und Vorratsflascheninhalt wird nicht beachtet. Die nadellose Injektion mittels Überdruckes verbietet sich für die Daueranwendungen wegen der erheblichen Gewebsbeschädidigungen. Überhaupt wird der Notwendigkeit, eine Reinigung von Gerätebestandteile durch den Patienten zu vermeiden, bisher immer noch zu wenig Rechnung getragen.

Vorteile der Erfindung:

Für den insulinspritzenden Zuckerkranken wie auch für den Insulinpumpenträger ist es vorteilhaft, wenn er ohne zusätzliche Selbstbeschädigung durch Ritzung der besonders an Schmerzpunkten reichen Haut der Fingerbeere und ohne die umständliche Prozedur der Aufschichtung eines Bluttropfens auf einen Teststreifens und der kolorimetrischen Ablesung desselben, während der Insulineinspritzung oder nach Wahl auch vor der Betätigung des Spritzenkolbens seine Stoffwechsellage erfährt. Selbst eine nachträgliche Reflexphotometrie in der Kanülenspitze oder eines aus dem Kanülenlumen entfernten Farbindikatorstreifens kann durch diätetisches Verhalten des Patienten genutzt werden. Durch die Aufzeichnung der Meßergebnisse und der verabreichten Insulindosen können sich Patient und Arzt über die Therapiesituation Rechenschaft geben und leichter verständigen. Bei den Einzelmessungen- sei es über den Sensorbelag der Kanüle, als Stromquelle oder über die Messung von Leitfähigkeitsänderungen unter dem Einfluß von Glucose- mittels der ständig erneuerten Kanülen entfallen Störungsquellen, wie Eiweiß- oder Elektrolytanlagerung an der Meßsonde, wie sie eine Daueranwendung erschweren. Die Sauginjektion bietet im Zusammenhang mit der stoffwechselkontrollierenden Sensorkanüle den besonderen Vorteil, daß die Dauer des Hautkontaktes automatisch gesteuert wird; außerdem ist es für die Meßanordnung vorteilhaft, in dem Saugglockenrand in Kanülennähe eine Vergleichselektrode von sicherer Haftung zu besitzen, welche nicht gesondert angebracht werden muß. Die Auffräsung des Kanülenschaftes zu einer Art Skelettkanüle, welche den Arzneiführungskanal und den Sensor aufnimmt, erspart eine Verdickung der Kanüle durch den Sensorbelag, welcher durch die durch die Schmerzhaftigkeit des Einstiches einen wesentlichen Vorteil zunichte machen würde, den die modernen feinen Dreieckschliffkanülen und die Sauginjektion mit sich bringen. Durch Teilung des Kanülenschaftes in den metallenen Skeletteil und einen angelagerten Sensorteil -welcher auch auf in den rinnenförmigen Kanülenschaft eingeführtem Schläuchen aufgebracht sein kann- besteht die Möglichkeit, zwischen zwei elektrisch geteilten Anteilen mit eigenen Ableitungswegen zu trennen und dadurch eine weitere Bezugselektrode zu gewinnen. Ein in eine solche halboffene Kanüle eingeschobener Schlauch kann beim Durchstechen der Haut eine geringe Lichtung aufweisen, um dank seiner Elastizität unter dem Druck der Injektionsflüssigkeit ausgeweitet zu werden. Es kann so eine raschere Injektion ermöglicht werden(bzw.eine solche von zählflüssigeren Lösungen oder von Suspensionen, wie Depotinsuline sie oft darstellen). Ein weiterer Vorteil kann sich daraus ergeben, daß der Schlauchoberfläche aufgelagerte Fremdstoffe durch kapilläre Ansaugung zwar mit Gewebsflüssigkeit oder Blut in Berührung kommt, eine Einführung solcher Fremdstoffe in den Köper aber vermieden werden kann. Die automatische Dosierungsangleichung an die jeweils ermittelte Stoffwechselsituation schließt Fehlberechnungen und -einschätzungen sowie Fehlbedienung durch den Patienten aus und erlaubt die Berücksichtigung unterschiedlicher Insulinempfindlichkeit. Darüber hinaus gestattet eine Programmautomatik die Korrektur einer groben Fehldosierung durch den Arzt beziehungsweise eine Anpassung an neue Lebens-und Ernährungsverhältnisse ohne ärztliche Mitwirkung. Die Anwendung von sofortwirkendem Altinsulin und von Depotinsulin nebeneinander ermöglicht ähnlich wie bei den Insulinpumpen eine kurzfristige Stoffwechselkorrektur neben einer Langzeitversorgung des Körpers. Dabei entfallen aber die Belästigungen durch ein am Körper getragenes Gerät und vorallem durch die in die Bauchhöhle oder ins Unterhautgewebe eingeführte und dort fixierte Kanüle mit der bei Zuckerkranken noch erhöhten Gefahr der Wundinfektion. Die Programmierung mittels auf dem Gehäuse drehbar angeordneter in Blöcken gegliederter Schaltringe erleichtert den Überblick. Die Wahl von Profilzahlen erlaubt den Abdruck der gesamten Programmzeile in die Patientenkartei ohne komplizierte technische Hilfsmittel. Abschließbarkeit der Programmierung und die Möglichkeit der Einschränkung von Kompensationsmöglichkeiten und Programmänderungen durch den Patienten sind unabdingbare Voraussetzungen zur Vermeidung ernsthafter Zwischenfälle infolge von Fehlvorstellungen, Eigenwilligkeiten und Uneinsichtigkeit des Patienten oder mutwilliger Eingriffe Dritter. Auf dem Gebiet der Vakuumerzeugung erscheint deren Vorverlegung in die Phase der industriellen Verpackung der Kanüle vorteilhaft, insbesondere dann, wenn der höhere Aufwand der Auslösevorrichtung für ein den Unterdruck bewahrendes, die Kanüle umgebendes Speichertöpfchen zum Einmalgebrauch auf den immer wieder verwendeten Injektor verlegt wird. Oberflächenveränderungen des Speichertöpfchens bei der Hautansaugung lassen sich über Tastelemente des Injektors in Steuerungsimpulse umsetzen, so daß die direkte Hautberührung eines Tasters für Schaltzwecke entfallen kann. Es bietet sich besonders bei elektrisch betriebener Dosisabgabe die Lösung der Unterdruckerzeugung über die Luftverdünnung innerhalb einer beheizten Kammer an. Der Einsatz mehrerer Kammern erhöht die Unabhängigkeit vom Stromnetz und damit die Gestaltungsfreiheit im Tagesablauf. Die Vorteile eines bedarfsgesteuerten Dauervakuums kann innerhalb eines handlichen Apparates durch eine Gasstrahlpumpe in Verbindung mit einer Haushalts-Kohlensäure-Patrone genutzt werden, nachdem Probleme wegen des hohen Gasaustrittsdruckes und der Ventilvereisung bei der Gasentspannung durch ein besonderes Schleußenventil beseitigt wurden. Schließlich kann auch in anbetracht des geringen Unterdruchbedarfes die Vakuumerzeugung in einer federbetriebenen Zylinder-Kolben-Pumpe ins Auge gefaßt werden, wenn durch die Fillterung der Luft aus der Saugglocke verhindert wird, daß Kolben und Zylinder von den feinsten Quarzkörnchen aus den Hautporen in ihrer Oberflächengüte beeinträchtigt werden. Zweckmäßigerweise wird die Unterdruckvorbereitung an die Bewegung eines Verschlußdeckels für die Saugglocke gebunden, wobei auch der Mechanismus zum Kanülenwechsel betätigt wird. Bowdenzüge erlauben eine raumsparende Kraftübertragung.

Die Einseitigkeit der Feststellung eines Saugkolbens durch eine seitliche Rastervorrichtung mit der Gefahr der Kolbenverkantung wird dadurch entgegengewirkt, daß der Saugkolben durch Magnete und Federsperre bereits annähernd in der Rasterstellung gehalten wird. Der Kraftaufwand zur Auslösung von der Saugglocke her wird dadurch erheblich gesenkt. Auch tritt eine Verzögerung der Pumpbewegung ein, welche zum Schluß eines Überdruckventiles genutzt werden kann. Letzteres dient der Ableitung der durch die Hautannäherung an die Kanüle in der Saugglocke verdichteten Luft zur Sogersparnis. Erlaubt schon die Verdünnung der oberen Hautschichten durch die Hautanhebung eine Verkürzung des Kanülenschaftes, so trägt die Kanülenstapelung in Schlauch oder Röhre zur weiteren Raumersparnis bei. Die Höhe der Stapelkanüle kann noch dadurch vermindert werden, daß bei entsprechender Sicherung gegen Drehung im Verhältnis zu den seitlichen Arzneieintrittsstellen- beispielsweise durch einseitige Abplattung des Kanülenmagazins, was noch eine dichte Anlage der seitlichen Kanülenöffnung sichertder Flüssigkeitskanal, der mit der Arzneiaustrittsöffnung des Gerätes in Deckung kommt als Rinne in oder an der Wandung des Kanülengehäuses abwärts verläuft, ehe er in den Kanülenschaft eintritt. Eine Gelenkverbindung zwischen den Stapelkanülen -welche ein Faden sein kann- erlaubt einen Kanülentransport durch Zug, eine im Magazin ein gelagerte Feder oder ein Dichtkörper vor einer Druckgasleitung eine Beförderung durch Druck.

Der seitliche Arzneieintritt über eine Ringfurche des Kanülenkörpers erleichtert die Beschickung des Kanülenschaftes mit mehreren Arzneisorten, sowie die Kanülenentfernung durch eine klauenartige Deckelvorrichtung. Auch die Kanülenanordnung in Reihe dient der Erleichterung der Kanülenentfernung nach Gebrauch, wobei platzsparend der Raum der ehemals ungebrauchten durch die gebrauchten Kanülen eingenommen werden kann.

Die Verschiebung der Kanüle nach der Injektion noch vor ihrer Entfernung bewirkt einen Ventilverschluß des Arzneizuflußkanales. Die Distanzierung zwischen Haut und Kanüle im Augenblick der Wiederbelüftung der Saugglocke mittels Rückkehr einer zuvor durch Raster federnd fesjgehaltenen Saugglocke oder durch einen Zentralstift innerhalb der Saugglocke vermeidet gefährliliche Hautschnitte bei unbeabsichtigter Entfernung des Gerätes nach Benutzung tangential zur Haut. Vorteilhafterweise kann auch das Kanülenmagazin als Ganzes -beispielsweise unter Sogeinwirkung in der Saugglocke auf ein mit dem Magazin verbundenes Septum- vor der Injektion der Haut entgegen abgesenkt und bei Wiederbelüftung wieder angehoben werden.

Auf dem Gebiet der Funktionssteuerung, insbesondere der Ablaufsicherung zwischen Hautansaugung, Arzneieinspritzung und Wiederbelüftung der Saugglocke werden ebenfalls Fortschritte angegeben. So vermittelt ein Zentralstift innerhalb der Saugglocke in Nähe der Kanüle den Zustand der Hautanhebung . Auch die drei um den Saugglockenrand verteilten Auslösestifte in anderen Beispielen wurden durch geschicktere Raumanordnung und dadurch verbessert, daß ihre Rückfederung auf die Haut nach deren Anhebung aufgehoben oder abgeschwächt wird. Die Distanzierung zwischen Haut und Kanüle nach der Injektion zur Vermeidung von Schnittwunden, wird durch -teilweise während der Injektion noch gesperrte- Rückfederung der Saugglocke sichergestellt. Die Wiederbelüftung der Saugglocke erfolgt mit Sicherheit erst nach Be-

- 13 -

endigung der Injektion (in den mechanisch gesteuerten Beispielen), wenn ein zuvor gespannter Federmechanismus erst nach Auflaufen der die Dosierung steuernden Verschiebeteile auf das dosisbegrenzende Hindernis aktiviert wird. Dem Federmechanismus ist eine magnetische Anziehung des Belüftungsventiles gleichzusetzen, wobei ein Flattern des Ventiles bei Anwendung eines Dauersoges mittels eines besonderen Sperrmechanismus verhindert wird. Vorteilhafterweise besetzt der Zentralstift einen Mittelpunkt einer eliptischen Saugglocke, während die Kanüle den anderen einnimmt, um die Dichtungen um Zentralstift und Kanüle sowie den Saugglockenrand im notwendigen Abstand zu halten. Die Anwendung eines Quellstiftes zur Hebung einer Gaskapsel in den Öffnungsdorn erspart einen verhältnismäßig gewichtigen Schraubverschluß und eine krafterfordernde Bedienung. Im Bereich der Arzneilagerung und -zufuhr wird für einen gleichmäßigen Arzneifluß zu der Dosiervorrichtung gesorgt. Ein Nachfüllvorgang, um einen Restvorrat auf eine verwendbare Dosis aufzubauen erspart teueres Hormon, insbesondere dann, wenn eine Verriegelung die Auswechslung des Vorratsbehälters erst dann gestattet, wenn der gebrauchte nahezu restlos entleert ist. Auf dem Gebiet der Dosierung wird die Dosiergenauigkeit durch Vermeiden von längeren Verbindungsschläuchen mit Windkesselfunktion zur Kanüle hin gehoben. Wurde die Dosiervorrichtung als Kurzhubkolben- oder Manschette in das Ende des Arzneizufuhrschlauches aus dem Behälter kurz vor die Kanüle verlegt und von außerhalb des Schlauches betätigt, so kann in anderern Lösungen in melkender Bewegung durch von außerhalb des Schlauches periodisch ausgeübten Druck die Aufgabe in elektromagnetischer Ausführung so erfüllt werden, daß bewegliche Teile im Schlauch entfallen. Lediglich ein Ringleisten tragender Stift innerhalb des Schlauches als Gegendrucklager ist dabei nützlich. Kurze rüttelnde Bewegungen zur Aufschüt-

- 14 -

telung von Zinkchlorid als Zusatz zu Depotinsulin ist erforderlich, um eine injizierbare Suspension zu erhalten. Die Steuerung eines Ausflußventiles in Verbindung mit einer Durchflußmessung wird unter dem Gesichtspunkt der Raumersparnis erwogen. Piezzoelektrisch oder über Schalter an Magnete vermittelte Unterschiede der in der Hubgeschwindigkeit oder ein Motorrücklauf, welcher zum Antrieb eines Schaltgetriebes benutzt wird, sparen erheblich an Gewicht für weitere Kraftantriebe für mehrere Dosiervorrichtungen ein. Die Verwendung von Hilfsflüssigkeit anstelle der Arznei zur Dosierung(unter Ausnutzung des Verdrängungseffektes) erspart oder erleichtert die Reinigung der Dosiervorrichtung. Wird dabei die Hilfsflüssigkeit hinter den Kolben einer Stufenspritze gebracht, so verhindert die höhere Viskosität der Hilfsflüssigkeit deren Zumischung zur Arznei. Erheblich an Raum für die Hilfsflüssigkeit kann gespart werden, wenn ein dem Trennkolben nachrückender Sperrkolben angewandt wird, so daß immer dieselbe begrenzte Hilfsflüssigkeitsmenge zwischen Dosiervorrichtung und Stufenspritze hinundherpendelt.

Die Erfindung beinhaltet noch eine Reihe in der dargestellten oder in neuer Kombination für die Lösung der Aufgabe wesentliche Vorteile, welche aus dem nachfolgenden Text in Verbindung mit den Zeichnungen hervorgehen.

Lösung der gestellten Aufgabe:

Die gestellte Aufgabe wird dadurch gelöst, daß eine der Eingabe von Flüssigkeiten dienende Kanüle zugleich als Sensor ausgebildet ist, welcher bestimmungsgemäß nach Einführung der Kanüle in den Körper eines Säugers physikalisch-chemische Zustandsänderungen im Körper dieses Säugers in Meßsignalveränderungen wandelt, welche einem Meßinstrument zur Auswertung zugeführt werden, um die physikalisch-chemischen Zustandsänderungen sofort oder später erkennbar zu machen. Hierzu kann der Sensor selbst als elektrische Spannungsquelle (z.B. beim sogenannten katalytischen Sensor) dienen, dessen Spannungsänderungen oder Stromstärkenabgabe zur Meßsignalverarbeitung dienen; der Sensor kann jedoch auch unter physikalisch-chemischer Beeinflussung durch den Zustand des Körpers seine Leitfähigkeit für von außen zugeführte elektrische Ströme ändern und dadurch Meßdaten an das Meßinstrument liefern. Wenigstens auf Teile des Kanülenschaftes wird hierzu in der Regel mindestens eine Schicht aufgebracht, welche die Sensoreigenschaft hervorbringt. Der Hauptanwendungsmöglichkeit der Erfindung gemäß reagiert dieser Sensorbelag auf dem Kanülenschaft hauptsächlich mit der Glukose des Körpers, um mit dieser als eine Art elektrischer Zelle zu reagieren oder durch Anlagerung von Glucose oder deren Reaktions- oder Syntheseprodukte (wie Glykogen, Stärke oder auch niedrigerer Kohlehydratketten) die elektrische Leitfähigkeit über die Kanüle oder an der Kanüle vorbei in Abhängikeit von der Glucosekonzentration von Blut oder Gewebe (je nach Lage der Kanüle innerhalb eines Blutgefäßes oder im Unterhautzellgewebe oder in der Bauchhöhle) zu verändern. Durch Entfernung eines Teiles des Kanülenschaftes hinter der Kanülenspitze unter Erhaltung von deren Schlifffläche, kann die Schaftwandung durch Kunststoffe ergänzt werden, welchen Sensoreigenschaften vornehmlich in elektrischer Isolation zum Kanülenschaft beigebracht wurden. Es kann auch die Flüssigkeitsabgabe über einen Schlauch mit Sensoreigenschaften an der Oberfläche erfolgen, dessen Lichtung in dichter Verbindung zur Bohrung des Spritzenansatzstückes oder -wo ein solcher nicht vorhanden ist- zum Infusionsschlauch in einem solchen rinnenförmig ausgehöhlten Kanülenschaft plaziert sein, ohne daß dessen freies Ende sich unbedingt über die Kanülenspitze selbst entleeren muß (bei sehr kurzem Kanülenschaft kann dies allerdings zweckmäßig sein, um die Arzneiinjektion unter die Haut zu garantieren). Wenn auch eine derartige oder ähnliche Sensorkanüle bei der Dialyse von Patienten mit Nierenfunktionsschwäche einsetzbar erscheint (bei quantitativer Erfassung von im Körper zurückgehaltenen Stoffwechselprodukten oder des Wasser- und Salzhaushaltes), so erscheint bei der Möglichkeit der Meßwerterfassung über längere Zeit auch die Anwendung in Verbindung mit Insulinpumpen mit liegenden Kanülen besonders vorteilhaft. Dabei kann kennzeichnenderweise die Unterteilung des Kanülenschaftes in mindestens zwei elektrisch voneinander isolierte Compartimente dazu geeignet sein, eine zusätzliche Vergleichselektrode außerhalb der Kanüle zu ersparen. Stromzu- bzw.abführung erfolgen über Leitungen, welche zweckmäßigerweise streckenweise innerhalb des Infusionsschlauches (in und durch dessen Wandung isoliert eingebettet) mit dem Meßgerät verbunden sind. Beim Einsatz der Sensorkanüle zur Einmalinjektion bietet sich die erfindungsgemäß neue Möglichkeit, daß der Sensorbelag der Kanüle unter dem Einfluß von Stoffwechselprodukten, insbesondere von Zucker oder spezieller von Glukose irreversibel verändert wird und aus dem Ausmaß dieser Veränderungen die Meßparameter gewonnen werden. Insbesondere kann Glukose an den Kanülen belag (beispielsweise an Pflanzenkohle oder fermentativ) gebunden werden und durch die Anreicherung des als elektrischer Nichtleiter isolierenden Zuckers die Leitfähigkeit der als guter Leiter gewählten Sensorbeschichtung in Abhängigkeit vom Zuckerangebot unter Berücksichtigung der Zeitfunktion herabgesetzt werden. Eine weitere Lösung ist die Miniaturisierung optischer Messungen und ihre Verlegung in den Bereich der Kanüle. Hierzu wird Licht durch wenigstens eine Lichtleitfaser oder direkt durch den Kanülenschaft hindurch gegen eine optische Indikatorzone gerichtet, und das von der unter Einfluß von Stoffwechselprodukten verändert zurückgeworfene Licht gemessen und ausgewertet. Eine andere Lösung ohne Farbindikatoren verwendet polarisiertes Licht, und die Eigenschaft mancher Stoffwechselprodukte (darunter auch der Glukose), die Polarisationsebene solchen Lichtes in Abhängigkeit von ihrer Konzentration zu verändern. Das polarisierte Licht wird erfindungsgemäß von einem Medium anderer Dichte, welches vorzugsweise im Bereich innerhalb der Kanülenspitze eingebracht wird, nach Kontakt mit dem Stoffwechselprodukt zurückgelenkt und mittels geeigneter Detektoren mit einem Referenzstrahl oder einem Eichstandart verglichen. Die Einhaltung der zur Messung und Injektion notwendigen Zeitspanne wird am besten bei der Anwendung der Sauginjektion gewährleistet, also dann, wenn durch Unterdruck innerhalb einer die Kanüle umgebenden Saugglocke die Haut in diese hineingehoben und solange festgehalten wird, bis die Saugglocke (automatisch gesteuert) wiederbelüftet ist. Bei Anwendung der Sauginjektion kann der Rand der Saugglocke (beispielsweise mit Silberchlorid-Silber belegt oder unter Anwendung eines Kontaktgeeles oder eines doppelseitig klebenden Elektrodenringes) als Vergleichselektrode benutzt werden. Anstelle der Hautanhebung durch Sog kann eine solche auch durch Pflasterzug erreicht werden. Der Abschluß der Messung und des Rechenvorganges zur Angleichung der zu verabreichenden Dosierung kann als Auslösesignal für die Dosierpumpe dienen. Damit entfällt die an und für sich vorteilhafte Injektionsauslösung durch Kontaktstifte oder durch Erdschluß der Kanüle innerhalb der hochgehobenen Haut. (Damit aber auch die Abhängigkeit eines "Erdschlusses", als eines Kontaktes mit dem Körper als einem Sender, welcher von der Nähe äußerer elektrischer Felder abhängig ist). Bei Ausfall der Meßvorrichtung erfolgt die Injektionsauslösung erfindungsgemäß mittels des Stromschlusses über die Haut. Die Kompensation einer schlechten Stoffwechseleinstellung innerhalb eines ärztlich programmierten Rahmens über als günstig errechnete zusätzliche Altinsulindosen oder durch den errechnet günstigen Abzug von Depotinsulindosen gehört zur wesentlichen Ausgestaltung der Erfindung. Aus ärztlicher Erfahrung bei der Patientenführung entspringt auch die Aufgabe, die Freigabe einer willkürlichen Zusatzdosierung von Altinsulin vor geplanten Mahlzeiten in ihrer Höhe ärztlich beschränken zu können. Andererseits ergeben sich Situationen, in welchen eine Programmautomatik zweckmäßig ist, welche gestattet, automatisch und ohne Mitwirkung des Arztes( insofern dieser diese Möglichkeit nicht bei ungeeigneten Patienten mittels der dafür vorgesehenen Vorrichtung sperrt) Fehlprogrammierungen allmählich richtig zu stellen und die Programmierung(wenn nötig auch ohne Mitwirkung eines Arztes) geänderten Ernährungs- und Lebensumständen anzupassen. Die gemessenen Werte und die verabreichten Dosen sowie die Art der Einflußnahme auf das eingestellte Programm können nach einer Ausgestaltung der Erfindung im Gerät ausgedruckt werden. Es kann auch zusätzlich oder anstelle des Druckers eine elektronische Speichervorrichtung für die Meßwerte (und die Dosierungen) vorgesehen sein, welche extern beim Arzt über einen Bildschirm sichtbar und in übersichtliche Kurven verwandelt werden. Selbst bei Anwendung einer Dauerinfusionskanüle kann die Möglichkeit einer Einzelmessung( zur Bestimmung der Stoffwechselausgangslage) nützlich sein. Ein erfinderischer Fortschritt ist auch die Programmierung über gerastert drehbare Schaltringe, welche zu Programmblöcken zusammengestellt sind, da sie den simultanen Überblick über den Ausgangspunkt der Programmierung festhalten. Eine drucktypenartige Erhabenheit der Programmsymbole und deren Aufstellung zu einer queren Programmzeile insgesamt erlaubt den Abdruck des vorgesehenen Programmes in der Patientenkartei oder anderwärts ohne besondere technische Anlagen. Die Sperrung gegen Verdrehung der Schaltscheiben über die eingestellte Raststufe hinaus durch seitliche Verklemmung mittels einer durch Schlüssel zu bedienenden Verschraubung wird durch seitliche Profilbildung auf den den Schaltringen untergelagerten Trägerringen erleichtert. Zwischen Trägerring und Rastring besteht jeweils ein rückgefedertes Spiel bei Kontaktführung zur Steuereinheit, so daß- auch etwa durch die Programmautomatik veränderte- Einstellwerte auf der Geräteanzeige sichtbar gemacht werden können. Es sind vom Patienten beeinflußbare Vorwarnsignale für die drohende Vorratserschöpfung von Arznei(oder auch Kanülen) vorgesehen und unter Rechnerkontrolle eine Wiederauffüllung eines nahezu erschöpften Arzneivorrates auf eine Enddosis, welche die fast völlige Entleerung des Vorratsbehälters erlaubt. Eine Verriegelung (elektromagnetisch oder mechanisch) kann die Nachfüllung durch den Patienten erzwingen, wobei dieser jedoch zu einer Verschiebung des Zeitpunktes durch Nachfüllung einer größeren Arzneimenge imstande sein soll.

Für die Fälle, in denen beispielsweise für Leitfähigkeitsmessungen die Möglichkeit der Beschichtung nur der Kanülenoberfläche sich ergeben würde (um den Kanülendurchmesser nicht verletzungserhöhend vergrößern zu müssen), ist noch vor Eindringen der Kanüle in die Haut von Seiten der Dosierpumpe die Abgabe einer klei-

nen Arzneimenge (Beispielsweise in Form eines einzelnen volumenverminderten Grundtaktes der Dosiervorrichtung) vorgesehen, um zu verhindern, daß es zu einer kapillären Ansaugung von Blut oder Gewebsflüssigkeit und zu einer Stromleitung im Nebenschluß über diese kommt. (Vorausgesetzt die Arznei ist praktisch ein Nichtleiter). Bei der Verwendung feinster Schläuche und Kapilaren innerhalb der Kanülenlichtung wird mit der Injektion zweckmäßigerweise schon vor Abschluß der Messung begonnen, um die Zeit des Injektorkontaktes in Grenzen zu halten. Der Injektionsdruck ist nämlich begrenzt wegen der Gefahr der Gewebsschädigungen. Der Rechner kann noch nach Injektion einer Grundrate eine begrenzte Sofortkorrektur einleiten.

Die Vorrichtung zur Sauginjektion selbst wurde erfindungsmäßig weiterentwickelt, nicht zuletzt zu deren Verkleinerung, welche es erst erlaubt ihr zusätzlich die Meß-und Steuereinheit für die Sensorkanüle noch anzugliedern, ohne daß die notwendige Handlichkeit verloren geht.Da der Unterdruckbedarf der Saugglocke verhältnismäßig gering ist und die menschliche Hand erheblicher Kraftkonzentrierung fähig ist, bleibt eine Kolben-Zylinder-Pumpe bei Kraftspeicherung in einer Feder eine wirtschaftliche Methode. Allerdings muß ein Sandfilter zwischen Vakuumpumpe und Saugglocke in den Sogkanal eingeschaltet werden, um den Zerstörungen der Kolben-Zylinderoberfläche durch feinste Quarzkörnchen vorzubeugen, wie sie aus den feinen Hautporen durch den Sog herausgerissen werden. Die Betätigung einer solchen Pumpe erfolgt raumsparend über Bowdenzüge. Dabei wird der Spannvorgang für die Feder zweckmäßigerweise mit der Schwenkbewegung des Deckels über der Saugglocke verbunden. Die Arretierung des Saugkolbens erfolgt von der Saugglocke aus unter Abschwächung der Losbrechkraft der Feder mittels einer Klemmvorrichtung oder der Haftung von

Dauermagneten. Auf diese Weise wird die Gefahr der Verkantung gemildert, wenn die Auslösung der Sogerzeugung einseitig exzentrisch von der Sauglocke her erfolgt. Die durch diesen Entlastungsmechanismus bewirkte Verzögerung begünstigt den Schluß eines federbeaufschlagten Überdruckventiles, welches die beim Aufsetzen der Saugglocke auf die Haut und bei Anhebung der Saugglocke gegen die Kanüle entstehende Luftdruckverdichtung aus gleicht. Die Anhebung des Saugglockenrandes gegen Federdruck und die Lösung der Arretierung der Rückbewegung der Saugglocke während deren Wiederbelüftung bewirken eine Distanzierung zwischen Kanüle und Haut nach der Injektion. Dieselbe Aufgabe kann auch durch einen Zentralstift gelöst werden, welcher zentral in der Saugglocke(wegen der Nähe der Kanüle günstigerweise im Zentrum einer eliptischen Saugglocke) gegen diese gedichtet während der Hautansaugung gehoben und während der Wiederbelüftung der Saugglocke unter Federeinwirkung gegen die Haut gedrückt wird. Die Federspannung für solche mit erheblicher Kraftaufwendung verbundenen Hilfsfunktionen wird günstigerweise beim Schließen einer Deckelklappe(mit Erinnerungsfunktion für mangelnde Einsatzbereitschaft des Gerätes) bewirkt, so daß der Druck des als Auslöser der Vakuumpumpe dienenden Zentralstiftes auf die Haut gemildert werden kann. Überhaupt wurden die Möglichkeiten erprobt, den Druck von Auslösestiften, welche von der Umgebung des Saugglockenrandes her diesen zunächst überragen, nach Aktivierung der Vakuumquelle von der Haut abzulenken. Dies geschieht durch die Einwirkung von Keilschrägen auf einen gefederten Ring, welcher reibungsarm nach Passieren der Keilschrägen die Stiftbewegung nach oben frei gibt. Eine Rückstellbewegung bis zur Einwirkung des gefederten Ringes auf die Keilschrägen erfolgt über eine elastische Manschette im Anschluß an den Saugglockenrand. Diese ermöglicht die Auslöserbewegung unter Sicherung des Hautabschlusses der Saugglocke,wobei die Federung

der Auslösestifte die gebotene Stabilität für den Saugglockenrand bewirkt, damit keine Außenluft unter dem Saugglockenrand hindurch während der Sogentwicklung eindringt. Bei den Unterdruck bewahrenden Speichertöpfchen, bei welchen der erforderliche Sog bereits bei der industriellen Fertigung vorbereitet wird, wird die Freisetzung desselben nach Sperrung während der Lagerung durch die Haltefunktion eines Deckels kurz vor der Injektion auf einen Auslösemechanismus im Gerät übertragen. Da sich die Stauwirkung der Haut gegenüber einem Ventilstift in einem Röhrchen parallel zu Kanüle als unzuverlässig erwiesen hat, wurde dieser Mechanismus dadurch verbessert, daß vom Ventilstift ausgehend ein federnder Bügel bogig die Saugglocke zur Haut hin überragt. Die Bügelenden liegen dem Sauglockenrand dabei auf. Bei manuellem Andruck de Töpfchens gegen die Haut wird der Bügel gespannt und schließlich ganz plötzlich in die Gegenrichtung umgebogen, wobei die Enden vom Saugglockenrand gezogen und in die Saugglocke gehoben werden. Der Ventilstift schnellt nach oben und durchbricht mit seiner Spitze die Membran über dem Ende seines Röhrchens, nachdem sie zuvor etwas ausgereckt wurde. Damit erfolgt der Luftdruckausgleich zwischen Unterdruckspeicher und Saugglocke.

Da die Wiederbelüftung der Saugglocke erst eintreten darf, wenn die Injektion abgeschlossen ist, wurde ein Federmechanismus vor der Anwendung des Gerätes auf der gespannt, welcher mit dem Ablauf des Injektionsmechanismus von diesem ausgelöst wird. Bei der einfacheren elektronisch verzögerten Auslösung wird ein Belüftungsventil vorgeschlagen, dessen doppelwirkender Magnet während der Sogerzeugung die Ventilmembran auf dem Belüftungskanal zur Anlage bringt(die Fixation besorgt dann der Unterdruck), um während der Wiederbelüftung die Ventilmembran vom Belüftungskanal entfernt zu halten. Der Magnet braucht auf diese Weise nicht gegen eine Rückstellfeder anzuarbeiten. In einem mechanich betriebe-

nen Beipiel unter Anwendung einer Dauervakuumquelle wird das Wiederbelüftungsventil durch die Betätigung eines Ventilstößels in der letzten Phase der Spritzenentleerung innerhalb eines sich zusammenziehenden Faltenbalges geöffnet, unterstützt durch die Anziehungskraft von Dauermagneten, welche auch ein Ventilflattern durch den Wechsel von Öffnungs- und Schlußstellung verhindern. Schließlich kann bei Konservierung eines größeren Vakuumspeicheraumes in ständiger Verbindung zur Saugglocke unter Betätigung des Unterdruckgenerators für die Dauer der Injektion der Wiederbelüftungsmechanismus durch eine Düse ersetzt werden, durch welche Luft allmählich nach Sogabschaltung in die Saugglocke eindringt. Auf dem Gebiet der Dosierung erscheint noch die quantitative Beförderung der Arznei über drei in bestimmter Reihenfolge verschlossene Ventile vorteilhaft, wozu vorzugsweise über von Randringen begrenzten Dosierkammern auf einem Stift im Arzneischlauch nahe dem Kanülenansatz von elektromagnetisch betriebenen Pumpen Schlauchringe mit einer Hilfsflüssigkeit gefüllt werden. Auch den Schlauch umgebende piezo-elektrische Stellelemente können als dosierende Ventile eingesetzt werden. Der Betrieb einer Dosierpumpe zur Beförderung einer(in der Regel zähflüssigeren)Flüssigkeit wie Paraffin, vermeidet den Kontakt von Pumpenteilen mit der Arznei und erspart die Reinigung. Es kann die Hilfsflüssigkeit auch hinter den Kolben einer Stufenspritze eingebracht werden, wobei der Effekt einer Dosierhydraulik mit kraftvollem und langsamem Vorschub des Kolbens der Stufenspritze entsteht. Der Kolben kann aus Wachsbestehen oder von einem wachsartigen Mantel umgeben sein, wodurch die kostspielige schleifende Passung vermieden wird. Ein besonderes Koppelungsventil bestehtaus einem Schieberventil mit Rückfederung im Geräteteil und einer Art verschieblicher Manschette über einer seitlichen Öffnung für den Zutritt von Hilfsflüssigkeit in die Stufenspritze, wobei die Manschette zwangläufig bei Einfügung der Stufenspritze in das Gerät verschoben wird.
Auf diese Weise wird eine luftfreie Koppelung beider
Flüssigkeitssysteme gewährleistet. Der Raumbedarf für
die Hilfsflüssigkeit kann im Wesentlichen erspart werden, wenn nur eine den einzelnen Dosierhub und den Leckverlust überschreitende Menge über die Dosiervorrichtung zwischen Trennkolben und einem Stützkolben eingespritzt wird, und der Stützkolben dem Trennkolben über
der Arznei jeweils nachgeführt und dann bewegungsgesperrt wird. Die Hilfsflüssigkeit pendelt auf diese
Weise zwischen Dosierpumpe und Zwischenkolbenraum und
Trennkolben und Stützkolben bewegen sich abwechselnd.
Auf dem Gebiet der Vakuumerzeugung ist noch eine Art
Schleußenventil im Anschluß an die Rinne auf dem Öffnungsdorn einer Druckgaspatrone von Bedeutung, worin
ein kleindimensioniertes Sitzventil innerhalb des Tellers eines größeren Sitzventiles zunächst mit geringerem Kraftaufwand aufgestoßen wird. Das unter hohem
Druck stehende Gas staut sich dann in einer Kammer,
welche hinter den Sitzventilen von einem Schieberventil verschlossen wird. Infolge des Druckausgleiches
in der Kammer kann dann auch das große Sitzventil
mühelos aufgestoßen werden. Über das geöffnete Schieberventil gelangt der Gasstrom dann zur Gasstrahlpumpe. Die Anhebung der Gaskapsel in den Öffnungsdorn
kann mittels eines Quellstiftes (beispielsweise aus
Agar-Agar) unter Wasseraufnahme erfolgen.
Ein automatischer Kanülenwechsel wird durch eine Stapelkanüle erleichtert, wobei bei vertikaler Anordnung
innerhalb eines Magazinrohres oder -schlauches der jeweils vorausgehende Kanülenschaft in einer Aushöhlung
des Körpers der nachfolgenden Kanüle liegt und die
Arzneizufuhr seitlich über eine Ringnut im Kanülenkörper mit sich in den Kanülenschaft öffnender Bohrung erfolgt. Am Ende der Kammer im Körper für die Aufnahme
des benachbarten Kanülenschaftes kann ein elastisches
Septum von letzterem zentral durchbrochen sein. Zur Ermöglichung einer Lagerung in gebogenen Magazinen kann auch ein Einschnitt zwischen zwei Kanülenkörperhälften vorhanden sein, um welchen die Abbiegung bei Elastizität des Materials erfolgen kann. Die Arzneizufuhr kann auch über vertikale Rinnen oder Kanäle entlang des Kanülenkörpers erfolgen. Verklebungen oder fadenförmige Anheftung zwischen Kanülenkörpern kann einen Transport durch Zug ermöglichen, welcher vorzugsweise von den Klauen am Ende des Aufnahmeröhrchens innerhalb des Saugglockendeckels ausgeht. Druckwirkung auf die Stapelkanülen kann von einer Druckfeder ausgehen, welche durch Schubwirkung verbrauchter Kanülen gespannt wird, wenn diese im Kreislauf aus dem Aufnahmeröhrchen über das Magazinschlauchende manuell nachgeschoben werden. Diese Schubbewegung hat für das Nachrücken einer Stapelkanüle in eine Öffnungsstellung in Deckung mit den Arzneiaustrittsstellen im Magazin Bedeutung. Als Schaltmechanismus kann eine mittels eines Schaltstiftes am Deckel betätigte Blattfeder dienen.

Auch die Anhebung des Zentralstiftes in der Saugglocke kann über einen Kugelraster das Tiefertreten einer Kanüle erlauben, was in diesem anderen Lösungsbeispiel unter Einwirkung von Druckgas auf einen Dichtkolben im Magazinschlauch geschieht.(Fig.18,Fig.19). Abplattung oder ovale Formgebung von Magazin und Kanülen können als Drehsicherung dienen und erlauben die Wirksamkeit der Gelenkelemente zwischen den Kanülen sowie eine bessere Anlage und Abdichtung der arzneiführenden Kanäle(Fig.27).

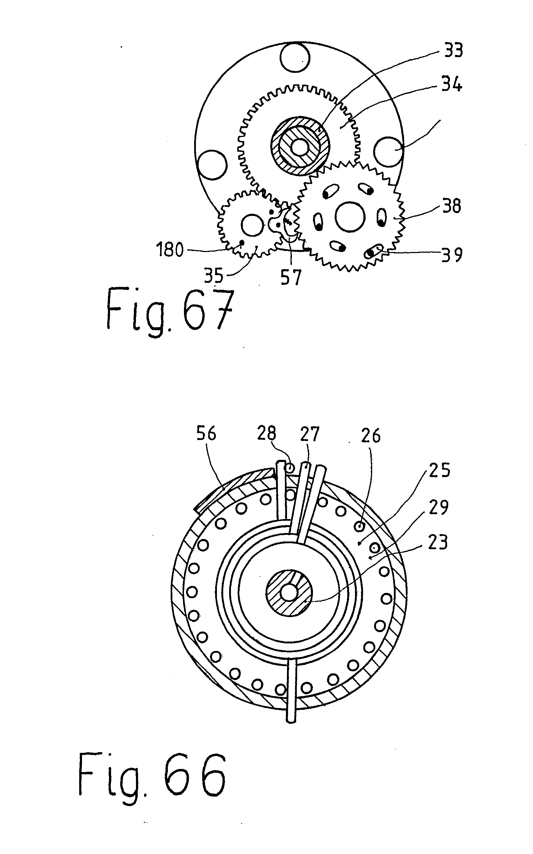

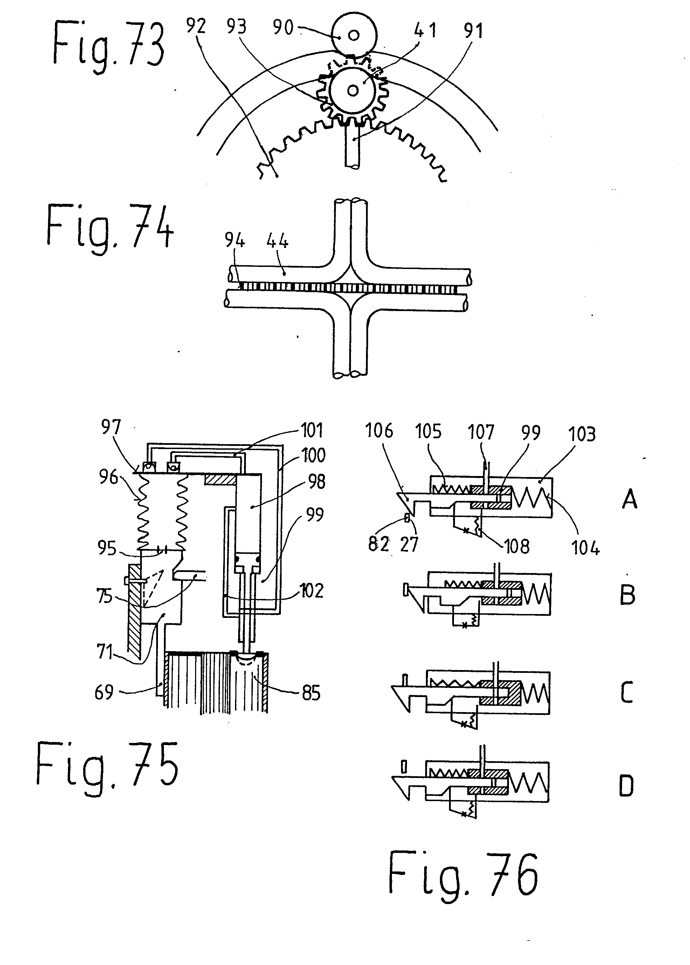

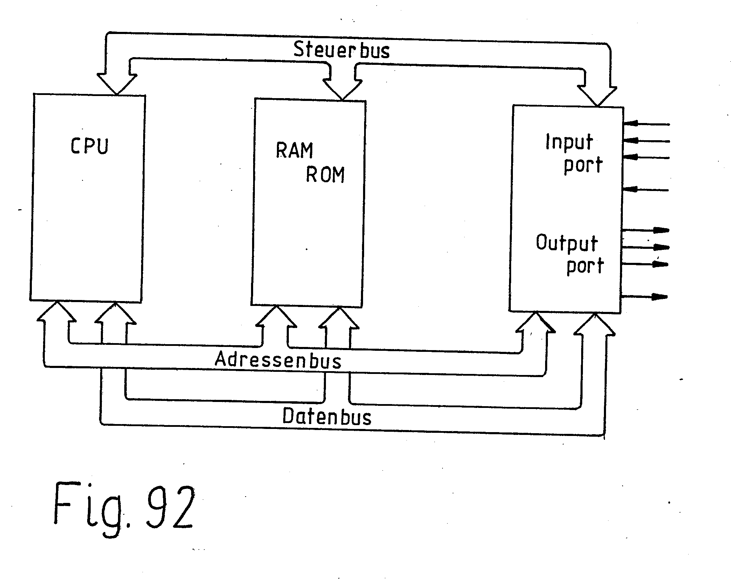

Zur Durchmischung der Insulinbestandteile im Bereich einer Schlauchpumpe, welche mittels einer Spiralfeder betrieben wird, wird auf der Achse derselben mittels einer zweiten Spiralfeder mindestens ein auf einer Scheibe befestigter Stift so über eine zahnradartige Unebenheit der gegenüberliegenden Fläche der die Rollen der Schlauchpumpe tragenden Scheibe bewegt,daß eine ruckartige,sich wiederholende Ausweichbewegung der Schlauchpumpe gegen eine Feder bewirkt wird. Die Dosiergenauigkeit bei kleinen Umfangsmaßen wird dadurch erhöht, daß zwei Schläuche parallel über zwei gleichartig wirkende Schlauchpumpen geführt werden; weiterhin dadurch,daß die Schläuche sich innerhalb der Pumpe nur zwischen den Auskehlungen der die Rollen tragenden Scheiben ausdehnen können und dadurch,daß der beispielsweise wieder vereinigte Schlauchabschnitt zwischen Pumpe und Kanüle zur Verringerung der Windkesselfunktion mit einem festen Mantel umgeben wird. Eine noch bessere Lösung ergibt sich aus der Einbringung einer kleinen Kolbenpumpe unmittelbar vor dem Kanülenansatzstutzen, wobei diese in vorprogrammierten Pumpentakten entweder über die Kulissenführung einer federkraftbetriebenen Scheibe oder durch dosisentsprechend wiederholte Betätigung eines Elektromagnetes die Arzneiabgabe bewirkt. Die Lagerung der Kolbenpumpe innerhalb des Arzneischlauches bei Betätigung von außen über die Vermittlung einer Querplatte am Pumpenstössel erspart die Reinigung und erleichtert das Auswechseln des Dosiersystems gemäß der Gesamtaufgabe der Erfindung. Besonders vorteilhaft hinsichtlich der Hersstellungkosten erweist sich der Ersatz eines Paßkolbens durch eine elastische Manschette, deren Aushöhlung gegen eine Anlagefläche beispielsweise eines durchbohrten mit dem Schlauch seitlich verschweißten Stopfens mittels des Stößels zur Dosisabgabe gepreßt wird, während das Ende des durch die Bohrung des Stopfens hindurch geführten Stößels verbreitert ist und im Gegentakt die Stopfenbohrung zum Kanülenansatzkonus hin verschließt. In besonders vorteilhafter Weise kann ein besonderes Pumpengehäuse erspart werden, wenn der Stopfen(114) direkt in eine Schlaucherweiterung einvulkanisiert wird, wobei die Schlaucherweiterung sich innerhalb einer Achsenbuchse des Injektors nur begrenzt ausdehnen kann(Fig.92). Der Antrieb der Dosiervorrichtung übernimmt hier zusätzlich die Funktion des Rüttlers zur Arzneidurchmischung und vermeidet die Dauerquetschung des Schlauches durch die Pumpenrollen, welche auch dann noch problematisch bleibt, wenn wie in dem aufgezeigten Beispiel eine Klemmung des Schlauches durch die Rollen immer am gleichen Ort praktisch durch Ablauf der Rollen in einer Richtung unter Addition der Dosierstrecken mit hoher Wahrscheinlichkeit vermieden wird. Jeweils einen Schlauch zwischen sich einschließende Auskehlungen der Trägerscheiben für die Dosierrollen vermindern die Dehnung der Schläuche innerhalb der Pumpe, welche mittels Federzuges am Ende der aus der Pumpe austretenden Schläuche zusätzlich ausgeglichen wird. (Fig.66). Es können auch jeweils zwei Rollenpaare radiär so übereinander angeordnet sein, daß sie die Schläuche zwischen sich einschließen(Fig.74). Auch eine Fixierung des Schlauchpaares über einen strickleiterartig für einen feststehenden Zahnkranz unterbrochenen Quersteg zwischen den Schläuchen, kann der Zugwirkung der Rollen begegnen(Fig.75).

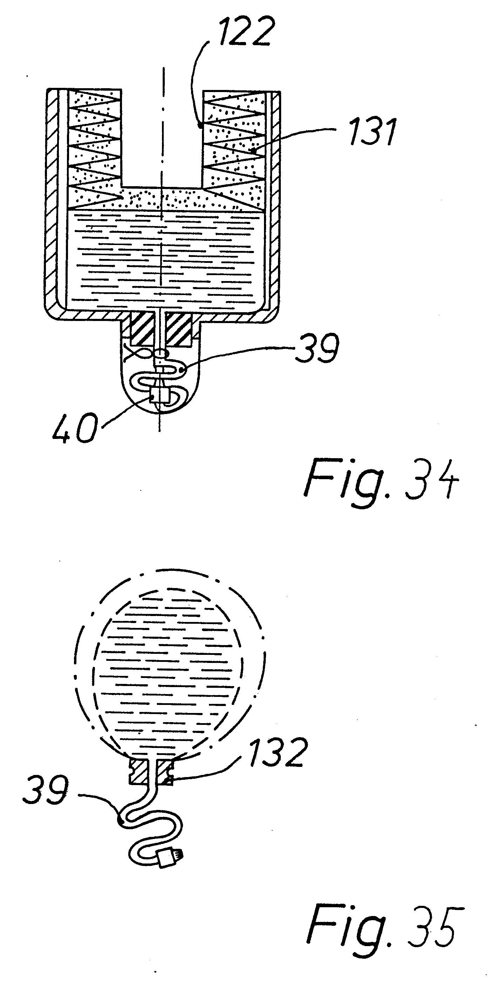

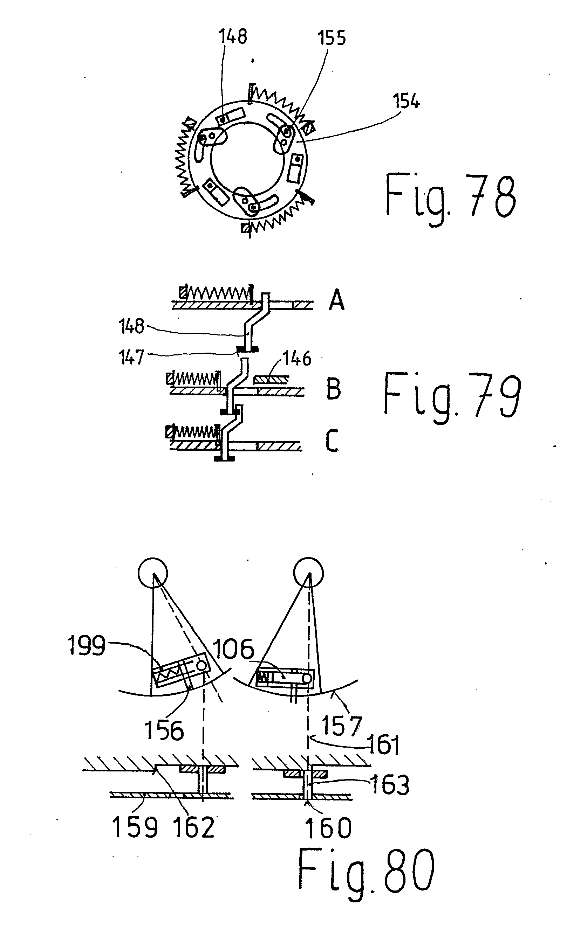

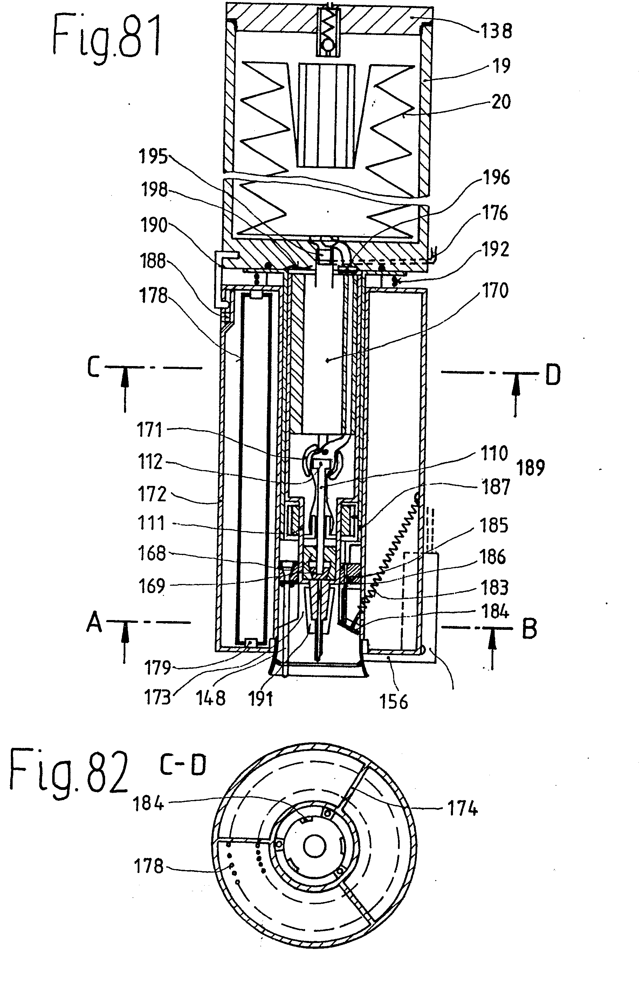

Auf dem Gebiet der Arzneilagerung im Vorratsgefäß wurde als Vereinfachung eine sich leicht zusammenziehende dünnwandige Membran, also ein einfacher Beutel in Betracht gezogen(Fig.66). Zur Arzneibeförderung kann dieser innerhalb eines weiteren starrwandigeren Beutels angeordnet sein bei Druckgaseinlagerung zwischen den Beuteln(Fig.35). Da aber Arzneivorschub mittels Druckwirkung im Behälter ein luftblasenfreies Dosieren(Luft kann auch bei Unterdruck im Schlauchsystem von der Kanüle her eindringen oder es könnte eine Rücksaugungswirkung über die Kanüle in Richtung Körpergewebe entstehen) wesentlich erleichtert, wurden Verbesserungen auch hier getroffen und zwar in der Weise, diesen Druck auf den Arzneivorrat gleichmäßig zu gestalten vom Beginn bis zum Ende der Arzneivorratserschöpfung. Dies wird durch ein im Verhältnis zu den abzugebenden Flüssigkeitsdosen erfolgendes Herabschrauben des Deckels gegen den arzneiführenden Faltenbalg bewirkt(Fig.78). Dadurch wird eine Grobdosierung ähnlich wie bei Verwendung einer Stufenspritze erzielt, welche jedoch durch die Kolbenpumpe für die Feindosierung ergänzt wird. In einem zweiten Beispiel mit Druckgaspolster(Fig.82) wird dieses durch verzögerte Freisetzung von Kohlesäuregas bei Einwirkung von verdünnter Salzsäure auf ein Karbonat bewirkt, wobei letzteres auf zusammengerollter Polyäthylenfolie durch einen von Salzsäure allmählich abgebauten Pektinfilm geschützt werden kann. Ein Überdruckventil verhindert ständig übermäßigen Druckanstieg. Der den Faltenbalg aufnehmende Vorratszylinder kann zweckmäßigerweise ein Gerätebestandteil sein, während der Faltenbalg mit der Arzneiflüssigkeit jeweils ausgetauscht wird. Für die Erzeugung von Kohlensäure in einer Menge, welche zur Austreibung von 50ml Flüssigkeit bei einem Restdruck von 1 atü sind 375 mg Natrium-Bikarbonat und 815 mg 20%ige Salzsäure erforderlich. Bei Verwendung von Calzium-Karbonate wären hiervon 446 mg erforderlich. Für die Bindung an eine Folie ist das hitzebeständigere Calzium-Bikarbonat vorzuziehen, da es erhitzt über die Folie geblasen werden oder auf eine auf 100 bis 120 Grad Celsius erhitzte Polyäthylenfolie aufgestreut werden kann.

Die Vorwarnung vor dem Verbrauch des Arzneivorrates ergibt sich bei der Lösung durch einen allmählich abgesenkten Schraubdeckel - was in einem Zylinderschlitz entlang einer Dosierskala verfolgt werden kann- aus der Anbringung eines entfernbaren Hinder-

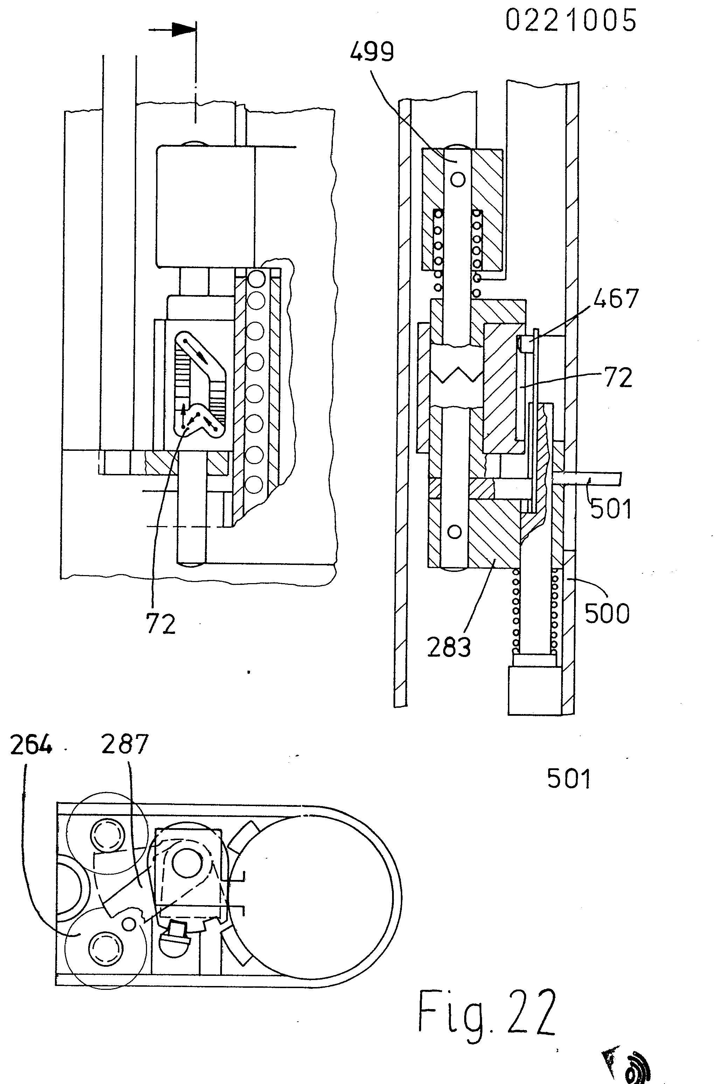

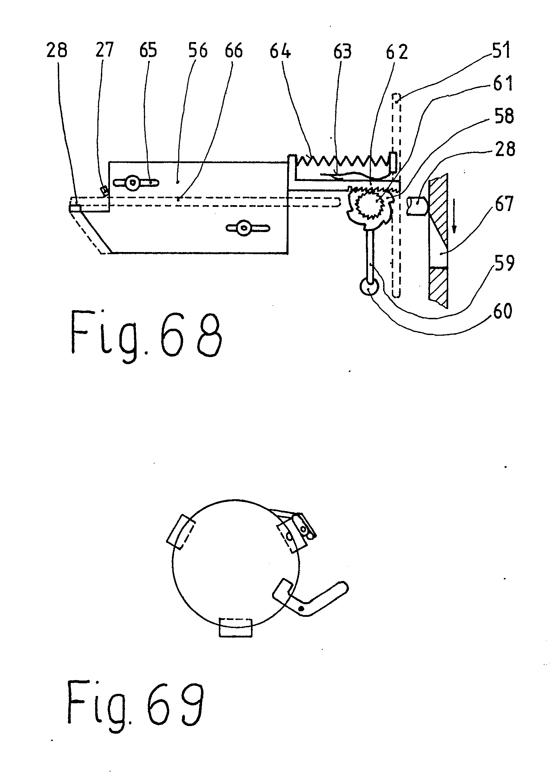

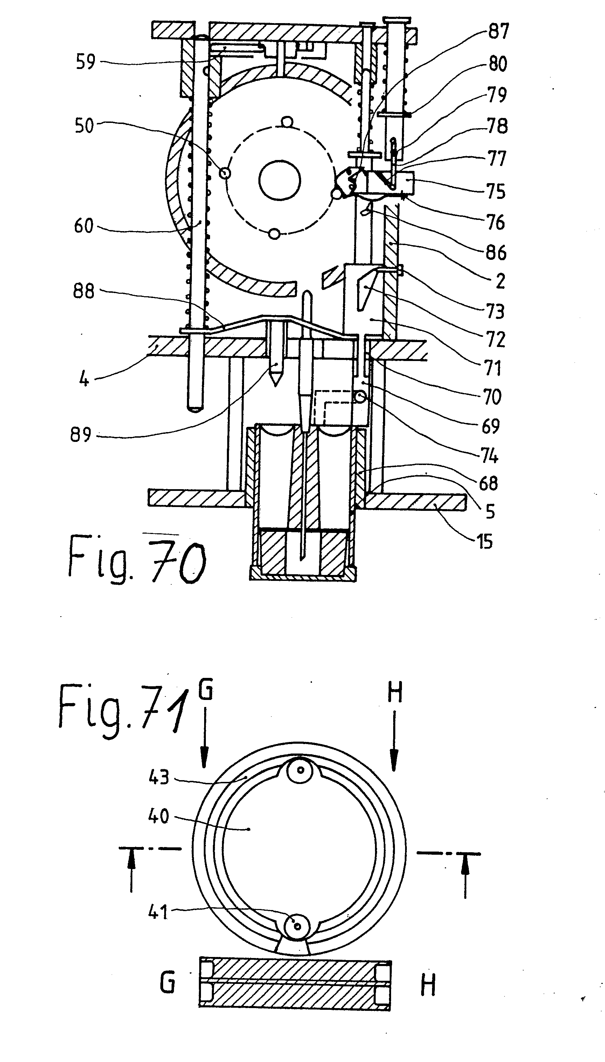

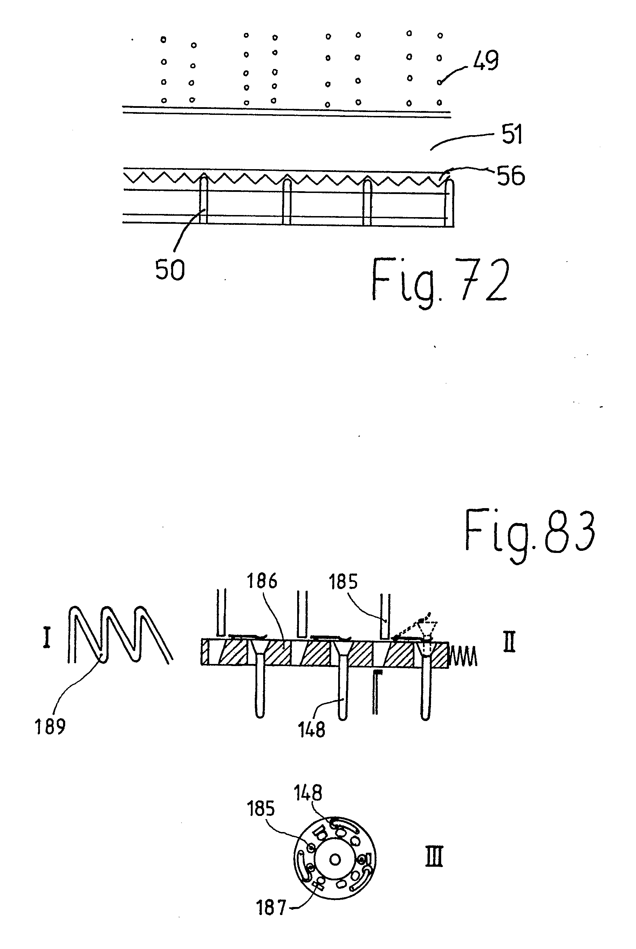

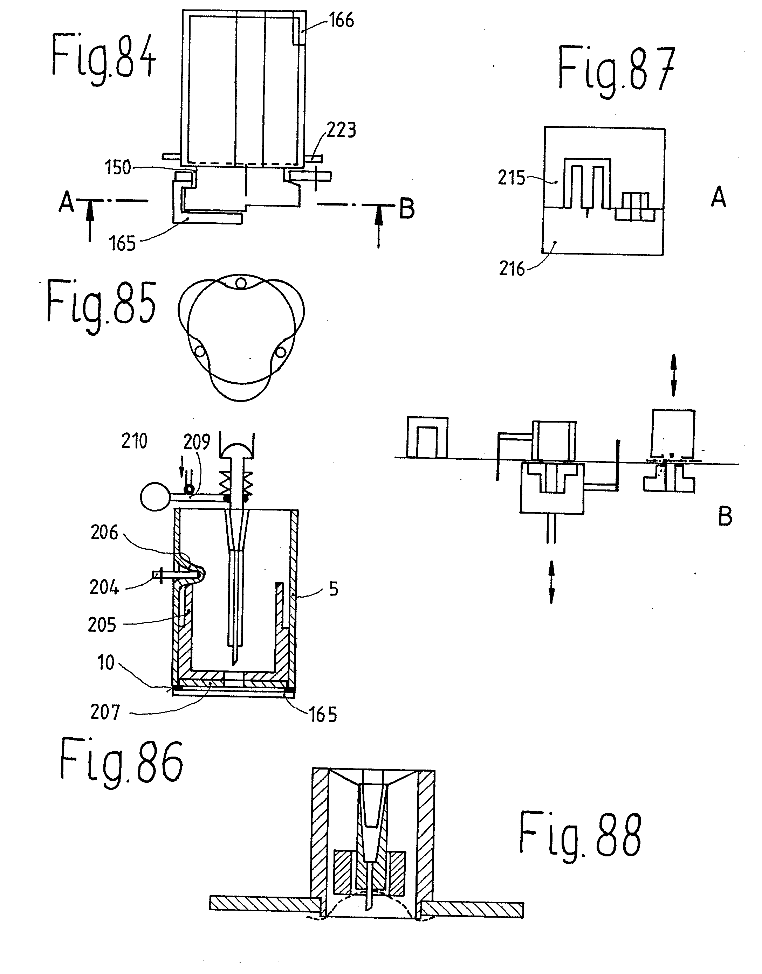

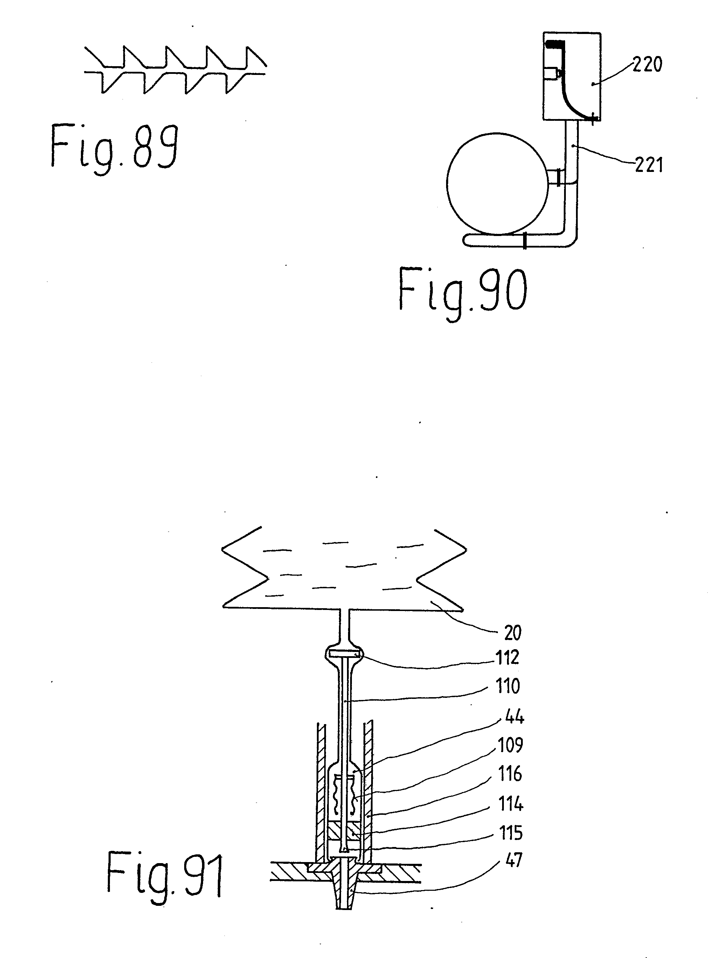

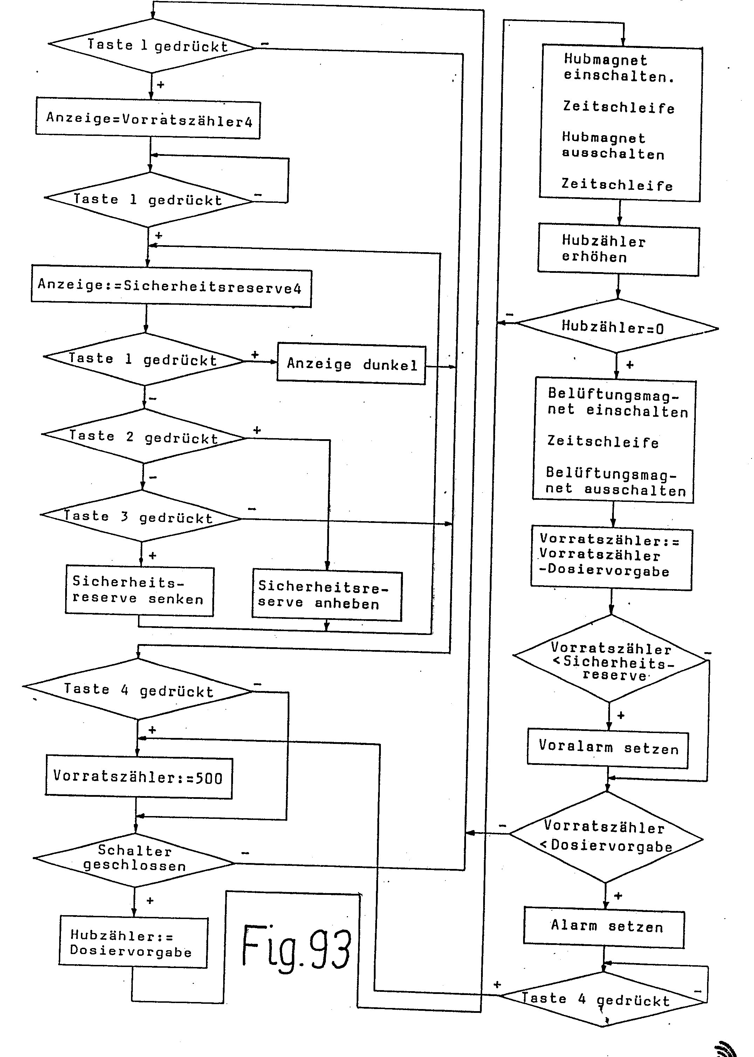

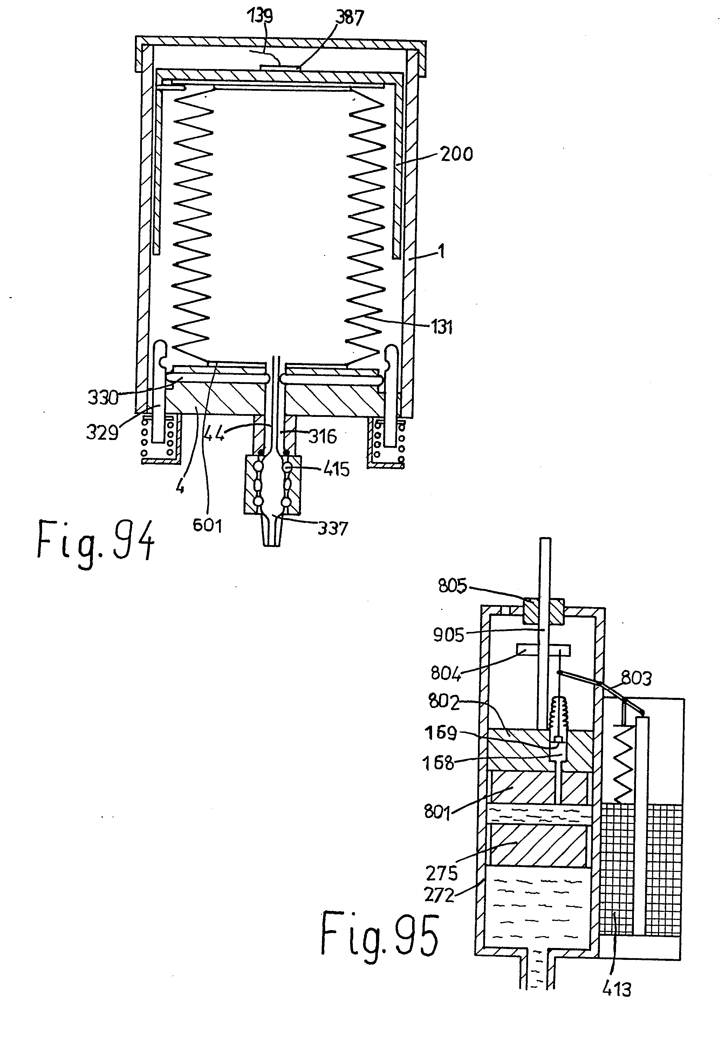

nisses gegen den herabtretenden Schraubdeckel im Punkte des unteren Grenzstandes für eine noch ausreichende Einzeldosis vor der Vorratserschöpfung(Fig.78). Es kann auch -besonders innerhalb der schlecht einsehbaren Druckgasspeicherflaschen(Fig.82)- zwischen Faltenbalg und Behälterdeckel ein Faden befestigt sein, welcher bei Straffung eine Warnvorrichtung betätigt, wie sie als elektronische Melodiegeber in Glückwunschkarten heute preiswert erworben werden können. Schließlich kann der Arzneiverbrauch auch elektronisch rechnerisch überwacht(Fig.93) und ablesbar(Fig.94) werden. Dabei kann ein Vorwarnungsspielraum gewählt und die Funktion des Injektors bei die Einzeldosis unterschreitender Restmenge gesperrt werden. Die Funktionssteuerung wurde vielfältig verbessert. So wird bei den federmechanisch betriebenen Lösungen, wie bei der Schlauchpumpe, die Steuermimik für Auslösung der Einspritzung oder Wiederbelüftung zugleich mit dem Einschub des kanülentragenden Unterdruckspeichers vorgespannt; es muß also nicht umständlicherweise ein besonderer Hebel betätigt werden, sondern als Stellglieder findet sich mindestens ein Stift, welcher nach unten den Kanülenansatzstutzen überragt. Zur Kontrolle des Hautkontaktes des Saugglockenrandes finden sich drei Stifte in Buchten außerhalb des kleblattartig gestalteten Saugglockenrandes in einem Beispiel(Fig.86), was ermöglicht, die Auslösestifte mehr dem Gerätezentrum zu nähern ohne die Haftung durch geringere Hautkontaktfläche zu mindern. Innerhalb der Saugglocke gelagerte Stifte müssen zum Saugglockendach(Fig.22) hin gedichtet werden. In dem dargestellten Beispiel bewirken solche Stifte zunächst die Drehung eines Ringes gegen dessen Federung,um dann reibungsarm nach oben treten zu können(Fig.80). Durch die Drehung jenes Ringes kommen von der Gegenrichtung her bewegte Stifte mit Öffnungen im Ring in Eingriff und die durch manuellen Druck bewirkte Senkung kann das Ventil zwischen Saugglocke und Unterdruckspeicher eröffnen. In dem geschilderten Beispiel wird der Unterdruck in drei gleich großen Kammern erzeugt, welche ringförmig um und über der Saugglocke angeordnet sind(Fig.83) und zwar über die Luftvertreibung mittels elektrischer Heizdrähte über das Lichtnetz. Die Kammerbeheizung wird über einen Temperaturfühler(beispielsweise Bimetallschalter) oder einen Kurzzeitschalter nach Erreichen der Höchsttemperatur abgestellt, die Kammern dann abgekühlt. Gefederte Ventilklappen lassen die Heißluft aus den Kammern über die Saugglocke austreten, was als Nebenwirkung noch deren Reinigung dient, während Stifte von oben jeweils eine der Ventilklappen für den Luftdruckausgleich zwischen Kammer und Saugglocke aus ihrem Sitz hebeln. Da ein breiter hutkrempenartiger Rand um den Saugglockenrand ohnehin nützlich ist,um das Verkippen eines gestreckt gebauten Injektors auf der Haut und damit einen ungewollten Lufteintritt in die Saugglocke zu vermeiden, fällt der höhere Raumbedarf der Heizkammern nicht so ins Gewicht gegenüber der Ersparnis des industriell evakuierten Speichertöpfchens um die einzelne Kanüle herum. Die Aufteilung des ringförmigen Mantelraumes um und über der Saugglocke in drei Einzelkammern erlaubt die dreimalige Benutzung des Injektors nach jeder Aufheizung. Auch die unterdruckbewahrenden Speichertöpfchen wurden wesentlich verbessert. So erlaubt ein membranöser Deckel um den Kanülenöffnungskonus nach Lösen einer Abstützung an der Kanüle oder am die Kanüle spritzgußtechnisch bedingt umschließenden Stift(Fig.66,Fig.89) ein Tiefertreten der Kanüle in die Haut. Bei anderen Beispielen wird die Saugglocke durch eine gummielastische Manschette des Injektors gebildet(Fig.78) in welche der Unterdruckspeicher zu diesem gedichtet hineingeschoben wird. Ein solcher Speicher des Zweikammersystems, bei welchem zwischen Speicherraum und Saugglokkenraum unterschieden wird und die Kanüle ungedeckt hervortritt, ähnelt dann einem Speicher des Einkammersystems, bei welchem die Kanüle insgesamt und bis zur Spitze hinter der Abschlußmembran liegt. Der Totraum um den freiliegenden Teil des Kanülenschaftes kann sogsparend dadurch verkleinert werden, daß er von einen Ringkolben ausgefüllt wird. Auf ein besonderes Ventil zwischen Saugglocke und Vakuumspeicher kann verzichtet werden, wenn als Trennwand zwischen Ringkolben und Unterdruckspeicher eine dehnbare Membran verwendet wird, wie sie etwa als Haushaltsfolie(z.B."Frapan") Verwendung findet(Fig.78,Fig.85). In diesem Falle muß der Ringkolben aber vor dem Gebrauch zum Unterdruckspeicher hin festgehalten werden. Erfindungsgemäß kann er hierzu eine Quernut aufweisen, in welche von einer Haltemanschette oder von Deckelausläufern aus Haltezungen vom unteren Rand des Unterdruckspeichers in diese Quernut sich erstrecken und so die Einwärtsbewegung des Ringkolbens unter Vakuumeinfluß vor Gebrauch verhindern. In Abschnitte der ringförmigen Quernut zwischen diesen Haltezungen schnappen bei Einschub in den Injektor Haltestifte oder -lamellen, welche nach Entfernung der Haltemanschette oder des Saugglockendeckels das Festhalten des Ringkolbens bis zur Injektionsauslösung übernehmen. Herstellungstechnisch kann die aufwendigere Quernut dadurch ersetzt werden, daß in derselben Form mit dem Unterdruckspeicher ein Ringkolben mit offener Ringkerbe gefertigt wird. Letzterer wird dann über ein Förderkarussell zu einer Klebevorrichtung -beispielsweise einem Ultraschallkopf- transportiert und mit einem kreisförmigen dehnbaren Folie verbunden. Dann wird der Ringkolben wieder unter einen Unterdruckspeicher gebracht und die Ränder der kreisförmigen Folie mit dem Rand des Unterdruckspeichers verklebt. Dieser Rand ist hierbei erfindungsgemäß hutkrempenartig verbreitert

0221005

- 32 -

und kann zugleich eine lineare Dichtungsberührung mit
der Saugglockenmanschette des Injektors aufweisen.
Die Quernut beziehungsweise die Ringkerbe kann in abwechselnden Sektoren waagerecht oder schräg gestaltet
sein um einerseits die Sperrwirkung der Deckelzungen
oder Manschettenstifte zu unterstützen, andererseits
aber das Entsperren der Halte-stifte oder-lamellen des
Injektors zu erleichtern(Fig.85). Da der Deckel des
Unterdruckspeichers in diesem Lösungsbeispiel starr gehalten ist, erfolgt bei Gebrauch die Kanülenannäherung
außer durch die Anhebung der Haut durch die Nachgiebigkeit der Saugglockenmanschette. Die Vergrößerung des
Bewegungsspielraumes zwischen Haut und Injektor kann
zur Freisetzung der Unterdruckwirkung auf die Haut innerhalb der Saugglocke dienen aber auch auf einer nachfolgenden Schaltstrecke der Auslösung der Dosiervorrichtung zur Einspritzung. Im Beispiel der Figur 66 ist
die korrekt senkrechte Bewegungsführung des Injektors
gegen die Haut dadurch Voraussetzung der Auslöserbetätigung, daß die Anhebung der Saugglockenmanschette nur
gleichzeitig mit der Anhebung eines Zylindersegmentes
erfolgen kann, welches zur Außenfläche der zylindrischen Aufnahme für den Unterdruckspeicher einen engangepaßten Ring aufweist, welcher bei einseitiger
Bewegungsführung sich gegen die Wandung der Aufnahme
verklemmt. Auf diese Weise ist gewährleistet, daß die
Auslösung der Einspritzung erst nach sicherem und
durch Unterdruck unterstütztem Kontakt der Saugglocke
mit der Haut erfolgt. Im Beispiel der Figur82 erfolgt
die Aktivierung der Rotationspumpen erst nach Feststellung der Unterdruckwirkung auf die Haut, wie schon
in früheren eigenen Patentanmeldungen. Als "Sogschalter" dient aber in diesem Falle ein Taster, welcher
Formveränderungen des Unterdruckspeichers kontrolliert. Diese Formveränderungen betreffen in den dargestellten Beispielen den Deckel und den Kanülenöffungskonus. Dieser kann -wie geschildert- membra- nös gestaltet sein und sich vor der Injektion absenken. Eine umschriebene elastische Partie wird unter dem Einfluß des Speicherunterdruckes nach innen gezogen; nach der Verminderung des Unterdruckes durch Ausgleich mit dem Saugglockenraum wird diese Membran sich leicht anheben. Die dabei nutzbare Leistung ist aber gering. Deshalb wurde ein bei geringer Reibung arbeitendes Schieberventil als Schaltglied vorgesehen. Der zugehörige Taster wird zunächst mittels eines pneumatischen Kolbens gegen die elastische Membran vorgeschoben, welcher durch einen Faltenbalg betrieben wird, welcher beim Einschieben des Unterdruckspeichers in den Injektor zusammengedrückt wird und solange zusammengedrückt bleibt, bis über das genannte Schieberventil eine Wiederbelüftung erfolgt. Die fakultativ noch durch eine Zusatzfeder verstärkte Wiederausdehnung des Faltenbalges betätigt das Schaltglied für die weiteren Funktionen des Gerätes(Fig.76). Ein leichter Randüberstand einer aufgeschweißten Deckelmembran kann einer federnden Haltevorrichtung Widerstand bieten, wodurch sich eine kostspieligere Querrinne im Kolben vermeiden läßt. Andererseits kann eine keilförmig auf den Deckel seitlich einwirkende Vorrichtung für die Entfernung des Unterdruckspeichers nach Gebrauch nützlich sein(Fig.70). Auch das Einkammersystem wird wieder wirtschaftlich tragbar, wenn der aufwendige Auslösemechanismus für den die Membran tragenden Saugglockendeckel auf den Injektor selbst übertragen wird(Fig.87). Eine dehnbare elastische Membran überspannt einen Bodenring innerhalb des Unterdruckspeichers, welcher seinerseits einen hohen Führungszylinder aufweist, der locker in jenes Unterdruckspeichertöpfchen eingeschoben ist. Ein innerer Teil jenes Bodenringes ist mit einem weiteren ringförmigen Deckelring verklebt, welcher mit seinem Rand dem unteren Rand des Unterdruckspeichertöpfchens aufliegt und nach Evakuierung des letzteren eine Hebung des Bodenringes solange verhindert, bis ein Randstreistreifen jenes Deckelringes längs einer Sollbruchlinie durch Herabziehen entfernt wird. Bestimmungsgemäß erfolgt dies erst, nachdem vom Injektor aus Stifte oder Rastsegmente in der Höhe oberhalb jenes inneren Führungszylinders an dessen Rand durch materialaussackende Wandverdünnungen des Unterdruckspeichertöpfchens hindurch die Abstützfunktion für die Bodenmembran übernehmen. Beim Einkammersystem liegt die gesamte Kanüle hinter der Deckelmembran geschützt, wobei der Nachteil der Abstumpfung der Kanülenspitze beim Membrandurchtritt bei der früher verwendeten Gummimembran bei der hochelastischen Kunststoffolie weniger ins Gewicht fällt. Der Umstand, daß nach Eröffnung des Membranverschlusses durch den Kanülenansatzstutzen in diesen hinein der Speicherunterdruck wirksam wird, kann dazu ausgenutzt werden, den Unterdruck seitlich durch Kollaps eines elastischen Bällchens sichtbar zu machen. Wird durch die für tiefere Injektionen vorbestimmte längere Kanüle ein Blutgefäß getroffen, so füllt sich nach Lösen der Klemme(210) das Bällchen mit Blut auf. Die Fehlinjektion in das Blutgefäß kann in einem solchen Falle durch Abbruch der Injektion verhindert werden(Fig.87). Das Einkammersystem weist den größten Unterdruckspeicherraum im Hinblick auf die Gesamtgröße des Unterdruckspeichertöpfchens auf. Auch ohne äußere Verpackung ist Sterilität der Kanüle gewährleistet. Die Funktionssteuerung für die Wiederbelüftung der Saugglocke wurde dadurch verbessert, daß jeweils ein leicht zu betätigendes Schiebeventil zunächst unter Kraftspeicherung für die Rückführung des Ventilstößels vor Betätigung der Dosiervorrichtung geschlossen wird. Der Ventilrückstellmechanismus wird dabei aber solange blockiert, bis der Dosiermechanismus vollständig abgelaufen ist, wobei jener durch Auslenkung eines bewegten Sperrgliedes an einer Steilflanke der Kulissenführung am Gehäuse aktiviert wird(Fig.69,Fig.77,Fig.81).

Auf diese Weise wird der Gefahr einer Wiederbelüftung noch vor Verabreichung der der gesamten Arzneiflüssigkeitsmenge begegnet und dem zusätzlichen Risiko, daß während des Rückzuges der Kanülenspitze durch die oberen Hautschichten auch dorthin- also intrakutan- Arznei eingespritzt wird. Die Durchmischung der Arzneibestandteile kann auch dadurch bewirkt werden, daß der Summer der elektronisch gesteuerten Warnvorrichtung mechanisch so mit den arzneiführenden Schlauchteilen um die Pumpe gekoppelt wird, daß sich dessen Schwingungen dem Schlauchinneren mitteilen(Fig.91) und zwar auch gerade kurz vor der Injektion. Ein Randring(223) am unterdruckbewahrenden Zylinder(5) dient sowohl der besseren Anbringung der Grenzmembran (6) als auch der günstigeren Abdichtung zum Injektor hin(Fig.85).

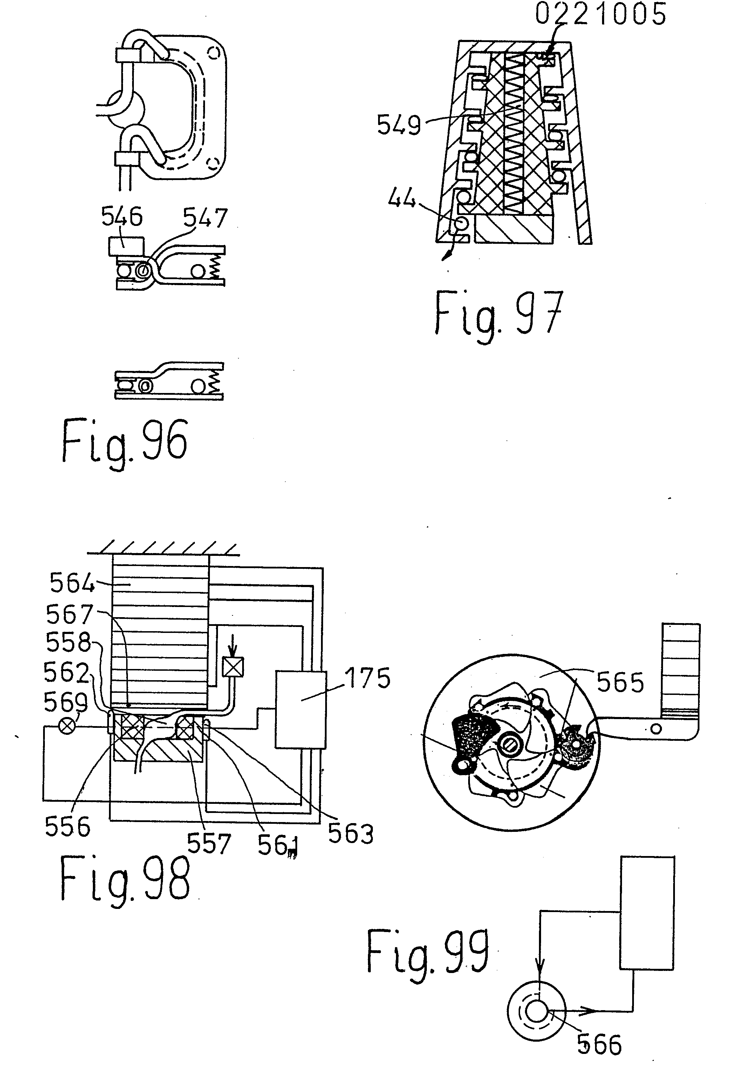

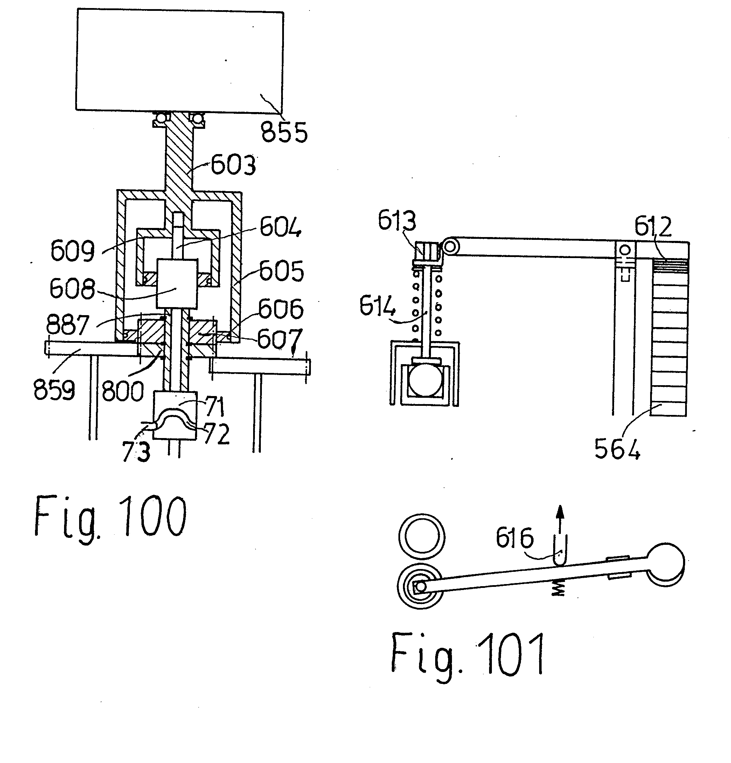

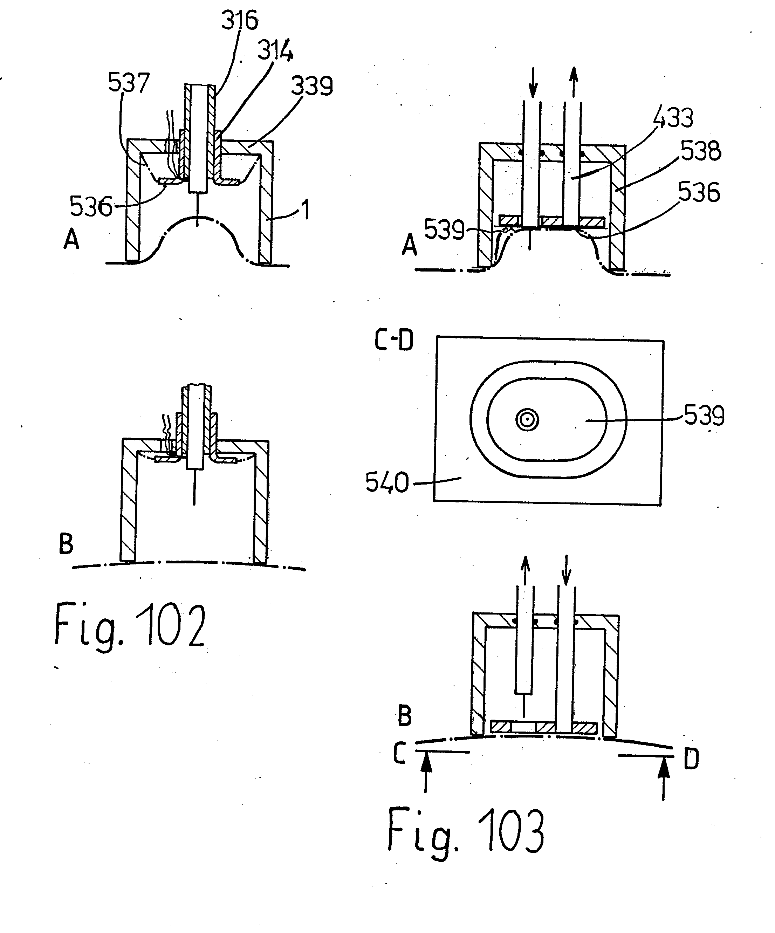

Im Bereich der Dosierung wird bei entsprechender Wiederstandsfähigkeit des Arzneischlauches(beispielsweise aus Silikon) die Dosierung mittels einer Art magnetgetakteten Wippe erwogen, wobei durch die Art der Klemmung der Zufluß aus dem Vorratsgefäß während der Ausquetschung des Schlauches gehindert(beispielsweise auch innerhalb einer Dosierschnecke, deren Windungen nacheinander über dem Schlauch diesen abplattend verengt werden.Fig.99). Die Möglichkeit, die Ausdehnung eines Körpers als Druckgeber(sei es Magnethub oder Piezoeffekt) in Stufen auf einen Schlauchteil einwirken zu lassen als Mittel der Dosierung, erlaubt zugleich die Absperrung eines Zuflußschenkels in die Schlauchkammer, erspart also ein Ventil(Fig.101). Von der Möglichkeit, die Geschwindigkeit von Magnethub oder Piezoausdehnung(durch sprunghafte Zuschaltung von Einzellamellen) zu ändern und zur Umschaltung auf eine andere Arzneisosrte zu nutzen, wurde bereits oben abgehandelt(Fig.98) Die Durchflussmessung(beipielsweise unter Zeitschaltung des Arzneiaustrittsventiles(das auch durch Schlauchring-Verengung(Fig.102) oder piezoelektrisch druckbeaufschlagte Blendenlamellen

um einen elastischen Stift im Schlauch nach Art eines Fotokamera-Zentralverschlusses bewerkstelligt werden kann) in Verbindung mit einer kritisch hohen Durchflußgeschwindigkeit oder eines konstanten Arzneidruckes vom Vorratsbehälter her oder auch die Ausnutzung der elektrischen Feldbilkdung oder der Kernresonanz zur Messung des Durchflusses bilden Möglichkeiten, die zur Verkleinerung der Dosiermechanik in Betracht gezogen werden. Derzeit jedenfalls scheint die Verwendung in ihrer Längenausdehnung gut steuerbarer Piezoelemente wegen der erforderlichen Hochspannung nur in Verbindung mir dem Stromnetz möglich(Bei der thermischen Vakuumerzeugung besteht ja nur eine zeitweitweilige Abhängigkeit). Jedenfalls kann nur unter erherblichem Kostenaufwand nach dem derzeitigen Stand der Technik entschieden werden, welche Zusammenstellung von Funktionsteilen den zur Aufgabe gemachten Injektor verwirklicht.

0221005

- 37 -

<u>Kurze Beschreibung der Ausführungsbeispiele:</u>

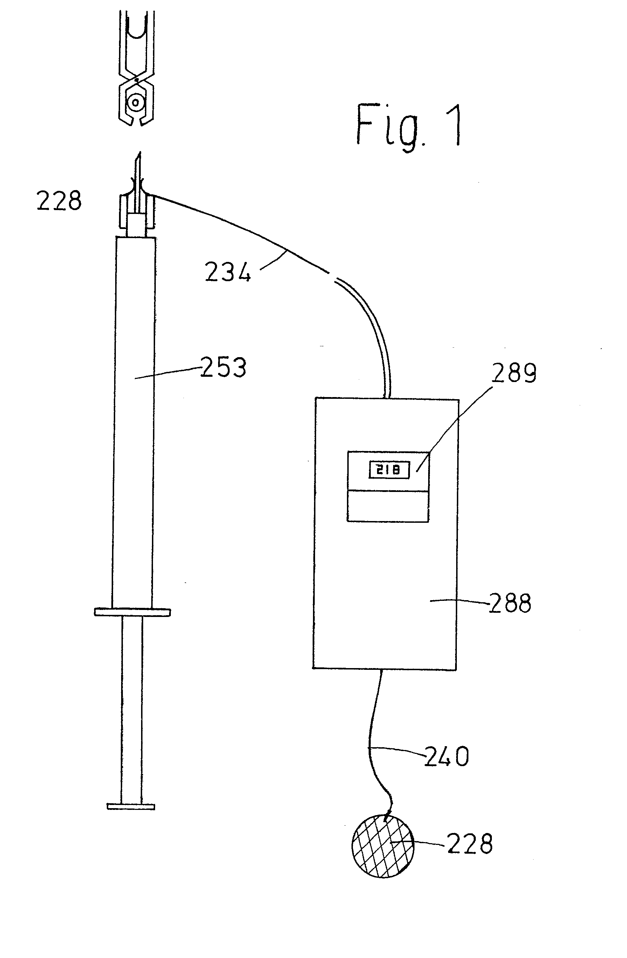

Figur 1 zeigt in einer Draufsicht eine gefüllte Injektionsspritze mit Sensorkanüle in Verbindung mit einer Meßvorrichtung.

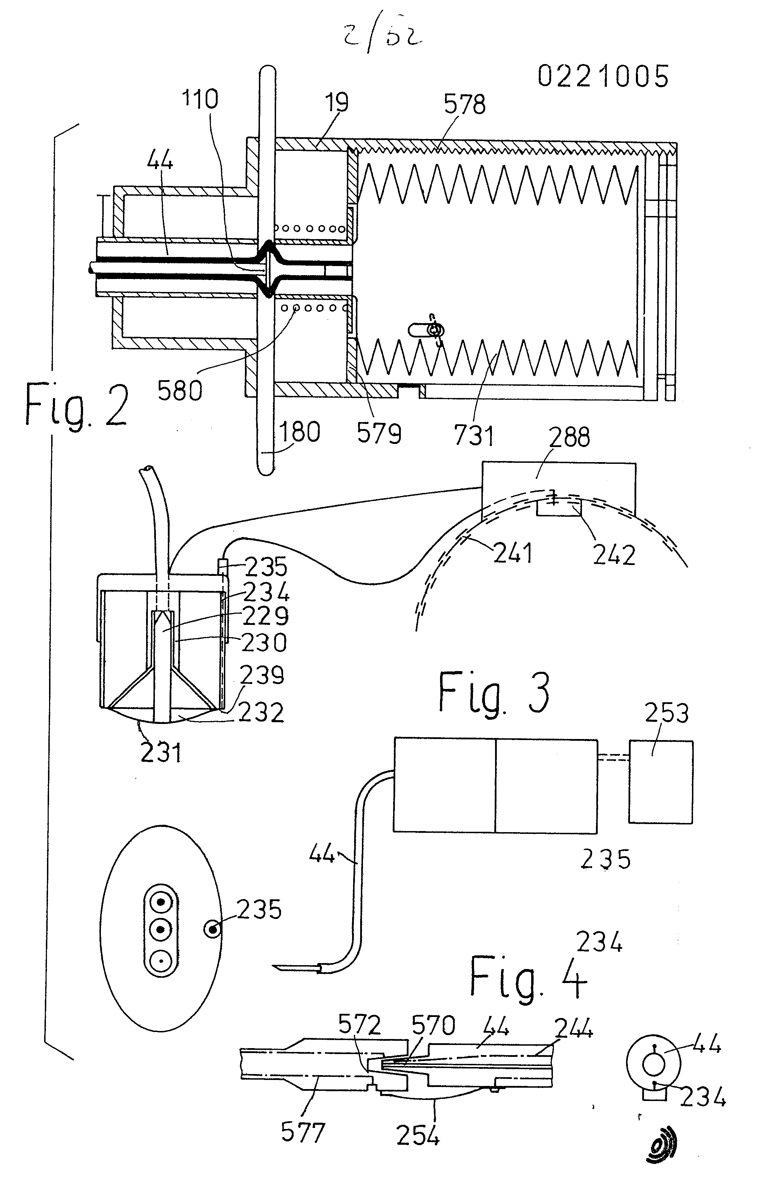

Figur 2 zeigt im Längsschnitt ein unterdruckbewahrendes Speichertöpfchen mit Sensorkanüle in Verbindung mit einer handbedienten Dosiervorrichtung und einer Meßvorrichtung.

Figur 3 zeigt in einer Längsansicht eine Insulinpumpe in Verbindung mit einer Sensorkanüle und Meßvorrichtung.

Figur 4 zeigt im Längsschnitt und rechtstehend im Querdas Detail eines Kanülenansatzstutzens in Verbindung mit einem Schlauchkoppelungsstück.

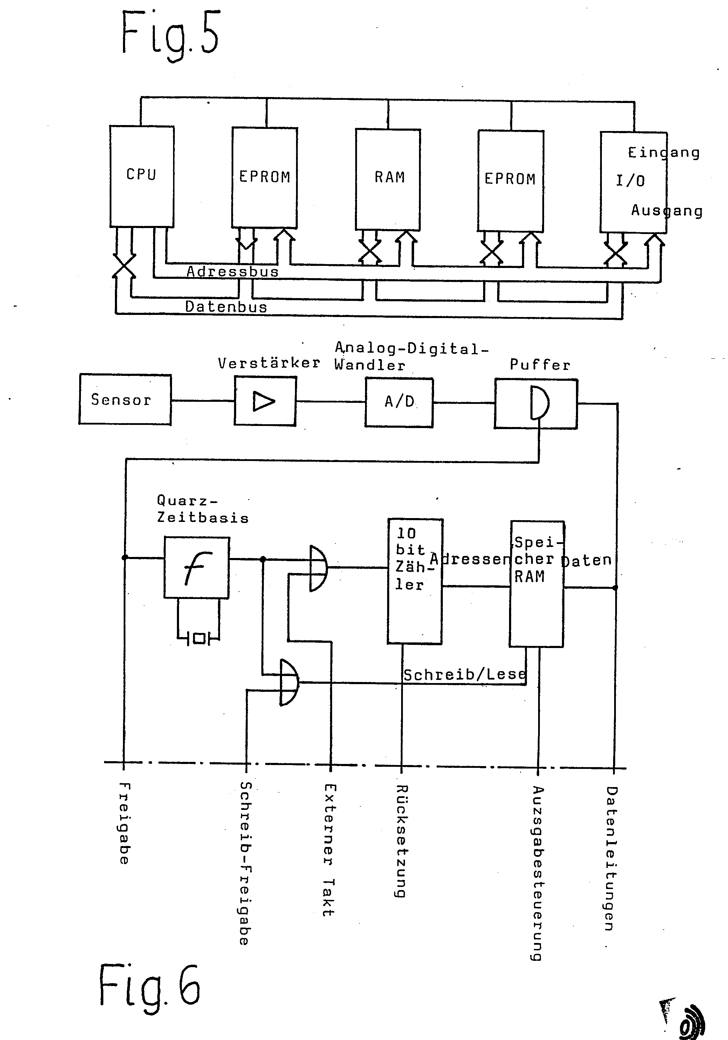

Figur 5 gibt ein Blockschaltbild für Datenspeicherung und -lesung bei einer Vorrichtung nach Fig.1 bis 3.

Figur 6 gibt eine Verfeinerung des Blockschaltbildes der Figur 5.

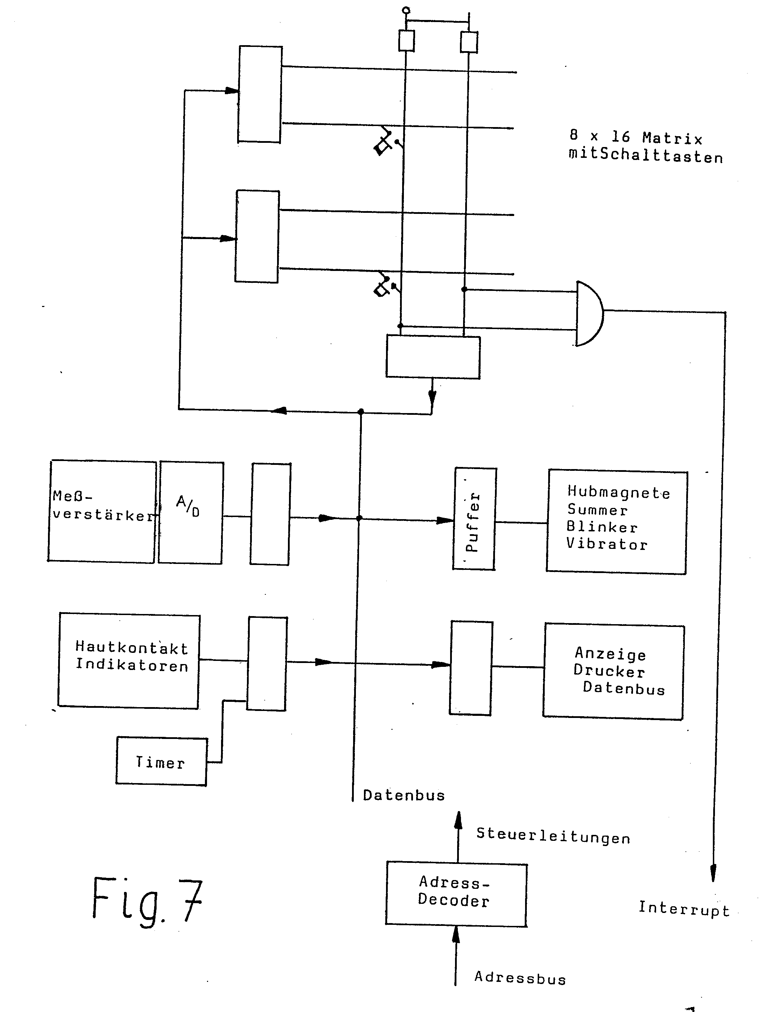

Figur 7 zeigt eine Schaltskizze des Lesers einer Vorrichtung wie in Fig.5 und Fig.6.

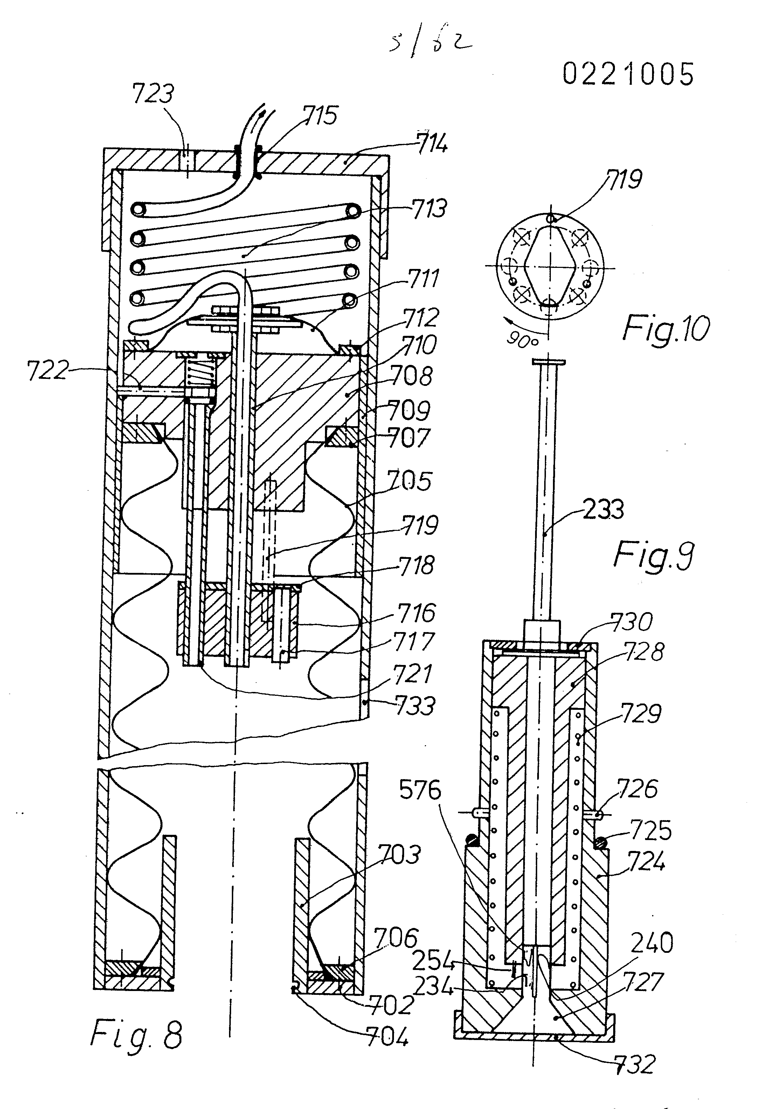

Figur 8 zeigt in natürlicher Größe bis auf eine Verkürzung um etwa 40 mm den Gehäusezylinder mit Ableitungsschlauch, Faltenbalg und Aufnahmerohrstutzen für den Einschubzylinder im Längsschnitt.

Figur 9 gibt den von unten in den Gehäusezylinder der Figur 8 einführbaren Einschubzylinder mit einer maximal gefüllten Injektionsspritze im Längsschnitt wieder.

Figur 10 zeigt einen Querschnitt durch die den Einschubzylider nach oben abschließende Eisenplatte, wobei die Lage der sechs Dauermagnete und des Auslöserohres für das Wiederbelüftungsventil unterbrochen eingezeichnet ist.

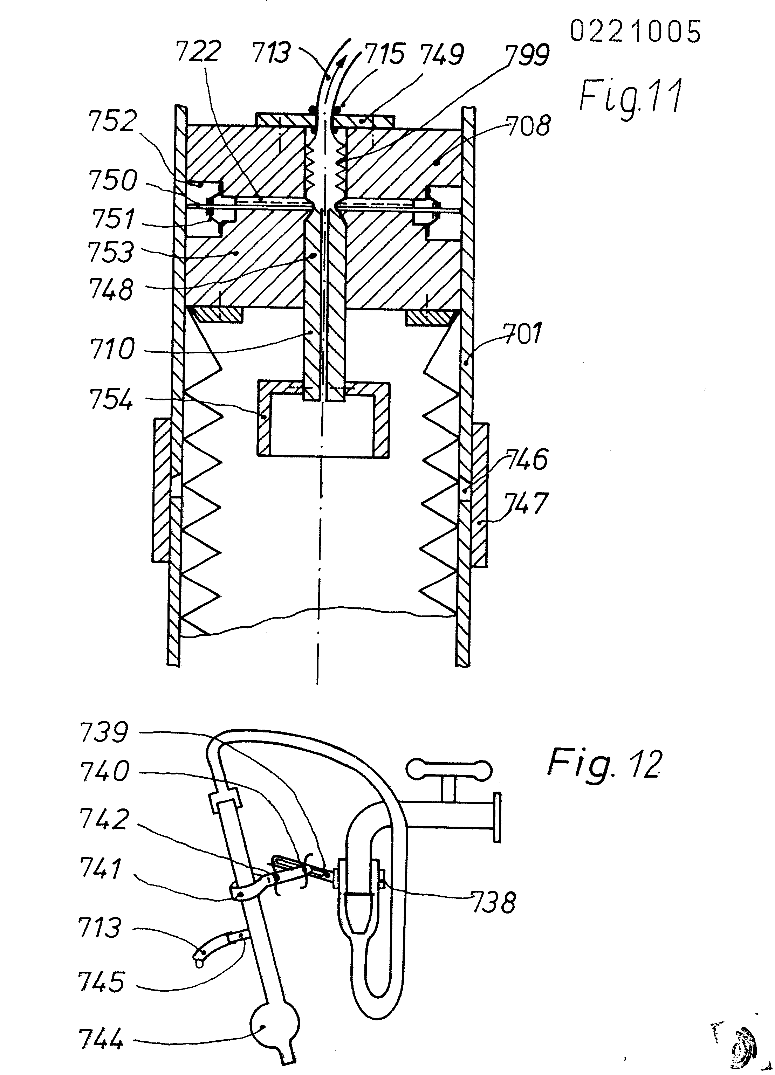

Figur 11 zeigt im Längsschnitt Teile eines Gehäusezylinders ähnlich demjenigen in Figur 8 mit Abweichungen in der Gestaltung des Wiederbelüftungsventiles.

Figur 12 zeigt in einer Verkleinerung etwa im Maßstabe 1 : 5 eine für den Betrieb der Vorrichung geeignete Wasserstrahlpumpe mit Leitungsanschluß in Längsansicht.

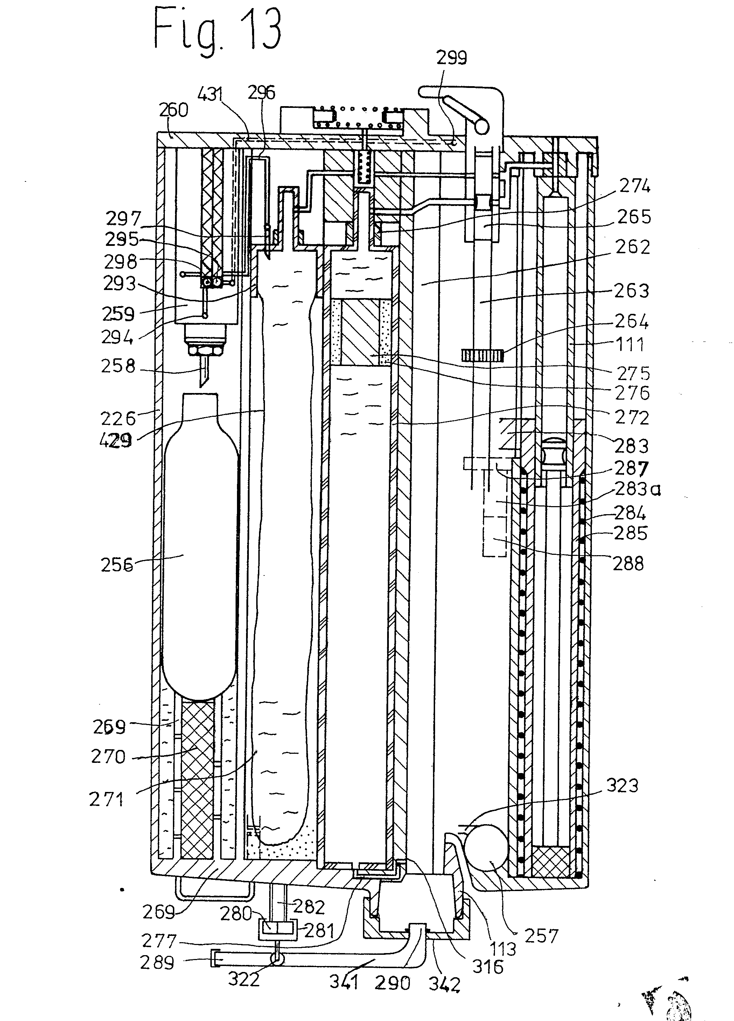

Figur 13 zeigt einen Längsschnitt durch einen Injektor, welcher mittels einer $CO_2$-Druckgaskapsel betrieben für eine Arzneisorte mit automatischem Kanülenwechsel.

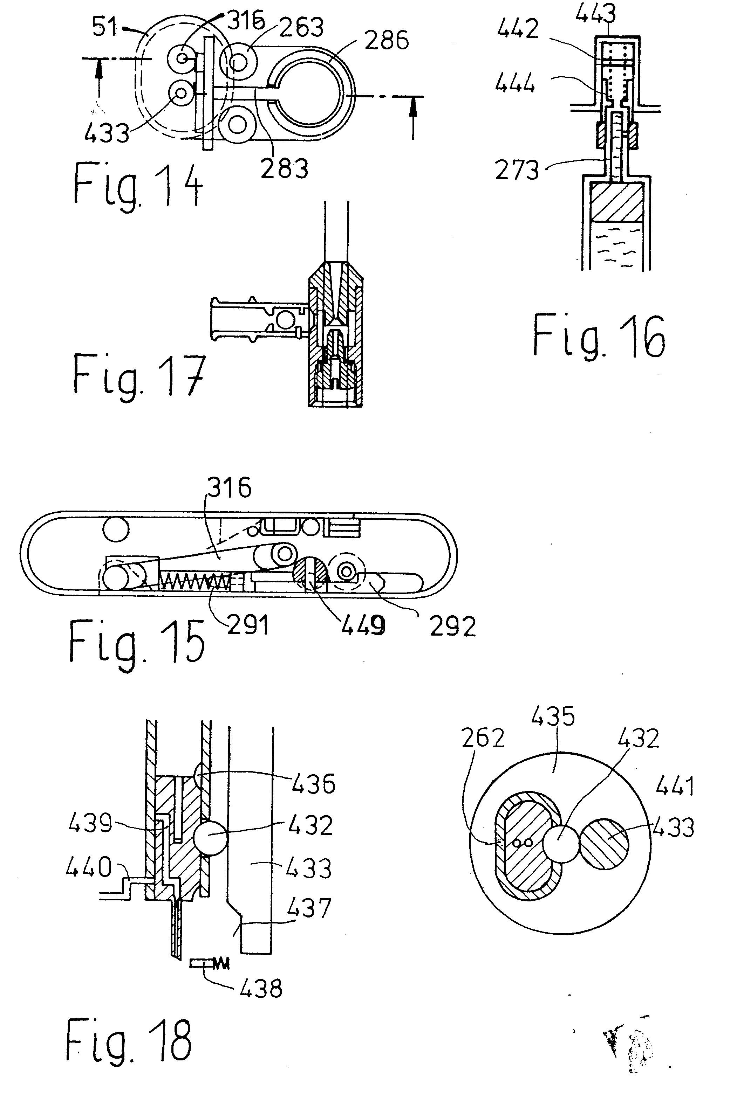

Figur 14 zeigt einen Querschnitt in Höhe A - B der Fig.13.

Figur 15 zeigt einen Querschnitt in Höhe C - D der Fig.13.

Figur 16 zeigt einen Längsschnitt durch das Detail eines der beiden Flüssigkeitskoppelungsventile zu Fig.13.

Figur 17 zeigt einen Querschnitt durch die Gasstrahlpumpe der Fig.13.

Fig.18 zeigt links im Längsschnitt und rechts im Querschnitt ein Detail am Ende des Kanülenmagazins im Maßstab 5 : 1 zu Figur 13.

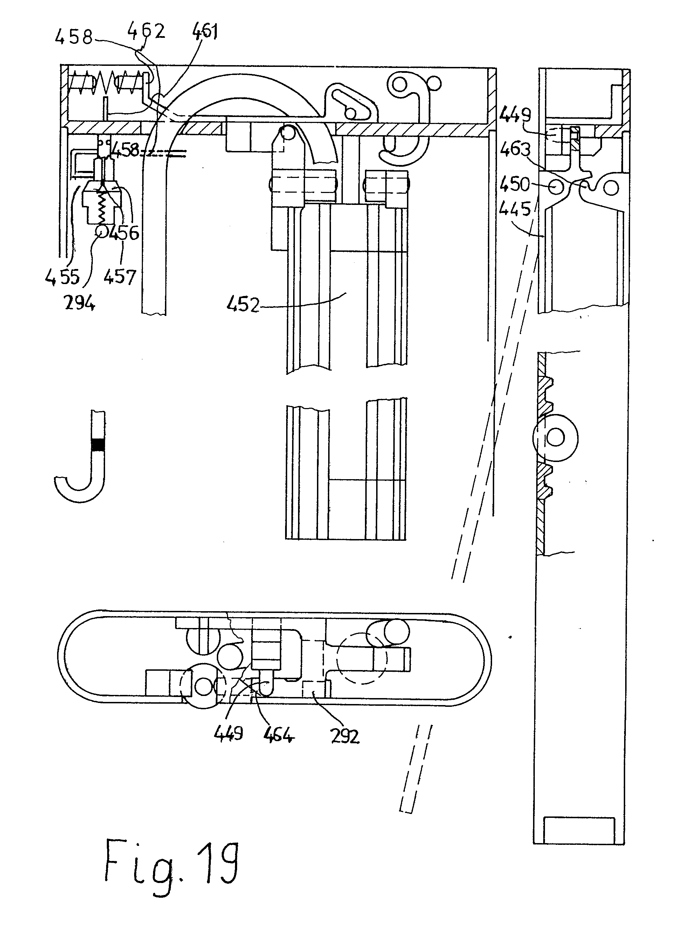

Figur 19 zeigt eine Seitenansicht auf die Deckelklappen einer Seite zu Fig.13; rechts davon ist ein Vertikalschnitt und darunter ein Querschnitt in Höhe A - B abgebildet.

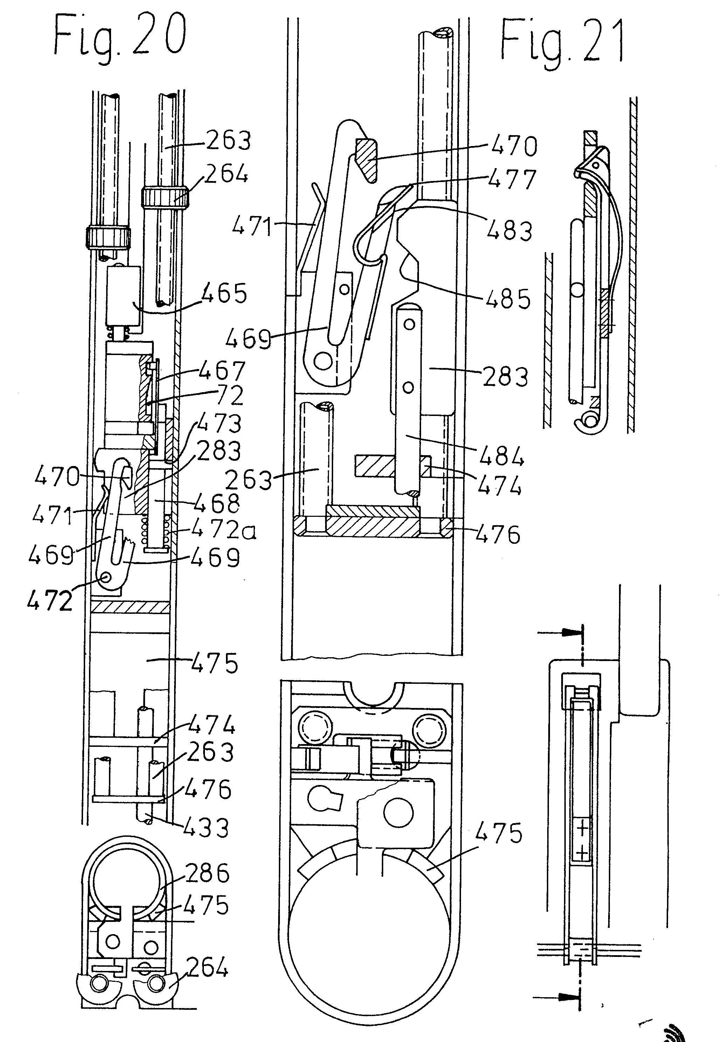

Figur 20 zeigt im Vertikalschnitt längs E - F der Fig.13 Einzelheiten der Steuerung der Auslösung und des Wechsels der Dosierung. Darunter ist ein Querschnitt zu sehen.

Figur 21 zeigt im Maßstabe 2 : 1 Details der Fig.20. Darunter ist wieder ein Querschnitt längs A - C zu sehen. Rechts sind in zwei um je 90 Grad gedrehten Ansichten von Einzelheiten der Lage der Überholfeder des Auslösers zu erkennen.

Figur 22 zeigt in teilweiser Ansicht oben und unten im Längsschnitt und in der Mitte in Querschnitten A - B und C - D Einzelheiten der Steuerungsmechanik.

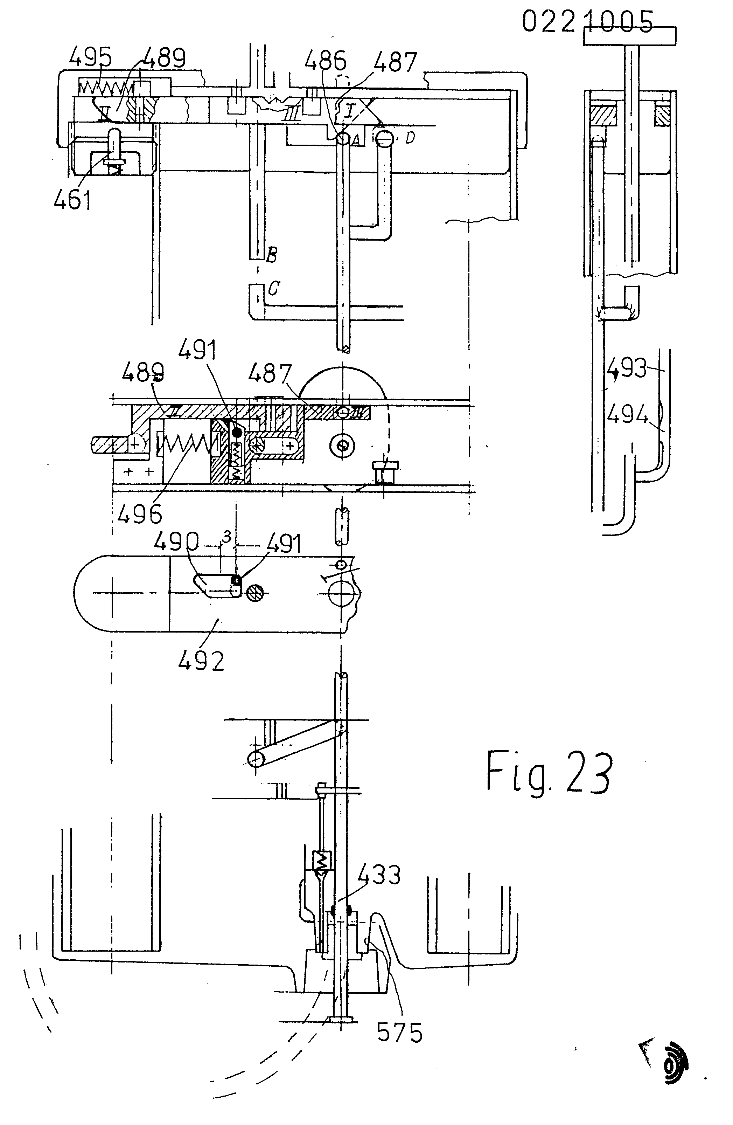

Figur 23 zeigt die von der Halbrinneausgehenden Steuerarme und die von ihnen betätigten Deckelschie-

0221005

- 39 -

schieber mit Zähl- und Sperrvorichtung in Teildarstellung im Längsschnitt und unter dieser in einer Draufsicht. Rechts wird noch ein Vertikalschnitt gezeigt.

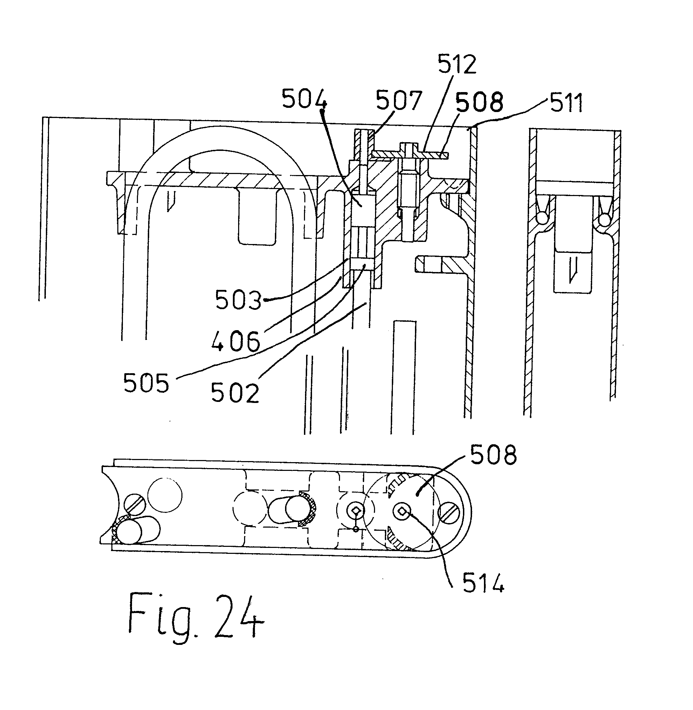

Figur 24 zeigt im Längsschnitt den Zähl- und Sperrmechanismus der Vorrichtung nach Fig.13 und folgende.
Darunter liegt der Querschnitt in Höhe A - B.

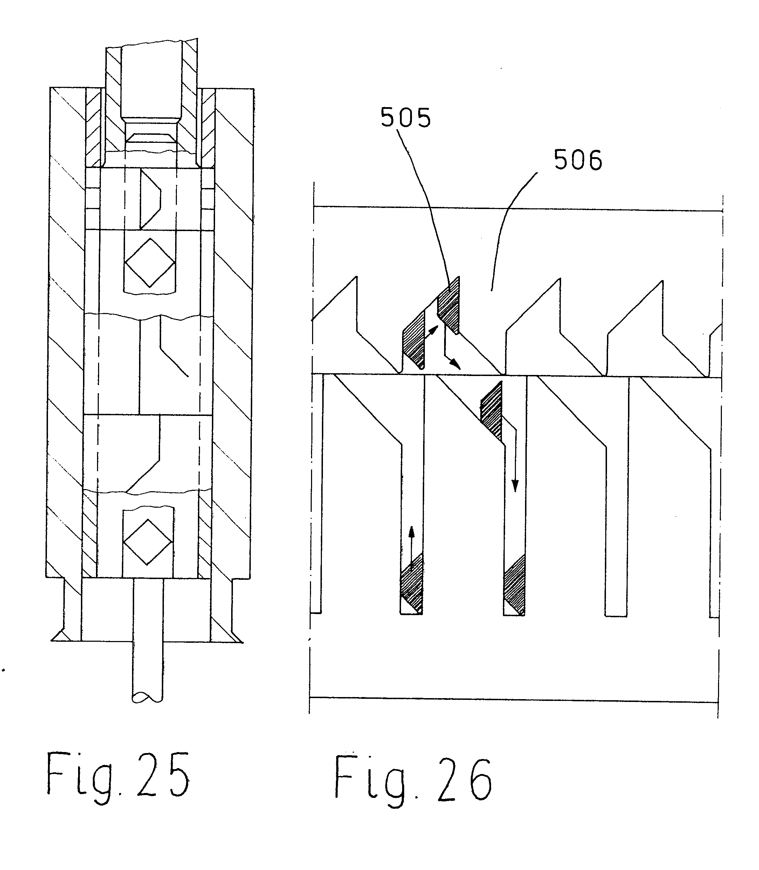

Figur 25 zeigt im Maßstabe 5 : 1 das Detail der Umsetzung der Hubbewegung in die Drehung des Zählwerkes.

Figur 26 gibt in einer Aufwickelung im Maßstabe 10 : 1
die Nuten in der Führungsbuchse wieder.

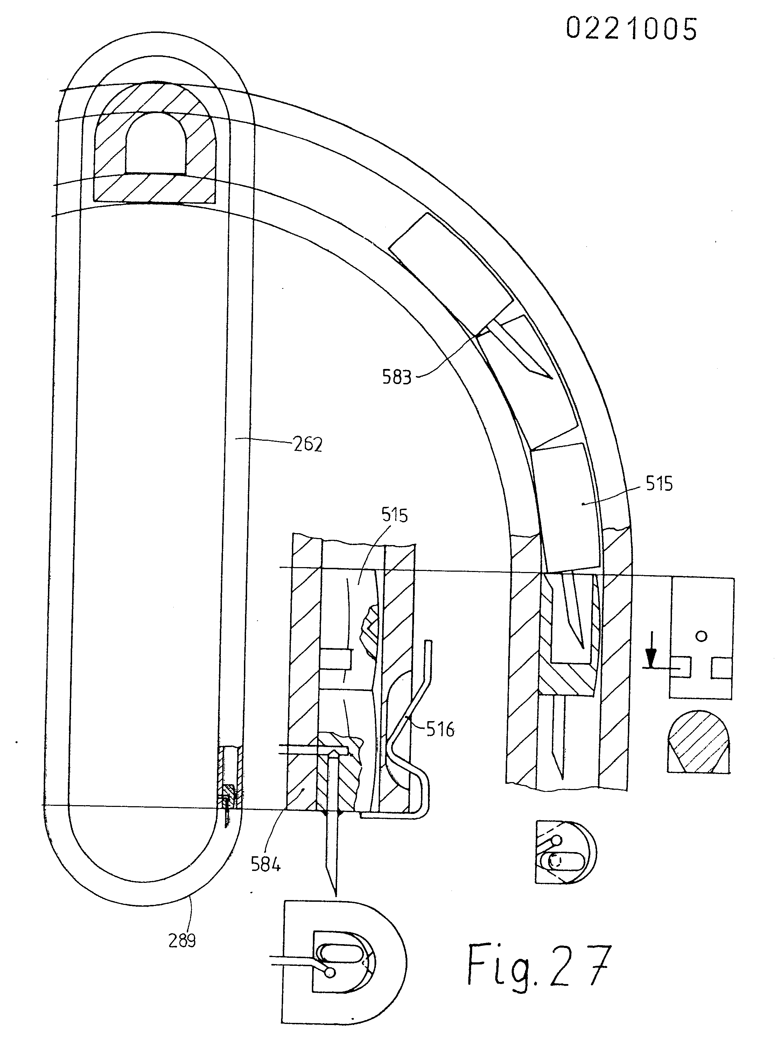

Figur 27 gibt links in einem Längsschnitt, der einen
Querschnitt im Maßstabe 5 : 1 umschließt, und rechts
davon im Längsschnitt ebenfalls im Maßstabe 5 : 1
ein Kanülenmagazin und Variationen von
Stapelkanülen wieder.

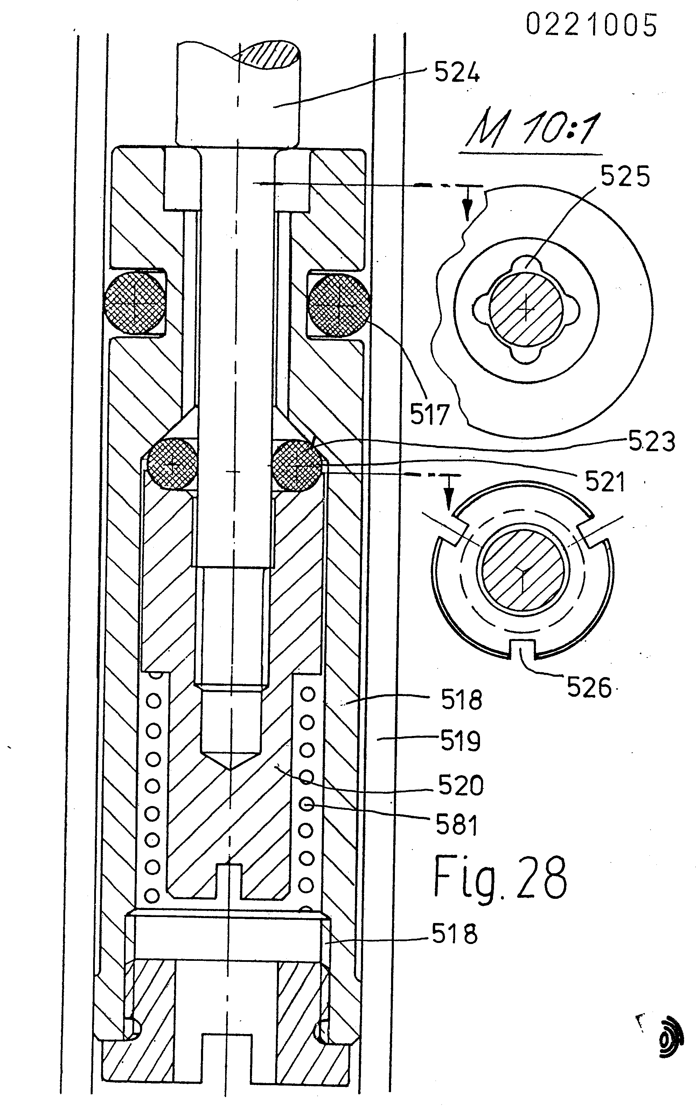

Figur 28 zeigt im Maßstabe 10 : 1 links im Längsschnitt
und rechts in zwei Querschnitten verschiedener Höhe
eine Dosierpumpe für Hilfsflüssigkeit.

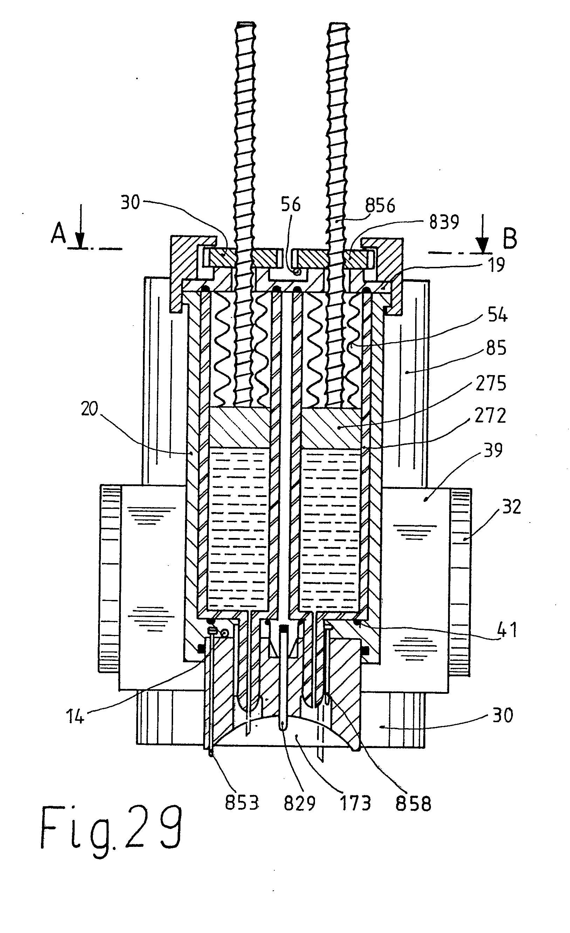

Figur 29 zeigt im Längsschnitt einen elektrisch betriebenen Injektor mit Stufenspritzen für zwei verschiedene Arzneien.

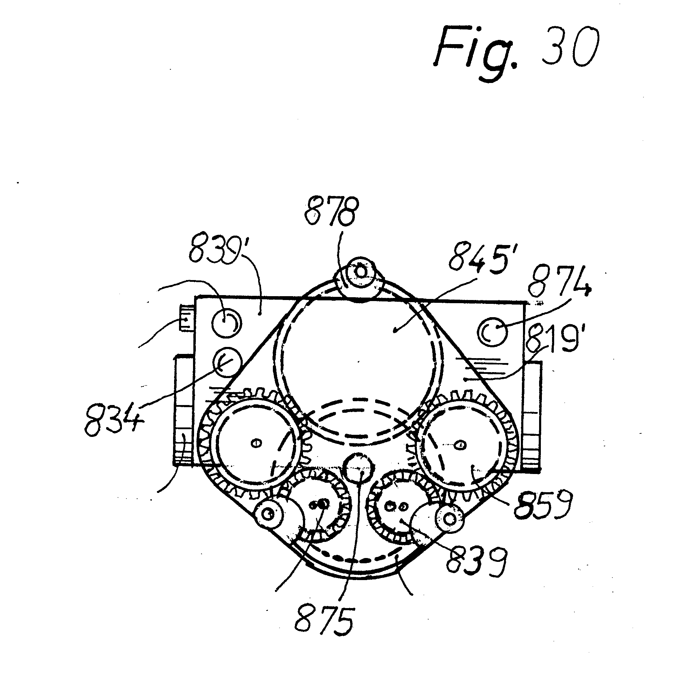

Figur 30 zeigt einen Querschnitt in Höhe A - B der
Fig.29.

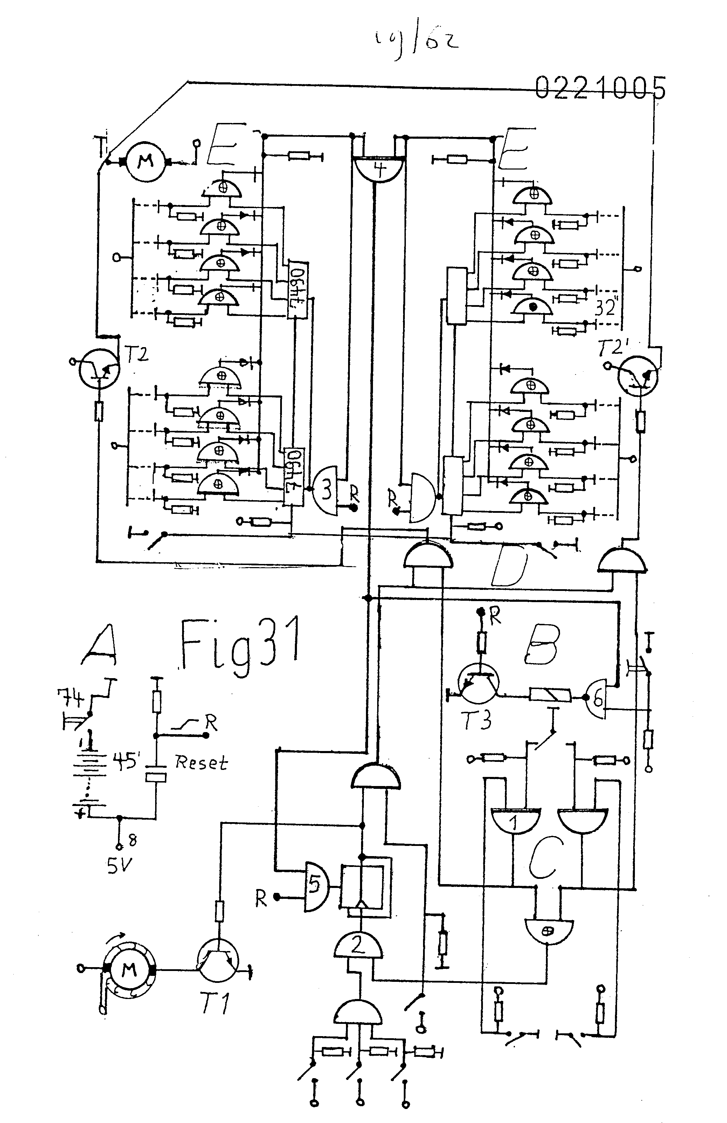

Figur 31 zeigt eine Schaltskizze in TTL zu Fig.29,30.

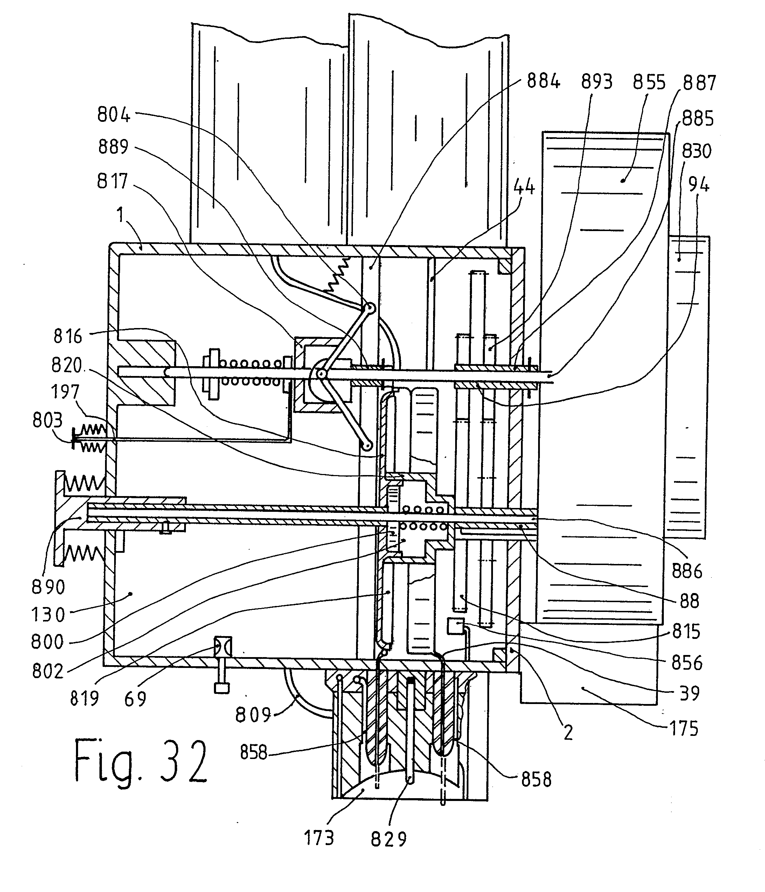

Figur 32 zeigt im Längsschnitt eine Variante zu Fig.29,
welche für zwei dosierende Schlauchpumpen einen Motor
mit Schaltgetriebe aufweist.

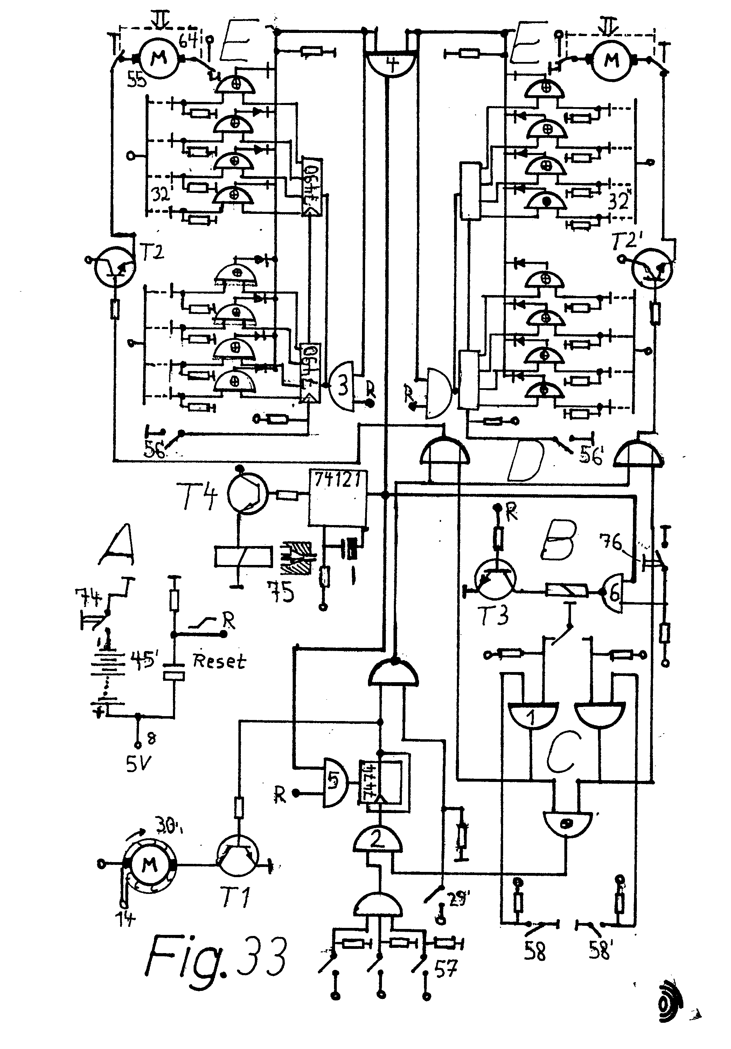

Figur 33 gibt die TTL-Schaltskizze zu Fig.32.

Figur 34 zeigt eine Arzneivorratsflasche mit Druckgas
innerhalb eines Faltenbalges im Längschnitt.

Figur 35 zeigt einen Längsschnitt durch einen Arzneibeutel innerhalb eines Druckgasbeutels.

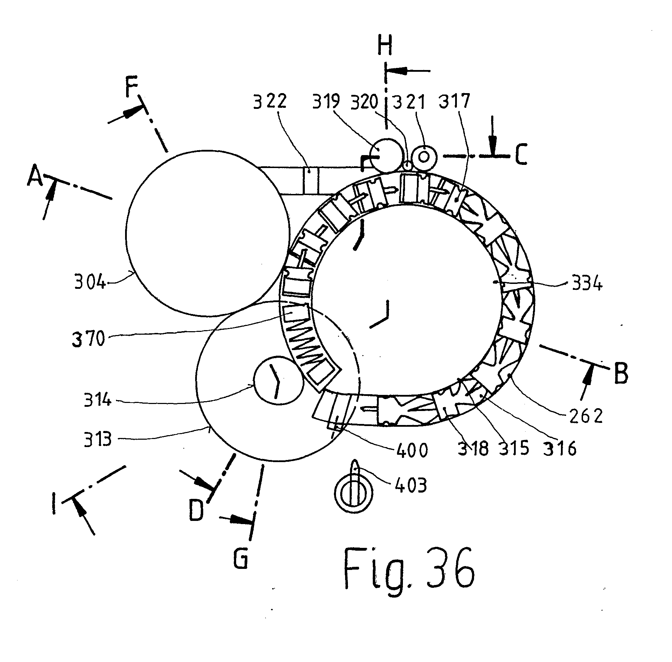

Fig.36 gibt eine schematische Draufsicht auf einen
Sauginjektor als bevorzugte Ausgestaltung der Erfindung. Im Magazinschlauch ist die Lagerung von Stapelkanülen zweier verschiedener Konstruktionen im Längsschnitt dargestellt.

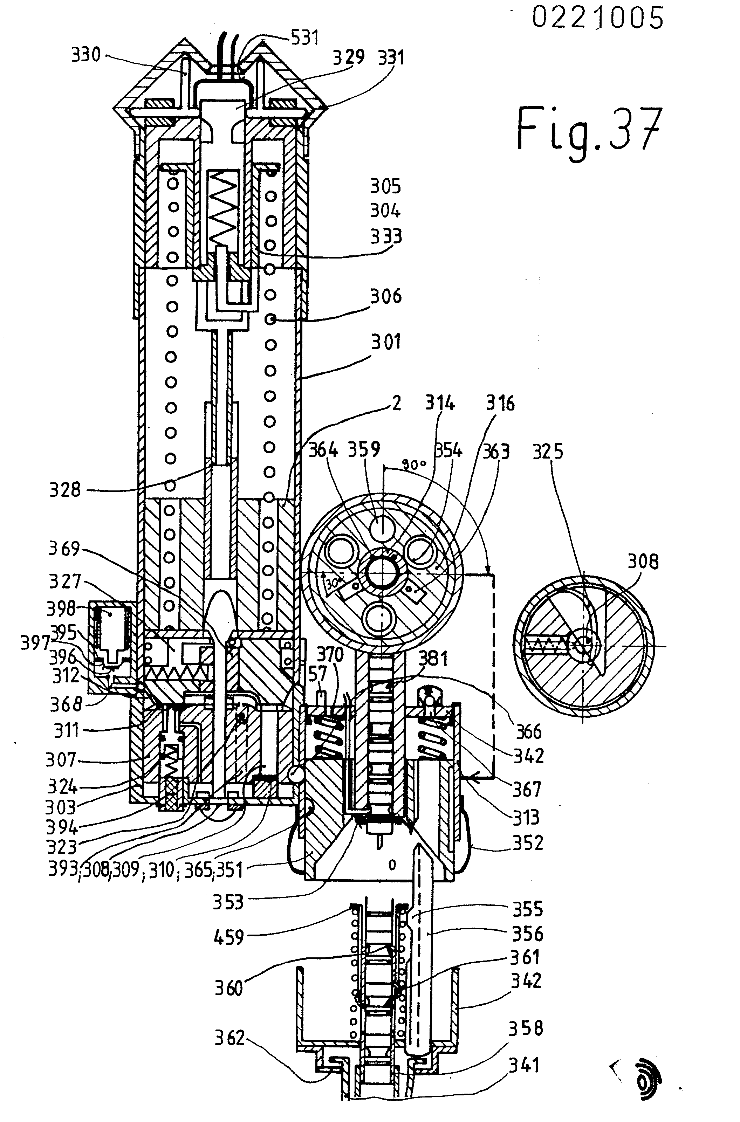

Figur 37 zeigt einen Längsschnitt durch den Sauginjektor, welcher der Linie F - G auf Figur 36 folgt.

Über der Saugglocke ist ein Querschnitt durch dieselbe (Höhe durch Hilfslinie bezeichnet) und rechts ein Querschnitt unterhalb des Sogkolbens zu erkennen.

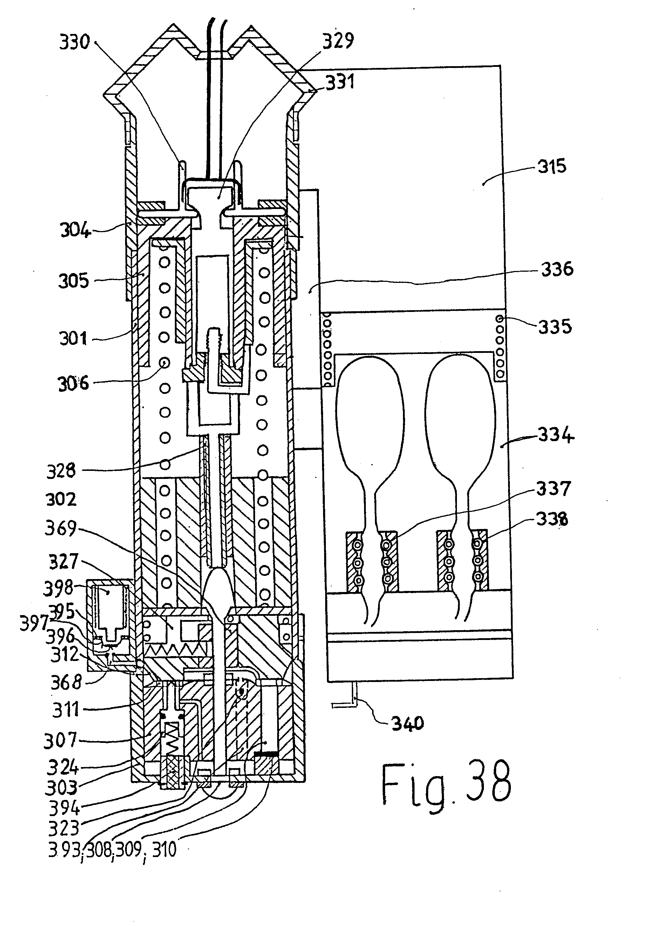

Figur 38 zeigt einen Längsschnitt längs der Linie A - B der Fig.36 durch den Injektor.

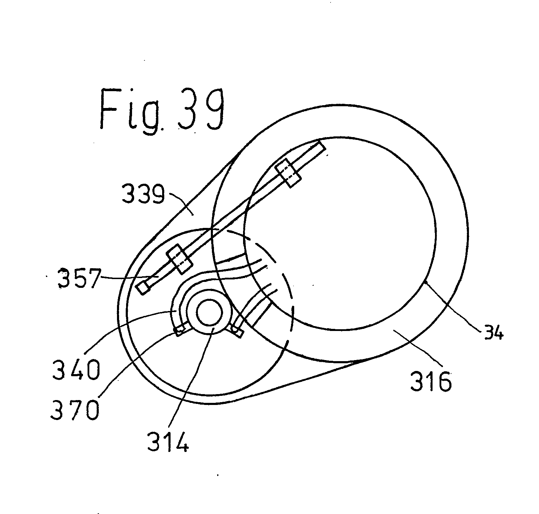

Figur 39 zeigt eine Draufsicht auf das Saugglockendach nebst nebst Zylinder(304) mit Kanülenmagazin.

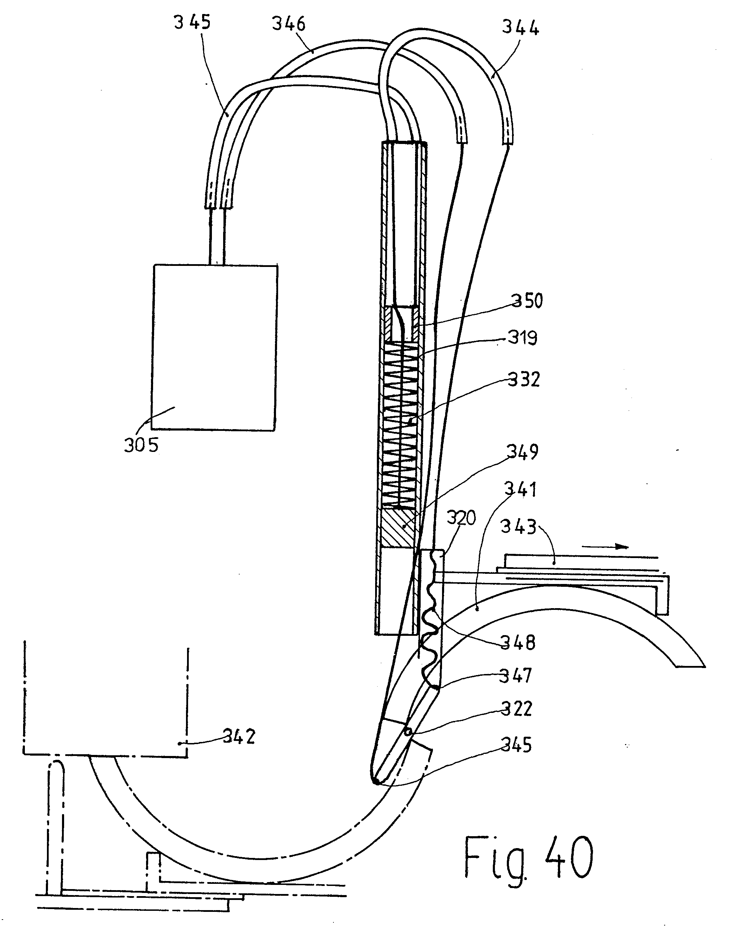

Figur 40 zeigt einen schematischen Längsschnitt längs der Linie C - D zur Erläuterung des Kolbenführungsmechanismus über die Bewegung des Saugglockendeckels in Wartestellung vor der Injektion.

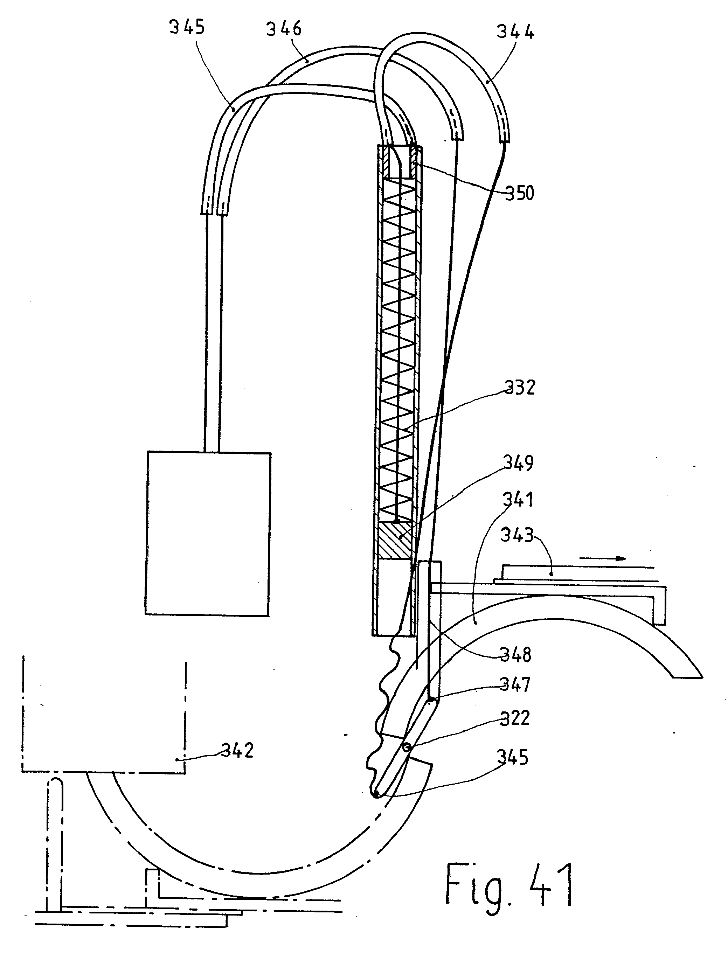

Figur 41 entspricht weitgehend der Fig.40, zeigt aber das Stadium nach der Injektion.

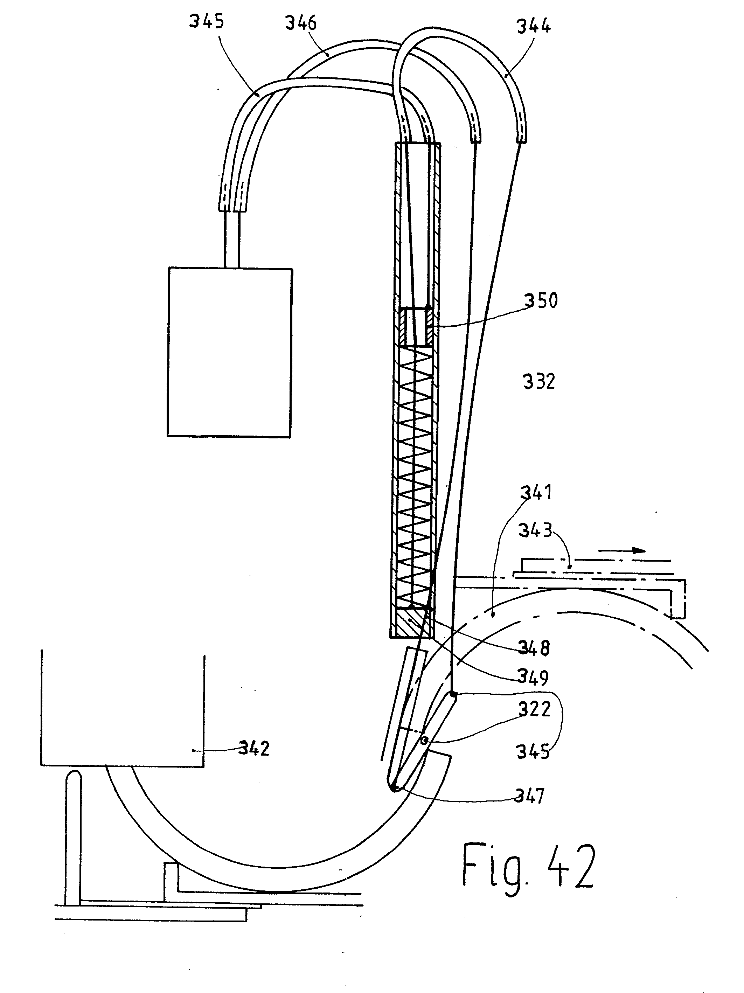

Figur 42 zeigt das Stadium nach Deckelschluß.

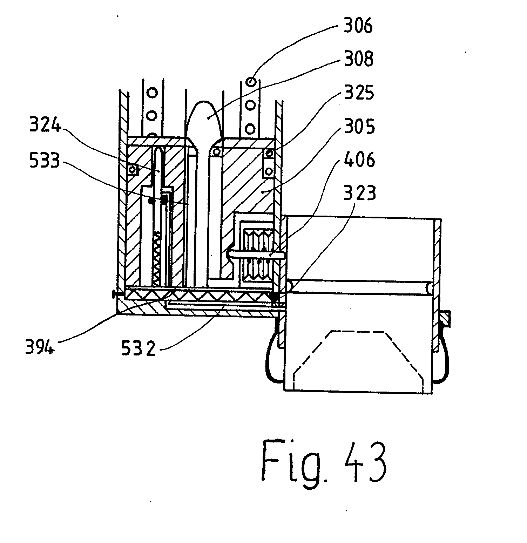

Figur 43 zeigt eine Variation des Auslösemechanismus für den Sogkolben bei Linienführung des Längschnittes wie in Fig.26; die Saugglocke ist in Seitenansicht gezeigt.

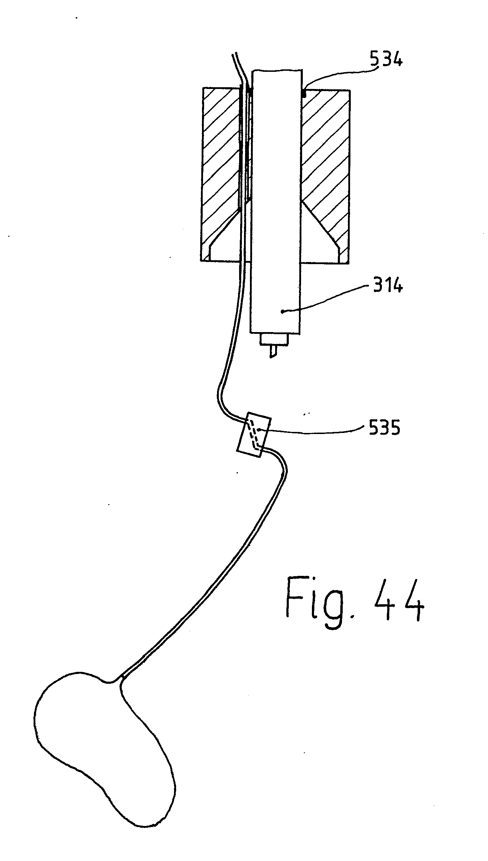

Figur 44 zeigt in schematischen Längsschnitt eine Verbindungsmöglichkeit zwischen altem und neuem Vorratsbeutel zur teilweisen Wiederauffüllung des alten.

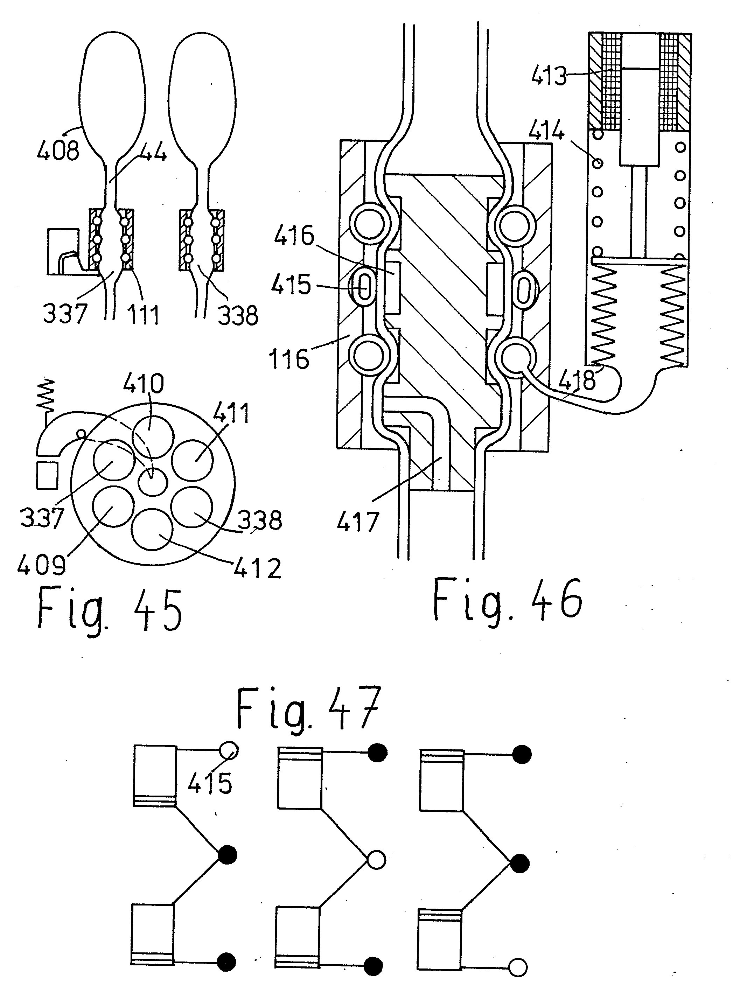

Figur 45 zeigt oben im Längsschnitt, darunter im Querschnitt zwei Dosierpumpen für den Injektor nach Fig.26 ff sowie eine Verriegelung der Vorratsbeutel.

Figur 46 zeigt im Längschnitt im Maßstabe 5 : 1 das Detail einer Dosierpumpe in Verbindung mit einer der magnetbetriebenen Dosierpumpen.

Figur 47 zeigt schematisch den Ablauf einer peristaltischen Dosierbewegung bei einer Variation der Pumpenanordnung zur Einsparung von Dosierpumpen.

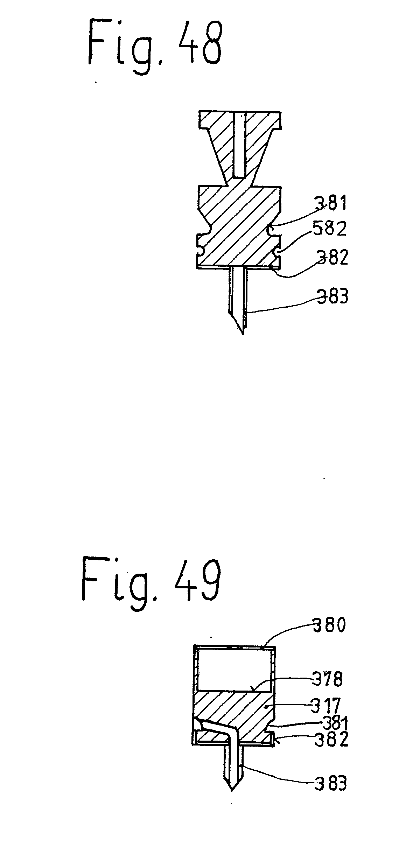

Figur 48 zeigt in einem Längsschnitt im Maßstabe 5 : 1 eine Stapelkanüle nach Fig.25.

Figur 49 entspricht der Fig.48 weitgehend und stellt eine Kanülenvariation dar.

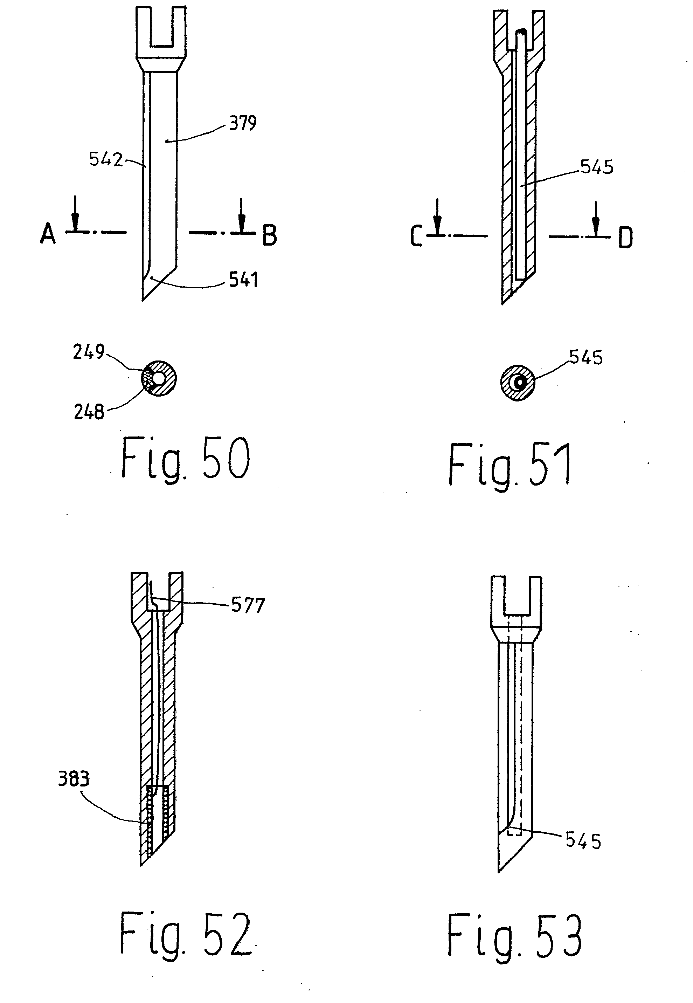

Figur 50 zeigt im Längsschnitt und darunter im Querschnitt längs der Linie A - B eine besondere Sensorkanüle mit teilweiser Schaftersetzung durch Kunststoff im Maßstabe 5 : 1.

Figur 51 zeigt eine Kanüle, welche in der Darstellungsweise der Fig.50 entspricht, mit eingelegtem Sensorröhrchen oder -faden bei intaktem Schaft.

Figur 52 zeigt eine Kanülenvariation mit Innenbeschichtung im Längsschnitt und im Maßstabe 5 : 1.

Figur 53 zeigt eine Kanüle, welche in der Darstellungsweise der Figur 50 entspricht, wobei in den aufgespaltenen Schaft ein Schläuchchen eingelegt wurde.

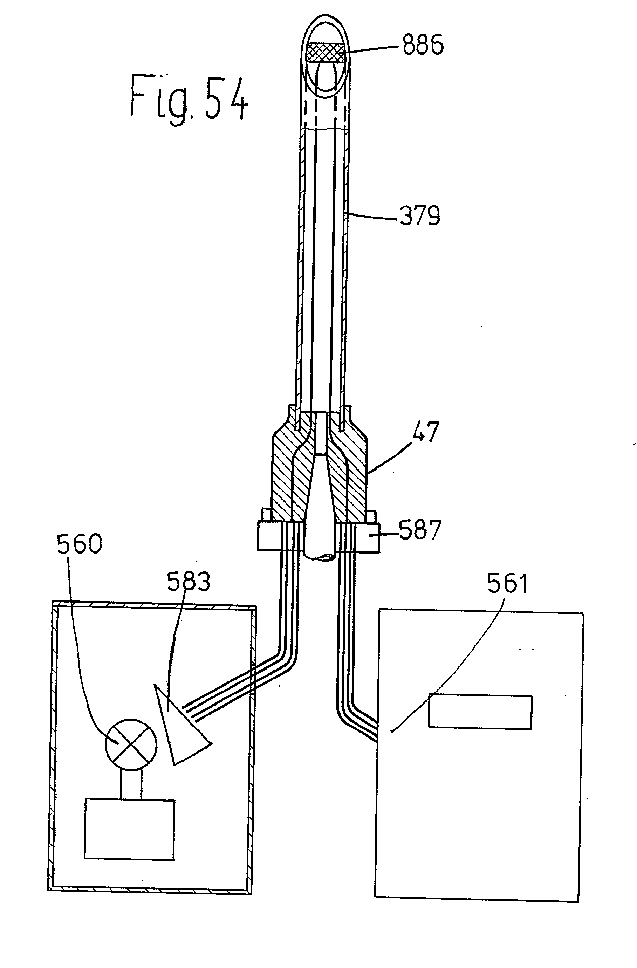

Figur 54 zeigt im Längsschnitt im Maßstabe 10 : 1 eine Sensorkanüle für reflexfotometrische Meßung.

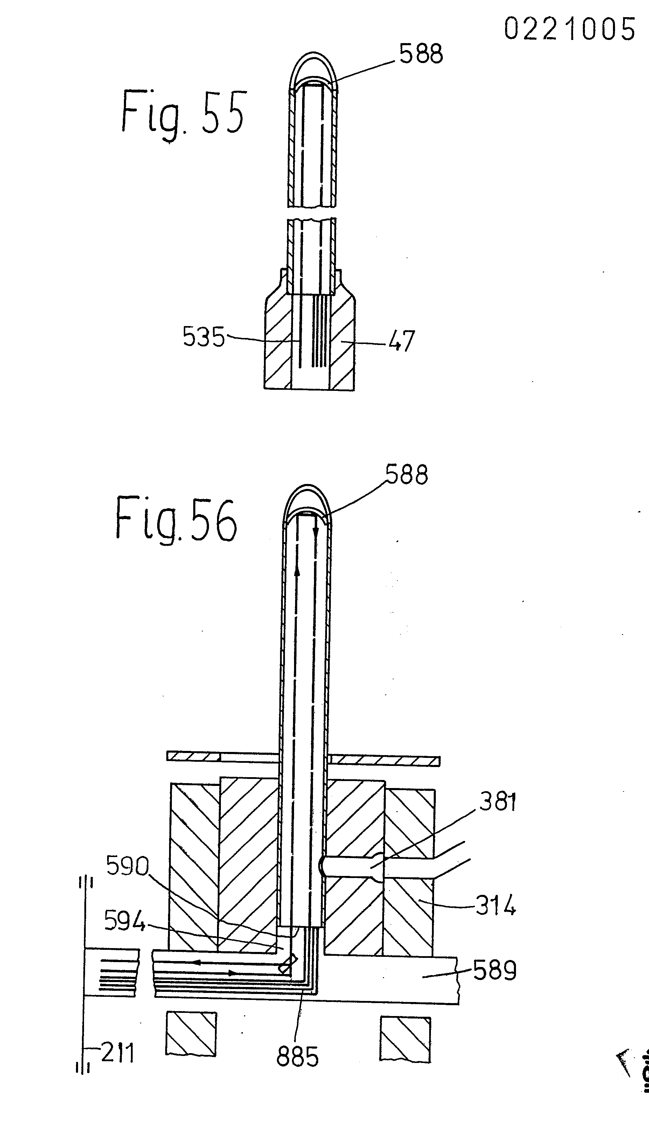

Figur 55 zeigt im Längsschnitt im Maßstabe 10 : 1 ei- Sensorkanüle für optische Messungen mit Lichtleitfasern nur bis zum Schaftansatz.

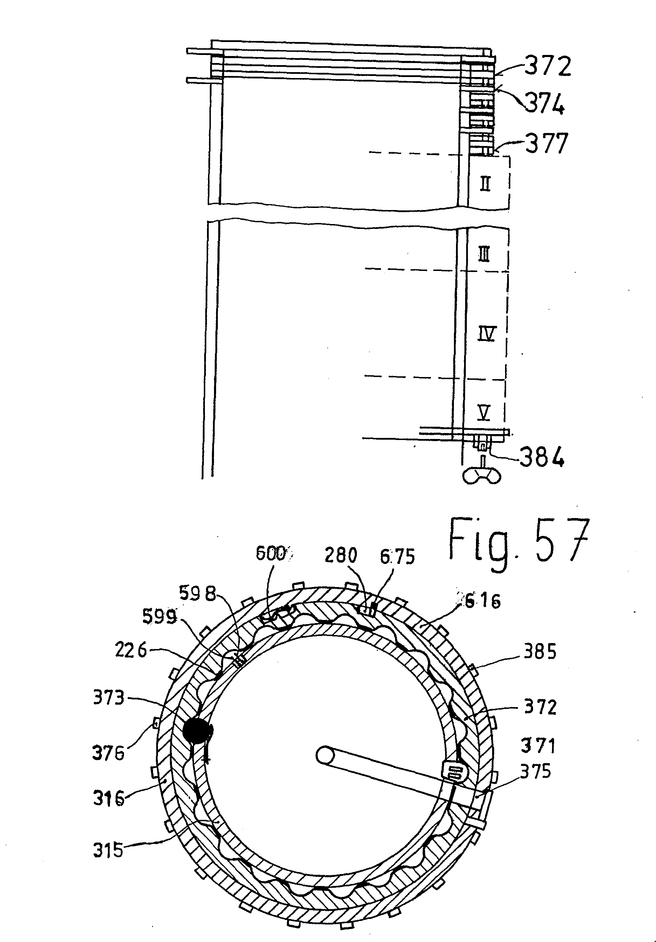

Figur 56 zeigt im Längsschnitt im Maßstab 10 : 1 die Variation einer Stapelkanüle entsprechend Fig.48,49 innerhalb einer Gerätebohrung mit Lichtzuführung über ein Schwenksegment.

Figur 57 zeigt oben im Längs- und unten im Querschnitt die Schaltringanordnung auf einem Gerätezylinder für eine Vorrichtung nach Fig36 ff.

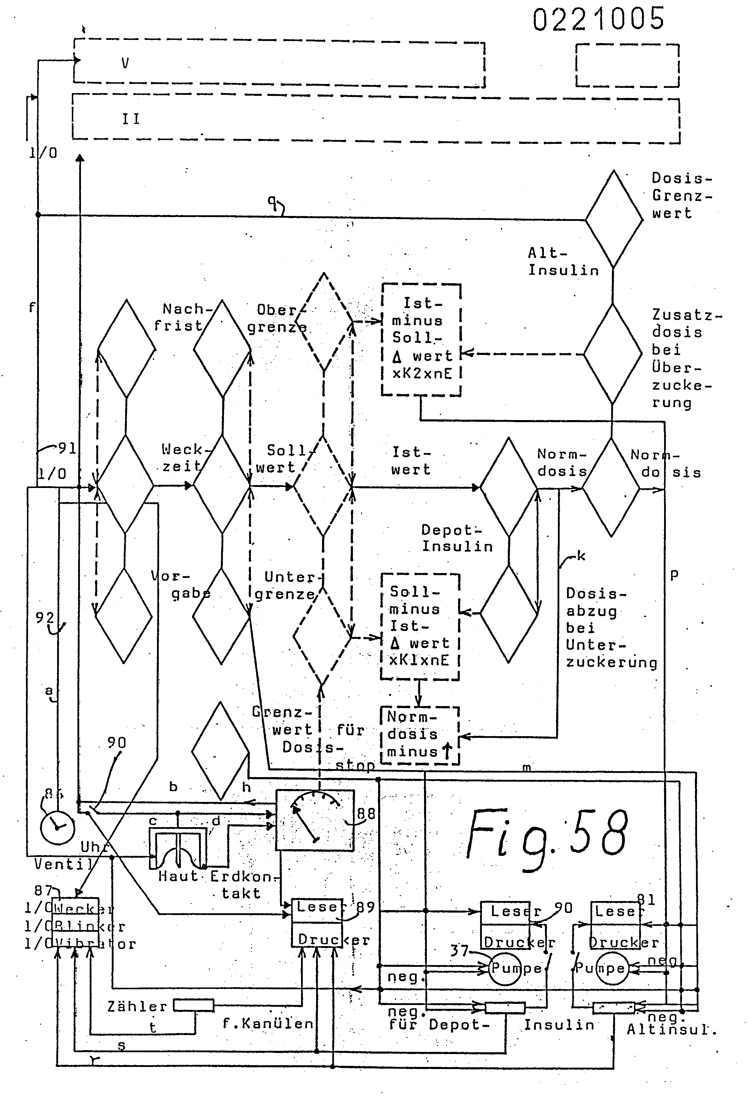

Figur 58 zeigt ein Funktionsdiagramm für die elektrische Steuerung und Programmierung.

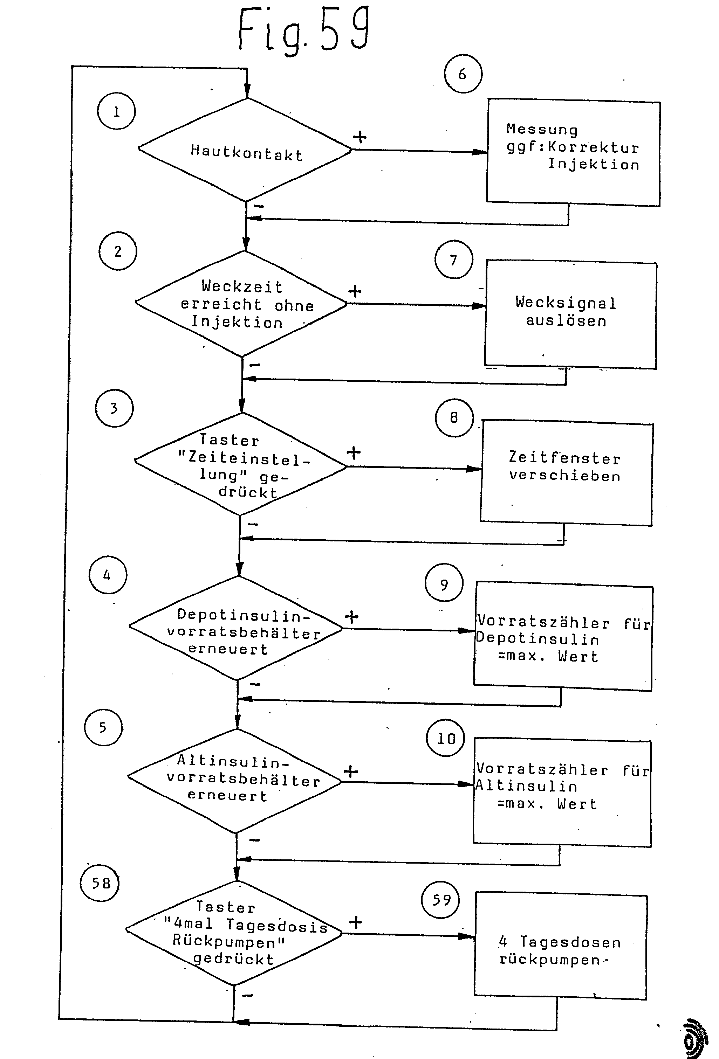

Figur 59 zeigt einen prozessorgesteuerten Programmablauf für einen Injektor nach Fig.36 ff.

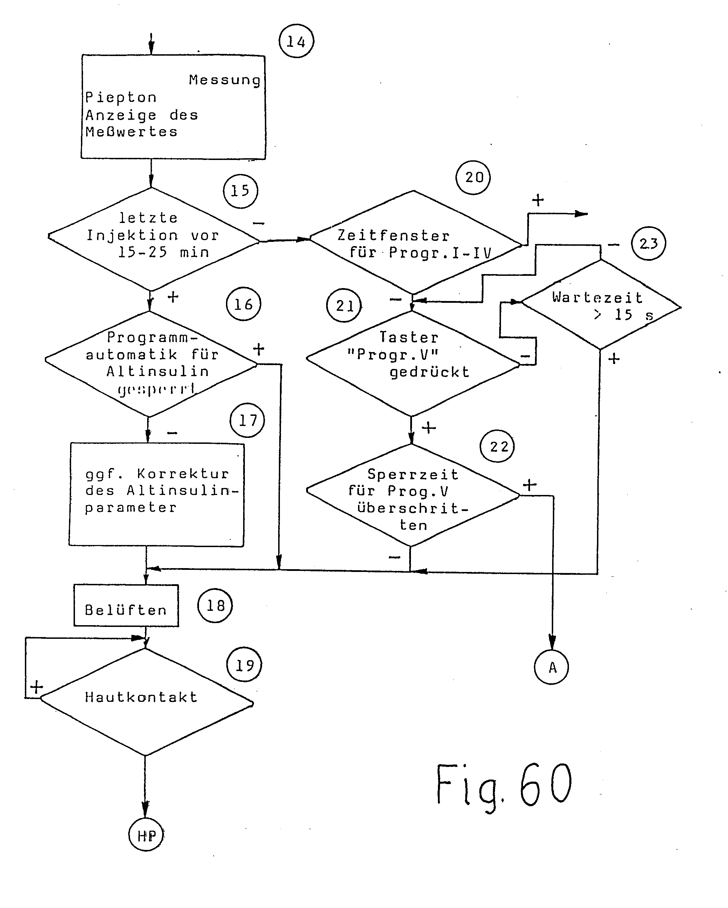

Figur 60 gibt eine Verfeinerung von Block 6 aus Fig.59.

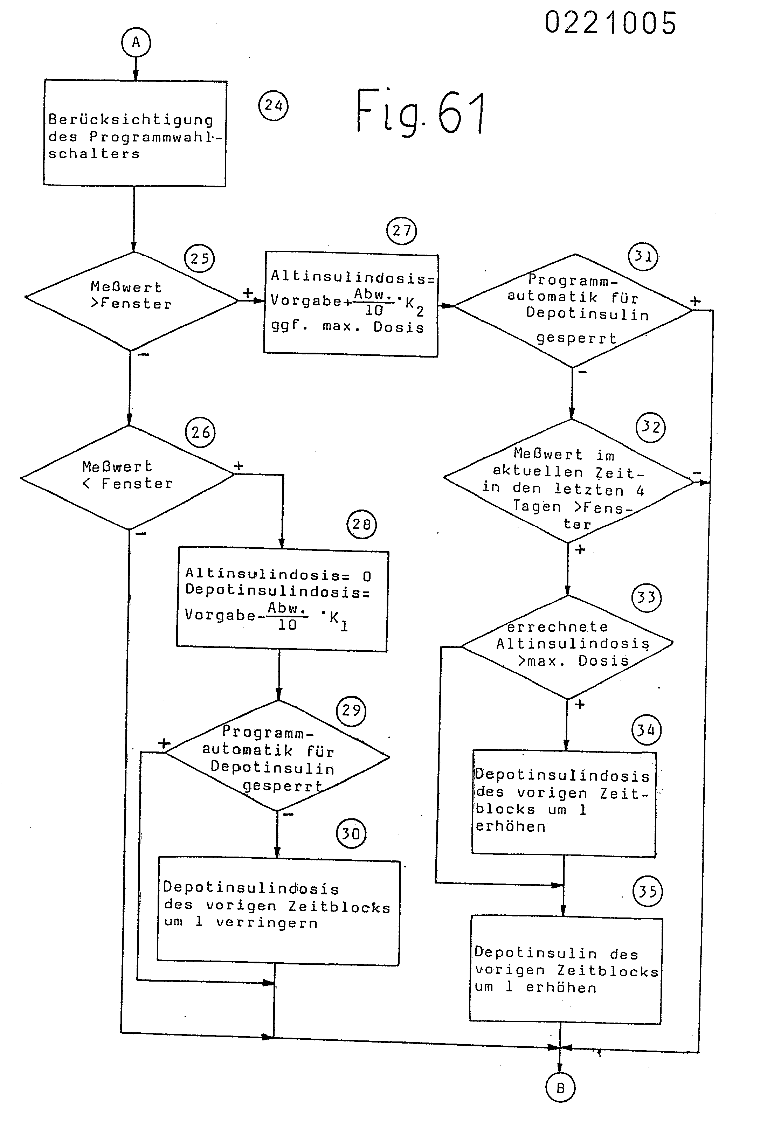

Figur 61 ist eine Fortsetzung der Figur 60.

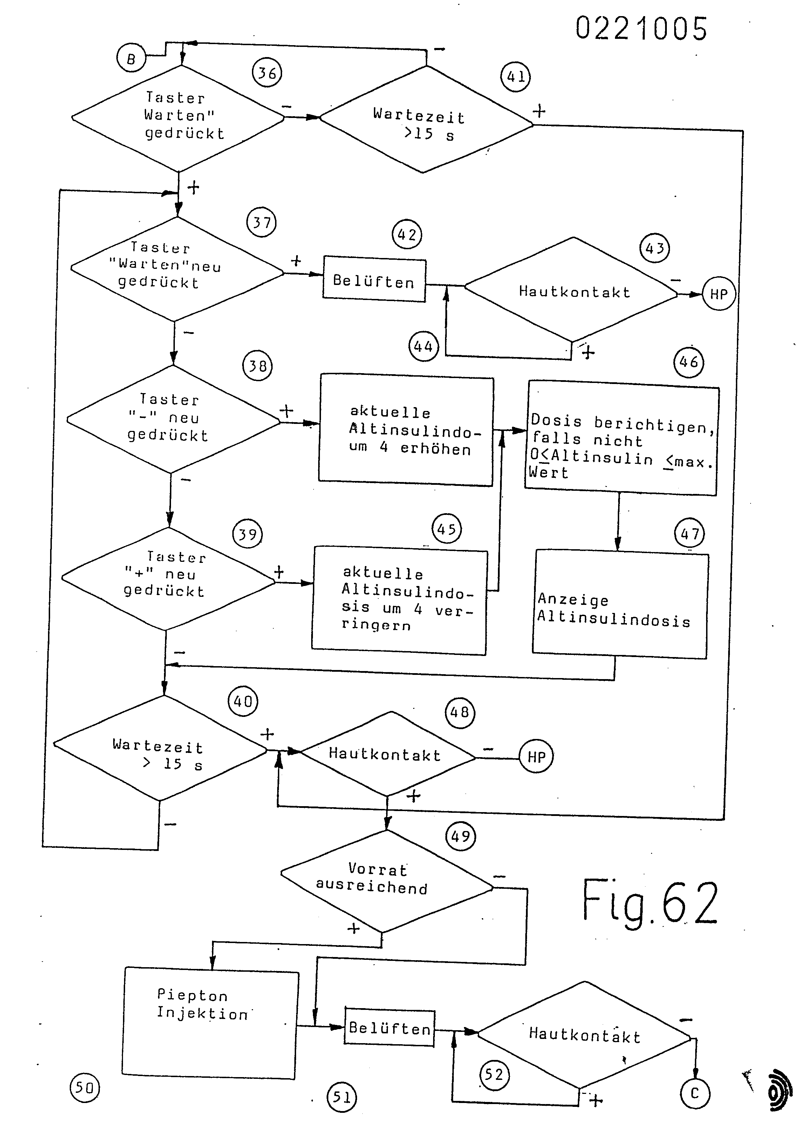

Figur 62 ist eine Fortsetzung der Fig.61.

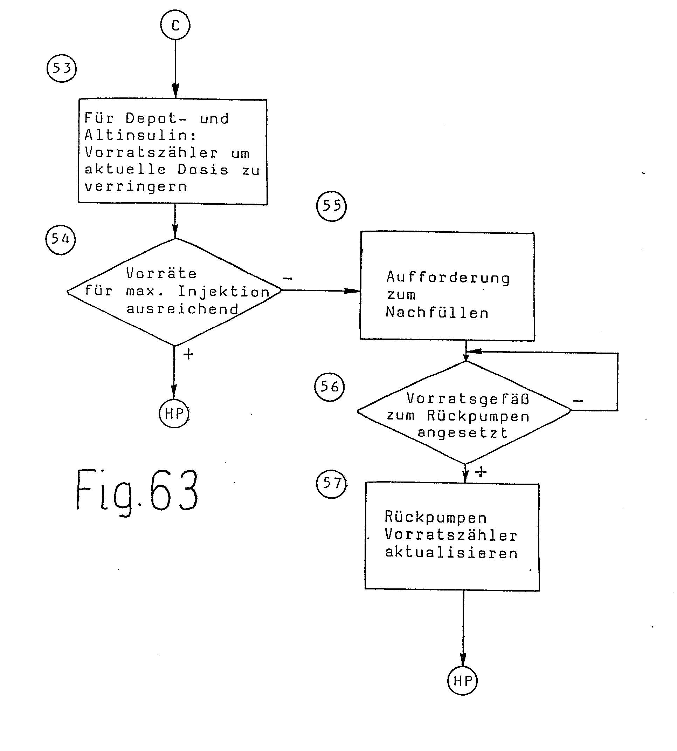

Figur 63 ist eine Fortsetzung der Fig.62.

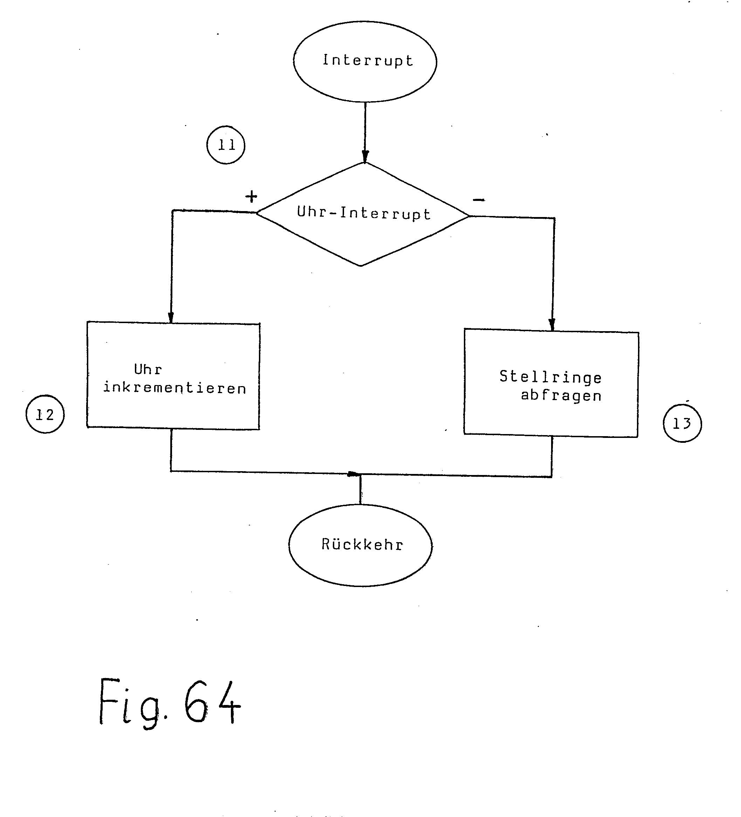

Figur 64 ist eine Fortsetzung der Fig.63.

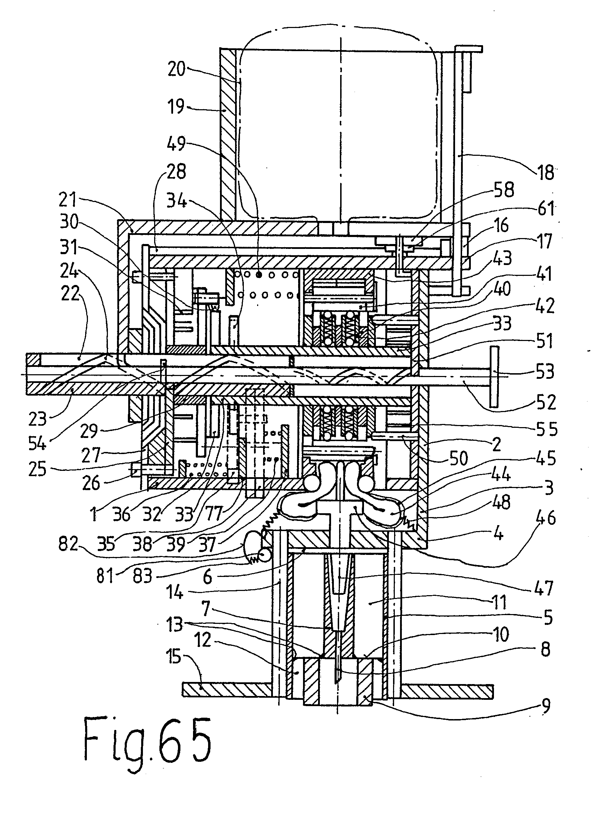

Figur 65 zeigt ein Beispiel eines Injektors mit zwei parallel und identisch betriebenen Schlauchpumpen als chen nach dem Zweikammerprinzip als Vakuumquelle. insgesamt. im Längsschnitt dargestellt.

Figur 66 beschreibt in einem Querschnitt A - B der Fig.65 den Mechanismus. welcher die Dosisvorwahl für jeweils bis zu drei Benutzungen ermöglicht.

Figur 67 zeigt im Querschnitt C - D der Fig.65 das Dosiszählwerk mit Sperrvorrichtung.

Figur 68 zeigt in einem Vertikalschnitt E - F der Fig.65 den Auslösemechanismus für die Wiederbelüftung und für die Dosisabgabe nach dem Rüttelvorgang.

Figur 69 zeigt im Querschnitt G - H durch den obersten Bereich des Unterdruckspeichertöpfchens der Fig.65 mit dem Mechanismus zum Festhalten des letzteren im Injektor sowie einen in Fig.65 nicht dargestellten Mechanismus zur Entfernung desselben.

Figur 70 zeigt im Querschnitt J - K der Fig.65 den Mechanismus für die Auslösung der Rüttelvorrichtung und den Auslösemechanismus für die Wiederbelüftung mittels eines federbewegten Dornes.

Figur 71 zeigt einen vereinfachten Querschnitt L - M durch die Fig.65 zur Darstellung der Dosierpumpen. Darunter ist ein Querschnitt zur Veranschaulichung der Schlaucheinbettung in den Trägerscheiben der Rollenachsen abgebildet der Pfeilrichtung entsprechend.

Figur 72 zeigt in schematischer Aufrollung längs der Draufsicht O - P der Fig.65 die Rüttelvorrichtung.

Figur 73 zeigt in der Vergrößerung 2 : 1 einen Querschnitt durch eine Pumpenvariante zu Fig.65 mit vier jeweils je einen der beiden Schläuche zwischen sich einklemmenden Rollenpaaren.

Figur 74 zeigt in natürlicher Größe eine schematische aufgerollte Draufsicht in Pfeilrichtung der Fig.73. wobei die Schlauchführung sichtbar wird.

Figur 75 zeigt in stark schematisiertem Längschnitt eine Variation des Auslösemechanismus für die Rüttelvorrichtung und das obere Teil eines Unterdruckspeichertöpfchens.

0221005

- 42 -

Dosiervorrichtung, mit einem Vorratsbeutel großer Wandnachgiebigkeit und einem Unterdruckspeichertöpfchen.

Figur 76 zeigt stark schematisiert vier Funktionsstadien A bis D eines Mechanismus für die Wiederbelüftung mittels eines Schiebeventiles.

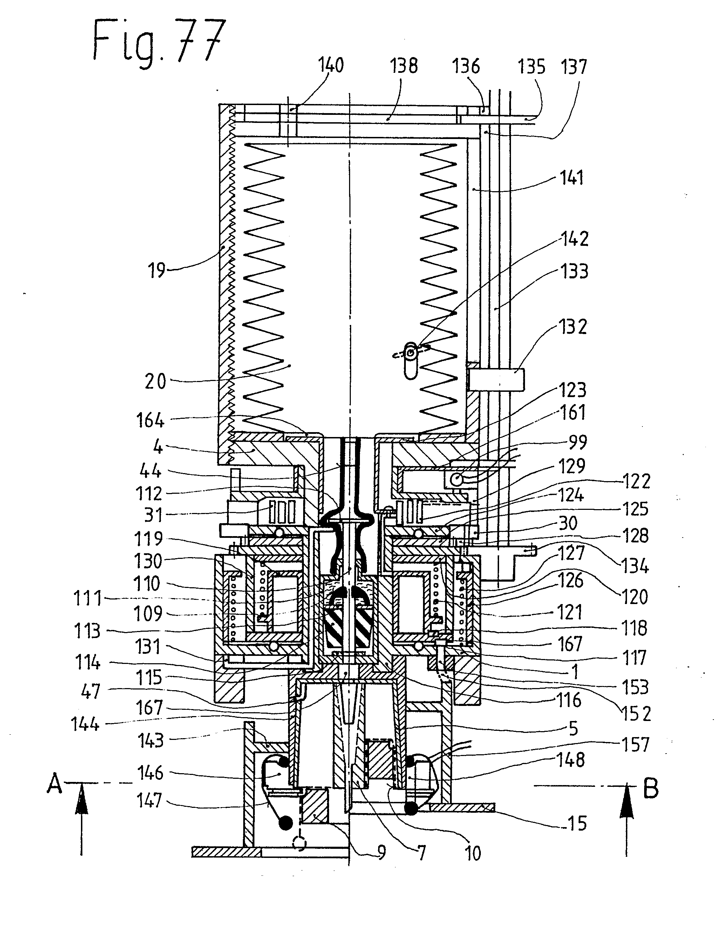

Figur 77 zeigt zeigt einen Längschnitt durch einen Injektor mit einer innerhalb eines Schlauches integrierten Dosiervorrichtung nach einer Art Kolben-Zylinder-Pumpe, einen Schraubdeckel mit Mechanismus zur automatischen Verkleinerung des arzneispeichernden Behälters und einen Saugglokkenrand mit elastischen Lippen, welcher dem Injektor zugehört und einen ebenfalls dem Injektor zuzurechnende Auslösevorrichtung für den Bodenzylinder eines Unterdruckspeichertöpfchens, welche die Haltefunktion eines vor dem Gebrauch im Injektor entfernten töpfcheneigenen Abstützvorrichtung übernimmt.

Figur 78 zeigt im Querschnitt A - B der Fig.77 einen Auslösemechanismus.

Figur 79 stellt einen schematisierten Längsschnitt der Fig.78 in Aufrollung dar.

Figur 80 zeigt stark schematisiert zwei Funktionsstadien A, B in Fig.77 eines Mechanismus zur Wiederbelüftung mittels eines Schiebeventiles.

Figur 81 zeigt einen Längsschnitt durch einen Injektor mit elektromagnetisch betriebener schlauchintegrierter Pumpe ähnlich derjenigen der Fig.77, wobei ein eine Folienrolle als Chemikalienträger für die allmähliche Druckgasbildung in Verbindung mit einem Überddruckventil gleichmäßigen Arzneivorschub gewährleistet und eine Vorrichtung zur thermischen Erzeugung des Unterdruckes für die Saugglocke in drei gesondert aktivierbaren Speicherkammern vorhanden ist.

Figur 82 zeigt einen Querschnitt in Richtung C - D durch Unterdruckspeicherkammern und Saugglocke der Fig.81.

Figur 83 zeigt eine schematische Abrollung des Mechanismus zur Eröffnung der Ventilklappen zu den Unterdruckkammern.

Linksstehend(I) in Fig.83 ist ein Teil der schematischen Aufrollung der Rillenführung für die Nocke des Schaltzylinders der Fig.81 dargestellt, um die sektorale Drehung desselben nach Senkung des Arzneibehälters gegen das Pumpengehäuse zu erklären.

Darunter(III) wird im Querschnitt A - B zu Figur 82 die Auslösefunktion erklärt.

Figur 84 zeigt eine Variation eines Unterdruckspeichertöpfchens ähnlich demjenigen in Fig.77. Auf der linken Bildhälfte ist der in der Quernut sperrende Bodendeckel zu erkennen, in der rechten Bildhälfte nach Entfernung des letzteren die Arretierung des Ringkolbens durch eine Sperrlamelle des Injektors.

Figur 85 zeigt einen schematischen Querschnitt durch die Saugglockenmanschette eines Injektors mit kleeblattähnlich gelapptem Querschnitt und die Lage der Auslösestifte außerhalb der Saugglocke.

Figur 86 zeigt im Längschnitt ein Unterdruckspeichertöpfchen nach dem Einkammersystem mit einer entfernbaren Abdeckelung der Saugglocke bei schematisierter Darstellung eines Sperrmechanismus für den den Bodenring tragenden Einschubzylinder, welcher dem Injektor zugehört, und einer Vorrichtung zur Kontrolle des Kanülensitzes im Hinblick auf deren Blutgefäßberührung.

Figur 87 zeigt stark schematisiert die Verfahrensschritte zur Herstellung eines Unterdruckspeichertöpfchens nach Fig.85.

Figur 88 zeigt ein Unterdruckspeichertöpchen der in Fig.65 verwendeten Art innerhalb des Führungszylinders im Stadium der durch Luftdruckausgleich hochgezogenen Haut im Längsschnitt.

Figur 89 zeigt im Maßstab 2 : 1 eine schematische Aufrollung der beiden gegeneinander gerichteten Profilleisten für die Ablenkung des Ventilstößels in einem Injektor nach Fig.77 in funktionsverdeutlichender Annäherung aneinander.

Figur 90 zeigt in schematischem Längsschnitt einen Befestigungswinkel als starre Verbindung zwischen einem elektrischen Summer und einem Ring um eine Dosierpumpe als Vorrichtung zur Arzneidurchmischung.

Figur 91 zeigt im Längsschnitt einen arzneiführenden Faltenbalg, welcher sich über einen Schlauch mit diesem integrierter Dosierpumpe ohne besonderes Pumpengehäuse entleert.

Figur 92 zeigt ein Blockschaltbild einer Vorwarnvorrichtung hinsichtlich der Arzneivorratserschöpfung für Injektoren wie sie in Fig.37,77,81 beschrieben wurden.

Figur 93 gibt die Schaltskizze zu Fig.92.

Figur 94 zeigt im Längsschnitt eine Abschließvorrichtung des Arzneivorrates bis zur Vorratserschöpfung.

Figur 95 zeigt im Längsschnitt eine Variante der Dosierhydraulik.

Figur 96 bringt unten eine Draufsicht, oben einen Längsschnitt in der Schnittlinie A - B der Draufsicht einer Dosiervorrichtung, welche durch einen elektrischen Magneten(546) druckbeaufschlagt ist.

Figur 97 bringt im Längsschnitt eine schneckenförmige Dosiervorrichtung ähnlich derjenigen in Fig.96.

Figur 97 zeigt im Längsschnitt eine Dosiervorrichtung mittels Piezo-Druckgeber unter fotoelektrischer und schaltergesteuerter Einflußkontrolle besonders für den Einsatz mit enger Ausflußstrombahn.

Figur 99 zeigt im Querschnitt ein Schlauch-Drosselventil in Verbindung mit einer elektronischen Durchflußmessung.

Figur 100 zeigt im Längsschnitt eine Dosisumschaltung mittels eines Schaltgetriebes, also eine Dosiervorrichtung für zwei Arzneien mittels eines Motores.

Figur 101 -auch im Längsschnitt dargestellt- erfüllt dieselbe Aufgabe mittels verschiedener Anzugsgeschwindigkeit eines Piezodruckgebers.

Figur 102 zeigt im Längsschnitt in zwei Funktionsstadien die Bewegung des Kanülenmagazins zur und von der Haut mittels Soges.

Figur 103 zeigt im Längsschnitt zwei Funktionsstadien einer Injektion unter Hautanhebung durch Pflasterzug.

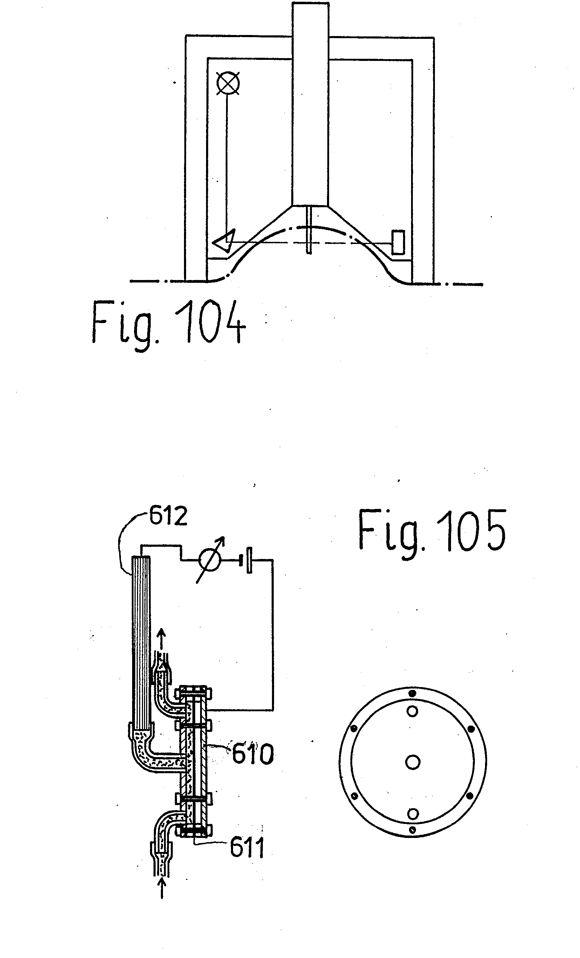

Figur 104 zeigt die polarisations-photometrische Stoffwechselkontrolle durch die in einer Saugglocke hochgehobene Hautkuppe.

Figur 105 zeigt den Grundversuch über die Beeinflussung der Leitfähigkeit einer Membran mit Sepharose-Concanavalin A durch Glukose.

Ausführliche Beschreibung der Ausführungsbeispiele:

Figur 1 zeigt in einer Draufsicht eine gefüllte Injektionsspritze(233), welcher eine Klammer(227) aufgesetzt ist, von welcher Kontaktklemmen(228) ausgehen, welche dem Sensorbelag der Kanüle(326) aufsitzen. Von den Kontaktklemmen führen Leitungsdrähte(234) zum Gehäuse mit der Meßvorrichtung(288) und der Lesevorrichtung(289) mit Drucker. Das Kabel(240) verbindet die Meßvorrichtung mit dem Kontaktpflaster (228) für die Haut, welches als Vergleichselektrode dient.

Figur 2 zeigt eine Vorrichtung ähnlich wie diejenige der Fig.1. Die Injektionskanüle(326) mit dem Sensorbelag liegt aber innerhalb eines den unterdruckbewahrenden Speichertöpfchens(wie es im Canadischen Patent No.1189412 vom 25.Juni 1985 in Fig.3 und 4 und in der Offenlegungsschrift O 103 664 der Europaanmeldung dargestellt wurde). In Kontakt mit dem Kanülenbelag führen die bei der Herstellung eingossenen Leitungsdrähte(234) zu Steckbuchsen(235) im Deckel. Der Ventilstift(229) liegt innerhalb seines Führungsrohres(230), das ins Zentrum des Speichertöpfchens(hier bei eliptischer Form desselben in einen von dessen Mittelpunkten) verlegt wurde und weist als weitere Neuerung eine dem Saugglockenrand aufliegende Blattfeder(231) auf. Diese ist zur Haut hin konvexbogig gestaltet und im Mittelpunkt am Ventilstift befestigt, dessen angespitztes Ende zur reckbaren Deckfolie(232) in Distanz liegt. Die Kanüle liegt neben dem Ventilstift aber nicht in Projektion auf die Blattfeder. Das Kontaktpflaster ist durch die ringförmige Klebeelektrode(239) am Saugglockenrand mit seiner eingegossenen Kontaktleitung (236) zur Steckbuchse ersetzt. Vor der Injektion werden die Stecker der Zuleitung(236) und des Kabels(240) zur Meßvorrichtung(388) in die Steckbuchsen(235) eingebracht. Die in üblicher Weise gefüllte Injektionsspritze(233) wird auf den Kanülenansatzstutzen aufgesteckt, der Schutzfolienring(237) von der Klebeelektrode(238) auf dem Saugglockenrand abgezogen. Wird dann das Speichertöpfchen zur Injektionsvorbereitung gegen die Haut gedrückt, so wird der Ventilstift angehoben. Die Blattfeder nimmt Spannung auf und schlägt schließlich nach oben um wobei ihre Enden den Saugglockenrand verlassen. Der Ventilstift wird dabei explosionsartig nach oben geschossen und durchbricht mit seiner Spitze die Deckfolie(nachdem sie ausgereckt wurde)was zum Luftdruckausgleich und zur Anhebung der Haut in die Saugglocke führt.(Die Ausreckung der Deckfolie verhindert, daß die Ventilöffnung vor sicherem Abschluß des Saugglockenrandes durch die Haut erfolgt). Der Schutzfolienring(237) muß vor Gebrauch vom Saugglockenrand abgezogen werden. Die Meßvorrichtung(288) kann auch vorzugsweise an einem federnden Gliederarmband(241) tragen werden und die Klebeelektrode kann dann durch ein Kontaktkissen(242) unterhalb der Meßvorrichtung ersetzt sein. Die Dosiervorrichtung enthält ähnlich wie diejenige der Figuren 78 oder 82 eine taktweise durch Handschluß über die Griffleiste(180) zu betätigende Pumpe. Diesselbe ist in den Schlauch(44) eingebaut und wird vom Behälterzylinder(19) mit geschlitzten Wandungen mittels der Verschaubung(578) festgehalten. Der arzneiführende Faltenbalg(731) ist mittels eines Befestigungsringes(579) am Behälterzylinder befestigt. Der Befestigungsring dient der Druckfeder(580) als Gegenlager, um den Stössel(110) zurückzubewegen und damit den Arzneiausstoß zu bewirken. Nach Haftung des Speichertöpfchens auf der Haut kann das Meßergebnis abgewartet und auch eine von

- 48 -

von einem Rechner ermittelte korrigierte Dosis taktweise verabreicht werden.

Figur 3 zeigt die Kombination der Sensor- und Dosis-Kompensationseinrichtung(also des Rechners) mit einer handelsüblichen Insulin-Infusionspumpe(245). Die Meßvorrichtung(288) sowie Lese-(289) und Rechner(246) sind dem Gehäuse der Infusionspumpe angegliedert. Die mit den Teilen der Sensorkanülen verbundenen Leitungsdrähte(234) werden eine Strecke weit innerhalb der Wandung des Zuleitungsschlauches(236) isoliert geführt und dann weiter mit eigener Isolierung zu den Steckbuchsen(235) im Gehäuse der Steuereinheit(175).

Figur 4 zeigt im Längsschnitt und rechtstehend im Querschnitt das Detail eines Kanülenansatzstutzens in Verbindung mit einem Schlauchkoppelungsstück.

Der Kanülenansatzstutzen(47) steht in Beinahekontakt mit dem Steckkonus(570) des Zuleitungsschlauches(236). Die Halteklammer mit dem Schleifkontakt(254) innen ist noch nicht in die Ringnut(581) kanülenwärts eingerastet, von welcher aus über das Drähtchen(577) Kontakt mit dem Kanülenschaft(79) hergestellt wird. Vom Schleifkontakt führt das Kabel(240) eine Strecke weit in der Wandung des Zuleitungsschlauches isoliert zur Meßvorrichtung. Vom Sensorbelag führt führt ein anderes Drähtchen durch den Kanülenschaft zum Innenkontakt (253) innerhalb der Bohrung des Kanülenansatzstutzens. Dort tritt dieser mit der leitenden Oberfläche des Steckkonus in Berührung und damit über den Leitungsdraht(234) mit der Meßvorrichtung.

Vorzugsweise für die Einrichtung nach Figur 3 ist eine Meßwert-Speicher-Vorrichtung nützlich, um das Verhalten der Meßwerte bei geringer Gewichtsbelastung durch das Gerät für den Patienten,für den Arzt durch eine Wiedergabeeinrichtung(255) in Zahlen und eventuell als Kurven auf einem Bildschirm sichtbar zu machen.

Figur 5 stellt die Blockschaltskizze einer derartigen Einrichtung dar. Als Hardware wurde ein prozessorge-

0221005

- 50 -

tels eines Halterringes(706) mit dem Bodenring(702)
dicht verschraubt und mittels des Befestigungsringes
(707) mit dem Kolben(708), dessen Führungshülse(709)
ein Verkanten verhindert. In der zentralen Bohrung
des Kolbens(708) befindet sich in lockerem Gleitsitz
das Schieberohr(710), welches mittels Verschraubung
zur zeltartigen Membran(711) gedichtet ist, die mittels des Ringes(712) dicht auf dem Kolben(708) ver
schraubt ist. Das Schieberohr(710) setzt sich oben in
den Ableitungsschlauch(713) fort, welcher dank vorgefertigter Spannung in Spiralen unter der Deckelkappe
(714) liegt und diesen zu einer Wasserstrahlpumpe oder
einer anderen Sogquelle hin über die Befestigungsbuchse(715) verläßt. Der Trägerring(716) für die Dauermagnetstifte(718), welche oben durch den Eisenring(718)
magnetisch zusammengeschlossen sind wird durch die
Schraubstifte(719) am Kolben(708) befestigt. In seiner
Zentralbohrung läßt der Trägerring(716) das Schieberohr(710) durch, dessen unteres Ende zum besseren Luftdurchtritt geschlitzt ist. Durch eine periphere Bohrung tritt ebenfalls in Gleitsitz das Auslöserohr(721)
für den Ventilstößel mit Dichtung, der zur besseren
Luftführung als Achtkant in der gedeckelten Ventilbohrung leicht druckfederbeaufschlagt gleitet. Der Kanal
(722) führt aus der Ventilbohrung seitlich zu einem
nach oben offenen Schlitz der Führungshülse(709) heraus, um die Außenluft durch die Kappenbohrung(722)
hereinzulassen.
Figur 9 zeigt im Längsschnitt den Einschubzylinder
(724) mit einer Schulter für die Dichtung(725), den
beiden Bajonettstiften(726) und der Saugglockenöffnung(727) nach unten hin. Innerhalb der Einschubhülse ist mit kolbiger Verdickung nach oben hin die
Schiebehülse(728) angeordnet, welche durch die schwache Druckfeder(729) gegen die auf die Einschubhülse
aufgeklebte Eisenplatte(730) gedrückt wird und dabei
die Spritze (233) an ihrem Flansch nach oben hält.

Die oft entbehrliche Abdeckkappe(732) für die Saugglocke besteht aus gummielastischem Material, während die übrigen Teile- abgesehen noch vom Schieberohr(710) und Auslöserohr(721), welche auch aus Metall sein können- zweckmäßigerweise aus einem wenig zerbrechlichen Acetatkunststoff, Delrin oder ähnlichem gefertigt sind. Ein Schleifkontakt(254) im Einschubzylinder richtet sich gegen den Kanülenschaft, eine Kontaktfeder(576) gegen den Kanülenansatzstutzen einer geeignet kontaktierten Sensorkanüle.(Anstelle des leitenden Überzuges auf dem Steckkonus (570,wie in Fig.4) ist dazu die Außenfläche des Kanülenansatzstutzens leitend). Der Leitungsdraht(234) und das Kabel(240) verbinden den Injektor mit der Meßvorrichtung.

Zur Vorbereitung der Injektion wird die Spritze in üblicher Weise aus dem Fläschchen gefüllt, dann mit der Sensorkanüle versehen und mit der Spitze nach vorn durch die Öffnung in der Eisenplatte(330) des Einschubzylinders so eingeführt, daß der Flansch des Spritzenzylinders durch die ovale Bohrung(siehe Fig.10) der Eisenplatte hindurchtritt. Zweckmäßigerweise -insbesondere dann wenn der Spritzeneinschubzylinder mit dem Spritzenkolben nach unten in den Rohrstutzen(703) des Gehäusezylinders eingeführt wird- wird die Spritze dann am Kolben axial um etwa 45° gedreht, um ihr Herausfallen zu verhindern. Durch Drehung des Einschubzylinders während seiner Befestigung im Rohrstutzen(703) bis zum Anschlag der Bajonettstifte wird die Dichtung(725) gegen den Bodenring(702) gepreßt und dadurch zwischen Gehäusezylinder und Einschubzylinder wirksam. Zweckmäßigerweise erfolgt eine etwaige Lagerung innerhalb eines sauberen Leinentuches. Der Sauginjektor wird am besten ständig mit einer Dauersogquelle, beispielsweise einer Wasserstrahlpumpe, verbunden, wie dies auf Figur 12 dargestellt ist. Zu diesem Zwecke wird bevorzugterweise ein konisch erweitertes Ende eines Wasserschlauches solange verkürzt, bis es gerade über den Teil des im Haushalt vorhandenen Wasserhahnes mit der Austrittsöffnung stramm aufgeschoben werden kann, wo es mittels einer Bandschelle(738) festgeklemmt wird, welche den Haltearm(739) trägt. Dieser wiederum weist die mittels Flügelschraube feststellbare Achse(740) zu den Klemmbacken(741) auf. In diese kann das Rohr(743) der Wasserstrahlpumpe in beliebiger Höhe eingeschoben und mittels der Flügelschraube(742) festgeklemmt werden. Da die Mündung der Strahlpumpe so beliebig ausgerichtet werden kann und der Wasserstrahl zuvor über eine Verwirbelungsvorrichtung(744) geleitet wird, läßt sich der Hahn leicht benutzen, wobei jeweils über den Stutzen(745) mit dem Ableitungsschlauch(713) Luft angesaugt wird. Zur Injektion hat der Patient den an einem Haken hängenden oder auf einer Ablage befindlichen Injektor aus dem Abdecktuch zu nehmen und die Saugglocke gegen eine beliebige Köperpartie zu richten(soweit sie sich nicht schon von der Empfindlichkeit und Beschaffenheit her zur Injektion als ungeeignet ausweist, wie Schädelbereich,After und Sohlen oder Handflächen), nachdem er den Wasserhahn geöffnethat und damit die Strahlpumpe nach Anordnung der Fig.12 betätigte. Es wird dann durch den Sog zunächst die Haut in die Saugglocke gehoben und dort festgehalten, dann die Membran (711) mit dem leichtgängigen Schieberohr(710) und schließlich der Kolben (708) mit dem Faltenbalg(705) gesenkt und damit zunächst die Injektionsspritze(233) gegen die Druckfeder (729) gepreßt. Nach Anschlag der Schiebehülse(728) auf dem Boden des Einschubzylinders(724) und Eindringen der Kanüle in die hochgezogene Haut wird der Spritzenstempel bis zum Anschlag gesenkt und die Spritze entleert. Auf den letzten Millimetern der Wegstrecke des vom Kolben(708) mitgenommenen Trägerringes(716) werden dessen Magnetstifte von der Eisenplatte(730) angezogen, was die Anhebung des Auslöserrohres(721) und damit die Öffnung des Wiederbelüftungsventiles begünstigt, so daß die Außenluft über den Kanal(722) eindringen kann, womit sich durch Aufhebung des Vakuums unter der Membrane(711) die Spritze unter Wirkung der Druckfeder(729) hebt, während sich die Haut aus der Saugglocke zurückzieht, während Faltenbalg mit Kolben(708) weiterhin durch die Magnetkraft unten festgehalten bleiben. Dies kann der Patient bei Durchsichtigkeit des Gehäusezylinders(701) oder durch den seitlichen Längsschlitz(733) in diesem notfalls unter Betastung mit dem Finger vor Ablage des Injektors feststellen. Wird die Verschraubung des Einschubzylinders(724) unter Rückdrehung der Stifte(720) in der Bajonettrillenführung(704) gelockert, so entfernt sich die Eisenplatte(730) von den Magnetstiften und der Faltenbalg dehnt sich durch seine Eigenelastizität wieder aus, und das Rückschlagventil wird durch seine Druckfeder geschlossen.

Es ist noch der Andruckstift(574) nachzutragen, welcher an der Schiebehülse(728) befestigt ist und bei deren Senkung gegen den Schleifkontakt(254) stößt und diesen dem Kanülenschaft nähert. Auf diese Weise wird eine Verschmutzung des später in die Haut eindringenden Kanülenabschnittes vermieden.

In Figur 11 ist ein Teil einer Einrichtung ähnlich derjenigen der Figuren 8 bis 10 dargestellt, welche eine Variante des Wiederbelüftungsventiles und dessen automatischer Bedienung zeigt. Der Gehäusezylinder(701) wird durch eine Lücke(746) mit Wandabschrägung in einen oberen und unteren Teil getrennt, welche durch die Befestigungsmanschette(747) zusammengehalten werden. Der Kolben(708) ist mit dem Faltenbalg(705) verbunden und enthält in seiner zentralen Bohrung das Schieberohr(710), welches oben eine Einkerbung(748) aufweist und dessen Innenlumen sich nach oben hin in einen kleinen Faltenbalg(749) fortsetzt. Dieser wiederum setzt sich in den Ableitungsschlauch(713) fort, welcher mittels Befestigungsbuchse(715) in der Deckplatte(749) gedichtet ist. In einer Querbohrung des Kolbens(708) sind zwei Stifte (750) mit gerundeten Endkuppen verschieblich gelagert, deren elastische Dichtkappen(751) innerhalb der Bohrungen(752) die Ränder zum Eingang in die kleineren Bohrungen(753) abdecken können. Aus den kleineren Bohrungen(753) führen Kanäle(722) zur Wiederbelüftung nach oben. Das untere Ende des Schieberohres(710) erweitert und verlängert sich zu einem Topf(754). Bei entspannten Faltenbalg(705) streckt sich der kleine Faltenbalg(799), die Stifte liegen über die Wandschrägung der Lücke(746) bei der Aufwärtsbewegung nach innen verschoben innerhalb der Tiefe der Kerbe(748) und die Dichtkappen(751) werden den kleinen Bohrungen dichtend angepreßt. Unter Sogwirkung kann sich nach Verschluß der Saugglocke durch die Haut der Kolben(708) senken; nach Berührung mit der Deckelplatte (30) des Einschubzylinders wird der Topf(754) und damit auch das Schieberohr(710) angehoben, wobei die Schräge der Kerbe(748) die Stifte(750) nach außen drängt und zwar gerade in dem Stadium der Kolbensenkung, in welchem ihre Enden in die Lücken(746) des Gehäusezylinders ausweichen können, wodurch eine aufwärtsbewegung des Kolbens solange verhindert wird, bis nach Entfernung des Einschubzylinders das Schieberohr(710) wieder nach unten ausweichen kann, was durch die Elastizität des kleinen Faltenbalges(799) noch begünstigt wird. Der Moment der Ablenkung der Stifte(750) nach außen bei tiefstem Kolbenstand ist auch derjenige, bei der die Elastizität der Dichtkappen(751) überfordert wird, so daß deren Ränder von ihrem Sitz vor den Eingängen der kleinen Bohrungen (753) abgerissen werden. Dadurch wird der Luftstrom am Kolben(708) vorbei ins Innere des Faltenbalges (705) zur Wiederbelüftung freigegeben.

Figur 13 zeigt im Längschnitt eine Injektionsvorrichtung, welche mittels einer CO2-Druckgaskapsel(256) über die Gasstrahlpumpe(257) den Unterdruck für die Saugglocke(173) erzeugt, welcher dort vom Rückschlagventil (323) aufrechterhalten wird. Die Entnahme des Druckgases erfolgt über den Dorn(258) mit Rinne, welcher mit dem Ventilblock(259) dicht verschraubt ist. Von der Deckelbrücke(260) aus schließen sich nach unten stehend an den Ventilblock zwei Koppelungsventile an, dann ein Steuerschacht mit Kanülenmagazin(262), je eine vordere und hintere Dosierspindel(263) mit Gewinde für die zugehörigen Dosiermuttern(264) -wie Fig.14 im Querschnitt zu sehen ist-, Schieberventil(265) und verschiebliches Pumpengehäuse(111) für den auf einer Stange feststehenden Kolben der Dosiervorrichtung. Alle bisher genannten Bestandteile und noch andere gehören dem wieder zu benutzenden Geräteteil an, während im Gehäuse(226) des wegzuwerfenden Einschubteiles(269) die CO2-Druckgaskapsel(256) über dem siebartig durchlöcherten Aufnahmerohr(269) für den Quellstift(270), den elastischen Beutel(271) mit einer Hilfsflüssigkeit und den Stufenspritzenzylinder(272) aus silikoniertem Glas enthält. Letzerer endet oben in dem Ventilrohr(273) über dem die Schiebemanschette(274) liegt. Der Trennkolben (275) zwischen Arznei-und Hilfsflüssigkeit weist den Wachsmantel(276) auf und endet in das seitlich abgehende und von unten in eine Bohrung des Kanülenmagazins(316) reichende hakenförmige Auslaßrohres(277). Einschubteil und Geräteteil sind mittels der Befestigungsspindel(279) mit einander verschraubt. Zum Anziehen der Gegenmutter (280) mit Vierkant dient der gefederte Ring(281) auf welchem der Rohrstutzen(282) mit dem Scharniergelenk (322) mittels Drehbewegung des Haltearmes(341) mit dem Scharniergelenk(322) für den Saugglockendeckel (342) einwirken kann. Zur Feststellung der Dosierpumpe dient der Winkelarm(283) mit Kraftübertragung zwischen der Arretierleiste(283) im Steuerschacht und dem über dem Zylinder des Pumpengehäuses(111) gegen die Druckfeder(284) verschieblichen Schiebezylinder(285), welcher im Außenzylinder(286) geführt wird. In Einschnitten der Arrettierleiste ist um eine Längsachse schwenkbar der Keil(287) für den Dosiswechsel angeordnet und der Halteschlitz für die Zugschlaufe(288). Das nach der Lippendichtung(290) pfeifenartig erweiterte Kanülenstaurohr(289) wird mittels des Haltearmes beim Deckelschluß mit dem Ende der gebrauchten Kanüle in Kontakt gebracht. Oben ist noch die Druckfeder(291) für den Deckelschlitten(292) und die Aufnahmehülse(293) für den Beutel(256) mit der Hilfsflüssigkeit zu erkennen. Von den Gaskanälen sieht man den Querkanal(294) nach dem Schleußenventil -das in Figur 19 näher beschrieben ist-, welcher hinter der Sperrkugel des Rückschlagventiles(295) über den Kanal(296) durch die Kanüle(297) innerhalb einer Stopfenmembran in der Trägerhülse(293) in den Gasspeicherraum(429) des Einschubteiles führt. Über das parallel zum Rückschlagventil(295) angeordnete Rückschlagventil(298), welches über eine Düse in die Schlauchverbindung(431) zum Ende des Kanülenmagazins die Druckgasleitung fortsetzt,führt der Kanal (299) zur Gasstrahlpumpe(257).

Figur 14 zeigt einen teilweisen Querschnitt durch den Injektor Fig.13 entlang der Schnittebene A - B dort. Es sind die elliptische Saugglocke(51) mit dem Zentralstift(433) und dem Kanülenmagazin(316) im Inneren, der Stufenspritzenzylinder(272), der Außenzylinder(286) der Dosierpumpe für die Hilfsflüssigkeit, die Dosierspindeln(263) mit Dosiermuttern(264) und die Arretierleiste(283) dargestellt. Figur 15 zeigt im Querschnitt in der Schnittführung A - B der Fig.19 den Deckelschlitten(292) mit seiner Druckfeder(291) und den Bolzen(449) des Klappenscharniers. Außerdem wird der U-Bogen des transversal verlaufenden Kanülenmagazins(316) sichtbar gemacht. Figur 16 zeigt im Längsschnitt eine Flüssigkeitskoppelungsvorrichtung in Annäherung des Einschubes an die Deckelbrücke. Der Flüssigkeitsaustrittsöffnung(442) im oben belüfteten Aufnahmezylinder(443), welche durch die gefederte Schiebehülse(444) verschlossen ist, entspricht die Flüssigkeitseintrittsöffnung(262) im Ventilrohr(273) des Stufenspritzenzylinders(272).Diese ist durch die Schiebemanschette(274) verschlossen und wird erst bei Annäherung der Schulter des Aufnahmezylinders zur Öffnung verschoben, wobei der Deckel des Ventilrohres auch die Schiebehülse(444) verdrängt.

Figur 17 zeigt in einem Querschnitt die Gasstrahlpumpe(257) mit dem Gaszustromkanal(446), dem zur Saugglocke führenden Saugstutzen und dem sogerhaltenden Rückschlagventil(448).

Figur 18 zeigt im Maßstab 2 : 1 rechts im Längsschnitt den Kanülenwechselmechanismus. Die Rasterkugel(432) wird vom Zentralstift(433) in seine Bohrung(434) innerhalb des Aufnahmekonus(435) für das Gehäuse(575) des Einschubteiles durch einen seitlichen Schlitz im Kanülenmagazin hindurch in die Mittelkerbe(436) des Kanülenkörpers gedrängt. Letztere kann über den Gasdruck, welcher hinter dem Kanülenmagazin für kurze Zeit aufgestaut ist, erst gesenkt werden, wenn der Zentralstift ganz gehoben ist. Dabei läßt dessen seitliche Abplattung(437) die Rasterkugel aus der Kanüle zurücktreten. Die Kanüle senkt sich bis zur gefederten Sperrklinke (438) Wird der Zentralstift wieder gesenkt, so verdrängt er die Rasterkugel wieder, da sie in die obere Einkerbung der tieferen und in die untere Einkerbung der oberen Kanüle ausweichen kann. Wird durch den Schaltstift (356) im Deckel die Sperrklinke betätigt, so kann die Kanüle von der Lippendichtung in das Kanülenstaurohr übernommen werden. Da eine leichte Hebung des Zentralstiftes beim Deckelschluß wieder eine Lücke im Zentralstift die Rasterkugel passieren läßt, tritt die nachfolgende Kanüle tiefer. Das Kanälchen(439) im Kanülenkörper kommt in der Stellung desselben auf der Sperrklinke in Deckung mit der Arzneiaustrittsöffnung(440) aus der Stufenspritze. Die Darstellung rechts im Querschnitt zeigt die ovaläre Gestalt von Kanüle und Kanülenmagazin(262) und die Lippendichtung(441) oberhalb des beschriebenen Rastmechanismus für den Zentralstift(433).

In Figur 19 sind auf einem oberflächennahen Längschnitt die Klappenscharniere mit den(unterbrochen dargestellten) Klappen der Vorderseite zu sehen. In der Klappenmitte ist ein rechteckiger Schlitz(452) für den Bewegungsspielraum der Dosiermuttern freigelassen. In einer mehr hinten gelegenen Ebene ist links das"Schleussenventil" abgebildet. Der Gaskanal(294) aus der $CO_2$-Druckgaskapsel führt in die Überdruckkammer(455), welche von dem großen Ventilteller(456) verschlossen wird. In dessen zentraler Bohrung sitzt an dem schmal endenden Stift ein kleiner Ventilkegel vor einer Anschlagplatte(457). Der Stift setzt sich in eingeschliffen in den Schieberventilzylinder(458) fort und weist eine Ringnut auf. In den Schieberventilzylinder wird das Gas aus der Überdruckkammer(455) seitlich eingeleitet und nach Senkung des Kappenstiftes(461) unter Einwirkung der Keilschräge des Schiebers(462) über die Ringnut zum Querkanal weitergeleitet. Zuvor kam es nach Aufstoßen des kleinen Ventilkegels bei noch geschlossenem Schieberventil zum Gasstau in der Überdruckkammer, welcher das Aufstoßen des großen Ventiltellers mittels der Andruckplatte erlaubte. Rechtsstehend unter (II) in einem Vertikal-Schnitt längs der Schnittlinie A - B des Längsschnittes werden die geschlossenen Klappen(445) mit den Klappenscharnieren(450) gezeigt, deren Stützarme(463) sich berühren und von denen der linke den Bolzen(449) trägt. Links ist gestrichelt die geöffnete Klappe skizziert.

Unter (III) ist die linke Klappe in einer Draufsicht in Höhe des Schlitzes(452) mit der Dosiermutter zu sehen. Darunter unter (IV) ist ein Querschnitt durch die Deckelbrücke gezeigt. Deutlich sieht man die Beeinflussung des Deckelschlittens(292) über dessen Keilschräge(464) mittels des Bolzens(449).

Die Figur 20 zeigt in einem Vertikal-Schnitt durch den Steuerschacht die beiden Dosierspindeln(263) mit Dosiermuttern(264), die Befestigungsangel(465) für die Achse(466) des Keiles(287) für den Dosiswechsel mit dem Noppenfeder(467) in dessen Kulissenführung(72). Darunter sieht man den gefederten Stift(468) zur manuellen Umschaltung zwischen den eingestellten Dosierungen. Der Auslöser(469) weist die Profilnase(470) für den Eingriff in die Rastkerbe der Arretierleiste (283) auf, an welche er mittels der Blattfeder(471) herangedrückt wird, wobei er um die Achse(472) drehbar ist. Rechts sieht man die Rückholfeder(472) des die Noppenfeder tragenden Bolzens, welcher durch den im Schlitz gleitenden Führungsstift(473) drehgesichert ist. Im unteren Steuerschacht oberhalb der Saugglocke überträgt die Trägerplatte(474) die Bewegungen des Zentralstiftes(433) auf die dem Außenzylinder (286) anliegende teilweise in zwei Schenkel aufgeteilte Halbrinne(475) zu den Deckelschiebern. Darunter ist die gegenüber dem Zentralstift höhenverschiebliche Verbindungslasche(476) für die drehgesicherte Befestigung der Dosierspindeln(263) dargestellt. Der teilweise Querschnitt unter Figur 20 macht die Lage der Teile noch deutlicher.

Figur 21 stellt im Maßstab 2 : 1 den Auslöser für die Flüssigkeitsinjektion im Detail dar. Die Auflage der Profilnase(470) auf einem Vorsprung der Arretierleiste (283) wird unten im Querschnitt gezeigt. Im Längsschnitt oben kann man die Überholfeder(477) erkennen, welche sich verschieblich gegen die Gabelöffnung(483) anlehnt(wie in einer Ansicht in Pfeilrichtung A rechtsstehend und darunter um 90 Grad gedreht gezeigt wird). Der Auslöseschieber(484) am Ende des Zentralstiftes bewirkt bei Anhebung seiner oberen Keilschrägen gegen die Überholfeder die Ausweichbewegung des Auslösers(469), wobei er mit seiner Gabel in die Einkerbung(485) zu liegen kommt unter dem Einfluß seiner Blattfeder(471). Kehrt der Zentralstift nach der Injektion gegen die Haut gerichtet zurück, so schiebt die obere Anschrägung der Einkerbung(485) die Überholfeder zurück, und der Auslösschieber kann innerhalb der Gabelöffnung(483) den Auslöser überholen.

Die Figur 22 zeigt in einem Vertikal-Schnitt durch den Steuerschacht -welcher den Zwischenraum zwischen Einschubteil und Dosierpumpe bildet- oberhalb des Auslösers im Maßstab 2 : 1 den Mechanismus für die Dosierstufenauswahl. Man erkennt, daß der Stift(468) für die Umschaltung der Dosierung mit seiner Lagerbuchse an der Arretierleiste(283) angehoben werden kann, während der Gegenstift(499) mit seiner kräftigeren Druckfeder feststeht. Am Querbolzen(501) kann er in der Führung des Wandschlitzes(500) gesenkt werden. Dabei rückt der Stift(468) unter Wirkung seiner Feder nach unten nach und die Noppenfeder(467) bewirkt durch ihren Ablauf innerhalb der Kulissenführung(72) des Schaltzylinders(welcher linksstehend herausgezeichnet wurde) eine Achsenschwenkung des Schaltzylinders und damit auch des in Abwinkelung an diesem befestigten Keiles für die Dosierung. Der Wechsel der Dosierstufenauswahl kann so ohne Auslösung der Dosierung vollzogen werden.

Auf dem Querschnitt unterhalb ist die Winkelverschiebung zwischen Schaltzylinder und dem Keil(287) deutlich erkennbar sowie auch dessen Lage oberhalb der Dosiermutter(264). Die unterbrochene Keildarstellung gibt dessen Lage nach der Umschaltung wieder.

Die Figur 23 zeigt die von der Halbrinne ausgehenden Steuerarme und die von ihnen Deckelschieber mit Zähl- und Sperrvorrichtung in Teildarstellung im Längsschnitt und in der Darstellung in der Mitte im Querschnitt und unter diesem in einer Draufsicht. Rechts wird noch ein Vertikal-Schnitt gezeigt.

Die Endrolle(486) des Steuerarmes des Zentralstiftes wirkt bei Anhebung desselben auf die Keilschräge des Schiebers(487) diesen nach links verschiebend. Dessen gefederter Rasterbolzen(488) nimmt im Kraftschluß den Ventilöffnungschieber(489) mit sich, welcher den Kappenstift(461) mittels seiner Keilschrägen senkt und damit die Ventile nach der CO2-Gasdruckkapsel öffnet. Die Profilführung(490) der Deckelplatte(492) bewirkt durch Ablenkung des Führungsstiftes(491) für den Rasterbolzen dessen Verschiebung entgegen seiner Federung. Dadurch werden die beiden Schieber(487,489) gegenseitig entriegelt. Wie besonders deutlich auf dem rechtsseitigen Vertikal-Schnitt zu erkennen ist weist der vom Zentralstift ausgehende Kantstab(493) eine seitliche Schaltnoppe(494) auf, welche bei ihrer Anhebung eine Hubststrecke lang in einen Einschnitt des Ventilöffnungsschiebers zu liegen kommt und damit dessen Rückbewegung unter Wirkung der Druckfeder(495) verhindert. In der Höchststellung des Zentralstiftes verläßt dessen Schaltnoppe jenen Einschnitt im Ventilöffnungschieber, so daß letzterer unter Ventilschluß wieder in die Ausgangsposition zurückkehren kann. Der Schieber(487) kann unter dem Einfluß der Druckfeder(496) erst dann wieder in die Ausgangslage zurück, wenn dessen Keilschräge die Endrolle(486) nach Senkung des Zentralstiftes die Keilschräge des Schiebers verlassen hat. Diese Rückwärtsbewegung erfolgt erst unter Wirkung der Druckfeder(291) durch die Bewegung des Deckelschlittens(292) -welcher auf Fig.13 dargestellt ist- über die Einwirkung der Keilschrägen auf die Endrolle (498). Der Deckelschlitten wiederum wird erst aktiviert, wenn über Anhebung einer Dosierspindel(263) wegen des Verbundes derselben über die Verbindungslasche(476) der Querstift(266) über eine Raststufe im Schrägschlitz(267) gehoben wurde(Fig.13,19). Mit der Anhebung der Verbindungslasche wird aber auch die an ihr befestigte Auslösestange(60) des Belüftungsventiles(324) gegen seine Federung angehoben und Saugglocke wiederbelüftet.

Die Figur 24 zeigt oben im Längs- und darunter im Querschnitt den Zählwerkmechanismus für die Zahl der Dosierungen. Der Stock(502), welcher in Verlängerung des Zentralstiftes von der Halbrinne ausgeht, betätigt den Zählwerktransport, indem er innerhalb der Schaltbuchse(503) das Ritzel(504) anhebt, dessen vier Schaltnocken(505) innerhalb der vier axialen Steuernuten (406) geführt werden. Hebung und Senkung bewirken zu gleichen Anteilen eine Drehung des hohen und schmalen Zahnrades(507), welche von diesem auf das flache und breite Zahnrad(508) ragen, daß als Zählrad dient und eine Nummernskala trägt. Das Zählrad ist auf dem Bolzen(512) mit Feingewinde montiert, welcher in seiner Gewindebuchse allmählich gehoben wird, bis die Nocke (509) -nach Entleerung eines Kanülenmagazins- gegen den Arretierstift(511) am nach links gefederten Sperrschieber(510) stößt. Durch Betätigung desselben kann die Sperrung(nach Erneuerung des Kanülenmagazins) aufgehoben werden. Ist der Insulinvorrat erschöpft, so stößt die Nocke(509) gegen die Sperrleiste(513) am Sperrschieber. Die Sperrung wird durch Zurückschrauben des großen Zahnrades(508) mittels des Steckschlüssels(514) aufgehoben. Die Zahl der Umdrehungen erfolgt gemäß einer am Gerät befestigten Tabelle, welcher die Zahl der Benutzungen der Höhe der beiden einstellbaren Insulindosen zugeordnet wird. Figur 25 zeigt im Längsschnitt im Maßstab 5 : 1 den Mechanismus des Zählwerktransportes mit Ritzel(504), Schaltnocke(505) an diesem, Schaltbuchse(506) und kleinem Zahnrad(507).

Figur 26 gibt im Maßstab 5 : 1 eine Aufwicklung der Innenseite der Schaltbuchse(506), wobei die Pfeile den Weg einer Schaltnocke(505) bezeichnen. Figur 27 zeigt im Längsschnitt im Maßstab 1 : 1 ein

Kanülenmagazin(262). Schematisch und gestrichelt ist unten das Kanülenstaurohr(289) zu einem solchen umgestaltet, das gebrauchte Kanülen ins Magazin zurückleitet. Ein Magazinquerschnitt innerhalb des oberen Magazinbogens im Maßstab 5 : 1 mit einer Abplattung setzt sich nach rechts im Bogen fort und zeigt im Längsschnitt die Lage von Stapelkanülen mit bogigem Rücken (515), welche auch im Quer- und Vertikal-Schnitt gezeigt wird. Die Andruckfeder(516) bewirkt durch eine Verdünnung der Magazinwand hindurch den Andruck gegen die Arzneiaustrittsöffnung.

Die Figur 28 gibt im Längsschnitt im Maßstab 5 : 1 eine Dosierpumpe wieder, bei welcher der Arzneidurchstrom von unten nach oben erfolgt. Eine solche Pumpe kann bei Einrichtungen nach Figur 13, aber auch nach Fig.78 oder 82 eingesetzt werden. Sie besteht aus einem Außenkolben(518), welcher mittels der Dichtung (517) gegen den Zylinder(519) gedichtet ist; weiterhin aus dem Innenkolben(520), welcher in der Innenbohrung des Außenkolbens verschieblich ist und gegen die konische Abschrägung(523) mittels des Dichtungsringes (521) durch Anhebung des Innenkolbens an der Kolbenstange(524) geschlossen werden kann. Bei der Senkung der Kolbenstange zur Ventilöffnung nach Beendigung des Dosierhubes(nach oben) findet der Innenkolben an der Druckfeder(581), welche der im Außenkolben eingeschraubten Anschlagbuchse(518) anliegt, Widerstand. Die beiden Kolben können so unter Flüssigkeitsdurchfluß zusammen gesenkt werden. Die Dosierung erfolgt durch Anheben der Kolbenstange(524) nach Ventilschluß am Dichtungsring(517).

Die Querschnittsdarstellung rechts vom Längsschnitt läßt die Flüssigkeitsdurchtrittskanäle(525,526) erkennen.

Zur Inbetriebnahme eines Injektors nach Fig.13 bis 26 wird zunächst eine schützende Deckelfolie über einem Einschubteil, welches als Ganzes erneuert wird, abgezogen. Es wird Wasser -günstigerweise aus einer vorbereiteten Spritze- an der $CO_2$-Gasdruckkapsel vorbei eingefüllt. Der Einschubteil wird dann unter die Deckelbrücke(260) gebracht, die Befestigungsspindel(279) in die Wandrinne(460) des Einschubteiles(siehe Fig.14) eingelegt und dieser bei Benutzung des Haltearmes(341) als Hebelarmes mit dem Geräteteil verschraubt. Dabei durchdringt die Kanüle(297) zum Gasspeicherraum(480) die zugehörige Dichtung und die luftfreie Flüssigkeitskoppelung zum Beutel(271) und zum Stufenspritzenzylinder(272) wird bewerkstelligt. Nach vorbestimmter Zeit hat der Quellstift(270) so viel Wasser aufgenommen, daß er die $CO_2$-Gasdruckkapsel gegen den Dorn (358) angehoben hat, wobei letzterer die Weicheisenplombe durchdrungen hat. Es wird dann von oben ein Kanülenmagazin eingeschoben. Bis zum vollständigen Sitz auch des Röhrchens mit der Arzneiaustrittsöffnung(440) in der Wandung des Kanülenmagazins(siehe Fig.14) muß der Zentralstift(433) kurz angehoben werden, um das Magazin an der Rasterkugel(527) vorbeizulassen. An den Dosierschrauben werden jetzt durch Höhenverstellung auf den Spindeln unter Vergleich mit einer Skala die Dosierstufen -beispielsweise für morgens und abends- eingestellt. Dann wird die Druckfeder(284) durch Herabziehen der Zugschlaufe(288) mit eingesteckten Fingern gespannt. Der Kolben(168) wird dabei gesenkt und füllt sich über die Zuleitung(528) und das Schieberventil(Fig.13) mit Hilfsflüssigkeit aus dem Beutel(272). Werden die Klappen(445) zusammengedrückt, so wird der Deckelschlitten gegen seine Druckfeder(291) nach links verschoben. Dabei wird der Kolben des Schieberventils(265) gesenkt und der Ableitungskanal(529) zum Stufenspritzenzylinder freigegeben. Über die Steckbuchsen(235), von welchen die Leitungen(364) zu den Schleifkontakten(584,Fig.27) für den Kanülenkontakt ausgehen, folgt der Anschluß zunächst zur Meßvorrichtung. Das Zählrad für die Funktionssperrung nach Kanülen- oder Insulinvorratserschöpfung muß zurückgedreht sein, da sonst die Bewegung des Zentralstiftes in der Schaltbuchse(503) gesperrt ist(Fig.24). Es wird jetzt beim Abschwenken des Saugglockendeckels(342) die letzte das Magazin verschließende Kanüle -im Magazinanfang ein schaftloser Kanülenkörper- über das Kanülenstaurohr(289) entfernt. Wird die Saugglocke gegen die Haut gepreßt, so wird noch vor der Saugglockenrandberührung der etwas vorstehende Zentralstift angehoben und der Gaszustrom zur Gasstrahlpumpe(257) freigegeben. Die Rasterkugel(527) tritt in die Abplattung des Zentralstiftes zurück(Fig.18). Die Kanüle tritt damit tiefer unter Einfluß dem Einfluß des Gasstaues zwischen dem Rückschlagventil(295) und dem Dichtkörper(585) am Ende des Kanülenmagazins- und der Haut entgegen, wobei die vorher unterbrochene HohlraumverbindungBei zwischen Kanülenschaft und Arzneiaustrittsöffnung(440) wieder hergestellt wird. Während der weiteren Anhebung des Zentralstiftes, welche wegen der Besonderheit der Keilprofilgestaltung(Fig.24) am Schieber(487) ohne Widerstand durch die Hautanhebung erfolgen kann, betätigt dieser den Auslöser(419,Fig.20,21). Damit wird der Schiebezylinder(285) mittels der Druckfeder (284) samt dem Kolben(268) auf dessen Kolbenstange (524) soweit bewegt, wie es der Keil(287) bis zu seinem Anschlag an der Dosiermutter(264) zuläßt. Die Hilfsflüssigkeit wird über das Schieberventil hinter den Trennkolben(276) gebracht und verdrängt mit diesem auch die darunter befindliche Arznei, welche sich über die Kanüle ins Gewebe entleert. Die konische Verengung des Stufenspritzenzylinders ist so gewählt, daß er den Wandabrieb des Wachsmantels (276) ausgleicht, ohne daß es zu einem Flüssigkeitsübertritt kommt. Wegen der höheren Viskosität der Hilfsflüssigkeit würde in Störfällen eher Arznei nach oben als Hilfsflüssigkeit nach unten in die Arznei verschoben. Das Bewegungsspiel der Dosierspindeln auf ihrer Verbindungslasche(476) wurde bei der Dosierstrecke mit einkalkuliert. Mit der Anhebung der Dosierspindeln wird auch das Schieberventil angehoben und dessen Querstift gerät über die kurze senkrechte Strecke im Schrägschlitz(267) hinaus. Die weitere Anhebung des Schieberventilestößels und die Betätigung des Belüftungsventiles(324) erlaubt die Schiebekoppelung(269). Sie wird durch die Rechtsbewegung des Deckelschlittens unter Klappenöffnung bewirkt. Will der Patient nach Ablesen des Meßergebnisses noch eine Dosiskorrektur durch Drehung an der Dosierschraube während des Hautkontaktes vornehmen, so hält er die beiden Klappen solange zusammengedrückt, bis er die Wiederbelüftung wünscht.

Die Figur 29 zeigt im Längsschnitt einen elektrisch betriebenen Injektor mit Stufenspritzen für zwei verschiedene Arzneien. Die beiden Stufenspritzenzylinder (272) sind in Behälterzylinder(820) eingeschoben. Über der Arzneiflüssigkeit liegen die Trennkolben (275), über diesen innerhalb von Schutzfaltenbälgen (854) und an diesen drehgesichert befestigt die Gewindespindeln(856). Höhenverschoben werden letztere durch die Innengewinde der kleinen Getriebezahnräder(839), welche durch die großen Zahnräder(859) über den Elektromotoren(855) angetrieben werden. Die Deckelplatte (819) hält die Getrieberäder auf dem Gehäuse fest. Während diese Motoren hinter der Schnittebene liegen, setzen sich die Stufenspritzen mit je eigenen Kanülenansatzstutzen in die Saugglocke(173) fort. Um deren Ränder sind die drei Auslösestifte(853) angeordnet, deren Druckkontakte geschlossen sein müssen, um den Kompressor(830) einzuschalten, welcher hinter der Saugglocke angeordnet ist. Die Kontaktstangen(858) für die Kanülen werden durch Aufstecken derselben auf den jeweiligen Kanülenansatzstutzen betätigt. Der zentrale Sensorstift(829) vermittelt durch Kontaktschluß mit der Haut deren Anhebung, was den Einspritzvorgang einleitet. Es genügt im allgemeinen hier eine Art Erdschlußkontakt zu wählen, ohne zusätzlichen Kraftaufwand für eine Schalterbetätigung. Zu beiden Seiten des Schaltgehäuses(839) mit der Steuereinheit sind die Schalträder(832) für die Dosiervorwahl angeordnet.

Die Figur 30 zeigtin einer Draufsicht den injektor der Figur 29. Zusätzlich zu den Getriebezahnrädern ist die Befestigungsschraube und Halterung(878) zu erkennen. Desweiteren sieht man den Umschaltknopf (834) zum Motorrücklauf, um die Gewindespindeln vor der Entnahme der Stufenspritze anzuheben, den Programmwechselschalter(864) und den Druckschalter(874) für das Belüftungsventil(875) mit dem Belüftungskanal(870). Die Lage der Batterie(845) ist zusätzlich angegeben.

Figur 31 zeigt eine Schaltskizze in TTL zu Fig.20 und 30. Abschnitt(A) bezeichnet die Energieversorgung, (B) die Wahl des Dosierprogrammes, (C) die Prüfung der Startvoraussetzungen, (D) Programmierung der Dosierung, (E) die Wiederherstellung der Ausgangsbedingungen. Durch Aufstecken einer Kanüle wird eine der Kontaktstangen(858) betätigt und der zugehörige Kontakt geschlossen und in Teil C mittels der beiden AND-Gatter(1) TTL SN 7408 und des EXOR-Gatter TTL SN 7486 auf Übereinstimmung hin verglichen. Die Leitung führt von dort zum AND-Gatter(2) TTL SN 7408, welches den Stromkreislauf zum Kompressor(830) unterbricht. Wird das AND-Gatter am Saugzylinder dreifach geschlossen (durch Aufsetzen auf die Haut), so schaltet das Flipflop TTL SN 7474 über den Transi-

- 68 -

stor(1) den umgekehrt,d.h. saugend, laufenden Kompressor(830) ein. Es ensteht jetzt Unterdruck über ein Sogrohr in der Saugglocke, die Haut wird in diese gehoben und betätigt ihrerseits die Kontaktstange(829) und damit das NAND-Gatter TTL SN 7400 von Stellung low nach high. In Teil D zur Schrittmototauswahl wird über die NOR-Gatter TTL NN 7402 je nach Stellung des bistabilen Relais über Transistor(2) oder(2) der Schrittmotor zu Dosierspindel 856 oder 856´von low nach high gebracht, wenn bistabiles Relais und Kompressor eingeschaltet sind. Der Nockenkontakt(856,856) - auf Fig.29 zu erkennen- wird bei jeder Umdrehung des Dosierrades (859, 859) betätigt und führt in den Dezimal-Zähler TTL SN 7490. Der Vergleich des Zählers mit dem Vorwahlschalter erfolgt mit EXOR-Gatter TTL SN 7486 mit jeweils zugeordneten Dioden IN 4148 bis an Punkt E der Stromfluß von high auf low geschaltet wird. Von low bei E wird über AND- Gatter(3) der Zählerauf Nullstellung gesetzt. Über AND-Gatter(4) wird der Kompressor abgeschaltet, über AND-Gatter(5) der Flipflop in Ruhestellung gekippt. Über Transistor(3) wird mit Hilfe des Einschalt-Resets sichergestellt, daß das bistabile Relais nicht durch Ein- und Ausschalten des Gerätes an Taste(874) seine Schaltstellung wechselt. Über Monoflop TTL SN 74121 wird für die hier vorbestimmte Dauer mittels Transistor(4) das Magnet-Belüftungsventil(368) betätigt, wobei Luft in den Saugzylinder eintritt. Zur Wiederbelüftung kann auch über BEtätigung des Druckschalters(874) über manuelle Öffnung eines Sitzventiles der Lufteintritt in die Saugglocke über den Belüftungskanal(870) freigegeben werden.
Die Figur 32 zeigt·im Längsschnitt eine Variante zu zu Fig.29. Der Aufbau der Vorrichtung versteht sich folgendermaßen:
Zwischen der Abschlußplatte(2) und der Zwischenwand (884) des Gehäuses(1) finden sich die Vierkantwellen (885,886) in ihren Lagerbuchsen(887,888,889,890). Die Vierkantwellen sind durch Klemmringe gegen ein Herausfallen gesichert; die Welle(885) ist mit der Antriebswelle des Elektromotors(855) verbunden und diese wiederum mit dem Kompressor(830). Das erste große Getrieberad(893) sitzt auf der Vierkantwelle(885) und wird von dieser mitgenommen, die übrigen Getrieberäder laufen auf den Lagerbuchsen bis auf das letzte große Rad(815), das auf der Vierkantwelle(886) befestigt ist und nun verlangsamt die Drehbewegung an die Dosierscheiben(816, ,325) weitergibt. Dies geschieht über ein auf der Vierkantwelle(886) verschiebliches Schaltritzel(800), welches durch die Lagerbuchse(890), welche zugleich als Stößelhülse unter Fixierung durch eine in eine Ringnut einrastende Klinke(801) hier in gezogener Lage im Eingriff mit der innen verzahnten Buchse(802) steht. Diese Buchse steht in fester Verbindung mit der Dosierscheibe(816), deren über dem Arzneischlauch und dessen Scheibenlager mittels seiner Rollen schlauchklemmend einwirkt und die Arznei zum zugehörigen Kanülenansatzstutzen verdrängen kann. Wird der Stößel der Achsenbuche (890) gedrückt, so verschiebt sich das Schaltritzel nach rechts und kommt mit der Innenverzahnung der Schaltbuchse(820) in Eingriff, welche fest mit der Dosierscheibe(325) verbunden ist. Es wird jetzt aus dem anderen Vorratsgefäß über den Schlauch(44) dosiert. Nach Zug am Starthebel(803) wird der Fliehkraftregler, dessen gewichtsbelastete Schwenkflügel(804) von der Strebe der Zwischenwand(884) zurückgezogen werden, unter Vermittlung der Vierkantwelle vom Motor angetrieben. Hierzu wird mit der Betätigung des Starthebels zugleich der elektrische Startkontakt(197) aktiviert. Die mit den Flügelarmen auf der Vierkantwelle verschiebliche topfartig ausgehöhlte Buchse, welche den Befestigungskopf der Flügelarme umfaßt, wird dabei gegen ihre verstellbare Feder nach links verschoben. Dieser Federmechanis-

- 70 -

mus bewirkt bei Nachlassen der Umdrehungszahl des Motors durch Abschaltung desselben in kurzer Zeit eine Sperrung der Rotation der Flügelarme an der Gehäusestrebe. Die Dosierung erfolgt unter Kontrollzählung über die Schaltnocke(856) und über die elektronische Steuereinheit(175). Das gedichtete Gehäuse ist über den Sogkanal(809) mit der Saugglocke verbunden und weist die Belüftungsdüse(869) auf. Starthebel(803) und Stößelhülse(890) sind durch Faltenbälge zum Gehäuse gedichtet. Gefederte Kontaktstifte(858) befinden sich neben den beiden Kanülenansatzstutzen. Der Sensor(829) ist zwischen den Kanülenansatzstutzen angeordnet und wird durch die vom Vakuum hochgezogene Haut betätigt. Drei Auslösestifte(853) finden sich in der Nähe des Saugglockenrandes zum Start des Motors. Die Programmwahl erfolgt über Kontakte, welche mit den Endstellungen der Stößelhülse(890) aktiviert werden. Die Energieversorgung ist nicht dargestellt.

Figur 33 zeigt hierzu eine vereinfachte elektronische Schaltskizze. An den mit 5 V bezeichneten Kontaktstellen soll die angegebene Spannung, an den mit R bezeichneten die Spannung des Einschalt-Reset liegen. Die Verdrahtungen sind der Übersichtlichkeit wegen ebenso weggelassen wie die Dioden gegen die Sperrströme. Die Handbetätigung des Einschalt-Reset, nach Einstellung der Programmvorwahlschalter für die Dosierung, bewirkt, daß auf einem Kondensator langsam die Spannung 5 V aufgebaut wird, um die Schaltung in die Nullstellung zu bringen. Das bistabile Relais bie B steht auf dem Programm I oder II und kann über die Programmwechseltaste manuell umgestellt werden. Der Druckkontakt(858 oder 858) wird durch Aufsetzen der Kanüle auf den Kanülenansatzkonus geschlossen und in Teil C mittels der beiden AND-Gatter(1) TTL SN 7408 und des EXOR-Gatter TTL SN 7486 auf Übereinstimmung hin verglichen. Die Leitung führt von dort zum AND-Gatter(2)TTL SN 7408, welche den Stromkreislauf zum Kompressor(830) unterbricht. Wird das AND-Gatter am Saugzylinder dreifach geschlossen(durch Aufsetzen auf die Haut), so schaltet das Flipflop TTl SN 7474 über den Transistor T(1) den umgekehrt, d.h. saugend, laufenden Kompressor ein. Es entsteht jetzt Unterdruck über Schlauch(809) in der Saugglocke(173); die Haut wird in dieser gehoben und berührt den Sensorstift(829) und betätigt damit das NAND-Gatter TTL SN 7400 von Stellung low nach high. In Teil D zur Dosierprogrammauswahl wird über die NOR-Gatter TTL NN 7402 je nach Stellung des bistabilen Relais über Transistor(2 oder 2) der Motor für die Dosierung von low nach high gebracht, wenn bistabiles Relais und Kompressor eingeschaltet sind. Der Nockenkontakt(856) wird bei jeder Umdrehung des Zahnrades(815) betätigt und führt in den Dezimal-Zähler TTL SN 7490. Der Vergleich des Zählers mit dem Vorwahlschalter erfolgt mit EXOR-Gatter TTL SN 7486 mit jeweils zugeordneten Dioden IN 4148 bis an Punkt E der Stromfluß von high auf low geschaltet wird. Von low bei E wird über AND-Gatter(3) der Zähler auf Nullstellung gesetzt. Über AND-Gatter(4) wird der Kompressor abgeschaltet, über AND-Gatter (5) das Flipflop in Ruhestellung gekippt. Über Transistor (3) wird mit Hilfe des Einschalt-Resets sichergestellt, daß das bistabile Relais nicht durch Ein- und Ausschalten des Gerätes an Taste 821 seine Schaltstellung wechselt.

Figur 34 zeigt im seitlichen Querschnitt eine Arzneidruckflasche, deren obere Wandbegrenzung so schüsselartig in den Faltenbalg(831) eingezogen ist, daß in die Schüsselnische(822) der Flaschenhals samt dem um den Schlauchansatznippel(838) gewickelten Schlauch(44) Platz findet, was die Stapelung solcher Flaschen erlaubt. Ein Klammerverschluß(823) verhindert den Arzneiaustritt in den Schlauch unter Gasdruck im Faltenbalg.

- 72 -

Figur 35 zeigt in einem seitlichen Längsschnitt eine Variation des Containers bestehend aus einer weniger elastischen äußeren Hülle(827) welche mit Druckgas gefüllt ist. In dessem Innerem findet sich ein weiterer membranöser Behälter(408) mit Flüssigkeit, welcher sich lediglich dann durch den Schlauch(44) entleert, wenn der Klammerverschluß(823) entfernt ist. Der Schlauch ist von einer Befestigungsplatte(838) zu dieser gedichtet zur Einbringung in einen Injektor umgeben.

Die Figur 36 gibt eine schematische Draufsicht auf einen Sauginjektor als bevorzugte Ausgestaltung der Erfindung. Im Kanülenmagazin(262), welches als spiralig gewundener Schlauch ausgebildet ist, ist die Lagerung von Stapelkanülen zweier verschiedner Konstruktionen im Längsschnitt dargestellt.(Die genannten Kanülen, welche einen automatischen Kanülenwechsel erlauben, werden in den Fig.48,49 näher beschrieben). Die Dosierung ist für zwei Arzneien vorgesehen, programmiert und in Abhängigkeit von den Meßergebnissen korrigierbar. Es werden die Schnittebenen für die folgenden Figuren angegeben. Dargestellt sind der Zylinder(304) für die Aufnahme des Saugkolbens, der Einschubzylinder (334) mit den Dosiervorrichtungen und Arzneivorratsbehältern, das Scharniergelenk(322) für den Haltearm des Saugglockendeckels, das zentrale Halterohr(314) für das Kanülenmagazin, der Führungszylinder(313), das Rohr(319) für die Druckfeder mit dem den Saugkolben rückführenden Bowdenkabel, das Schutzröhrchen(320) für das spannende Ende für jene Druckfeder und das Leitrohr(321) für den starken Bowdenzug zur Spannung der Zugfeder des Saugkolbens. Die Banderole(400) entspricht einem Magazinende vor der Benutzung. Sie hält mittels des durch die Wandung des Schlauches gesteckten Stiftes(403) die Kanülen, welche unter der Wirkung der gespannten Druckfeder mit den Dichtungskörpern (370) an ihren Enden stehen im Magazin zurück.

0221005

- 73 -

Die Figur 37 gibt im Längschnitt in Schnittrichtung A - B der Fig.36 den Sauginjektor bei schon geöffnetem Saugglockendeckel vor dem Gebrauch wieder. Der Saugzylinder(301) mit dem dichtend eingeschliffenen Saugkolben(302) in seinem Inneren wird an seinem unteren Rand vom Bodenzylinder(303), an seinem oberen Rand vom Zylinder(304) für den Spannkolben(405) gehalten. Die zwischen Saug-und Spannkolbensich erstreckende und an jenen befestigte Zugfeder(306) befindet sich im gespannten Zustand. Der Saugkolben steht in Nachbarschaft des Ringkolbens(307), welcher entlang dem auf dem Bodendeckel des Bodenzylinders feststehenden Zentralstift(308) etwas verschieblich ist und dessen konzentrisch angeordnete stiftförmig fest eingelassene Dauermagnete(309) auf einer ringförmigen Sockelleiste auf dem Bodenzylinder mit einem dort befestigten Eisenring(310) in Kontakt stehen. Die oberen Enden der Dauermagnete(309) sind in einen Weicheisenring(311) eingelassen, welcher gedichtet an einem elastisch-membranösem Septum(312) befestigt ist. Dieses Septum teilt den Zylinderraum luftdicht in einen größeren oberen und kleineren unteren Teil und wird durch das verschiebliche Rückschlagventil(323) unterbrochen, in dessen Zuleitungsbohrung das Sandfilter(393) eingeschoben ist. Auch um den Zentralsstiftist das Septum mittels Verschraubung gedichtet ist.Der Zentralstift weist an seinem Ende eine spindelförmige Auftreibung auf mit flachem Kurvenanstieg oben und steilerem unten. Die mit ihrer Kraftwirrichtung nach innen wirkende Biegefeder(325) liegt eben noch dem unteren Kurvenanteil an, und der Rasterwinkel(327) ist durch seine Druckfeder in einem Schlitz der Hülse(369), die am Saugkolben befestigt ist, eingerastet. Die zentralen Teleskoprohre(328) zwischen Saug-und Spannkolben sind auseinandergezogen. Der zentrale Sperrstößel(329) ist nach Streckung seiner Feder herabgezogen. Dadurch können die Sperrglieder(330) sich unter Wirkung der Zugfeder(306) nicht aus der Nischenschräge des Dachaufsatzes(331) zurückziehen. Die Abwärtsbewegung des Spannkolbens ist also gesperrt. Der Stößel des Belüftungsventiles(324) mit dem Sandfilter (393) ist vom Ringkolben her druckbeaufschlagt. Die Saugglocke(351) ist innerhalb des Führungszylinders(313) gegen drei Druckfedern(354) beweglich angeordnet, wobei die Bewegung nach unten durch die Rasterfeder(353) für die Kanülensteuerung mittels der Schaltnoppe(355) des Schaltstiftes(356) begrenzt wird. Zwischen dem unteren Rand der Saugglocke und des Führungszylinders spannt sich eine elastische Dichtmanschette(352). Neben den drei Bohrungen für die Aufnahme der Druckfedern(354), denen die Verriegelung (357) des Saugglockendeckels(342) und die Druckfeder(335) unter dem feststehenden Gehäuse(315,Fig.39) entgegenwirken, enthält die Saugglocke noch den nach unten trichterförmig sich erweiternden Durchlass(359) für den Schaltstift, wobei dessen Führung bei gewisser Elastizität des Haltearmes(341) für den Saugglockendeckel die senkrechte Annäherung des Aufnahmeröhrchens(358) für den Kanülenwechsel erlaubt. Eine auf diesem Aufnahmeröhrchen gegen eine Druckfeder verschiebliche kleine Röhre(459) weist an ihrem unteren Ende durch Schlitze des Aufnahmeröhrchens auf die Kanüle schiebend einwirkende Klauen(361) auf. In Gegenrichtung zu den beschriebenen Klauen gehen von den Enden des Aufnahmeröhrchens selbst Federklauen(360) aus, welche nach Zurückschieben der gefederten Röhre(459) durch Andruck gegen das zentrale Halterohr(314) während des Deckelschlusses in die Ringnut der zuletzt gebrauchten Kanüle einrasten, nachdem diese infolge der momentweisen Rückholung der Rasterfeder(353) durch den Schaltstift(356) unter dem Einfluß der Druckfeder(Fig.36) im Kanülenmagazin tiefergetreten war. Die Absenkung der Röhre(459) beim Deckelschluß bewirkt auch, daß die unten im Aufnahmeröhrchen steckenden Kanülen von den Klauen(361) nach unten weitergeschoben werden. Bei der Öffnung des Saugglockendeckels passiert die Schaltnoppe(355) wiederum die Rasterfeder(353), wodurch die nächste Kanüle im Kanülenmagazin tiefer treten kann. Diese liegt dann mit der Unterkante ihres Körpers der Rastfeder auf, während die Ringnut(381), welche zum Kanülenschaft in Hohlraumverbindung steht, über die Bohrung im Kanülenmagazin für die Abwinkelung(340) der Arzneizufuhrröhrchen zu liegen kommt. Die zuvor gebrauchte Kanüle wird mit der Deckelöffnung in dem Aufnahmeröhrchen liegend entfernt, wo bei die Federklauen(360) ein Herausfallen verhindern. Unter dem Saugglockendeckel(342) ist am Haltearm(341) die Bajonettkuppelung(362) zu erkennen.

Im Querschnitt über der Saugglocke ist neben der Lage von Kanülenmagazin(316) im zentralen Halterohr(314), die drei Druckfedern(354), der Durchlaß(359) für den Schaltstift zu erkennen. In beiden Schlitzen(363) die beiden Rohre mit der Abwinkelung(340) zur Arzneizuführung und im zentralen Halterohr die beiden elektrischen Leitungen(364) von den Schleifkontakten an der Kanüle zur Steuereinheit.

Im Querschnitt rechts ist die Lage und Funktion der Biegefeder(325) unter der kolbigen Endauftreibung des Zentralstiftes(308) erläutert.

Die Figur 38 gibt im schematischen Längschnitt längs der Schnittlinie C - D der Fig.36 eine Ergänzung des Geräteaufbaues. Im Zylinder(304) für den Spannkolben (305) ist dieser unter Einfluß Der Zugfeder(306) tiefergetreten. Der Saugkolben(302) im Saugzylinder(301) liegt wiederum unter Einrastung der Biegefeder am Zentralstift(308) und unter Wirkung der Dauermagnete unten über Septum mit Rückschlagventil(323) und Ringkolben(307). Unten im Einschubzylinder(334) liegen die beiden Dosiervorrichtungen(337,338), wobei eines der beiden elastischen Rohre mit der Abwinkelung(340) zu erkennen ist. Der Einschubzylinder wird von der Schiene(336) an einem Ring um den Saugzylinder gehalten und durch die starke Druckfeder(335) unter dem feststehenden Gehäuse(315) mit der Steuereinheit und elektrischen Batterie nach unten gepreßt. Die beiden Arzneivorratsbehälter liegen innerhalb des Einschubes. und damit auch gegen den Saugglockendeckel(339).

Figur 39 zeigt eine schematische Draufsicht auf eben diesen Saugglockendeckel(339). Um den Einschubzylinder ist darüber eine Windung des Schlauches des Kanülenmagazins(316) zu sehen. Die Rohre mit der Abwinkelung(340) treten durch die Dichtmanschetten(370) neben dem zentralen Halterohr(314) in die Saugglocke ein. Als Verriegelung(357) für das Saugglockendach ist ein Schieber eingezeichnet.

Figur 40 zeigt in einem schematischen Längschnitt längs der Schnittlinie E - F auf Fig.36 die Lage und Funktion der Teile für die Vorbereitung der Saugpumpenfunktion über die Schwenkung des Saugglockendeckels mittels des Haltearmes(341). Dieser wurde -unmittelbar vor der Injektion- aus der (gestrichelt dargestellten) Stellung mit Saugglockendeckelschluß um 180 Grad um das Scharniergelenk(322) zur Abdeckelung der Saugglocke in Fortsetzung des in Fig.37 wiedergegebenen Funktionsstadiums geschwenkt. Dabei war durch Zugwirkung des Bowdenzuges mit der Ummantelung(344), welcher nach Umbiegung über eine Schleife im Schutzröhrchen(320) weitergeleitet wird und schließlich am Ende des Hebelarmes(345) mit dem Scharniergelenk verbunden ist, die Druckfeder(332) im Zylinderrohr(319) unter Hebung des unteren Führungskolbens(349) in Spannung gebracht worden, ohne daß letztere ihr Bewegungsmoment über das Bowdenkabel mit der Mantelung(345), welches am zentral durchbohrten oberen Führungskolben(349) befestigt ist, hätte weitergeben können. Der obere Führungskolben war mit der Hebung des Spannkolbens(305) beim Deckelschluß bereits gesenkt worden. Die Spannkolbenhebung erfolgte dabei unter Zug am starken Bowdenkabel im Mantel(346) vom Ende(347) des Hebelarmes aus über das Leitrohr (321) Bei Anhebung des Spannkolbens innerhalb des Dachaufsatzes (also nahe von dessen oberer Endstellung) wurden dabei die Sperrglieder(330) durch Absenkung des Sperrstößels(329) bei Zug von den Teleskoprohren(328) in die nischenartige Ausladung des Dachaufsatzes gedrängt, wodurch die Rückbewegung des Spannkolbens unter Einfluß der Zugfeder(306) verriegelt bleibt(wie in Fig.37 dargestellt). Durch Selbstaufwickelung des saitenartigen Endes(348)in Fortsetzung des starken Bowdenkabels wurde die Öffnungsbewegung des Haltearmes(341) freigegeben.

Figur 41 gibt eine Teildarstellung des Funktionsstadiums nach der Injektion und nach der Spannkolbenrückstellung. Der Saugglockendeckel(342) ist noch weggeklappt. Nach Öffnung des elektromagnetischen Ventiles(324) für die Belüftung des Saugzylinders(301) wurde der Saugkolben(302) unter Wirkung der Zugfeder(306) dem hochstehenden Spannkolben genähert. Nach Entlastung vom Zug der Zugfeder(306) wurde der Sperrstößel(329) unter Wirkung seiner Feder angehoben, so daß die Sperrglieder(330) unter Wirkung der unteren Keilschräge in der Nische des Dachaufsatzes in die Ringkerbe des Sperrstößels auswichen. Jetzt konnte sich die Druckfeder(332) entspannen, indem sie mittels des Bowdenzuges in der Umhüllung(345) den Spannkolben(305), welcher bereits unter dem Einfluß des Saugkolbens und der Zugfeder(306) tiefer getreten war, vollends nach unten drückte. Zuletzt geriet der Saugkolben dabei unter den Einfluß der Dauermagnete(309) und die Biegefeder(325) rastete am Zentralstift(308) ein. Gleichzeitig wurde der Rasterwinkel(327) und damit der Saugkolben gegenüber der Rasterkugel(366) arretiert. Die Senkung des Ringkolbens(Figur 27) hatte die Rasterkugel in seine für diese bestimmte Nische ausweichen lassen. Unter der Wirkung der Druckfedern(354) wurde nun die Saugglocke(353) in ihrem Führungszylinder(313) gegen die
Haut gesenkt. Gleichzeitig öffnete sich auch das Belüftungsventil(324) durch Aufprall des Ringkolbens gegen dessen Ventilstößel und die Saugglocke wurde über
die Bohrung für die Rasterkugel(366) wiederbelüftet.
(Fig.37, bei Tiefstand des Spannkolbens wie in Fig.38).
Die Figur 42 gibt den schematischen Funktionsablauf
für den Saugkolbenspannmechanismus im Stadium des
Schlusses des Saugglockendeckels wieder. Der Spannkolben((305) ist an seinem Bügel(531) über das starke Bowdenkabel im Mantel(346) unter Ausreckung von dessen saitenartigem Ende(348) in seine obere Raststellung beim
Deckelschluß hochgezogen worden. Die Zugfeder(306) wurde dabei gespannt. Der Bowdenzug in der Ummantelung(344)
wurde entspannt und dabei der untere Führungskolben(349)
mit der entspannten Druckfeder abgesenkt, wobei auch der
obere Führungskolben(350) vom Bowdenzug in der Umhüllung(345) im Zylinderrohr(319 nach unten verschoben wurde. Zur Hebelarmverlängerung des Haltearmes
(341) kann für den Schluß des Saugglockendeckels die
ausgezogene Teleskopschiene(343) dienen.
Die Figur 43 gibt im Längsschnitt ein Detail einer Variante des Auslösemechanismus für den Saugkolben wieder, welche es gestattet, das Septum und den beweglichen Ringkolben zu vermeiden. Anstelle der Rasterkugel
(366) tritt ein durch einen Faltenbalg gedichteter
Stößel(406), welcher links mit einer Kerbe des Saugkolbens(305) in Eingriff steht. Die Biegefeder(325)
bremst wiederum am der kolbigen Auftreibung des Zentralstiftes(308) die Aufwärtsbewegung des Saugkolbens
unter Wirkung der Zugfeder(306). Das Belüftungsventil
(324) ist durch die Auflage des Saugkolbens gegen den
gefederten Ventilstößel geöffnet und damit auch die Verbindung der Saugglocke zur Außenluft über den Kanal
(532) über das Sandfilter(594). Die Bohrung für das
Rückschlagventil(323) verläuft parallel zum Stößel(406).
Wird die Saugglocke gehoben, so gerät deren Mulde(365) in den Bereich der Stößelkuppe, die in diese unter der Wirkung der Keilschrägen der Kerbe im sich hebenden Saugkolben eintritt. Nun ist die Saugglocke arretiert wobei der im Saugzylinder sich bildende Unterdruck bei Öffnung des Rückschlagventiles(323) die Luft aus der Saugglocke gefildert über den Luftkanal(533) ansaugt. Das Belüftungsventil(323) hat sich geschlossen und wird erst wieder durch Rückkehr des Saugkolbens unter Belüftung des Saugzylinders über das(nicht eingezeichnete) elektromagnetische Ventil geöffnet, während die Wiederbelüftung über den Luftkanal(533) bis dahin durch das Rückschlagventil(323) verschlossen ist.

Die Figur 44 zeigt schematisch im Längschnitt durch das Detail des Saugglockenbereiches eine Vorrichtung und die Funktion der Nachfüllung eines Vorratsbehälter für Arznei bei einem Gerät nach Fig.37. Das zentrale Halterohr(314) ist in seiner Dichtung(534) im Saugglocckendeckel abwärtsgeschoben. Das betreffende Arzneizufuhrrohr wird an seiner Abknickung(340) aus der Bohrung des zentralen Halterohres gezogen und in eine Bohrung der Steckbuchse(535) eingeführt, während die andere Bohrung der Steckbuchse mit dem Arzzneizufuhrrohr des neuen Vorratsbehälters verbunden wird und zwar über Einstecken des Konus am Ende des Schlauches(44).

Die beiden Dosierpumpen sind auf im Längsschnitt auf Figur 45 abgebildet. Vom Vorratsbeutel(408) führt der Arzneischlauch(44) zu dessen sackartige Erweiterung zwischen dem Pumpenzylinder(111) und den Dosierpumpen- stiften(337,338) und setzt sich in die Röhre mit winkliger Abknickung(340) fort, welche über die Querbohrung im zentralen Halterohr(314) über den Kanülenkörper mit dem Kanülenschaft in Verbindung tritt. Im unten abgebildeten Querschnitt längs der Linie A - B der Fig.45 ist neben den Dosierpumpenstiften(337,338) die Anordnung der Flüssigkeitspumpen(409,410,411,412) zu erkennen.

In Figur 46 erlaubt die Vergrößerung im Maßstab 5 : 1 den Aufbau einer Dosierpumpe nach Fig.44 deutlicher zu erkennen. Auf dem Pumpenstift stellen drei ringförmige Einkerbungen je eine Dosierkammer(416) dar. Der dieser zugeordnete Schlauchring(415) ist in einer rinnenförmigen Ausbuchtung der Achsenbuchse(116) höhenfixiert. Bei Aufblähung des Schlauchringes verdrängt dieser die Flüssigkeit aus der Dosierkammer und ihrer Umgebung und sperrt dann den Flüssigkeitsstrom, wobei er sich den Rillenkanten anlegt. Der Flüssigkeitsstrom zur Kanüle führt über den Kanal(417). Die Flüssigkeitspumpe in Fig.46 stellt eine Variante dar. Von den drei je einem Schlauchring zugeordneten Flüssigkeitspumpen für die Hilfsflüssigkeit ist nur eine dargestellt. Hubmagnet(413) und Rückstellfeder(414) wirken auf den Faltenbalg(418) nach der danebenstehenden Impulsfolge ein, welche bei I zur Injektion führt, während bei II ein Arzneirücklauf bewirkt wird.

In Fig.47 sind zwei Flüssigkeitspumpen für die Betätigung einer Dosierpumpe als doppelt-wirkende Kolben-Zylinder-Pumpen in Verbindung mit Hubmagnet(413) und Rückstellfeder(414) dargestellt, wobei die beiden Zylinderenden über Schlauchverbindungen mit wenigstens einem Schlauchring(415) in Verbindung stehen. Der mittlere Schlauchring ist mit jedem der beiden sich gegenüberliegenden Zylinderenden durch Schlauch verbunden. Fig.47 verdeutlicht auch die Pumpenfunktion. In der Grundstellung A sind beide Kolben unter Federwirkung gesenkt. Der oberste Schlauchring ist von Flüssigkeit entlastet, während der untere und der mittlere Schlauchring durch Flüssigkeitsüberschuß aufgebläht sind. In Stellung B erhielt der oberste Magnet einen Impuls, wodurch der oberste Schlauchring aufgebläht und der mittlere entlastet ist. In Stellung C werden beide Magnete erregt, wodurch der untere Schlauchring entlastet und die beiden anderen aufgebläht werden. Die Impulsfolge --,+-,++ wirkt sich so aus, daß eine bestimmte Arzneiquantität von oben nach unten zur Kanüle hin verdrängt und im übrigen der Flüssigkeitsstrom gesperrt.

Die Figur 48 zeigt in der Vergrößerung 3 : 1 eine Stapelkanüle(318) aus gummielastischem Material wie Perbunan. Für ihren Einsatz sind besondere Dichtungelemente insbesondere um den Arzneieintrittskanal oder im Kanülenmagazin nicht erforderlich. Obere und untere Kammer sind über eine gelenkartig wirkende Taille miteinander verbunden. Die obere Kammer für die Aufnahme des Schaftes der angelagerten Kanüle kann verhältnismäßig eng gestaltet werden, da Biegungen des Magazinschlauches in der Taille ausgeglichen werden. Der versilberte Streifen(382) erstreckt sich von der Bodenfläche zum Kanülenschaft als Leitungsbahn. Auf dem Kanülenschaft ist die Enzymbeschichtung(383) angedeutet. Die Kanülenkörper können vor Gebrauch an ihren Rändern miteinander verklebt sein, was Sterilität der Kanülenschäfte garantiert.

Figur 49 zeigt eine andere Stapelkanüle im Längsschnitt. Der becherförmige Kanülenkörper(317) ist durch eine Trennwand(318) in eine untere Kammer zur Befestigung des Kanülenschaftes (379) in deren Bodenplatte und in eine obere Kammer zur Aufnahme des Kanülenschaftes der nachfolgenden Kanüle geteilt. Die obere Kammer wird durch die im Zentrum durchbohrte Membran(380) abgeschlossen. Innerhalb der unteren Kammer biegt der Kanülenschaft winkelig zu einer den Kanülenkörper umgebenden Ringnut(381) um, in die er sich öffnet. Die Ringnut hat nach unten hin eine Steilfläche, welche den Klauen(361) des Kanülenaufnahmeröhrchens Halt gibt, und geht in sanftem Anstieg nach oben außen, um den Transport der Kanüle nach unten zu erleichtern, wobei ja die Klauen die Ringnut verlassen müssen. Die Strecke der Ringnut bis oberhalb derselben weist einen Streifen mit einer Versilberung auf, während der Kanülenschaft selbst platiniert ist, um ohne Spannungsverzerrungen Schwachströme an die Kontaktfeder im zentralen Halterohr für das Kanülenmagazin weiterzugeben.

Der Kanülenkörper ist aus gußplastischem Material wie beispielsweise Polyvinylchlorid. Die erforderliche Ringdichtung innerhalb des zentralen Halterohres(314, Fig.37) wurde nicht dargestellt. Die Enzymbeschichtung (383) ist eingezeichnet. Die Stromweiterleitung erfolgt über die Silberchlorid-Silber-Schicht der Rastfeder(353) für den Kanülenwechsel, während deren Unterflächeelektrisch isoliert ist, um einen Hautkontakt zu verhindern. Andererseits ist die Verwendung eines Kontaktgeles, wie bei Herzstromableitungen üblich, erforderlich, um von dem versilberten Rand der Saugglocke aus dioe Vergleichselektrode für Messungen zu gewährleisten. Es kann auch eine beidseits klebende ringförmige Klebeelektrode(Fig.94) angewendet werden, welche auf den Saugglockenrand aufgeklebt wird und zur Silberschicht gut leitend ist. Durch eine solche Klebeverbindung kann die Anwendungssicherheit erhöht werden, solange noch erhebliche Reaktionszeiten bis zum Abschluß der Messung erforderlich sind, da die Erhaltung des Unterdruckes in der Saugglocke noch besser garantiert ist.                Die Ringnut(582) in Fig.49 dient der Feststellung durch die Rastfeder(353).

Die Figur 50 zeigt oben im Längsschnitt und unten im Querschnitt im Maßstab 5 : 1 die Variation einer Senkanüle in Schrägansicht. Der Kanülenschaft ist hinter dem Bereich der noch geschlossenen Spitze(541) abgeschliffen, so daß die Kanülenbohrung als Mulde(542) offen wäre. Die Schaftwandung wurde aber durch die Kunststoffeinlage(543) mit Isolierzonen(544) zum Kanülenschaft hin geschlossen.

Figur 51 zeigt im Längschnitt und darunter im Querschnitt eine andere Variation der Sensorkanüle. Innerhalb des Schaftes ist ein fast kapilläres dünnes Schläuchchen(545) oder ein Faden eingelegt, dessen Oberfläche Sensoreigenschaften haben können Wie auch in den anderen kann neben Blut auch Gewebsflüssigkeit durch die kapilläre Wirkung (oder durch Soganwendung vom Arzneizufuhrrohr aus) zur Meßwertermittlung angesaugt werden.

Figur 52 zeigt einen Längsschnitt durch das Schaftende einer Kanüle, bei welcher die Wandung im Spitzenbereich von
innen verdünnt ist und mit dem Sensorbelag(383) beschichtet, so daß letzterer beim Einstich in die Haut nicht abgestreift werden kann. Die Stabilität des Kanülenschaftes
bleibt durch die wandstärkere Partie gewahrt. Im Schaftinneren ist ein Drähtchen(577) zur Stromableitung erkennbar.

Figur 53 zeigt im Längsschnitt eine Sensorkanüle ähnlich
derjenigen der Fig.50. In die offene Mulde(542) des Schaftes ist jedoch ein Schläuchchen(545) oder Faden mit Sensoreigenschaften eingelegt.

Da der Umgang mit den glukosespezifischen Enzymbeschichtungen die augenblicklichen Möglichkeiten des Erfinders
überstiegen wurde als Beschichtung zur Änderung der elektrischen Leitfähigkeit auf der Platiniumkanüle in Verbindung mit dem Meßgerät ein elektrolythaltiges Gel mit zukkerspezifischem Adsorbens als Dotierung vorgesehen(z.B.das
Adduct Sepharose-Concanavalin A der Pharmazia Freiburg-
Breisgau).

Figur 54 gibt eine photometrische Version der Sensorkanüle
beispielsweise für die Glukosebestimmung, wobei die Lichtquelle(560) und der optische Detektor(561) sowie die Kanüle
im Längsschnitt dargestellt sind. Innerhalb des Kanülenansatzstutzens(47) welche den Kanülenschaft(379) umschließt, führt mindestens ein Fiberglasfaden(885) im weiteren Verlauf in der Lichtung des Schaftes(379) liegend
zur Kanülenspitze, welcher unter Krümmung oder Neigung eine Indikatorschicht(886) aufgebracht ist. Mindestens ein
weiterer Fiberglasfaden verläuft von jener Indikatorschicht
durch den Schaft zurück. Um das arzneizuführende Schlauchende mit dem Kanülenansatzkonus liegt der Kontaktring(587),
von welchem Fiberglasbündel zur Lichtquelle mit Polarisationsfilter(583) und andere zum Detektor zurück führen.
Wird die Kanüle ins Gewebe eingeführt, so reagiert die Indikatorschicht mit der Gewebsglukose unter Verfärbung in Abhängigkeit von der Glukosekonzentration. Die Abschwächung der zurückgeleiteten Lichtsignale kann gemäß eines Eichvergleiches zum Meßwert ausgewertet werden. Es können auch je zwei Fiberglasfäden zu zwei qualitativ unterschiedlich beschickten Indikatorschichten und zurück führen, wobei letztere eventuell für unterschiedliche Meßzeitpunkte für verschiedene Konzenzentrationsränge empfindlich sind. Solche optische Indikatorschichten für Glukosebestimmung sind beispielsweise mit Glukoseoxydase und -Peroxydase imprägniertes Papier, welchem als Frabgeber 3,55.5´-Tetramethyl-benzidin-dihydrochorid bzw. 4-Aminoantipyrin-dihydrochlorid und Natrium-3.5-dichlor-2-hydroxy-benzolsulfonat zugesetzt ist.

Die Figur 55 zeigt im Längsschnitt eine Sensorkanüle, bei welcher anstelle eines Farbindikators ein halbmondförmiger Spiegel(588) im Spitzenbereich des Schaftinneren angebracht ist (der Spiegel kann aus aufgedampftem Silber bestehen, welches zum Schutz gegenüber Oxydation mit Siliziumdioxyd überzogen ist). Links ist die an eine Lichtquelle angeschlossene Lichtleitfaser(585) innerhalb des Kanülenansatzstutzens(47) zu erkennen. Der freie Lichtstrahl zum Spiegel ist gestrichelt dargestellt, wo er durch die Gewebsflüssigkeit hindurch von der anderen Spiegelseite zurückgeworfen wird und eine Faser des Lichtleitfaserbündels zum Detektor trifft. Die Drehung der Polarisationsebene durch die Glukose wird·im Rechner ausgewertet.

Figur 56 zeigt eine andere Ausgestaltung der Erfinduchg nach Fig.55. Um die problematische und aufwendige optische Koppelung zwischen den Lichtleitfasern im Kanülenansatztstutzen und denjenigen des Gerätes zu vermeiden, werden die Lichtstrahlen von der Sektorscheibe(589) direkt durch die Mittelbohrung einer Stapelkanüle(317,beispielsweise) in den Kanülenschaft

- 85 -

hineinprojiziert und von dort auch wieder im Geräteteil empfangen und zum Detektor in Lichtleitfasern
(885) weitergeleitet. Die Kanülenbohrung wird nach unten hin durch die Fenstermembran(590) abgeschlossen,
welche die Lichtstrahlen durchläßt, nicht aber die
Arznei. Letztere wird seitlich von der Ringnut(381)
her aus der Arzneiauslaßöffnung(440) durch das Halterohr(314) hindurch eingespritzt. Nach der Injektion
wird durch Senkung der Sektorscheibe(589) deren Haft-
Zentrierkonus(594) aus der Mittelbohrung gezogen und
durch Schwenkung um die(nur angedeutete) Achse nach
Art eines Transportkarussells(211) der Durchtritt
der nächsten Kanüle zur Arzneiauslaßöffnung hin
freigegeben. Der Spiegel(588) sei wie derjenige der
Fig.55 aus Silber oder Aluminium aufgedampft.

Figur 57 zeigt in einer Seitenansicht und darunter
in einem Querschnitt einen Teil der Schaltringe für
die elektronische Programmierung eines Injektors
wie er in Fig.36-44 beschrieben wurde. Auf der Schalterleiste(371) des Schaltgehäusezylinders befinden
sich je zwei benachbarte Druckkontakte für einen
Schaltring(372), welche bei wellenförmiger Gestaltung der Innenbegrenzung der Schaltringe nacheinander
nach jeder Ausnehmung betätigt werden und zwar je
nach Drehrichtung in der entgegengesetzten Reihenfolge, wobei der Kugelraster(373) die Verstellung
nur in ganzen Schaltschritten garantiert. Bei binärer Kodierung können jetzt die einzelnen Parameter,
wie Gewebszuckerwerte oder Insulinmengen zur Dosierung in ihrem jeweiligen Soll- oder Istwerten
erkannt, dargestellt und rechnerisch ausgewertet
werden. Für die Praxis erscheinen bereits 20 Schaltschritte als ausreichend. Bei der Zeit die Stundenabstände mit Pause zwischen 0 und 5 Uhr (wobei eine
Tag-Nachtumkehr durch Umstellung des Zeitgebers ausgeglichen werden kann), bei den Gewebszuckerspiegeln in mg/% 20,30,40...bis 100, dann 120,140,160... bis 300, dann 350 und 400, bei den Insulinmengen in Einheiten 4,8,12...80 sowie bei den Multiplikationskonstanten (K1 für Depotinsulin und K2 für Altinsulin) die Schaltstufen 0,1,2,3...bis 19. Als Bezugslinie für die Ablesung dienen Randvorsprünge an den auf dem Schaltzylinder feststehenden Zwischenringen(374) sowie Drahtbügel(375) oder an dessen Stelle eine durchsichtige Kunststoffleiste mit optischer Vergrößerungswirkung, welche aus dem Ablesebereich weggedreht werden kann, damit die erhaben gestalteten Zahlen zur Programmarchivierung abgedruckt werden können. Vorspringende Kanten(376) erleichtern das Drehen der Schaltringe und verhindern ein Überdrehen in einer Richtung. Benachbarte Schaltringe, welche funktionszugeordnet sind, können je einen Stift(377) mit hakenartiger Abbiegung aufweisen, welcher gegen die Kante(376) anstößt und so eine automatische Mitnahme der Schaltringe untereinander bewirkt. Es empfehlen sich 4 Programmierungsblöcke (I-IV) jeweils gleicher Zusammensetzung für vier vorprogrammierbare Verabreichungszeitpunkte mit jeweils einer nach Stunden vorwählbaren Vor- und Nachfrist sowie ein nach vorprogrammierbarem Mindestabstand nach Injektionen beliebig oft verfügbarer Ergänzungsblock(V) allein für die zusätzliche Verabreichung von schnell wirksamem Altinsulin vor allem zum Ausgleich von Diät- oder Einstellungsfehlern, wobei es Aufgabe des Arztes bleibt, die Depotinsulindosierung in vernünftiger Weise anzugleichen. Tabelle 1 zeigt eine Zusammenstellung beispielsweiser Programmierungen und deren Auswirkungen auf die Insulinbehandlung. Es handele sich um Patienten mit morgendlichen Insulingaben in den niedrigsten bis zu den höchsten Dosierungen entsprechend einem sehr unterschiedlichen Ansprechen auf diese Therapie. Über den Zeitplan sind hier keine Feststellungen getroffen, die Toleranzbreite der Sollzuckerspiegel wurde mit 90 - 120 mg% bei einem Idealwert von 120mg% festgesetzt. Auch die gemessenen Ist-Werte für Glukose sind mit einmal 180 mg%, ein andermal mit 50 mg% vereinfacht. Lediglich der Korrekturfaktor K2 für Depotinsulin wurde nach Schätzung variiert und dabei eine Korrektur mit Altinsulin nicht mit einbezogen; die Spalten der Dosiskorrektur, welche vom Rechner besorgt wird, ist zur Verdeutlichung dargestellt. Das Ergebnis der Korrektur, welches im Falle der Zuckerspiegel von 180 mg% in einer Erhöhung, im Falle der Zuckerspiegel von 50 mg% in einer Erniedrigung der ursprünglich vorgesehenen Dosen besteht, ist aus den letzten beiden senkrechten Zahlenreihen ersichtlich. Patient 2 würde theoretisch bei einem Blutzuckerspiegel von 180 mg% 56E Depotinsulin zusätzlich erhalten müssen, was aber wegen der Höchstdosisüberschreitung nicht möglich ist, sondern auf das Ergänzungsprogramm V mit Altinsulin hinführt. Derselbe Patient erhält bei bei einem Zuckerspiegel von 50 mg% überhaupt kein Insulin. Einem durch Unterzuckerung besonders gefährdeten Patienten muß dringend geraten werden, vor den Mahlzeiten zu messen und zu spritzen, um zusätzliche Altinsulinverabreichungen zu vermeiden. Andererseits kann ein anderer in der Absicht, eine reichlichere Mahlzeit zu sich zu nehmen, die Injektion auf eine Zeit nach der Mahlzeit verschieben und sich dadurch zusätzliche Altinsulineinheiten verschaffen.

Wenn auch die Programmierung durch den Arzt vorgenommen werden soll und deshalb eine Sperrung der Drehung der Schaltringe durch den Druck einer mit Schlüssel zu betätigenden Schraube(384) vorgesehen ist, was durch Rippen(385,Fig.57) auf den Planseiten der

- 88 -

der Schaltringe begünstigt wird, so hat der Patient doch eine Reihe von Mitwirkungspflichten und Einflußmöglichkeiten, was auch aus der Schilderung der Funktionsübersicht hervorgeht. Eine solche Funktionsübersicht, aus der jeder geschulte Fachmann unschwer ein elektronisches Blockdiagramm ableiten kann, gibt die Figur 58 wieder.

Unten sind mit Rechtecken und Kreisen die Funktionsorgane, wie Zeitgeber(386), Saugglocke(173) mit Kanüle im Erdschluß durch die mittels Unterdrucks hochgezogene Haut, Erinnerungs- und Warnvorrichtung(387) mit den Funktionen akustisches und optisches Signal und Vibrator gezeigt, um die Bedienung für Behinderte zu erleichtern (alle getrennt an Handschaltern abstellbar), die elektrische Meßvorrichtung mit der Meßwertanzeige und Drucker(389), Meßanzeige und Drukker für abzugebende Depotinsulineinheiten(390) und für Altinsulineinheiten(391) sowie die Dosierpumpen(337,338). In Rhomben finden sich darüber die Einstellbereiche der Schaltringe, in Rhomben und Rechtecken mit unterbrochenen Linien die Meßdaten der Ist-Glukosewerte und deren rechnerische Verarbeitung. Der Zeitmesser gibt die jeweilige Uhrzeit über die Linie(a) zum Vergleich an den Wecker. Ein Rechenelement bestimmt dort, wieviel Zeit vor der Alarmzeit und nachher noch ein Signal von der Meßvorrichtung (388) her in Richtung auf die Pumpen über die Linie(b) wirksam wird. Immer frei ist der Weg der Linie(d) von der Kanüle zur Meßvorrichtung(388) und deren Anzeige und Drucker. Bei Ausfall der Meßvorrichtung hat der Patient über Handschalter(390) in der Linie(c) noch die Möglichkeit eines Normdosisabrufes. Die eingestellte Weckzeit wird über Linie(e) der Warnvorrichtung(387) aktiviert. War der vorgesehene Zeitraum für eine Behandlung schon überschritten, so wird das Nachfolgeprogramm angewählt; dies ist auch möglich,

wenn Programm I am Handschalter(391) abgestellt ist.
Ist auch das Programm II von Hand abgestellt, so
erfolgt keine Dosierung. Die Wahlmöglichkeit zwischen zwei Programmen zum gleichen Zeitpunkt erhöht
die Anpassungsfähigkeit zwischen Lebensführung und
Behandlung. Nach der Zeitprogrammierung folgt (von
links nach rechts gelesen) über Linie(g) das Sollwert-Programm für den Gewebszuckerspiegel mit dem
gewünschten Idealwert in der Mitte und den Grenzwerten darüber und darunter, bis zu deren Erreichung keine Dosiskorrektur erfolgen soll; die gestrichelten
senkrechten Linien mit Pfeilen symbolisieren diese
rechnerische Feststellung der erlaubten Abweichung
im Vergleich mit der Vorprogrammierung. Der vierte
unterste Rhombus bezeichnet den (gefährlichen) äussersten Tiefstwert, von dem über die Linie(h) unter
Registrierung ein Stopp-Signal zu beiden Pumpen geht.
Liegen die Zuckerwerte höher so führt die Linie(i)
weiter in Richtung auf die Dosierprogramme. Bei Erniedrigung des gemessenen Zuckers unter den unkorrigiert zugelassenen Wert wird durch rechnerische Ermittlung der Abweichung vom festgesetzten Idealwert
und Multiplikation mit dem durch Entfernung des unteren Rhombus von dem der Normdosis festgelegten
Faktor K1 die Höhe des Abzuges an Depotinsulineinheiten ermittelt und nach Abzug von der Normdosis
gemäß Linie(k) werden über Linie(l) Anzeige, Registrierung und Dosierung vorgenommen. Über Linie(m)
ist die Altinsulinabgabe dabei abgeschaltet. Bei
gemessenen Zuckerwerten innerhalb und oberhalb der
zugelassenen Toleranz führt die Linie(n) zur Zuschaltung des Programmes für das Altinsulin, wobei
eine etwaig vorprogrammierte Altinsulingabe gemäß
dem rechnerischen Vergleich zwischen überhöhtem
Wert und Soll- oder Idealwert nach Multiplikation
mit dem Faktor K2 über Linie(p) zu Registrierung angeregt wird. Wird dabei die vorprogrammierte Höchstdosis überschritten, so wird über Linie(q) das Ergänzungsprogramm vorgemerkt, das innerhalb der dort programmierten Zeitgrenzen sich über die Warnvorrichtung in Erinnerung bringt und angewendet werden kann. Über die Linie(u) wird das elektromagnetische Ventil bei (368) zur Wiederbelüftung geöffnet. Wiederbelüftung erfolgt auch über Linie(v) nach alleiniger Messung des Gewebszuckerspiegels ohne Insulinabgabe(beispielsweise außerhalb der festgelegten Zeitgrenzen oder nach Willen des Patienten). Über den Druckschalter(392) kann der Patient nach Ansaugung der Haut im Verlauf der Messung den Befehlsablauf in Richtung auf die Injektion unterbrechen. Läßt er nach Kenntnisnahme der Meßwerte den Druckschalter los, so wird (noch etwas verzögert) eingespritzt. Betätigt er aber nach kurzer Zeit den Druckschalter ein zweites Mal, so wird die Injektion endgültig unter Wiederbelüftung abgebrochen. Durch additive Zählung der durch die einzelne Pumpe jeweils abgegebenen Mengen wird der Arzneiverbrauch kontrolliert und ist über die Linien(w,x) abfragbar. Nach vorprogrammiertem Mengenverbrauch erfolgt Registrierung und Alarm über die Linien(r,s). Auch der Kanülenverbrauch wird über einen Zähler nach Kontaktzählung am Kontakt(392) im Bereich der Saugglocke gemäß Betätigung durch den Schaltstift(356) über Linie(t) gemeldet und registriert. Hinsichtlich der Konstruktion der Sensorkanülen stützt sich die Erfindung auf die nachfolgenden Schriften und das in diesen angezogene Schrifttum:

I. Implantable Glucose Sensor, E.Wilkens and M.G.Wilkens: J.Bio.med.Eng.1983,Vol.5,October,309-315.

2. Problems in Adapting a Glucose-Oxidase Electrochemical Sensor Into an Implantabel Glucose-Sensing Device, Daniel R. Thevenot, Diabetes Care,Vol.5 No 3, May-June 1982,184-189.

3. Progress Towards an Implantable Glucose-Sensing Device, David A.Gough,John K.Leypoldt, and John C.Armour: Diabetes Care, Vol.5 No 3, May-June 1982,190-198.

Es ist noch eine elektronische Verzögerung eines erneuten Druckvorganges bei kontinuierlichem Transport des Registrierbandes anzumerken, um die ausgedruckten Daten lesbar zu halten. Vorteilhafter(wegen der Energieersparnis) ist der Nachlauf des Registrierstreifens gemäß der verflossenen Zeit jeweils bei der Benutzung. Es ist auch eine Programmautomatik vorgesehen, um offensichtliche Fehlprogrammierungen von Seiten des Arztes, welche immer wieder gleichlaufende Dosiskorrekturen notwendig machen zu bereinigen. Eine solche Programmautomatik ist auch dann von Vorteil, wenn sich die Lebensumstände des Patienten verändern, ohne daß er ärztliche Hilfe in Anspruch nehmen kann. Auch kurzfristig muß der vernünftige Patient die Möglichkeit haben, innerhalb vom Arzt festgelegter Freiräume die Insulindosen zu verändern.

Mittels der(vom Arzt verschließbaren) Taste(586) kann der Patient impuls- oder taktweise zusätzlich Altinsulin zur Injektion hinzufügen, sofern er dieses in der nächsten Minute nach dem Befehl durchführt. Die absolute Höhe der gesamten Ersatzdosis kann vom Arzt innerhalb eine ohnehin eingeengten Spielraumes begrenzt werden. Für die eine Patientenwahl begrenzenden Befehle ist ein besonderer Schaltring vorgesehen. Beispielsweise bedeutet dessen Stellung 1 = Sperrung der Programmautomatik(siehe unten) für Altinsulin, 2 = Sperrung der Programmautomatik für Depotinsulin,3 = Sperrung der gesamten Programmautomatik, 4 = Begrenzung der Zusatzdosis auf 15 Takte,5 = Begrenzung auf 10 Takte,7 = Sperrung der Zusatzdosen,8 = Begrenzung der Zusatzdosen auf 15 Takte und Sperrung der Programmautomatik für Altinsulin,9 = Begrenzung der Zusatzdosen auf 10 Takte und Sperrung der Programmautomatik für Altinsulin,11 = Sperrung der Zusatzdosen und der Programmautomatik für Altinsulin,12 = Begrenzung der Zusatzdosis auf 15 Takte und Sperrung der Programmautomatik für Depotinsulin,15=Sperrung der Zusatzdosen und der Programmautomatik für Depotinsulin,16 = Begrenzung der Zusatzdosen auf 15 und Sperrung der gesamten Programmautomatik usw. Eine Entschlüsselungsscheibe(595) ist auf dem Gerätedeckel angebracht, wobei dieser zweckmäßigerweise selbst als Schaltring drehbar ist. Unter "Programmautomatik" ist die Einrichtung zu verstehen, welche eine Korrektur von Fehlprogrammierungen für die Dosierungsausgleichungen vollzieht. Wird wegen überhöhter Meßwerte viermal hintereinander im selben Zeitblock eine Korrekturdosis abgerufen, so wird die Depotinsulindosierung für den den Meßzeitpunkten vorausgehenden Zeitpunkt jeweils um 1 Takt erhöht. Erreicht die programmierte Korrekturdosis den eingestellten Höchstwert ohne volle Kompensation(wird also zu einer Zusatzinjektion aus dem Block V aufgefordert), so wird die Depotinsulindosierung des vorhergehenden Zeitblockes um 2 Takte erhöht. Die Erhöhung erfolgt innerhalb eines jeden Blockes nun bei jedem Anlaß nach Messung. Wird bei irgendeiner Benutzung des Gerätes die Untergrenze zur Dosiskorrektur unterschritten, so erfolgt eine Kürzung der Depotinsulindosis für den Zeitblock, welcher dem Meßzeitpunkt vorausgeht, um 1 Takt. Wird der untere Grenzwert für den Dosisstop erreicht, so erfolgt eine Kürzung um 2 Takte, insofern in den abgelaufenen zwei Stunden das Ergänzungsprogramm V nicht betätigt wurde.

Zur Betätigung der Programmautomatik für das Altinsulin ist die Mitwirkung des Patienten erforderlich. Mit Betätigung der Programmtaste(597) wird die Zeitblockzuteilung außer Kraft gesetzt und Block V angewählt, sofern in den letzten 6 Stunden keine Depotinsulininjektion erfolgte. Der Patient soll etwa 15 bis 20 Minuten vor der Tastenbetätigung eine Mahlzeit mit 3 Broteinheiten leicht resorbierbarer Kohlehydraten zu sich nehmen und wird dazu aufgefordert innerhalb von 15 bis 20 Minuten nach der erfolgten Altinsulindosierung, deren Höhe dem vorprogrammierten Korrekturprogramm entspricht, die Kontrollmessung vorzunehmen. Übersteigt der Meß-(Ist-)Wert den Ideal-(Soll-)Wert um mehr als 40mg%, so wird der Dosiskorrekturfaktor(K2) um 1 erhöht. Unterschreitet der Istwert den Sollwert um mehr als 20mg%, so wird der Dosiskorrekturfaktor(K2) um 1 gesenkt. Über die Anzeige wird in allen Korrekturfällen der Patient zu gelegentlicher Wiederholung der Programmtestung aufgefordert. Der Stand der wirklichen Programmeinstellung (in etwaiger Abweichung von der durch die Schaltringstellung ablesbaren Ausgangsposition) kann durch leichte Drehbewegung an den betreffenden Schaltringen auf den zugeordneten Anzeigefeldern sichtbar gemacht werden. Der Arzt kann durch Nachstellung der Schaltringe nach Rückstellung über die Nullstellung(unter gleichzeitiger Betätigung beider Kontaktstifte der Schalterleiste(371) die veränderten Programmwerte auch durch die Schaltringstellung sichtbar machen. Hierzu ist der dem Schaltring(372) jeweils aufgelagerte Randring(316) gegen ersteren drehbar angeordnet, wobei das Ausmaß der Beweglichkeit durch den Sperrstift(275), der vom Randring aus in einen Schlitz auf dem Trägerring reicht, auf eine Strecke unterhalb der Abstände der einzelnen Raststufen des Kugelrasters (373) begrenzt wird. Der Schleifkontakt(226) an einem Wellengipfel des Innenprofils des Schaltringes trifft bei Drehung des Randringes auf die Kontakte(598,599) einer Kontaktleiste auf dem Gehäusezylinder(315) und schließt den Kontakt zur Steuereinheit. Die Rückstellfeder. (600) zwischen Randring und Schaltring unterbricht diesen wieder.

Es muß bei der Aktivierung der Programmautomatik für Altinsulin unterschieden werden, ob die Anpassung des Korrekturfaktors für Programm V an die Stoffwechselverhältnisse beabsichtigt wird( und diese Art der Korrektur war oben dargestellt), oder ob die vorprogrammierten Altinsulinmengen der einzelnen Blöcke geändert werden soll. Die Korrektur der letzteren Zusatzdosierungen von Altinsulin zu den Depotinsulingaben (ausnahmsweise auch die alleinige Altinsulingabe) erfolgt automatisch, wenn der Patient den Injektor 15 bis 20 Minuten nach einer Injektion nochmals anwendet (insofern eine Korrektur nicht gesperrt ist). Beschreibung des Programmablaufs: (Fig.59 - 64) In der Hauptprogramm(HP)schleife wird abgefragt, ob die Kanüle Hautkontakt(1) hat, so daß eine Messung(6) beginnen kann , ob die Weckzeit(2) eines aktiven Zeitblocks erreicht ist, so daß der Benutzer (durch Wecksignal) aufmerksam gemacht werden muß, ob der Benutzer eine Verschiebung(8) der Zeitblöcke wünscht(3),ob(4,5) einer der Vorratsbehälter ersetzt wurde, so daß der Vorratszähler auf den Wert eines neuen Behälters gesetzt (9,10) werden muß.(Fig.59)

Die Bedienung(12) der internen Uhr und der Stellringkontakte(13) ist so zeitkritisch, daß sie bei Bedarf das Hauptprogramm unterbricht.(Fig.60)

Die Fig.61-64 sind eine Verfeinerung des Blocks(6). Nach der Messung(14) wird entschieden(15,16), ob die Programmautomatik(17) für Altinsulin wirksam werden soll. Falls das nicht der Fall ist, die Zeit in keinem Zeitfenster(20) der Programme I - IV liegt und der Benutzer innerhalb der nächsten 15 s (23) nicht die Taste (21) "Programm V" drückt oder zwar Programm V gewählt wurde, aber die Sperrfrist seit der letzten Injektion noch nicht überschritten(22) ist, wird der Vorgang durch Belüften(18,19) abgebrochen.(Fig.61) Ist die Wahl(24) zwischen zwei Programmen möglich, dann wird die Stellung des Programmwahlschalters abgefragt,
wenn nicht am selben Tag schon eine Entscheidung getroffen· wurde. Liegt der Meßwert nicht im Toleranzfenster, so erfolgt eine Korrektur(27,28) der Insulindosen. Bei Meßwertüberschreitung folgen die Funktionsblöcke 27,31 bis 35. Außerdem wird die Programmautomatik für Depotinsulin entweder 29,30 oder 31 bis 35
wirksam,wenn sie nicht vom Arzt gesperrt(29,31) wurde.
(Fig.62). Die Injektion erfolgt nach 15 s(41), falls
der Benutzer nicht den Taster"Warten"betätigt. Zweimaliges Drücken(37) dieser Tast(392,Fig.58)führt zum Abbruch(42,43) ohne Injektion. Mit den Tasten"+" und"-"
kann die Altinsulindosis verändert werden(44,45,46).
Sind die nach der Injektion verbleibenden Vorräte(53)
nicht für die Injektion verbleibenden Vorräte(53)
nicht für eine Injektion mit vollen Dosen ausreichend
(54), so wird der Benutzer aufgefordert, die Differenzmenge in die Vorratsbehälter pumpen(56,57) zu lassen. Wenn 15 s keine Tasten betätigt wurden und noch
Hautkontakt(48) besteht und der Insulinvorrat ausreichend(69) ist, wird injiziert, andernfalls erfolgt Abbruch durch Belüftung(51). Bei versehentlich noch bestehendem Hautkontakt bleibt das Gerät in Wartestellung(52)(Fig.65).
Figur 65 zeigt im Längsschnitt ein mit Federkraft
betriebenes Beispiel, bei welchem eine Schlauch-Rollen-Pumpe als Dosiervorrichtung verwendet wird. An
dem Gehäusezylinder(1) ist über die ihn verschließende Abschlußplatte(2) über die Besfestigungszunge(3)
eine Befestigungsplatte(4) als Gegenlager für ein auswechselbares zylindrisches Unterdruckspeichertöpfchen
(5) angebracht. Letzteres weist einen membranösen
Deckel(6) um den Kanülenlffnungskonus auf,welcher im
Zentrum des Stifte(7) gelegen ist und sich in den Kanülenschaft fortsetzt mit dessen freiem Ende(8), welches von dem Ringkolben(9) umgeben ist, Die Grenzmembran(10) teilt das Töpfchen.

in den Unterdruckspeicherraum(11) und die Saugglocke (12) und weist die Sollbruchrillen(13) auf. An der Befestigungsplatte(4) ist über vier Schraubstifte(14) der Abstützring(15) befestigt. Parallel zu diesem und zur Befestigungsplatte ist auf der Gegenseite des Gehäusezylinders über den Sockel(16) die Grundplatte(17) mittels der Steckverriegelung(18) befestigt, welche Platte den Behälterzylinder(19) trägt mit einem seitlichen Schlitz für die Einführung des Arzneivorratsbeutels(20). Über den Winkelarm(21) greift von der Grundplatte her eine Nocke in die Längsnut(22) der Schiebehülse(23) ,um deren Drehung zu verhindern. In deren Spiralnut(24) bewirkt ein Mitnehmerzapfen der Dosierscheibe(25) deren Drehbewegung. Auf dem Umfang der Dosierscheibe finden sich gleichmäßig verteilt Klemmstifte(26), zwischen denen Federstahlzungen(27) am Auslöserstift(28) arretiert werden. Der Antrieb der Dosierscheibe(25) durch Verschiebung der Schiebehülse überträgt sich über das Rohrsegment(29) auf die Scheibe mit der Rastklinke(30) und auf die Spiralfeder(31). Die Rastklinke(30) wirkt auf das Sperrzahnrad(32) auf der Rohrmanschette(33). Das auf letzterem befestigte Ritzel(34) betätigt das Zählwerk, wobei es in den Zahnkranz des Zählrades(35) eingreift, welches hinter der Bildebene liegt und mittels der Druckfeder(36) gegen eine Stützlamelle(77) am Gehäusezylinder gehalten wird. Gefederte Stifte auf dem Zählrad(38) dessen Druckfeder(39) der Stützwand(37) angelehnt ist, bewirken über Reibungskontakt mit dem Ritzel die Drehung des Zählrades(38), wobei ein Anker auf der Stützlamelle(77) über die Betätigung durch einen Schaltstift auf dem Zählrad(35) nur jeweils die Drehung um einen von 100 Zähnen beim Zählrad(38) zuläßt, nachdem jeweils das Zählrad(35) eine ganze Umdrehung gemacht hat. Nach annäherndem Verbrauch des Arzneivorrates rastet einer der gefederten Stifte, welcher einen anderen Mittelpunktsabstand aufweist, in eine Bohrung des Ritzels ein und sperrt die Drehung auch der Dosierscheibe.(Fig.70). Über die Rohrmanschette(33) wird die Kraft der Spiralfeder(31) auf die Trägerscheiben(40) für die Achsen der beiden Rollen(41) übertragen. Letztere werden durch je zwei in Rohrbuchsen(42) gelagerten Kugeln gegen die Schläuche(44) gepreßt, wobei der ausgekehlte Ring(43) als Gegenlager dient. Letzterer weist unten zum Durchtritt der beiden Schlauchschenkel eine Lücke auf. In diesem Abschnitt sind die Schläuche , bis hin zum T-Stück(46) mit dem Kanülenansatzstutzen(47) von einem dehnungsfesten Mantel(45) überzogen. Zugfedern(48) strecken die Schlauchenden dort, wo sie aus der Pumpe austreten. Das Pumpenaggregat ist längs der Schiebehülse auf der Rohrmanschette(33) gegen die Druckfedern(49) verschieblich. (Die hierzu zweckdienlichen von den Trägerscheiben(40) ausgehen den Stifte in zugehörigen Querschlitzen in der Rohrmanschette sind nicht dargestellt). Die Schiebebewegung wird durch vier Rüttelstifte(50) bewirkt, welche auf ein ringförmiges Prismenprofil auf der Außenseite der Trägerscheibe(40) angeordnet ist(Fig.72). Die Rüttelstifte sind in die Scheibe(51) eingelassen, welche unabhängig von der Rohrmanschette drehbar ist und mit einer Nocke in die Spiralnut der Achswelle(52) eingreift. Am freien Ende der Achswelle befindet sich die Fußplatte(53), am anderen Ende ein Nockenstift(53) ,welcher in die Längsnut der Schiebehülse eingreift und die Achsendrehung verhindert. Dem Antrieb der Scheibe(51) dient die Spiralfeder(55).

Figur 66 zeigt im Querschnitt längs der Linie A - B der Figur 65 den Mechanismus der Dosiereinstellung. Drei Federstahlzungen(27), welche von auf dem Rohrsegment(29) drehbaren Scheiben ausgehen, liegen zwischen Klemmstiften(26).Eine der Federstahlzungen liegt dabei dem Auslöserstift(28) für die Dosierung an,nachdem er die Keilprofilleiste(56) des Auslösemechanismus für die Wiederbelüftung(Fig.68) passiert hat. Zwei der dargestellten Federstahlzungen liegen in einer Art Blindzone des Drehsektors der Dosierscheibe(25), wie er sich aus der Notwendigkeit der Abstützung der Spiralfeder am Gehäusezylinder(1) und aus der Herstellungsmöglichkeit der Nuten in der Schiebehülse(23) ergibt.

Figur 67 zeigt im Querschnitt längs der Linie C - D das Dosiszählwerk, welches den Arzneiverbrauch anzeigt, und die Sperrvorrichtung zur Verhinderung eines Injektorgebrauches nach Arzneivorratserschöpfung. Auf der Rohrmanschette(33) ist das Ritzel(34) zum Antrieb des Zählrades(35) befestigt. Ein Nockenstift(180) auf diesem betätigt nach jeweils einer Umdrehung den gefederten Sperranker(57), welcher das Weiterdrehen des grossen Zählrades(38) um eine Zahnbreite unter Reibungseinfluß der mittels Blattfedern zurückstoßbaren Stifte steuert. Einer dieser Stifte hat einen anderen Abstand zum Zahnradmittelpunkt und wird durch die Druckfeder(39)(39) in eine Bohrung des Ritzels(34) gestoßen, sobald das Zählrad(38) eine ganze Umdrehung vollendet hat, was der Vorratserschöpfung entspricht.

Figur 68 zeigt in einem tangentialen Anschnitt längs der Linie E - F der Fig.65 den Auslösemechanismus für die Wiederbelüftung und für die Dosisabgabe nach dem Rüttelvorgang. Die Federstahlzunge(27) hat das Keilstück der Keilprofilleiste(56) zum dargestellten Zeitpunkt überwunden. Letztere wurde durch die Druckfeder(64) zurückgeführt. Dabei verhindert die Schlitzführung(65) mit den Nutstiften ein seitliches Abgleiten unter Wirkung der Blattfeder(63), welche die Sperrzahnstange(62) in die Sperrzähne des kleinen Zahnrades- dessen Drehung bewirkend- eingreifen läßt. Das Mitdrehen des mit dem kleinen Zahnrad verbundenen grossen Zahnrades(58) bewirkt,daß dessen Sägezähne den Schiebestift(59) dann in eine Nut der Auslösestange eingreifen lassen, wenn letztere(wie gezeigt) angehoben ist.

Rechts von der Fig.68 ist zum Maßstab 2 : 1 vergrössert ein Teil der Scheibe(51), welche die Rüttelstifte trägt im Detail herausgezeichnet, um einen der vier Durchbrüche(67) darzustellen, in welche die Rundkuppe des Auslösestiftes(28) unter dem Einfluß der Schrägstellung der Federstahlzunge ausweichen konnte, um nach der Steilflanke über eine Schrägung wieder auf die Scheibenoberfläche zurückgeführt zu werden. Auf diese Weise wurde die Bewegung der Dosierscheibe bis zum Anschlag der nächsten Federstahlzunge freigegeben.

Figur 69 zeigt in vereinfachtem Querschnitt längs der Schnittlinie G - H der Fig.70 die drei Andruckpuffer (81), welche sowohl durch einen Federstahlbügel(82) zur Wand des Unterdruckspeichertöpfchens hin geführt als auch durch die Druckfeder(83) gegen dessen überstehenden Rand gepreßt wird. Zur Wiederentfernung des derart befestigten Töpfchens dient ein Schwenkhebel (84), welcher einen Keil zwischen Befestigungsplatte (4) und Deckelmembran(6) einzwängt. Letztere wird auf dem teilweisen Längsschnitt deutlicher, welcher unter dem Querschnitt gezeigt wird.

Figur 70 zeigt im Querschnitt längs der Linie J - K , in welchen die Mitte des Unterdruckspeichertöpfchens zu J hin einbezogen ist, den Mechanismus für die Auslösung der Rüttelvorrichtung und den Auslösemechanismus für die Wiederbelüftung des Unterdruckspeichertöpfchens. Der Abstützring(15) ist hier als Variante zu einem nach oben führenden Führungszylinder zur Erleichterung der Einführung der Unterdruckspeichertöpfchen ausgebildet und weist einen seitlichen Schlitz(68) auf. Durch diesen reicht der abgeplattet geformte Ladestock(69) herab. In der Abbildung wird dessen Breitseite gezeigt, wobei die Unterkante der Wandung des Unterdruckspeichertöpfchens(5) aufliegt, während der Tasterwinkel(85) nach hinten sich erstreckt (weshalb er gemäß seiner späteren Stellung gestrichelt dargestellt ist). Über den Durchlaß(70) setzt sich der Ladestock in die Trommel(71) fort. Diese weist die eingesenkte Kulissenführung(72) auf, in welche der Fixierbolzen(73) von der Abschlußplatte (2) hineinragt. Das Lager für die Trommel ist der Klarheit der Zeichnung wegen weggelassen ebenso wie die Lagerung des Rasterkeiles(75) für die Blockierung der Rüttelstifte(50). Auf dem Rasterkeil ist eine um ein Scharnier federnde Kappe(76) aufgesetzt, welche die Rüttelstifte bei Rechtsdrehung der Scheibe zum Spannen der Spiralfeder(55) passieren läßt, nicht aber bei Linksdrehung. Der Rasterkeil weist einen Schrägschlitz(77) zur Aufnahme eines Drahtbügels(78) auf, dessen Vierkantachse innerhalb der Gelenkbohrung (79) eines Schlitzes im federnd gelagerten Haltebolzen (80) drehbar ist. Die Auslösekerbe(86) im Schaft oberhalb der Trommel ist entsprechend der Stellung des Fixierbolzens in der Kulissenführung vom Querstift(87) des Rasterkeiles weggedreht, was bei Anhebung der Trommel sich mit deren Drehung längs der Kulisse noch verstärkt. Wird das Unterdrucktöpfchen ganz nach oben geschoben, so schwenkt der Tasterwinkel um 90 Grad und kommt auf den Rand des Stiftes(7) um den Kanülenschaft zu liegen. Die Absenkung der Trommel wird so verhindert. Der Ladestock dreht ebenfalls und findet nun auf seine Schmalseite gedreht seitlich der Wandung des Töpfchens Platz, um sich an diesem vorbei abzusenken. Der Auslösemechanismus für die Wiederbelüftung besteht aus der Auslösestange(60) mit seiner Druckfeder und dem die Drehung verhinderden Brückensteg(88) zwischen dem in einer Buchse geführten Dorn (89) und der Befestigung unterhalb der Trommel(71). Im Gegensatz zur Darstellung in Fig.68 befindet sich der Schiebestift(59) noch nicht in Raststellung innerhalb der Auslösestange(60).

Figur 71 zeigt im Querschnitt längs der Linie L - M der Fig.65 die Lage des Schlauches(44)zwischen Rollen(41) und gekehltem Ring(43). Ein Schnitt durch die Dosierscheibe(25) in Pfeilrichtung zeigt darunter deren Profilaushöhlung für die Aufnahme der beiden Schläuche.

Figur 72 zeigt in einer Aufrollung den Rüttelmechanismus der Fig.65, wobei vier Rüttelstifte(50) auf der Scheibe(51) mit deren Drehung gegenüber der Keilprofilleiste(56) der Dosierpumpe deren ruckartige Verschiebung gegen die Druckfedern(49) bewirken.

Zur Inbetriebnahme der Vorrichtung nach den Figuren 65 bis 72 wird die Steckverriegelung(18) gezogen. Darnach läßt sich die Achswelle aus der Schiebehülse herausziehen. Dabei wird der Injektor in Hälften zerlegt, deren rechte Abschlußplatte(2),Befestigungsplatte(4) und Schraubstiften(14) samt Abstützring(15) aufweist, und das Pumpenaggregat einschließlich des gekehlten Ringes(43) kann von der Rohrmanschette(33) abgezogen werden. Nach Ausbiegung des Ringes(43) lassen sich die Schläuche in die Profilaushöhlungen der Dosierscheibe einschieben und die austretenden Schlauchenden am Beginn des Mantelschlauches(45) in die Zugfedern(49) einhängen. Der Arzneivorratsbeutel(20) wird über den Schlitz im Behälterzylinder(19) in diesen eingeführt. Letzterer gehört mit seiner Befestigung zur linken Injektorhälfte, welche durch Zusammenstecken der Teile wieder genähert sein muß. (Der nicht dargestellte Arretierstift muß dabei in seinen Längsschlitz in der Rohrmanschette(33) eingeschoben werden). Der Kanülenansatzstutzen(47) wird durch die Bohrung der Befestigungsplatte gesteckt, zuletzt die Steckverriegelung(18) geschlossen. Die Dosierung wird dann durch leichtes Anheben von Federstahlzungen(27) und Drehung in die durch Nummernmarkierung bezeichneten Zwischenräume zwischen je zwei Klemmstifte(26) vorgewählt. Durch Druckausübung gegen die Fußplatte(53) am Ende der Achswelle(52)(beispielsweise gegen eine Tischplatte) bewegt sich die Scheibe mit den Rüttelstiften(50) über die Keilprofilleiste(56), wobei die Kappe(76) federnd ausweichend die Rüttelstifte passieren läßt bis zur Höchstspannung der Spiralfeder(55). Die Bewegungsumkehr wird durch den Rasterkeil(75) gesperrt. Durch Druckausübung gegen die Schiebehülse(23) wird die Spiralfeder(31) gespannt, ohne daß sich diese Bewegung dank der Rastklinke(30) auf die Pumpe überträgt. Die Federstahlzungen schleifen dabei elastisch ausweichend dank der Schrägstellung ihrer Enden über das Ende des Auslöserstiftes(28) und der Keilprofilleiste(56), wobei die Federstahlzunge für die erste Dosierung dann die Keilprofilleiste zurückdrängend vor dem Auslöserstift steht durch letzteren arretiert. Einwärts gegen die Druckfeder(64) kann die Keilprofilleiste nur bewegt werden, nachdem die Auslösestange(60) angehoben ist und deren Einkerbung über die Bewegung des Schiebestiftes die Bewegung des großen Zahnrades(58) freigibt. Dies geschieht nach dem Einschieben eines Druckspeichertöpfchens durch Anhebung des Ladestockes bis zu dessen Drehung um 90 Winkelgrade über die Kulissenführung(72) an der Trommel. Die Einkerbung(86) umgeht dabei überholend den Querstift(87),um zuletzt über diesem auf dem Scha Schaft oberhalb der Trommel zu stehen. Wird zur Anwendung der Abstützring(15) auf die Haut aufgesetzt, so wird bei starker Druckausübung auf den überragenden Ringkolben(9) derselbe gehoben bis die ringförmigen Sollbruchlinien(13) der Grenzmembran (10) brechen, während der Saugglockenrand bereits die Haut um die Saugglocke abschließt. Der vom Speicherraum her wirksame Unterdruck hebt die Haut in die Saugglocke, zugleich aber nähert sich der Stift um den Kanülenschaft (seiner Stütze beraubt) der Haut(Fig.88). Damit wird aber auch der Tasterwinkel(74) gesenkt und die Auslöserkerbe(86), in welche der Querstift(87) ausweichen kann(Fig.70). Dies geschieht unter der Schubwirkung des der Kappe(76) anliegenden Rüttelstiftes. Der unter Spannung des Haltebolzens(80) stehende Drahtbügel(78) dringt daraufhin in den Schrägschlitz (77) ein und bewirkt die Rückkehr des Rasterkeiles(75) in die Sperrstellung vor der Passage des nächsten Rüttelstiftes. Nach Durchmischung des Schlauchinhaltes im Pumpenbereich durch die Rüttelbewegungen tritt der Auslösestift(28) über die Steilflanke des Durchbruches (67) in der Scheibe(51), wodurch die Sperrung der Federstahlzungen(27) kurzfristig aufgehoben und die Einspritzung eingeleitet wird. Nach Überschreiten der Steilflanke an der Keilprofilleiste(56) durch die nächste Federstahlzunge wird eine Sektordrehung des großen Zahnrades unter Wirkung der Druckfeder(64) frei gegeben und infolge des Zurückweichens des Schiebestiftes(59) senkt sich die Auslösestange unter Federwirkung,und der Dorn(89) durchbricht die Deckelmembran des Unterdruckspeichertöpfchens zur Wiederbelüftung desselben. Zur Funktion des oben beschriebenen Drahtbügels(78) ist nachzutragen, daß er während der Anhebung des Haltebolzens (80) in der Endphase der Anhebung des Ladestockes(69) über Kraftschluß an den Auflageplatten für die Druckfedern aus dem seine Bewegung sperrenden Kantschlitz mit seinem dem Drahtbügel parallelen Endarm in die Gelenkbohrung(79) herabgezogen wird und seitlich aus dem Schrägschlitz (77) herausgezogen wird. Durch die nicht besonders dargestellte sich verengende Schlitzführung im Haltebolzen wird die Rückführung des Haltebügels in die Senkrechte bei Druckbelastung gesichert(Fig.70). Nach dem Wechsel des Arzneivorratsbeutels ist die Zählvor-

richtung in die Nullstellung zurückzuversetzen. Hierzu können die Zählräder(35,38) jeweils entgegengesetzt gegen die ihnen zugeordneten Druckfedern vom Zahneingriff befreit und beliebig gedreht werden.

Figur 73 zeigt stark schematisiert im Maßstab 2 : 1 paarweise gegeneinander wirkende Rollen(41), wobei die äußeren Gegenrollen(90) in diesem Beispiel lediglich freidrehend passiv bewegt werden, was durch ihre gummielastische Oberfläche erleichtert wird. Je zwei Rollen sind an einem gemeinsamen Schwenkarm(91) durch Achsen befestigt, wobei der Antrieb über ein auf einem größeren Zahnrad(92) laufendes Ritzel erfolgt.

Figur 74 zeigt in natürlicher Größe eine schematische Aufrollung bei Aufblick auf eine Dosierpumpe(etwa in Pfeilrichtung auf Fig.73). Es wird die Möglichkeit der Schlauchführung bei einem Schlauchpaar unter Verwendung von Rollenpaaren wie in Fig.73 demonstriert und zwar durch seitlichen Abgang der beiden Schlauchschenkel, welche auf der Trägerscheibe(40) dadurch fest aufliegen, daß sich zwischen ihnen eine Art Zahnrad befindet, zwischen dessen Zahnkerbungen sich strickleiterartige Brückenfäden(94) zwischen den beiden Schläuchen(44) ausspannen.

Figur 75 stellt eine Variante des Beispieles der Fig. 65 bis 74 dar, wobei die Abweichung sich auf die Art des Startmechanismus für die Dosierung bezieht. Die schematische Zeichnung gibt den oberen Teil eines Unterdruckspeichertöpfchens wieder mit einem elastisch verschlossenen Fenster(85) in der starren Deckelmembran. Gemäß der Drehstellung des Fixierbolzens in der Kulissenführung der Trommel(71) zeigt der Ladestock (69) mit seiner Breitseite zur Wandung des Töpfchens und hat sich an diesem vorbei gesenkt. Der infolge der Achse(95) gegenüber der Trommel stillstehende Faltenbalg(96) ist am Galgen(97) befestigt gemeinsam und parallel zu dem pneumatischen Zylinder(98), dessen Kolben zugleich den Stößel des Schiebeventiles(99) darstellt. Das zur Außenluft offene Schiebeventil ist über die Leitung(100) und ein offenes Rückschlagventil mit dem Faltenbalg verbunden. Über ein geschlossenes Rückschlagventil führt die Leitung(101) vom Faltenbalg in den pneumatischen Zylinder, welcher über die Leitung(102) mit der hier gerade verschlossenen Öffnung des Schiebeventiles verbunden ist. Die jetzige Position des Schieberventiles folgt aus der Anhebung des Ventilstößes mit der elastischen Membran des Fensters (85). Er wurde angehoben, da durch Druckausgleich während der Hautanhebung in der Saugglocke der die Membran anziehende Unterdruck sich abschwächte. Wird beim Einschub eines neuen Töpfchens der Ladestift gehoben, so wird der Faltenbalg zusammengedrückt, indem die Luft durch die Leitung(101) entweicht und den pneumatischen Kolben betätigt. Dabei wird dessen Stößel in das Fenster(85) gesenkt und über die Leitung(102) der Überdruck in der pneumatischen Pumpe abgelassen. Der Faltenbalg kann solange sich nicht entfalten und die Trommel unter Drehung und Betätigung des Auslösers für die Dosierung(75) senken, bis durch leichte Hebung des Ventilstößels durch die Fenstermembran die Leitung (100) wieder belüftet wird.

Figur 76 zeigt stark schematisiert in den Funktionsstadien A - D eine Variation des Mechanismus zur Wiederbelüftung der Saugglocke, wie er für Vorrichtungen zweckmäßig ist, die wie diejenige in Fig.75 nicht durch die Zerstörung der Deckelmembran eines Töpfchens wieder belüftet werden können. Hier ist das Schiebeventil(99) als ganzes auf einer Schiene(103) gegen die Druckfeder(104) verschieblich. Der Ventilstößel(106) mit Keilprofil zur beispielsweisen Betätigung durch eine Federstahlzunge(27,Fig.65) weist eine gesonderte Druckfeder(105) auf. Der Belüftungsschlauch(107) führt von dem Schiebeventil in den Saugglockenraum, was am einfachsten zu bewerkstellen ist, wenn die Saugglocke ein Teil des Injektors ist (Fig.77).Ein wippenartiger Sperrschieber(108) wird längs einer Kulisse am Ventilstößel betätigt. Im Stadium A steht der Ventilstößel links und das Schiebeventil ist geschlossen und linksständig für den Zustand während der Injektion. Im Stadium B ist das Schieberventil rechtsverschoben und noch geschlossen. Im Stadium C wird das Schieberventil in der Rechtsstellung durch den Sperrschieber noch festgehalten, während der Ventilstößel unter Wirkung seiner Feder nach links verschoben und dadurch geöffnet wird. Während dieser Schiebebewegung des Ventilstößels wird die Rückbewegung des Schieberventiles als Ganzes durch seine Feder(104) durch Betätigung des Sperrschiebers aus gelöst. Im Stadium D ist dadurch der Zustand des Stadiums A wiederhergestellt.

Figur 77 zeigt im Längschnitt einen Injektor, dessen Dosierpumpe aus einer topfartig ausgeformten elastischen Manschette (109) besteht, welche unverschieblich auf einen Stößel(110) montiert ist, welcher an seiner Oberfläche eine bohrerartig gewundene Rillenvertiefung aufweist zum Durchlaß der Arznei. Gelagert ist der Stößel mit seinem unteren Ende innerhalb des Kanülenansatzstutzens(47) mit seinem oberen Ende im Halsabschluß des Pumpengehäuses(111). Oben endet der Stößel in einem für den Arzneidurchtritt durchbohrten Teller(112), welcher den Schlauch (44) an dieser Stelle ausweitet, der die Strecke zwischen dem als Faltenbalg gestalteten Arzneivorratsvorbeutel(20) und dem Halsabschluß des Pumpengehäuses überbrückt. Die Manschette(109) ist mit ihrer Höhlung gegen die mit dem Pumpengehäuse befestigten Ringplatte(113) gerichtet. Letztere setzt sich in einen nach unten konisch verjüngten elastischen und zentral durchbohrten Stopfen (114) fort. Unterhalb des Stopfens trägt der Stößel die ringförmige Ventilplatte(115). Das Pumpengehäuse ist in die zentrale Achsenbuchse (116) eingeschoben, um welche drehbar die Teile für die Betätigung der Pumpe angeordnet sind, und die sich nach außen hin in den Gehäusezylinder(1) fortsetzt. Auf dessen Boden ist auf einem Kugellager das Rad(117) gelagert, mit seiner nach oben gerichteten ringförmigen Keilprofilleiste(56) ,welche nach Funktionsweise und Gestaltung der Rüttelvorrichtung in den Figuren 65 und 72 ähnelt. Anstelle des Stiftes mit einer die Gleitfähigkeit erhöhenden Kugel am Ende werden hier drei über den Kreisumfang gleichmäßig verteilte Rollen (von denen nur eine zur Darstellung kommt) über Achsstifte unterhalb des äußeren unteren Randes des napfkuchenartigen doppelwandigen Zylinders(119) befestigt, dessen Innenwand auf der Achsenbuchse verschieblich, aber nicht drehbar ist. Zwischen dem Randring dieses Doppelzylinders und der an der Achsenbuchse befestigten Ringplatte(120) ist die Druckfeder(121) gelagert. Vom oberen Rand des Doppelzylinders erstrecken sich durch Schlitze in der Achsenbuchse drei auf den Umfang verteilte Winkelstücke bis seitlich oberhalb des Tellers(112), um dort mit Kopfnuten in längliche Verriegelungsschlitze der Randsegmente der Sperrhülse(123) einzugreifen. Letztere überträgt die Stoßbewegungen der Rollen(118) von oben auf den Teller. Auch die Ringplatte(124) ist fest mit der Achsenbuchse verbunden. Auf einem Kugellager schließt sich dieser drehbar das Rad(125) mit einer Keilprofilleiste an, welche nach unten gerichtet ist. Gegen dieses richten sich drei Rollen auf dem gegen die anschließende Innenwandung des Gehäusezylinders in der Senkrechte verschieblichen Außenzylinder(126). Gegen dessen Ringplatte wirkt die Druckfeder(127), welche sich gegen den Boden des Gehäusezylinders(1) abstützt. Das Rad(125) setzt sich aus Teilen verschiedenen Randdurchmessers zusammen. Der obere Teil trägt außen einen Kranz von Sperrzähnen, in welchen die Rastklinke (128) eingreift. Letztere ist an der schmalringigen Scheibe(30) befestigt, welche um die Außenfläche der Ringplatte(124) drehbar ist. Über den Brückenwinkel (129) wird diese Drehung der Spiralfeder(31) mitgeteilt, welche so gespannt werden kann, ohne daß das Rad(125)mitgedreht wird. Nach der Bewegungsumkehr teilt sich die Spiralfederbewegung über die Rastklinke dem Rad(125) und von diesem über den zwischen Rad (125) und Rad(117) befestigten Zylindersegment(130) den beiden Keilprofilleisten mit. Die Pendelbewegung des Außenzylinders(126) überträgt sich über drei in Bohrungen der Achsenbuchse gelagerte U-Bügel(131) von unten auf den Teller(112). Der Behälterzylinder(19) ist mit einem Innengewinde auf die Befestigungsplatte (4) aufgeschraubt und trägt eine Führungsbuchse(132) für die Viereckstange(133). Das an dieser befestigte untere Ritzel(134) greift in den entsprechend gestalteten Zahnkranz des unteren Teiles des Rades(125) ein, während das obere Ritzel (135) auf der Viereckstange verschieblich ist und zwischen den überstehenden Scheiben(136,137) des Deckels(138) in dessen Zahnkranz eingreift. Der Deckel weist ein Außengewinde auf und ist hiermit im Innengewinde des Behälterzylinders(19) verschraubt. Die Bohrung(140) dient der Belüftung des Behälterinnenraumes über dem Faltenbalg. Für den Durchgriff des oberen Ritzels durch den Behälterzylinder ist in diesem ein Schlitz(141) vorgesehen. In Schlitzen des Behälterzylinders längs einer Skala verschiebliche Schraubbolzen(142) erlauben es die Arzneirestmenge, welche einer entsprechenden Dosishöhe entspricht, vor Beginn der Benutzung eines neuen Faltenbalges festzulegen, indem eine weitere Senkung des Deckels(138) durch deren Anschlag auf den Schraubbolzen verhindert wird.

In der Fig.77 entspricht unten die linke Bildhälfte dem Zustand vor, die rechte dem Zustand während der Benutzung des Injektors zur Injektion. Der Abstützring(15) setzt sich in einen Zylinder fort, welcher einen Schiebering(143) zum Aufnahemezylinder(144) für das Unterdruckspeichertöpfchen(5) aufweist. Der am Aufnahmezylinder befestigte Ring(145) dient der Befestigung der gummielastischen Manschette(147), welche den Saugglockenrand trägt. Außerdem enthält er die Führungsschlitze für die Auslöseststifte(148) und dient zur Befestigung des Auslösemechanismus für die Sogeinwirkung auf die Haut. Hierzu sind drei Sperrlamellen(149) in die Einkerbung(150) des Ringkolbens eingeschoben(linke Seite der Fig.77). Durch Drehung der Ringscheibe(151) durch die Anhebung der kantigen und bajonettförmigen Auslösestifte bei Anhebung des Randes der Manschette(147) werden die Sperrlamellen ähnlich wie die Lamellen des Zentralverschlusses einer Kamera aus der Einkerbung des Ringkolbens herausbewegt, was durch eine Abschrägung der Einkerbung begünstigt wird, und der Ringkolben bewegt sich unter dem Einfluß des Unterdruckes zwischen Haut und Unterdruckspeicherraum nach oben bis zum Druckausgleich. Die Grenzmembran(10) wird dabei gereckt. Der Saugglockenrand, welcher durch den Druck von seiten der Federung der Auslösestifte her zunächst gegen die Haut gepreßt wird, wird mit dem Abstützring noch eine weitere Strecke angehoben. Dabei betätigt der obere Rand des Zylinders(151) die Wippe(152) gegen deren Druckfeder, wobei der Stift(153) aus einer unteren Keilprofilleiste des unteren Rades(117) herausgezogen und damit die Drehung desselben und die Dosierung ausgelöst wird.

Figur 78 zeigt den Haltmechanismus für den Abstützring(15) des Unterdruckspeichertöpfchens im Querschnitt. Die Wirkungsweise der Auslösestifte(148) wird im Längsschnitt in Figur 79 verdeutlicht. Der Steuerungsring(154), welcher mittels dreier Kopfschrauben(155) am Ring(146) in Schlitzen drehbar befestigt ist, wird mittels der Druckfedern von den Befestigungspunkten(145) am Ring her in der Endstellung A gehalten. Wie auf der schematischen Aufrollung unter dem Querschnitt ersichtlich stehen die bajonettförmig abgeknickten Auslösestifte(148) dabei mit ihrer Schräge einem Schlitz im Steuerungsring an. Bei Anhebung der Auslösestifte wird der Steuerungsring zunächst im Stadium B nach links verdrängt unter Anspannung der Druckfedern. Während weiterer Anhebung im Stadium C liegt der Steuerungsring den Auslösestiften an, so daß die Rückführungswirkung der Druckfedern auf die Auslösestifte nicht wirksam ist. Die erste Phase der ihrer Rückführung wird durch die Elastizität der Manschette bewirkt, an der sie befestigt sind. Die Sperrlamellen(149) werden entgegen ihrer Sperrfunktionsseite von den Kopfschrauben(155) gehalten. Ein zweiter Achsstift(158) befestigt sie drehbar mit dem Steuerungsring(154).

Die Figur 80 zeigt schematisch einen Mechanismus zur Wiederbelüftung zu Fig.77 durch den Schlauch(156). Dieser verläuft im Schlitz(157) des Zylinders des Abstützringes. Das zugehörige Schiebeventil(99) wird durch einen sektoral angeordneten Verzögerungsmechanismus betätigt.Mit der Bewegung des Handhebels (161), welcher als um die Dosisanzeigescheibe(159) mittels eines Rohrsegmentes drehbare federelastische Zunge ein nach oben gerichtetes Bewegungsmoment aufweist, wird der Ventilstößel(106) eines Schiebeventiles gegen seine Druckfeder(105) dergestalt verschoben, daß die Belüftungsöffnung zum Schlauch(256) verschlossen wird, da der Pumpenzylinder auf der Dosisanzeigescheibe befestigt ist. Der Handhebel muß nun gesenkt werden, um die Steilflanke(162) des Kulissenringes unter der Befestigungsplatte(4) passieren zu können. Dazu weicht der Mitnehmerstift(163) in die Mitnehmerbohrung(160) aus, wodurch die Rückbewegung des Ventilstößels solange verriegelt bleibt, bis der Handhebel die Steilflanke nach Rückkehr in die 0-Stellung wieder passiert hat. Der Mitnehmerstift verläßt dann dank der Elastizität des Handhebels die Mitnehmerbohrung, und die Druckfeder(105) kann das Schiebeventil zur Wiederbelüftung der Saugglocke wieder eröffnen. Es besteht über den Brückenwinkel(129) eine starre Verbindung zwischen der Dosisanzeigescheibe(159) und der Rastklinke(Fig.77).

In Figur 80 bezeichnet das Stadium A den Zustand nach Rückkehr des Handhebels und bei wieder offenem Schiebeventil. Das Stadium B bezeichnet den Zustand kurz vor Rückkehr des Handhebels in die 0-Stellung mit noch geschlossenem Schiebeventil.

Zur Inbetriebnahme eines Injektors nach den Figuren 77 bis 80 wird zunächst die Vierkantstange(133) vom unteren Ritzel(134) gelöst und aus der Führungsbuchse(132) herausgezogen. Der Behälterzylinder(19) kann jetzt von der Befestigungsplatte(4) abgeschraubt werden. Unter Drehung am Faltenbalg kann die Verbindung zwischen Sperrhülse(123) und Winkelstück(122) gelöst und das Pumpengehäuse(11) mit dem Kanülenansatzstutzen(47) nach oben aus der Achsenbuchse herausgezogen werden. Der Deckel(138) wird nach unten aus dem Behälterzylinder herausgeschraubt. Ein neues Pumpengehäuse wird eingeschoben und durch Drehung an der mit dem Faltenbalg adhäsiv fadenartig verbundenen Sperrhülse die Verriegelung mit den Winkelstücken vorgenommen. Das obere Ritzel(135) wird zwischen die Scheiben(136,137) des Deckels und dann auf die Vierkantstange geschoben, nachdem der Behälterzylinder(19) auf die Befestigungsplatte wieder aufgeschraubt wurde. Die Vierkantstange wird in die Führungsbuchse zurückgesteckt, nachdem zuvor der Behälterzylinder(19) auf die Befestigungsplatte(49) aufgeschraubt wurde. Es wird jetzt ein Undruckspeichertöpfchen der sterilen Verpackung entnommen und mittels Druckes gegen den Bodendeckel(165) in den Aufnahmezylinder(144) eingeschoben, wobei Führungsnuten(166) die richtige Radiäreinstellung sichern, da ihnen Nocken(167) im Führungszylinder entsprechen. Vor der Passage der Sperrlamellen müssen die Auslösestifte(148) kurz betätigt werden. Nach Einrasten der Sperrlamellen wird der Bodendeckel entfernt, jedenfalls aber vor der Injektion. Mittels Drehung des Handhebels(161) wird das Schiebeventil zur Wiederbelüftung der Saugglocke geschlossen und nach leichtem Niederdrücken des Handhebels dieser gemäß der anzuwählenden Dosis bei Zählen der Raststufen des Stiftes(153) entlang der Sperrzahnprofilleiste weiter verschoben. Die eingestellte Dosierung kann anhand der Stellung einer Markierung auf der Dosisanzeigscheibe zur Skala auf dem Behälterzylinder kontrolliert werden. Wird der Abstützring(15) auf die Haut aufgesetzt, so kann dieser wegen sonst auftretender Verklemmung zum Aufnahmezylinder hin nur bei streng senkrechter Schubrichtung angehoben werden. Die Anhebung des Saugglockenrandes an der Manschette(147) bewirkt die Anhebung der Auslösestifte(148) und damit den Rückzug der Sperrlamellen aus der Einkerbung des Ringkolbens(9), der mit der Haut hochgehoben wird. Die Anhebung des Saugglockenrandes setzt sich noch fort bis zur Betätigung der Wippe(152) und damit der Einleitung der Einspritzung. Diese vollzieht sich in gleichgroßen Flüssigkeitsstößen entsprechend der Pumpbewegung der Manschette(109). Wird diese mit ihrem Rand gegen die Ringplatte(113) gepreßt, so schließt sie durch Dichtung gegen dieselbe wie auch gegen die Innenwandung des Pumpengehäuses eine Flüssigkeitsmenge unter ihren Rändern ab, von der eine gewisse Menge -die sich nach der Bewegungsstrecke des Ventilstößels bemißt- in Richtung Kanüle ausgestoßen, da die Ventilplatte(115) sich von der unteren Stopfenfläche entfernt hat. Diesen Moment gibt die Figur 77 wieder. Die Rollen(118) stehen dabe tief in der Einsenkung ihrer Keilprofilleiste, während die Gegenrollen durch ihre Keilprofilleisten nach unten gedrückt werden. Die Ventilplatte(115) wird durch Senkung des Stößels(110) vom Stopfen entfernt und die Manschette(111) nähert sich mit ihren Rändern dem der Ringplatte(113). Bei weiterer Manschettensenkung wird eine bestimmte Qantität an Flüssigkeit zur Kanüle hin ausgestoßen. Bevor die Manschette aber von der Ringplatte in Gegenbewegung wieder abhebt, hat die Ventilplatte die Stopfenöffnung wieder verschlossen(bei entsprechender Rollenführung).

Die Figur 80 zeigt in einem Längsschnitt einen Injektor mit elektromagnetischer schlauchintegrierter Dosierpumpe ähnlich wie diejenige auf Fig.77, wobei eine Rolle dicht geschichteter und mit Chemikalien beschichteter Folie eine allmähliche Gasfreisetzung im Behälter unter Kontrolle eines Überdruckventils und damit einen gleichmäßigen Arzneivorschub gewährleistet; der Unterdruck wird durch drei gesondert zu eröffnende vor der Injektion elektrisch beheizte Kammern erzeugt. Auf Fig.81 sind außerdem die Schlaufen (191) am Kanülenansatzstutzen innerhalb der Saugglocke mit dem gummi-elastischen Rand und der Innenlippe (180) zu erkennen, darüber großer(168) und kleiner Ventilkolben(169) der Dosierpumpe mit Stößel(110) und durchbrochener Teller(112) innerhalb des Schlauches. Um den Teller(112) liegen die federnden Klemmbacken (171), welche vom rückgefederten Hubmagnet(170) bewegt werden. Zwischen Heizkessel und Hubmagnet liegt der Isoliermantel(177), welcher streckenweise auch den Schlauch(44) umschließt. Über den Feststellbolzen (195) und die Feststellschraube(194) ist der Arzneibehälter(19) befestigt. In dessen Deckel(138) findet sich das Überdruckventil(181) und im Innern der Faltenbalg(20) mit der Arznei und die Rolle(182) zur Gaserzeugung. Als Drehsicherung dienen die Klammern (190), während der Schaltzylinder(188) mit Kugellagerung zum Behälter hin gemäß der Rillenführung(193) mit seinem Stift drehbar ist. Kabelanschlüsse(176) führen von der Steuereinheit(175) zum Hubmagneten, zu den Heizdrähten,zum Startkontakt(197) und zur Sensorkanüle. Die Widerstandsheizdrähte(178) sind zwischen Isolatorringen(179) ausgespannt. Der Faltenbalg (20) mit Arznei befindet sich innerhalb des Gehäusezylinders(19), der mittels eines gedichteten Deckels (138) zugeschraubt ist. Zwischen Befestigungsplatte (4) und Schlauch befindet sich im Durchlaß für jenen die Dichtung(196). Der Faltenbalg(20) enthält in seiner zentralen schüsselförmigen Einsenkung ein eingerollten Festkörperpaket(182) zur Druckgaserzeugung. Schräg durch die Kammern sich erstreckende Zugfedern (183) sind an durch Bohrungen der Saugglockenschräge geführten Haltestiften von Ventilklappen(184) befestigt. Exzentrisch ausserhalb der Dichtungsringe der Ventilklappen werden diese jeweils von einem senkrechten Stößel(185) erreicht, welche vor dem Schaltring(186) gedichtet sind. Ihnen begegnen Auslösezungen(187), welche vom Schaltzylinder(188) aus nach unten reichen und die einen Stößel nur jeweils nach jeder dritten Absenkung des Schaltzylinders durch den Schaltring hindurch betätigen können. Hierzu ist Voraussetzung daß die drei Auslösestifte(148) innerhalb der Saugglocke gleichzeitig angehoben sind(Fig.83). Zur Inbetriebnahme des Injektors werden die Klammern (190) aufgebogen und der Behälterzylinder samt Pumpenaggregat bis hin zum Kanülenansatzstutzen samt einem Isoliermantel gegen Hitzeeinwirkung aus dem Schaltzylinder herausgezogen. Die Feststellschraube(194) wird gelockert, nachdem der Behälterzylinder von der Befe-

stigungsplatte abgeschraubt wurde und die Kanüle an ihren Schlaufen(191) aus der Saugglocke herausgezogen wurde. Nach Lösung vom Feststellbolzen(195) werden Faltenbalg, Befestigungsplatte, Schlauch und Pumpengehäuse(111) und Isoliermantel in Gesamtheit ausgetauscht, wobei die Klemmbacken(171) um den Teller (112) zu schließen sind, bevor das Pumpenaggregat wieder in den Schaltzylinder eingeschoben und die Verriegelungen vorgenommen werden. Über die Stecker des Netzanschlusses wird der Heizkessel für vorbestimmte Zeit beheizt, wobei erhitzte Luft über die durch Überdruck gegen die Zugfedern abgehobenen Ventilklappen in den Saugglockenraum entweicht.

Figur 82 gibt einen Querschnitt in Höhe A - B der Fig.81, welcher die Verteilung der Heizkessel(172) und ihre Querwände(174) sowie die Verteilung der Heizdrähte(178) zeigt.

Figur 83 verdeutlicht in schematischer Aufrollung die Drehung des Schaltzylinders über eine in Kulissenrillen(189) geführte Nocke(links I).

Die schematische Aufrollung im Bereich des Schaltringes(186)

zeigt, daß lediglich bei Anhebung aller drei Kegel durch die gefederten Auslösestifte(148) unter Wirkung der Druckfeder eine Linksverschiebung des Schaltringes zustandekommen kann. Nach dieser Drehung können die drei Aulösezungen(185) unter Senkung des Schaltzylinders in die Langlöcher mit Keilschrägen eindringen, wobei sie eine Rückdrehung des Schaltringes gegen seine Feder bewirken. Dadurch daß die drei Auslöszungen und die neun Langlöcher gleichen Abstand haben, aber die Ventilstößel unregelmäßig verteilt unter den Langlöchern angeordnet sind, kann trotz kurzem Drehmoment erreicht werden, daß jeweils eine Ventilklappe nach der anderen eröffnet wird. Die Klammern(190), welche in die Befestigungsplatte eingelassen sind, geben das notwendige Bewegungspiel zwischen Arzneibehälter und dem übrigen Injektor frei. In der letzten Phase der Abwärtsbeweung des Schaltzylinders wird der Startkontakt(197) für die Dosierautomatik betätigt. Nach Abschluß der Dosierung mittels der vorprogammierten Pumpenstöße erfolgt die Wiederbelüftung der Saugglokke durch Eröffnung des Magnetventiles(368) durch Impuls von der Steuereinheit her. Für die nächsten zwei Benutzungen ist keine erneute Aufheizung erforderlich. Die Straffung des Seiles(139) löst den elektronischen Arlarmgeber unter dem Deckel aus, wenn der Arzneivorrat beinahe erschöpft ist.

Die Figur 84 zeigt im Längsschnitt ein Unterdruckspeichertöpfchen nach dem Zweikammersystem, wie es in dem Injektor nach Fig.77 verwendet werden kann. Es ist eine der drei Führungsnuten(166) eingezeichnet, welche schon vor Passage an den Sperrlamellen vorbei zu einer Drehung des Töpfchchens in eine bestimmte Winkelstellung zwingen. Die Nocken(167) sichern diese Winkelstellung im Endsitz des Töpfchens.

Die Figur 85 zeigt im Querschnitt durch den Saugglokkenbereich eines Injektors, daß der Saugglockenrand auch kleeblattartig gebuchtet sein kann, um die drei Auslösestifte(148) dem Injektorzentrum zu nähern, ohne sie nach oben abdichten zu müssen.

Die Figur 86 zeigt im Längsschnitt ein Unterdruckspeichertöpfchen nach dem Einkammerprinzip und einen vom Injektor ausgehenden Sperrstift(204), dessen Funktion einer Sperrlamelle entspricht. Die Grenzmembran(10) wird vom Bodenring des Stützzylinders(205) abgestützt, welcher in seiner oberen Wandung einen Kalibersprung nach innen aufweist, um Raum für die Passage der dünnwandigen Wandaussackung(206) zu lassen. Mittels der ringförmigen Schweißkante(207) sind innerer Rand des Bodenringes des Stützzylinders(205), Grenzmembran(10) und das Zentrum des Bodendeckels (165) miteinander verklebt. Der Rand des Bodendeckels stützt sich gegen den unteren Rand des Unterdruckspeichertöpfchens(5) ab und weist wenigstens eine ringförmige Sollbruchlinie auf, welche durch Zug an einer Lasche abgelöst werden kann, wenn im Injektor die Abstützung des Stützzylinders auf dessen Rand von den Sperrstiften(204) des Injektors bis zur Auslösung zur Hautansaugung übernommen wurde. Zum Wegwerftöpfchen gehört als dargestellte Erweiterung der Erfindung der Trichter für die Verbindung mit dem vom Kanülenansatzstutzen ausgehende elastische Schläuchchen(209), welches in ein Bällchen endet und auf welches sich Unterdruck durch den Kanülenschaft hindurch überträgt. Die Klemme(210) wird zur Kontrolle auf Unterdruck angehoben, wobei das Bällchen eingezogen wird. Nach der Hautansaugung kann die Klemme nochmals gehoben werden, um zu prüfen, ob sich das Bällchen mit Blut füllt. Während der Injektion verhindert die Klemme den Abfluß von Arznei in das Bällchen.

Figur 87 zeigt grob-schematisch einen Herstellungsprozeß eines Unterdruckspeichertöpfchens wie in Fig.19. Im Stadium A erfolgt der Spritzguß von Töpfchen und Ringkolben nebeneinander mit senkrechtem Auseinanderziehen der Formhälften(215,216). Im Stadium B werden beispielsweise mittels dreier sektoriell überlappender Transportkaruselle(211,212,213) die mittels Messerschnittes hergestellte Grenzmembran(10) dem Ringkolben genähert und mittels Ultraschallkopfes(214) mit dem Ringkolben verschweißt. Das Transportkarusell (212) bringt die vereinigten Teile unter eine Druckpresse zur Anbringung des Bodendeckels(165) und dann unter die Öffnung des Unterdruckspeichertöpfchens. Die vereinigten Teile werden in der Evakuierungskammer(217) bei Erzeugung des Unterdruckes durch Kompressor mittels des Ultraschallkopfes(218) im Berührung von Grenzmembran und Randring(219) verschweißt.

Die Figur 88 zeigt ein Unterdruckspeichertöpfchen

der in Fig.65 dargestellten Art innerhalb der Schiebehülse(23) im Zustand der Hautansaugung.

Die Figur 89 zeigt im Maßstab 2 : 1 in schematischer
Aufrollung die Profilleisten(225) an den beiden Rädern
(117,125) in Fig.77, wobei sie zur Verdeutlichung ihres gegenseitigen funktionellen Zusammenwirkens einander genähert sind.

Die Figur 90 zeigt stark schematisiert die Befestigung eines elektrischen Summers, der Warnvorrichtung
über die drohende Vorratserschöpfung, über den Träger
(221) mit einem Ring um den Austritt der beiden
Schlauchschenkel aus einer Dosierpumpe nach Fig.65.
Mit den Schwingungen des Summers werden auch die
Schläuche und die Trägerscheiben(40) für die Rollen
der Dosierpumpe in Schwingung versetzt, so daß die
Rüttelvorrichtung entfällt.

Die Figur 91 zeigt im Längsschnitt eine Variation der
Dosiervorrichtung von Fig.77 im Detail. Der vom Faltenbalg(20) herabführende Schlauch(44) erweitert sich
unterhalb dem von ihm umschlossenen vom Rand her sektorartig unterbrochenen Teller(112) um den Stößel
(111). Eine nochmalige stärkere Ausbuchtung des Schlauches ersetzt das Pumpengehäuse und wird von der Achsenbuchse(116) umgeben. Nach Passage der Bohrung im
Stopfen(114) wird das Ende des Stößels von einem Ventilkegel(115) gebildet, während als Dosierkammer eine Art Faltenbalg(109) dient. Der Schlauch umfaßt am
erweiterten Ende die Trägerplatte des Kanülenansatzkonus(47), welcher zum Einschubteil gerechnet werden kann.

Die Figur 92 zeigt das Blockschaltbild der Steuerung
des Injectors, wie er in den Fig.81 und 82 dargestellt ist, bestehend aus Central Processing Unit
(CPU), Memory Unit und Imput/Output-Port.

Die Figur 93 beschreibt den Programmablauf für eine Steuereinheit nach Fig.92.

Die Funktion der vier Tasten ist folgende:

Taste 1 schaltet beim ersten Druck den Inhalt des Vorratszählers auf die Anzeige. Beim zweiten Druck erscheint die Sicherheitsreserve in der Anzeige. Nur jetzt haben die Tasten 2 und 3 eine Funktion.

Taste 2 erhöht die Sicherheitsreserve.

Taste 3 verringert die Sicherheitsreserve des Vorratsbehälters.

Taste 1 schaltet beim dritten Druck die Anzeige ab.

Taste 4 wird beim Wechsel des Insulinsvorratsbehälters geschlossen.

In Wartestellung fragt der Prozessor die vier Tasten und den Schalter(Startkontakt 197) ab. Bei Betätigung der Tasten bedient der Prozessor die Anzeige und verändert gegebenenfalls die Größe der Sicherheitsreserve wie oben beschrieben. Der Schalter(197) löst die Einspritzung aus. Dann wird der Hubzähler mit dem Wert der Dosiervorgabe (es bestehen beispielsweise Speichermodule für 2 bis 20 Kolbenhübe zu je vier Einheiten Insulin) geladen und bestimmt die Häufigkeit der Dosierimpulse und damit die verabreichte Arzneiflüssigkeitsmenge. Nach beendeter Injektion wird ein Impuls auf den Magneten für das Magnetventil (368) zur Wiederbelüfung der Saugglocke gegeben, damit das Gerät wieder von der Haut gelöst werden kann. Der Vorratszähler wird aktualisiert und mit der Sicherheitsreserve und der Dosiervorgabe verglichen.

Ist die Sicherheitsreserve unterschritten, wird die Warnvorrichtung betätigt, das Gerät bleibt aber funktionsfähig. Ist die Dosiervorgabe unterschritten (d.h. Der Arzneivorrat unter die abzugebende Einzeldosis herabgesunken), so wird die Warnvorrichtung ausgelöst und die Gerätefunktion bleibt gesperrt bis ein neuer Arzneivorratsbehälter eingesetzt wird und dabei Taste 4 geschlossen ist.

Figur 94 zeigt im Längsschnitt eine Abschließvorrichtung des Arzneivorrates bis zur Vorratserschöpfung. Innerhalb des nach oben offenen Gehäusezylinders(1) befindet sich als Arzneivorratsbehälter(131) ein Faltenbalg, dessen Arzneischlauch(44) sich innerhalb des Arzneiableitungsrohres(316) mit dem Auflagering (601) befindet. Dieser liegt der Befestigungsplatte (4) auf, innerhalb derer die queren Sperrstifte(330) außen den gefederten Sperrbolzen(329) mit ihren Kuppen anliegen, während die inneren Kuppen in einer Ringnut des Arzneiableitungsrohres eingerastet sind. Der Schlauch(44) setzt sich nach unten in den Pumpenstift(337) mit dem Kanülenansatzstutzen fort; im Bereich der Schlauchringe(415,Fig.45-47) ist er von der Achsenbuchse des Gerätes umgeben. Oben ist der Faltenbalg mittels federnder Rasterlamellen innerhalb des Einschubzylinders(200) befestigt und durch seitliche Zylinderschlitze lösbar. Wird der Faltenbalg nach Befestigung im Schiebezylinder mit diesem zusammen in den Gehäusezylinder eingeschoben, so tritt der nicht ummantelte Teil des Schlauches um den Pumpenstift mit den Schlauchringen in Kontakt. Die Sperrstifte(330) rasten ein (wie abgebildet). Nach annäherndem Verbrauch der Arznei senkt sich der untere Rand des Schiebezylinders auf die Sperrbolzen und senkt diese. Die Sperrstifte können nun aus der Ringnut des Arzneileitungsrohres bei Zug am Seil(139) für die elektronische Warnvorrichtung(387) zurückweichen und der Faltenbalg samt Schlauch kann wieder herausgezogen werden.

Figur 95 zeigt eine Variante der Dosierhydraulik. Innerhalb eines Stufenspritzenzylinders(272) ist oberhalb der Arznei der Trennkolben(275) gelagert, darüber lagert Hilfsflüssigkeit, welche oben von einem Deckkolben(801) als Wegwerfteil begrenzt wird. Diesem ist als Gerätebestandteil der Stützkolben(802) aufgesetzt, welcher die Zylinderbohrung mit dem grossen(168) und kleinen(169) Ventilkolben enthält, welche sich in den Raum mit Hilfsflüssigkeit über eine Bohrung entleert. Zwischen dem Faltenbalg mit Hilfsflüssigkeit, der unter Senkung des Stützkolbens nach Leckverlusten aufgefüllt wird, und dem Ventilstößel befindet sich eine Dichtung. Die Ventilkolben werden mittels des Ventilstössels über den Schwenkhebel(803) vom Hubmagnet(413) betätigt. Das Ende des Ventilstössels greift in zwei Armen phasenverschoben in Schrägnuten(804) einer Scheibe ein. Letztere ist auf der Gewindestange(905) befestigt, welche in der Gewindebuchse(805) gelagert ist. Zur Dosierung wird bei Senkung des Ventilstößels und der Ventilkolbens(168, 169) eine Dosiseinheit an Hilfsflüssigkeit aus dem Faltenbalg in den Flüssigkeitsraum zwischen Trennkolben und Stützkolben gepumpt. Da der Stützkolben durch die Gewindespindel nach unten gehalten wird, wird der Trennkolben und damit die Arznei durch die Kanüle verdrängt. Bei Anhebung des Ventilstößels (dessen Gabelung auf verschiedene Schrägen der Scheibe in verschiedenen Phasen trifft) wird die Scheibe gedreht und und die Gewindespindel um den Betrag gesenkt, welcher der Verdrängung einer Dosiseinheit entspricht. Dabei läuft die entsprechende Menge Hilfsflüssigkeit in den Faltenbalg zurück.

Figur 96 bringt oben eine Draufsicht, unten und in der Mitte einen Längsschnitt in der Schnittlinie A - B der Draufsicht und unten ebenfalls in einem Längsschnitt in der Schnittlinie C - D der Draufsicht eine Dosiervorrichtung, welche durch den elektrischen Magneten(546) druckbeaufschlagt ist. Dieser Magnet sitzt über den kurzen Hebelarmen, welche jeweils zwei im Scharnier(547) zusammenklappbaren Platten(535) zugehören. Hebelarme und Platten überkreuzen sich nicht am unten abgebildeten Ende, wo der Schlauch(44) zur Kanüle hin herausgeleitet wird. Bei Klappenöffnung -wie dargestellt- ist der Arzneiabfluß durch Schlauchabklemmung zwischen den kurzen Hebelarmen gesperrt. An der Stelle der Arzneischlaucheinleitung überkreuzen sich Platten und kurze Hebelarme scherenartig, weshalb der Zufluß dort unbehindert ist. Zwischen den Platten liegt der Schlauch in einer Schleife. Werden die Platten einander unter Magnetwirkung genähert, so wird (bei Federung der kurzen Hebelarme) zunächst der Schlauch an der Zuflußstelle abgequetscht und dann im Bereich der Schleife zwischen den Platten. Der rückführende Schlauchschenkel ist über den Durchbruch(549) auf die Außenfläche der obersten Platte hinausgeleitet, um eine Abflußhemmung zu vermeiden. Die Druckfeder(549) dient der Rückstellung.

Figur 97 zeigt den Schlauch(44) in Windungen auf einem Spiralring gelagert, welcher außen auf einer elastischen Gummipyramide eingelassen ist. Die Gegenspirale befindet sich jeweils oberhalb der Schlauchwindungen im Eingriff und ist innerhalb eines kegelförmigen Bechers angeordnet. Bei Senkung desselben unter Magnetwirkung, werden entsprechend einer größeren Distanzierung der beiden Spiralringe unten als oben diese zuerst oben einander genähert bis zur Schlauchabklemmung oben. Der Schlauch wird peristaltisch nach unten(der Kanüle zu) entleert. Bei Anhebung des Kegelbechers durch die Druckfeder(549) wird zunächst der Überbecher(553) gehoben dessen unterer Rand den Schlauch unten abklemmt.

Die Figur 97 bringt im schematischen Längschnitt eine weitere Dosiervorrichtung für den Impulsbetrieb. Mit elektrischer Spannung betriebene piezoelektrische Druckgeber(564) sind gegen einen feststehende Gehäuseteil oben gelagert und liegen einem elastischen Mantelring(556) auf. Dieser enthält vor dem starren Ansatzstück(557) für das Ableiterohr eine Schlauchauftreibung
(558), aus welcher sich der Schlauch über eine seitliche Abflachung des Mantelringes zu einem Absperrventil
fortsetzt. Letzteres wird bei der Deckelöffnung beim Injektor freigegeben, um auch bei Spannungslosigkeit im
Bereich der Piezoelemente den Arzneiabfluß zu stoppen.
Die Oberfläche der linsenartig abgeplatteten Schlauchauftreibung ist fest mit einer Haftplatte(567) verbunden, welche ihrerseits mittels der Rastschieber(568) an
der Unterfläche des Druckgebers(564) befestigt sind.
Von der Lichtquelle(560) links führen optisch leitende
Fasern durch den Mantelring und richten sich auf die
Wandung der teildurchsichtigen Schlauchblase. Der Lichtquelle gegenüber verlaufen im Mantelring wiederum lichtleitende Fasern(unterhalb der durch Druck deformierten
Zone) zum optischen Sensor(561), welcher mit der Steuer-
einheit(175) elektrisch verbunden ist ebenso wie die
piezoelektrischen Schalter(562,563). Von der Steuereinheit führen Befehlsleitungen(555) zu den piezoelektrihen Spannung-Druckwandlern. Unter Kontrolle des höhenjustierbaren Schalters(562) erhalten Piezoelemente über
ihre Befehlsleitungen unter Vermittlung der Steuereinheit Spannungen, die zu ihrer Ausdehnung und zur Sperrung des Arzneizuflusses im Zuflußbereich der Schlauchauftreibung führen. Die Dosierimpulse werden nun in die
Anregung und Ausdehnung größerer Teile des Druckgebers
(564) umgesetzt, wodurch der Mantelring abgeplattet und
im Lumen verengt und die Schlauchauftreibung verkleinert wird und zwar solange, bis der Schalter(563) den
eingestellten Tiefstand des Druckgebers rückmeldet.
Nach Spannungsänderung zur Wiederausdehnung erfolgt
Schlauchauffüllung. Durch plötzlichen Rückzug des
Druckgebers tritt innerhalb der Schlauchauftreibung
zunächst ein Vakuum auf(wegen der größeren Trägheit
der Flüssigkeitsdynamik). Die Dichteabnahme und ihr Wiederverschwinden durch die Auffüllung wird über den optischen Sensor an die Steuereinheit zurückgemeldet und der nächste Dosierimpuls auf den Druckgeber geleitet, noch ehe Arznei ungehindert vom Arzneibehälter zur Kanüle durchströmen kann. Durch die Geschwindigkeit der Zuschaltung einzelner Abschnitte des Druckgebers(als Datenwort in der Steuereinheit) kann die Ausflußgeschwindigkeit geregelt werden(bzw. die Verteilung der Druckeinwirkung auf die Zeitkurve). Dadurch kann auch über feinere kapilläre Zuleitungen innnerhalb der Kanüle subkutan injiziert werden, ohne durch plötzlichen Überdruck Gewebszerstörungen zu verursachen. In einem solchen Fall besonders engen Ausflußkanales(wie er ja zugunsten einer schmerzarmen Injektion angestrebt wird), wird die Arneiabgabe(in verlangsamten Takten) schon vor Abschluß der Messung der Stoffwechselparameter eingeleitet. Es ist dann bereits eine gewissen Basisrate appliziert, ehe die weitere Abgabe eventuell gemäß einer errechneten Korrektur unterbrochen wird.

Die Figur 99 zeigt grobschematisch die Verbindung eines im Querschnitt gezeigten Drosselventiles(565) mit einer Steuereinheit, welche zugleich als induktives Durchflußmeßgerät ausgebildet ist und gemäß der über die Meßelektroden(566) rückmeldeten Durchflußmenge die Ventil steuerung bewerkstelligt. Solche Meßvorrichtungen sind in der Patentliteratur vielfältig beschrieben. So durch G.Geisler,A.Seebode in DE 33 14 954,K.Walter,F.Hofmann, W.Stelz,Ronald,Venlo in DE 24 10 407 oder von E.Prinz, G.Leiser in DE 27 44 845.

Figur 100 zeigt im Längsschnitt eine Umschaltvorrichttung von einer Dosiervorrichtung auf eine andere bei Einsatz nur eines einzigen Motors. Der Elektromotor (855) weist in seiner Antriebswelle(603) eine zentrale Bohrung zur Aufnahme der Schaltstange(604) auf. Die Kraftübertragungsbuchse(605) wirkt nur rechtsdrehend auf die Beförderung des Sperrzahnrades(607) über seine Sperrklinke(606). Mit dem um
die Lagerbuchse(887) frei drehbaren Schaltritzel
(800) ist das Sperrzahnrad fest verbunden. Vom
Schaltritzel wird das Getrieberad(859) an der Gewindestange einer Stufenspritze(beispielsweise)
getrieben, da es mit ihm in Eingriff steht. Auf
der Schaltstange ist das Sperrzahnrad(608) fest
montiert, welches über die Sperrklinke der kleinen
Kraftübertragungsbuchse(609) bei Linksdrehung angetrieben wird. Die Bewegung des Sperrzahnrades
(608) überträgt sich auf die Trommel(71) mit der
Kulissenführung(72) in welcher der Fixierbolzen
(73) eine Hebung und Senkung bei Drehung bewirkt
Diese Achsenverschiebung überträgt sich auf das
Schaltritzel und bedingt dessen Stellung zu den
Getriebezahnrädern. Zur Umschaltung auf das jeweils andere Dosierprogramm braucht der Motor also
nur eine halbe Linksdrehung durch Stromumpolung zu
verrichten. Dies bedeutet eine erhebliche Raum-
und Gewichtsersparnis.

In Figur 101 wird oben in einer Seitenansicht und
unten in einer Draufsicht eine Vorrichtung zum Dosiswechsel bei Verwendung von Magneten oder Piezodruckgebern als Dosierantrieb. Zwischen dem kurzen Ende des Schwenkhebels und dem Druckgeber(564)
besteht eine elastische Verbindung(612). Die
schwenkbar angeordnete Kappe(613) steht über dem
gefederten Stößel(614), wobei die Feder nach jedem Pumpentakt gegen die Schlaucherweiterung innerhalb einer Dosierkammer den Stößel hebt. Dabei bleibt er mit der Kappe in Kontakt. Wird aber
ein schneller Zugimpuls vom Druckgeber aus wirksam, so verläßt die Kappe den Stößel und eine mittels Druckhebels(616) gespannte Feder befördert
die Kappe unter Schwenkung über den Stößel der anderen Dosiervorrichtung.

Figur 102 zeigt im Längsschnitt eine Variation des Mechanismus für die vertikale Bewegung des Kanülenmagazins vor und nach der Einspritzung in den beiden Funktionszuständen A und B. Das Kanülenmagazin (316) ist hierzu zusammen mit dem zentralen Halterohr ohne Dichtung in einer Buchse des Saugglockendeckels leicht verschieblich. Das Ende des zentralen Halterohres verbreitert sich zu einer Ringplatte(536), welche über eine elastische Membran(537) zum Saugglockendach gedichtet ist. Das Stadium A zeigt,wie die Haut in die Saugglocke unter Sogeinfluß hochgezogen wurde und gleichzeitig die Ringplatte(536) abgesenkt ist, so daß sich Haut und Kanüle begegnen. Im Stadium B haben sich Haut und Kanüle nach Wiederbelüftung der Saugglocke dank der Elastizität von Haut und Membran wieder von einander entfernt.

Figur 103 zeigt im Längsschnitt eine Variation eines Injektors, bei welchem anstelle einer Saugglocke ein Mantel(538) zum Schutze der Kanüle vorliegt. Der Mantel ist als Ellipse ausgebildet, in deren einem Mittelpunkt das zentrale Halterohr(324) vertikal verschieblich angeordnet ist, während im anderen Mittelpunkt ein Zentralstift(433) eine Ringplatte(536) trägt, welche eine Bohrung für den Durchtritt des zentralen Halterohres aufweist und deren Unterfläche mit einer beidseits klebenden Folie(539) versehen wurde. Oben im Stadium A wurde der Zentralstift(ähnlich wie bei dem Beispiel in Fig.13) mit der Ringplatte und Haut angehoben, während das zentrale Halterohr mit der Kanüle abgesenkt wurde. Im Stadium B unten wurde umgekehrt das zentrale Halterohr angehoben und der Zentralstift gesenkt, so daß sich Haut und Kanüle getrennt haben. Eine Unterdruckanwendung wird so vermieden. Zwischen A und B ist in einer Draufsicht die Klebefolie(539)

- 127 -

mit der Bohrung für den Kanülendurchtritt zu erkennen, welche zwischen den beiden Schutzfolien(540) liegend zweckmäßigerweise in konkardenförmiger Anordnung vom klebenden Elektrodenring umgeben wird.

mit der Bohrung für den Kanülendurchtritt zu erkennen, welche zwischen den beiden Schutzfolien(540) liegend zweckmäßigenweise in konkardenförmiger Anordnung vom klebenden Elektrodenring(617) umgeben wird.

Die Figur 104 zeigt im Längsschnitt die in eine Saugglocke mittels Unterdruckes erhobene Haut, wobei durch die Hautkuppe polarisiertes Licht auf einen gegenüberliegenden Detektor geworfen wird.

Die so ermittelten Meßwerte könnten zur Dosiskorrektur herangezogen werden. Es muß erst noch bestätigt werden, inwieweit sich die im Gewebe langsamer verändernden Zuckerwerte zur Beurteilung der aktuellen Stoffwechsellage eignen.

Die Figur 105 zeigt links in Seitenansicht und rechts in Draufsicht den Grundversuch, welcher zur Änderung der elektrischen Leitfähigkeit an Grenzmembranen in Auftrag gegeben wurde. Auf die elktrolytisch definierte Silberchlorid-Silberplatte(610) wurde in seitlicher Abdichtung ein Filterpapier(611) aufgespannt und Sepharose-Concanavalin A dazwischen eingefüllt.

Die andere Seite der Membran wurde mit Kochsalzlösung verschiedener Glucose-Konzentration bespült und gegen die Kalomelektrode(611) gemessen. Es wurden 600 V Wechselstrom angewandt und signifikante Meßergebnisunterschiede bestätigt.

Weitere Meßdaten:
Membranfläche: $5,3cm^2$ ,ConA-Sepharose-Gel Sediment 2,1ml,(Pharmazia Freiburg Br.), darin enthaltene Menge ConA etwa 21mg,durchgesetzte Glukosemenge etwa 20mg, Effekteintritt nach etwa 2mg,Widerstand der Meßanordnung 10 Ohm, Widerstand der Anordnung (ConA-Sepharose/1n-NaCl-Glukose-Lös.-Kalomelelektrode 250 Ohm, Nennfrequenz 10 kHz. Trisaccharid-Raffinose-Lösung zum Vergleich war ohne Wirkung.

Beispiele automatischer Dosisangleichung

bei von der Erwartung abweichendem Gewebsglucosespiegel:

| Depot-Insulin | Sollwerte (Glucose-Toleranz +Nüchern-Idealwert) | Istwerte Glukose | Korrektur-faktor (Altinsulin / Depot-I.) | Dosiskorrektur inklusive Berechnung | Verabreichte Dosen Altinsulin | Verabreichte Dosen Depotinsulin |
|---|---|---|---|---|---|---|
| 80E | 90–120mg% | 180mg% | 10 | 10x1x8 | +80E | |
|  | 100mg% | 50mg% | 4 | 4x1x20 | | −20E=60E |
| 60E | 90–120mg% | 180mg% | 7 | 7x1x8 | +56E | |
|  | 100mg% | 50mg% | 12 | 12x1x5 | | −60=0 |
| 40E | 90–120mg% | 180mg% | 5 | 5x1x8 | +40E | |
|  | 100mg% | 50mg% | 4 | 4x1x5 | | −20E=20E |
| 20E | 90–120mg% | 180E | 3 | 3x1x8 | +24E | |
|  | 100mg% | 50mg% | 12 | 2x1x5 | | −10E=10E |
| 16E | 90–120mg% | 180mg% | 1 | 1x2x4 | +8E | |
|  | 100mg% | 50mg% | 2 | 2x1x5 | | −10E= 6E |

Anmerkung: Patient 1 (obere Zeile), Patient 2 (untere Zeile).

T A B E L L E  1

0221005

- 128 -

Patentansprüche:

A
1. Vorrichtung zur Einspritzung von Flüssigkeiten unter die Haut,
dadurch gekennzeichnet,
daß zumindest in Nähe der Kanüle ein Sensor angeordnet ist, welcher gestattet, Stoffwechselveränderungen im Körper zu messen und die Meßergebnisse zur Kenntnisnahme vorzubereiten.

A
2. Vorrichtung nach Anspruch 1,
dadurch gekennzeichnet,
daß die Kanüle als Sensor ausgebildet ist.

0221005

- 129 -

<u>P a t e n t a n s p r ü c h e :</u>

1. Vorrichtung zur Einspritzung von Flüssigkeiten unter die Haut,
dadurch gekennzeichnet,
daß die Kanüle als Sensor ausgebildet ist, welcher es ermöglicht, Stoffwechselveränderungen im Körper ohne zusätzliche Verletzung zu erfassen.

2. Vorrichtung nach Anspruch 1,
dadurch gekennzeichnet,
daß sie in Verbindung mit einer Sauginjektionsvorrichtung, bei welcher die Haut durch Unterdruck innerhalb einer Saugglockes in die Kanüle gehoben wird, angewandt wird(Fig.8-12,13-28,29-31,32-33, <u>36-49</u> und <u>57-64</u>,65-75,76-79 und 89,80-83,84,85,86, 87,88,91,102,104).

3. Vorrichtung nach Anspruch 1,
dadurch gekennzeichnet,
daß innerhalb einer Injektionsvorrichtung eine Platte mit hautadhesiver Beschaffenheit vorhanden ist, welche einen Raum freiläßt, um eine Kanüle vorbeizulassen, wenn diese Platte durch einen Mechanismus nach oben bewegt wird(Fig.103).

4. Vorrichtung nach Anspruch 1,2,3,
dadurch gekennzeichnet,
daß der Kanülenschaft hinter dem Anschliff den Ringschluß desselben erhaltend rinnenförmig eröffnet ist(Fig.50,53).

5. Vorrichtung nach Anspruch 1,2,3,
dadurch gekennzeichnet,
daß die Sensor-Oberfläche der Kanüle in Verbindung
mit der Körperflüssigkeit des Lebewesens und vermittels einer Gegenelektrode als aktives elektrochemisches Element wirkt.

6. Vorrichtung nach Anspruch 1,2,3,
dadurch gekennzeichnet,
daß Sensor-Oberfläche der Kanüle unter Einwirkung
der Körperflüssigkeit des Lebewesens ihren elektrischen Widerstand in gesetzmäßiger und reproduzierbarer Weise ändert, und wobei eine diesem Widerstand
propartionale Spannung, bzw. die zeitliche Änderung
dieser Spannung oder eine diesem Widerstand proportionale Stromstärke, bzw. die zeitliche Änderung
dieser Stromstärke zur Auswertung zur Verfügung
steht(Fig.105).

7. Vorrichtung nach Anspruch 1,2,3,
dadurch gekennzeichnet,
daß daß die Kanüle als optischer Sensor ausgebildet
ist, bei welchem innerhalb des Kanülenschaftes
Strahlen eingeführt werden(Fig.56).

8. Vorrichtung nach Anspruch 1,2,3,
dadurch gekennzeichnet,
daß im Bereich des Kanülenschaftes Lichtleitfasern sich befinden.(Fig.54,55).

9. Vorrichtung nach Anspruch 1,2,3,
dadurch gekennzeichnet,
daß an einer Indikatorschicht zurückgeworfenes
Licht zu Messungen ausgewertet wird(Fig.54,55).

10.·Vorrichtung nach Anspruch 1,2,3,
dadurch gekennzeichnet,
daß eine Kanüle an dem Ende, welches dem Kanülenschaft entgegengesetzt ist, eine Öffnung für die
Aufnahme des Schaftes einer anderen gleichartigen
Kanüle aufweist, und daß die Arzneizufuhr zum Kanülenschaft seitlich im Kanülenkörper erfolgt
(Fig.27,48,49).

11. Vorrichtung nach Anspruch 1,2,3,
dadurch gekennzeichnet,
daß Kanülen innerhalb eines schlauchförmigen Behälters so gespeichert werden, daß über einen Kanülenwechselmechnismus in der Abdeckelung für jenen Behälter ein Kreislauf eintritt, bei welchem benutzte
Kanülen den Raum früher unbenutzer Kanülen einnehmen
(Fig.37).

12. Vorrichtung nach Anspruch 1,2,3,
dadurch gekennzeichnet,
daß wenigstens eine elektronische Speichereinheit
vorhanden ist, deren gespeicherte Meßdaten über
einen Leser später außerhalb der Vorrichtung darstellbar sind(Fig.5-7).

13.·Vorrichtung nach Anspruch 1,2,3,
dadurch gekennzeichnet,
daß wenigstens eine Steuereinheit vorhanden ist,
welche eingangseitig eine Meßeinheit für die Aufnahme und Verarbeitung der vom Sensor
abgegebenen Signale umfaßt, des weiteren mindestens
eine programmeinheit mit Zeitgeber für die Vorbestimmung von Befehlen, wenigsten eine Rechnereinheit
für die Auswertung und Inbezugsetzung der von
Sensor und Programmeinheit gegebenen Daten und
wenigstens eine Befehlseinheit zur Betätigung wenigstens einer Dosiervorrichtung(Fig.58-64):

14. Vorrichtung nach Anspruch 1, 2 und 3, dadurch gekennzeichnet, daß mindesten eine Zeitprogrammiereinheit vorgesehen ist, welche mindestens je einen Termin in Vergleich mit einem Zeitgeber an eine Weckvorrichtung weiterleiten kann und darüber hinaus den Zeitraum für den Abruf einer bestimmten, wenn auch nach den ermittelten chemischen Parametern variable Arzneimenge, zwischen veränderbaren, aber jeweils festlegbaren Zeitgrenzen einengt.

15. Vorrichtung nach Anspruch 1, 2 und 3, dadurch gekennzeichnet,
daß mindestens eine Programmiereinheit vorgesehen ist, welche in veränderbarer, aber für den Einzelfall festlegbarer Weise die Abweichungen von gemessenen chemischen Parametern nach unten und oben bestimmt, innerhalb deren keine Anpassung der Dosierung von einer vorgesehenen Normaldosierung abweichend statthat, sowie dadurch gekennzeichnet,
daß innerhalb einer Programmiereinheit für mindestens eine Arzneiart entsprechend der Zuordnung zu einer Dosiervorrichtung eine Normaldosis für bestimmte Zeiträume festgelgt werden kann sowie deren Korrektur gemäß von wenigstens einem abgestuft wählbaren Korrekturfaktor in Verbindung mit den Abweichungen wenigstens eines ermittelten chemischen Parameters von dem als erwünscht festgelegten Normalwert, sowie dadurch gekennzeichnet,
daß für den Fall der Überschreitung veränderbarer, für den Einzelfall aber festgelegter Parameter wenigstens eine Programmiereinheit für ein Sonderdosierprogramm innerhalb veränderbarer aber für den Einzelfall festlegbarer Dosisgrenzen innerhalb festlegbarer Zeitgrenzen anwählbar wird.

-133-

16. Vorrichtung nach Anspruch 1, 2 und 3,
dadurch gekennzeichnet,
daß eine hinsichtlich ihrer Funktion abschließbare
Programmautomatik vorhanden ist, welche erlaubt bei
mehrfachen deutlichen Meßwertabweichungen mit Korrekturen, welche auf eine Dosisfehleinstellung
schließen lassen, das voreingestellte Dosierprogramm
zu verändern.

17. Vorrichtung nach Anspruch 1, 2 und 3,
dadurch gekennzeichnet,
daß bei mehrfachen Altinsulinzugaben gemäß der voreingestellten Dosierung die Depotinsulindosierung
des dem jeweilingen Zeitpunkt der Meßwerterhebung
vorangegangenen Injektionstermines in Stufen erhöht wird, während bei Meßwertunterschreitungen eine
Depotinsulinerniedrigung als künftige Dosis vorgemerkt wird.

18. Vorrichtung nach Anspruch 1, 2 und 3,
dadurch gekennzeichnet,
daß zur Korrektur der Dosiereinstellung für Altinsulin unter Mitwirkung des Patienten eine Programmautomatik wirksam wird, welche bei erhöhten
oder erniedrigten Kontrollmeßwerten nach einem regulären Injektionstermin eine stufenweise Anpassung
der Altinsulindosen im Dosierprogramm vornimmt.

19. Vorrichtung nach Anspruch 1, 2 und 3,
dadurch gekennzeichnet,
daß zur Korrektur der Einstellung des Korrekturfaktors für Altinsulin unter Mitwirkung des Patienten eine Programmautomatik wirksam wird, welche
nach erhöhten oder erniedrigten Meßwerten bei Meßkontrolle nach einem vom Patienten bestimmbaren
in bestimmtem Mindestabstand von einer Insulininjektion liegenden Termin und vorausgegangener Altinsulingabe die Einstellung des Korrekturfaktors in Stufen verändert.

20. Vorrichtung nach Anspruch 1,2 und 3, dadurch gekennzeichnet, daß wenigstens eine Zähleinheit vorhanden ist für Datenspeicherung über abgegebenes Vorratsgut, welche Daten so verarbeitet werden, daß nach Abgabe einer vorbestimmten Menge des Vorratsgutes eine Warnvorrichtung betätigt wird und daß die Funktion des Injektors gesperrt wird, sobald die Abgabe einer vorbestimmen Arzneimenge nicht mehr gewährleistet ist,soweit nicht eine teilweise Wiederauffüllung des Arzneivorratsbehälters durch Rückpumpen aus einem anderen Vorratsbehälter stattgefunden hat.und die erforderliche Kanüle vorhanden ist

21. Vorrichtung nach Anspruch 1,2,3, dadurch gekennzeichnet, daß Schalter und Verzögerungsvorrichtung vorhanden sind, welche dem Benutzer es ermöglichen, den Beginn der Einspritzung solange zu verzögern, bis er sich über die Meßergebnisse schlüssig geworden ist.

22. Vorrichtung nach Anspruch 1,2,3, dadurch gekennzeichnet, daß der Arzneivorratsbehälter erst dann zur Entnahme aus dem Injektor entriegelt wird, wenn der Arzneivorrat in ihm nahezu erschöpft ist(Fig.45,94).

23. Vorrichtung nach Anspruch 1,2,3, dadurch gekennzeichnet, daß der Einflußnahme des Patienten auf die Injektorfunktionen einschließlich einer willkürlichen Dosis-

veränderung in zeitlicher Nähe zum Injektionstermin von Befugten durch eine Sperrvorrichtung eingeschränkt werden kann.

24. Vorrichtung nach Anspruch 1,2,3, dadurch gekennzeichnet, daß daß Schaltringe um den Gehäusezylinder eines Injektors verschieblich in Reihen, vorzugsweise zu Zeitblöcken geordnet, angeordnet sind, welche über eine wellenfömige Gestaltung ihrer Innenfläche nebeneinander angeordnete Kontakte wenigstens einer Schalterleiste betätigen, so daß aus der Reihenfolge ihrer Betätigung die Matrix für die Schaltstellung festgelegt werden kann, ferner dadurch gekennzeichnet, daß die Schaltringe innerhalb der jeweils federgesicherten Raststellung zur Erkundung einer Einstellstufe zur Drehung etwas rückgefedertes Spiel haben, um dabei die aktuellen aus der Steuereinheit wirksamen Einstellwerte auf dem zugeordneten Leser über Schalterbetätigung abzufragen.

25. Vorrichtung nach Anspruch 1,2,3, dadurch gekennzeichnet, daß die Ziffern von Schaltringen erhaben gestaltet sind und so in eine Reihe gestellt werden bei der Programmierung, daß die Programmdaten über einen auch einfachen Kopierträger abgedruckt werden können.

26. Vorrichtung nach Anspruch 1,2,3, dadurch gekennzeichnet, daß in einer Saugpumpe, deren Saugkolben durch eine Feder bewegt wird, dieser Kolben während seiner Raststellung durch wenigstens eine Vorrichtung zur Haftungserhöhung in seiner Anfangsbewegung behindert wird(Fig.37(309,325).

27. Vorrichtung nach Anspruch 1,2,3, dadurch gekennzeichnet, daß in einem Injektor zwischen dem Saugglockenraum und dem Raum, in welchem sich ein Saugkolben in einem Zylinder zur Unterdruckerzeugung bewegt, wenigstens ein Sand- und Staubfilter vorgesehen ist (Fig.37(393,394).

28. Vorrichtung nach Anspruch 1,2,3, dadurch gekennzeichnet, daß die Feder für einen Saugkolben unter Vermittlung eines Bowdenzuges bei Betätigung des Haltearmes für den Saugglockendeckel gespannt wird.

29. Vorrichtung nach Anspruch 1,2,3, dadurch gekennzeichnet, daß ein Ventil während der Verkleinerung des Saugglockenraumes durch die Annäherung und das Eindringen der Haut eine Luftkompression in der Saugglocke verhindert(Fig.37(367).

30. Vorrichtung nach Anspruch 1,2,3, dadurch gekennzeichnet, daß als Sogquelle eines Injektors mindestens ein Heizkessel(172) dient, welcher durch Reibungselektrizität beheizt wird, wobei die erhitzte Luft durch Ventile(184) entweichen und der Unterdruck bewahrt werden kann, bis zu einer Ventileröffnung zwischen Heizkessel und Saugglockenraum zur Ansaugung der Haut(Fig.81,82).

31. Vorrichtung nach Anspruch 1,2,3, dadurch gekennzeichnet, daß im Anschluß an eine Druckgaskapsel eine Ventilkombination vorhanden ist, welche aus zwei Sitzventilen besteht, von denen das kleinere zuerst geöffnet wird, und aus einem Schieberventil, so daß in einer Überdruckkammer zu den Sitzventilen hin eine Gasdruckstauung entsteht, welche die Öffnung des großen Sitzventiles erleichtert(Fig.19).

32. Vorrichtung nach Anspruch 1,2,3, dadurch gekennzeichnet, daß in einem den Unterdruck bewahrenden Speichertöpfchen mit Saugglocke ein Ventilstift innerhalb eines Führungsrohres verschieblich ist, dessen oberes Ende durch eine Deckfolie verschlossen ist, während der Ventilstift den Saugglockenrand vor Benutzung überragt und eine Art Blattfeder aufweist, welche dem Saugglockenrand aufliegt und dazu geeignet ist, die Aufwärtsbewegung des Stiftes plötzlicher zu gestalten(Fig.2).

33. Vorrichtung nach Anspruch 1,2,3, dadurch gekennzeichnet, daß der Injektor eine Sperrvorrichtung(148,149,154, 155) für den Bodendeckel(9,165) eines Unterdruck-Speichertöpfchens trägt, wobei der Bodendeckel eine Befestigungsvorrichtung(165,2o7,208) besitzt, welche bestimmungsgemäß for dem Gebrauch des Injektors entfernt wird, nachdem die Sperrvorrichtung des Injektors in Tätigkeit trat(Fig.77,84,85).

34. Vorrichtung nach Anspruch 1,2,3, dadurch gekennzeichnet, daß als Sogquelle die Bewahrung von Unterdruck in einem der Saugglocke benachbarten Teil eines gedeckelten Zylinders(5) dient, wobei am Injektor eine Abstastvorrichtung(69-74) für die nach Nachlassen des Unterdruckes nach Hautkontakt an jenem Zylinder bewirkte Oberflächenveränderung vorhanden ist, welche den Fortgang des Funktionsablaufes des Injektors steuert(Fig.70,75).

35. Vorrichtung nach Anspruch 1,2,3, dadurch gekennzeichnet, daß die Saugglocke innerhalb einer Führungshülse gegen Federn verschieblich angeordnet ist, um von einer Rastervorrichtung solange festgehalten zu werden, bis die Wiederbelüftung des Saugglockeninneren erfolgt.(Fig.37).

36. Vorrichtung nach Anspruch 1,2,3, dadurch gekennzeichnet, daß ein Zentralstift innerhalb der Saugglocke nach Abschluß der Injektion und vor Abschluß der Wiederbelüftung der Saugglocke gegen die Haut bewegt wird, um diese aus dem Bereich der Kanülenspitze zu entfernen(Fig.23).

37. Vorrichtung nach Anspruch 1,2,3, dadurch gekennzeichnet, daß seitliche Klappen vorhanden sind, deren Schliessung eine Federspannung bewirkt, welche zur Steuerung von Gerätefunktionen eingesetzt werden kann und zugleich den Funktionszustand anzeigt(Fig.19).

38. Vorrichtung nach Anspruch 1,2,3, dadurch gekennzeichnet, daß die Haltevorrichtung für die Kanüle an ihrem Ende innerhalb der Saugglocke eines Injektors eine Ringplatte trägt, welche mit einer Membran verbunden

- 139 -

ist, um sich bei Sogeinwirkung innerhalb der Saugglocke mit der Kanüle abzusenken und durch Elastizität wieder nach Wiederbelüftung der Saugglocke in
die höhere Ausgangslage zurückzukehren(Fig.102).

39. Vorrichtung nach Anspruch 1,2,3,
dadurch gekennzeichnet,
daß in der Umgebung des Saugglockenrandes nur
geringfügig von diesem in der Fläche zurückspringend
eine Flächenauflage vorhanden ist, um ein Verkanten
während der Anwendung zu erschweren(Fig.65,77,81,88).

40. Vorrichtung nach Anspruch 1,2,3,
dadurch gekennzeichnet,
daß die Drehung des Steuerungsringes(154) von drei
Stiften im Saugglockengbereich aus Durchtrittsöffnungen für Auslösezungen(187) oberhalb des Steuerungsringes freigibta, so daß bei Senkung der Auslöszungen die weiteren Funktionen des Injektors
ausgelöst werden(Fig.83).

41. Vorrichtung nach Anspruch 1,2,3,
dadurch gekennzeichnet,
daß die Auslösestifte(148) für die weiteren Funktionen des Injektors innerhalb von Einbuchtungen
eines kleeblattartig gelappt geformten Saugglockenrandes außerhalb des Saugglockenraumes liegen
(Fig.85).

42. Vorrichtung nach Anspruch 1,2,3,
dadurch gekennzeichnet,
daß zur Wiederbelüftung des Saugglockenraumes ein
Schaltmechanismus vorgesehen ist, welcher vor Stillstand desdie Arzneiflüssigkeitsabgabe bewirkenden
Motors die Spannung einer Feder(64,104,105) vorsieht, wobei ein Sperrglied(59,99,159) durch einen Raster(58,62,108,160,161,163) solange festgehalten wird, bis dieses Sperrglied an der Steilflanke(162) einer stehenden Gehäusekulisse nach Abgabe der Arzneiflüssigkeit die Bewegung durch jene Feder(64,105) freigibt(Fig.70,7680).

43. Vorrichtung nach Anspruch 1,2,3, dadurch gekennzeichnet, daß als Dosiervorrichtung ein Stift sich innerhalb des Arzneischlauches vor einer starrwandigen Ableitung befindet, welcher einen Zuflußkanal zur starrwandigen Ableitung aufweist und wobei Schlauchringe zwischen jenem arzneiführenden Schlauch und einer festen Ummantelung vorhanden sind, welche von einer elektrisch betriebenen Pumpe aus teilweise nacheinander so mit Hilfsflüssigkeit gefüllt werden, daß der Arztneizufluß teilweise unterbrochen und Flüssigkeit in bestimmten Mengen in peristaltischer Bewegung nach einer Richtung hin verdrängt wird (Fig.38(338),45,46,47).

44. Vorrichtung nach Anspruch 1, 2 und 3, dadurch gekennzeichnet, daß ein Schlauchring um einen innerhalb des arzneiführenden Schlauches gelagert ist, wobei ein Schlauchring zwischen jenem Schlauch und einer starren Wandung sich befindet, welcher über eine elektrisch betriebene Pumpe mit Hilfsflüssigkeit gefüllt wird, wodurch der Arzneistrom im Schlauch gestoppt wird(Fig.99).

45. Vorrichtung nach Anspruch 1,2,3, dadurch gekennzeichnet, daß über einem elastischen Kegel mit spiraliger Randleiste, auf welcher der arzneiführende Schlauch gelagert ist, ein konischer Mantel mit spiraliger Innenleiste bei der Senkung über elektrischen Antrieb so auf den Schlauch einwirkt, daß die oberen Schlauchwindungen früher als die unteren gequetscht werden und eine peristaltische Dosierbewegung erfolgt, welche bei der Rückstellung des konischen Mantels durch die Einwirkung einer unter einer Schlauchwindung liegenden Barriere gesperrt wird (Fig.97).

46. Vorrichtung nach Anspruch 1,2,3,
dadurch gekennzeichnet,
daß ein elastischer Mantelring vorhanden ist, in dessen Höhlung eine Schlaucherweiterung lagert, dessen zuführender Schenkel flach über den Rand des Mantelringes geführt wird, wobei jene Schlaucherweiterung deckelseitig mit einer Platte verbunden ist, welche mit einem darüber gelagertem Druckgeber verbunden wird, so daß bei dessen Annäherung zunächst der zuführende Schlauchschenkel abgeklemmt und dann die Schlaucherweiterung teilweise durch die Kanüle entleert wird, wobei eine Lichtquelle mit einem Sensor zusammenwirkt, um den Füllungzustand der Schlaucherweiterung zu kontrollieren, während die Hubhöhe des Druckgebers durch piezoelekrische Schalter kontrolliert wird(Fig.98).

47. Vorrichtung nach Anspruch 1,2,3,
dadurch gekennzeichnet,
daß Meßelktroden an dem arzneiführenden Schlauch vorhanden sind, deren Meßdaten über elektromagnetische Zustandsänderungen in Abhängigkeit von der Durchflußgeschwindigkeit an eine Steuereinheit weitervermittelt werden, welche ein elektrisches Absperrventil befehligt, und wobei die Arzneiflüssig-

keit vom Vorratsbehälter her unter Druck steht (Fig.99).

48. Vorrichtung nach Anspruch 1,2 und 3, dadurch gekennzeichnet, daß in Nähe der Kanüle ohne Unterbrechung durch Schlauchabschnitte mit der Möglichkeit zu einer von dessen innerer Materialbeschaffenheit oder von außen ungehinderten Ausdehnung ein zylindrisches Pumpenge- häuse(111) zwischengeschaltet ist, in welchem ein Stößel(110) eingelagert ist, welcher ein Dichtungselement(109,168) und und einen Ventilverschluß (115,169) im Wechseltakt betreibt, wobei die Kraft von einem von Durchlässen für die Arzneiflüßigkeit durchbrochenen Teller(112) am Stößel über eine Klemmvorrichtung(113,171) und ein Gestänge(122,126) von einem Motor betrieben wird, wobei mittels des Mechanismus für eine Vorprogrammierung der Dosierung die Zahl der Pumpentakte des Stößels für die jeweilige Injektion festgelegt wird, wobei letztere ausgelöst wird, sobald die Haut in der Saugglocke hochgehoben ist(Fig.77,81,91).

49. Vorrichtung nach Anspruch 1,2,3,

welche aus einem Gehäusezylinder(1) besteht, einem Bodenring, einem von dessen Nähe zu einem Kolben sich erstreckenden Faltenbalg, einem Rohrstutzen zur Verbindung mit einem Einschubzylinder zur Aufnahme einer Injektionsspritze, dadurch gekennzeichnet, daß ein Wiederbelüftungsventil vorhanden ist und eine Vorrichtung zur vorrübergehenden Sperrung der Kolbenrückbewegung zur Wiederentfaltung des Faltenbalges, nachdem dessen Innenraum über ei- nen Ableitungsschlauch mit einer Sogquelle in

- 143 -

bindung gesetzt und die Saugglocke durch die Haut des Patienten verschlossen war (Fig.8-12).

50. Vorrichtung nach Anspruch 1, dadurch gekennzeichnet, daß der Injektor aus einem zusammenfaltbaren Arzneivorratsbeutel als Flüssigkeitsbehälter und als Mittel zur Regelung des Arzneidurchflusses durch den Schlauch gemäß einer vorwählbaren veränderlichen Dosierung aus einer Trägerscheibe(40), welche zwei einander gegenüber liegende Rollen(41) trägt und den Schlauch(44) in einer ausgekehlten Kammer einschließt und gegen ein ringförmig umgebendes Gegenlager(43) mit jenen Rollen zusammenpreßt und einen sektorförmigen Durchlaß aufweist, wobei jener Schlauch außerhalb dieses Durchlasses von einem dehnungsfesten Mantel(45) umgeben ist bis hin zur Kanüle(8) innerhalb der Saugglocke, wobei als Kraftquelle für die Bewegung jener Trägerscheibe eine Spiralfeder dient, und die Bewegung über ein Sperrzahnrad mit Sperrklinke(30) in der Entspannungsrichtung jener Spiralfeder erfolgt, und wobei als Mittel der Voreinstellbarkeit der Dosierung vor dem Gebrauch federnde Zungen(27) als Anschläge dienen, welche längs einer Skala feststellbar die Trägerscheibe dadurch hindern, daß sie einem Schaltstift(66) anliegen, welcher nach Ablauf einer Sektorenbewegung der Scheibe(51) durch eine Profilkulisse(67) kurzzeitig zurückgezogen wird, nachdem die von gesonderter Spiralfeder(55) angetriebene Scheibe(51) der Rüttelvorrichtung in regelmäßigen Abständen Stifte trägt, welche in die ringförmig durchlaufende Keilprofilleiste(76) längs der Trägerscheibe eingreift und diese in achsenquere Stoßbewegungen versetzt, sobald nach Ansaugung der Haut innerhalb der Saugglocke jene in die Kanüle gehoben wurde. (Fig.66).

Fig. 1

228

234

253

289

218

288

240

228

Fig. 2

Fig. 3

Fig. 4

3/62

0221005

# Fig.5

| CPU | EPROM | RAM | EPROM | Eingang I/O Ausgang |

Adressbus

Datenbus

Sensor

Verstärker

Analog-Digital-Wandler

Puffer

A/D

Quarz-Zeitbasis

$f$

10 bit Zähler

Adresse

Speicher RAM

Daten

Schreib/Lese

Freigabe

Schreib-Freigabe

Externer Takt

Rücksetzung

Auzsgabesteuerung

Datenleitungen

# Fig.6

0221005

8 x 16 Matrix
mitSchalttasten

| Meß-verstärker | A/D | |
|---|---|---|

Puffer

Hubmagnete
Summer
Blinker
Vibrator

Hautkontakt
Indikatoren

Anzeige
Drucker
Datenbus

Timer

Datenbus

Steuerleitungen

Adress-
Decoder

Interrupt

Adressbus

Fig. 7

723
715
714
713
711
712
710
708
709
707
705
719
718
716
717
721
733

722

703

706

254
234

576

730
728
729
726
725
724
240
727

719

90°

Fig.10

233

Fig.9

702
704

Fig. 8

732

0221005

*Fig.11*

722 713 715 749 799
752 708
750
751
753
748
710 701
754
746
747

*Fig. 12*

739
740
742
741
738
713
745
744

Fig. 13

0221005

Fig. 14

Fig. 16

Fig. 17

Fig. 15

Fig. 18

0221005

458   462   461

449

463

450

445

456

455   457

294

452

449   464   292

Fig. 19

Fig. 20

Fig. 21

11/62

0221005

499

467

72

501

283

500

501

72

264  287

Fig. 22

Fig. 23

0221005

Fig. 24

505

506

Fig. 25   Fig. 26

15/52

0221005

583

262

515

515

516

584

289

Fig. 27

524

M 10:1

525

517

523

521

526

518

519

520

581

Fig. 28

518

Fig.29

*Fig. 30*

Fig 31

Fig. 32

Fig. 33

122

131

39

40

Fig. 34

132

39

Fig. 35

23/52

0221005

Fig. 36

Fig.37

0221005

0221005

Fig. 38

Fig. 39

339

357

340

370

314

34

316

Fig. 40

0221005

345   346                    344

350

332

349

341

343

348

347

322

342                           345

Fig. 41

Fig. 42

Fig. 43

0221005

534

314

535

Fig. 44

Fig. 45

Fig. 46

Fig. 47

# Fig. 48

381
582
382
383

# Fig. 49

380
378
317
381
382
383

379

542

A ─┤─·─·─ ·─·─├─ B

541

249

248

# Fig. 50

545

C ─┤─·─·─ ·─·─├─ D

545

# Fig. 51

577

383

# Fig. 52

545

# Fig. 53

Fig.54

886

379

47

587

560

583

561

Fig. 55

588

535

47

Fig. 56

588

381

590

314

594

589

211

885

372
374
377

II

III

IV

V

384

Fig. 57

600  280  675
598          616
599
226
373          385
376          372
316          371
315          375

V

II

1/0

Dosis-Grenz-wert

Alt-Insulin

Zusatz-dosis bei Über-zuckerung

q

f

Nach-frist

Ober-grenze

Ist-minus Soll-Δ wert xK2xnE

91
1/0

Weck-zeit

Soll-wert

Ist-wert

Norm-dosis

Norm-do sis

92

a

Vor-gabe

Unter-grenze

Soll-minus Ist-Δ wert xK1xnE

Depot-Insulin

k

Dosis-abzug bei Unter-zuckerung

P

Grenz-wert für Dosis-stop

Norm-dosis minus ↑

m

90

86

b

h

c

d

88

*Fig.58*

Uhr
Ventil

Haut Erdkon-takt

Lese 89
Drucker

Leser 90
Drucker

Leser 81
Drucker

87
1/0 Wecker
1/0 Blinker
1/0 Vibrator

37
Pumpe

Pumpe

neg.

Zähler
t

f.Kanülen

neg.
für Depot-

neg.

neg.
Insulin

neg.
Altinsul.

s

r

Fig. 59

19/52

0221005

Fig. 59 — Flussdiagramm

① Hautkontakt
  + → ⑥ Messung ggf:Korrektur Injektion
  −

② Weckzeit erreicht ohne Injektion
  + → ⑦ Wecksignal auslösen
  −

③ Taster "Zeiteinstellung" gedrückt
  + → ⑧ Zeitfenster verschieben
  −

④ Depotinsulin-vorratsbehälter erneuert
  + → ⑨ Vorratszähler für Depotinsulin =max. Wert
  −

⑤ Altinsulin-vorratsbehälter erneuert
  + → ⑩ Vorratszähler für Altinsulin =max. Wert
  −

58 Taster "4mal Tagesdosis Rückpumpen" gedrückt
  + → 59 4 Tagesdosen rückpumpen
  −

Fig. 60

Fig. 61

(A)

(24) Berücksichtigung des Programmwahl-schalters

(25) Meßwert >Fenster

(27) Altinsulindosis= Vorgabe$+\frac{Abw.}{10}\cdot K_2$ ggf. max. Dosis

(31) Programm-automatik für Depotinsulin gesperrt

(26) Meßwert < Fenster

(32) Meßwert im aktuellen Zeit-in den letzten 4 Tagen >Fens-ter

(28) Altinsulindosis= 0 Depotinsulindosis= Vorgabe$-\frac{Abw.}{10}\cdot K_1$

(33) errechnete Altinsulindosis >max. Dosis

(29) Programm-automatik für Depotinsulin gesperrt

(34) Depotinsulindosis des vorigen Zeit-blocks um 1 erhöhen

(30) Depotinsulindosis des vorigen Zeitblocks um 1 verringern

(35) Depotinsulin des vorigen Zeitblocks um 1 erhöhen

(B)

B

(36) Taster "Warten" gedrückt

(41) Wartezeit >15 s

(37) Taster "Warten" neu gedrückt

(42) Belüften

(43) Hautkontakt → HP

(44)

(38) Taster "-" neu gedrückt

(45) aktuelle Altinsulindosis um 4 erhöhen

(46) Dosis berichtigen, falls nicht $0 \le$ Altinsulin $\le$ max. Wert

(39) Taster "+" neu gedrückt

(45) aktuelle Altinsulindosis um 4 verringern

(47) Anzeige Altinsulindosis

(40) Wartezeit > 15 s

(48) Hautkontakt → HP

(49) Vorrat ausreichend

(50) Piepton Injektion

(51) Belüften

Hautkontakt → C

(52)

Fig.62

(C)

(53)

Für Depot- und
Altinsulin:
Vorratszähler um
aktuelle Dosis zu
verringern

(55)

(54)

Vorräte
für max. Injektion
ausreichend

Aufforderung
zum
Nachfüllen

−

+

(HP)

(56)

Vorratsgefäß
zum Rückpumpen
angesetzt

−

Fig.63

(57)

+

Rückpumpen
Vorratszähler
aktualisieren

(HP)

44/52

0221005

Fig. 64

Fig.65

33

34

38

39

180

35

57

## Fig. 67

56  28  27  26

25

29

23

## Fig. 66

28  27  65  56  66  64  63  62  51
61
58
28
67
59
60

## Fig. 68

## Fig. 69

Fig. 70

Fig. 71

0221005

Fig. 73

Fig. 74

Fig. 75

Fig. 76

0221005

49/52

Fig. 77

0221005

Fig. 78

Fig. 79

Fig. 80

Fig.81

138
19
20

195
198
190
188
178

196
176
192
170

C

D

171
112
172
111
168
169

110  189
187
185
186
183
184

A

B

179
173  148  191

156

Fig.82  C-D

184

174

178

0221005

**Fig.72**

49

51

56

50

**Fig.83**

186   185

I

189   148

185   148

III

187

Fig.84

166

223

150

A

B

165

Fig.85

Fig.87

215

216

A

210    209

206

204

205

5

10

207

165

B

Fig.86

Fig.88

Fig.89

Fig.90

20
112
110
44
109
116
114
115
47

Fig.91

Fig. 92

57/52

0221005

Fig.93

0221005

Fig.94

Fig.95

0221005

549

44

## Fig. 97

546  547

## Fig. 96

564
567
558
562
569

556

557  561  563

175

565

## Fig. 98

## Fig. 99

566

Fig. 100

855
603
609
604
608
605
887
606
607
859 800
71
72
73

613
612
614
564

Fig. 101

616

Fig. 102

Fig. 103

Fig. 104

612

Fig. 105

610

611